# EUROPEAN PATENT APPLICATION

(11) **EP 4 799 624 A2**
(43) Date of publication of application: **02.09.2026**
(21) Application number: 26193369.1
(22) Date of filing: 29.11.2022
(51) Int. Cl.: A61K 31/497

(54) **USE OF PIPENDOXIFENE TO TREAT SARS-COV-2 INFECTION**

(30) Priority: 30.11.2021 US 202163284591 P; 10.05.2022 US 202263340363 P; 09.06.2022 US 202263350821 P
(62) Document number(s) of the earlier application(s) in accordance with Art. 76 EPC: 22902338.7 / 4 440 574
(71) Applicant: Model Medicines, Inc., La Jolla, CA 92037 (US)
(72) Inventor: HADERS, Daniel, La Jolla, 92037 (US); NICOLA, George, La Jolla, 92037 (US)
(74) Representative: Hutter, Anton

(57) **Abstract**

Methods and compositions for treating RNA viral infections, including behavior symptoms of the RNA viral infections, are disclosed herein. Also disclosed are methods and compositions for reducing the progression of clinical complications associated with RNA viral infections. The methods, for example, can include administering pharmaceutical compositions comprising Pipendoxifene or analogues thereof (e.g., a compound of Formula (I), Formula (II), or Formula (III)) to a patient in need. One or more additional therapeutic agents can also be administered to the patient in the disclosed methods.

## Description

### RELATED APPLICATIONS

The present application claims priority to U.S. Provisional Application No. 63/284,591, filed November 30, 2021; U.S. Provisional Application No. 63/340,363, filed May 10, 2022; and U.S. Provisional Application No. 63/350,821, filed June 9, 2022. The entire contents of these applications are hereby expressly incorporated by reference in their entireties.

### BACKGROUND

### Field

The present disclosure generally relates to the use of drugs for the treatment of RNA viral infections. More specifically, the disclosure describes methods, compositions and kits for the treatment of an RNA viral infection and/or treatment or prevention of symptoms of an RNA viral infection by administering pharmaceutical compositions or their analogues.

### Description of the Related Art

An RNA virus is a virus that has RNA (ribonucleic acid) as its genetic material. This nucleic acid is usually single-stranded RNA (ssRNA) but may be double-stranded RNA (dsRNA). Notable human diseases caused by RNA viruses include the common cold, influenza, SARS, coronaviruses, COVID-19, hepatitis C, hepatitis E, West Nile fever, Ebola virus disease, rabies, polio and measles.

A large respiratory outbreak originating from Wuhan, China in December 2019 is currently spreading across many countries globally. The infectious disease was determined to be caused by a newly identified human coronavirus, severe acute respiratory syndrome coronavirus 2 (SARS-CoV-2). As of May 25, 2020, there are approximately 4.5M confirmed cases of severe acute respiratory syndrome (SARS-CoV-2) globally with a 6.9% mortality rate (WHO). The main symptoms of this virus are cough, shortness of breath or difficulty breathing, fever, headache, sore throat, and loss of taste and/or smell. New symptoms caused by SARS-CoV-2 are surfacing frequently; examples of recently discovered effects on various organs and physiological systems can be viewed at Jason Gale 2020. For instance, a notably severe effect of the virus' infection in the respiratory tract is that it can induce Acute Respiratory Distress Syndrome (ARDS) in addition to general respiratory complications. Treatment that serves to block viral infection or attenuate symptoms of SARS-CoV-2 are of utmost interest.

SARS-CoV-2 is part of the genus Betacoronavirus and shares structural and sequence similarity with SARS-CoV and MERS-CoV. This novel coronavirus is an enveloped positive sense RNA virus. Its structure is mainly encompassed by a spike (S) glycoprotein, a small envelope (E) glycoprotein, membrane (M) glycoprotein, and a nucleocapsid (N) protein. The S Protein facilitates binding and fusion for host-cell entry. The S protein is composed of two subunits, S1 and S2, that require proteolytic activation by host enzymes furin and TMPRSS2. Once activated, the S1 subunit utilizes its receptor binding domain to recognize and bind to the host's angiotensin-converting enzyme 2 (ACE2) located in the type II alveolar cells of the respiratory tract. The S2 subunit contains fusion peptides that facilitate fusion of the viral and host membranes.

Following viral entry and fusion into the host cell, the virus releases its genome in a form that can be readily translated by the host's ribosomal machinery. Two virally encoded proteases, papain-like protease (PLpro or Nsp3) and 3C-like protease (3CLpro or Nsp5) are essential to process viral polyproteins pp1a and pp1ab which are necessary for production and maturation of nonstructural proteins (Nsp). The released Nsps are required for proper formation and execution of the virus' replication/transcription complex. Another important element of the replication/transcription complex is RNA-dependent RNA polymerase (RdRp or Nsp12). RdRp synthesizes a complete negative-strand RNA template that is then used to create more viral genomic RNA. Targeted inhibition of the key proteins furin, ACE2, TMPRSS2, 3CLpro, PLpro and RdRp could block cellular entry and propagation of SARS-CoV-2 and potentially other coronaviruses of the same genus.

The pharmaceutical candidates described herein have been investigated in various other diseases. In view of the large volume of data from the clinical investigation of these pharmaceutical candidates, and deep understanding of their clinical behaviors, it is beneficial to determine if these pharmaceutical candidates can be used to treat and/or prevent other disorders, for example RNA viral infections. There is an urgent need or compositions and methods for preventing, delaying the onset of, or treating an inflammatory effect of an infection or a disease caused by a RNA virus (e.g., SARS-CoV-2).

### SUMMARY

Disclosed herein includes the use of pharmaceutical compositions and pharmaceutical composition analogues for the treatment or prevention of disorders related to the modulation of one or more receptors related to RNA viral infections, for example coronavirus infections (including the abnormal behavioral symptoms related to coronavirus infections).

The RNA viral infection can be caused by, for example, a coronavirus. In some embodiments, the RNA viral infection is caused by SARS COV-1, SARS COV-2, the common cold, influenza, SARS, hepatitis C, hepatitis E, West Nile fever, Ebola virus disease, rabies, polio, measles, or a combination thereof. The International Committee on Taxonomy of Viruses (ICTV) classifies RNA viruses as those that belong to Group III, Group IV or Group V of the Baltimore classification system. Another term for RNA viruses is ribovirus. Viruses with RNA as their genetic material which also include DNA intermediates in their replication cycle are called retroviruses, and comprise Group VI of the Baltimore classification. Notable human retroviruses include HIV-1 and HIV-2, the cause of the disease AIDS. In some embodiments, the RNA viral infection is a results of viruses from Groups III, IV, V, or VI of the Baltimore classification system.

Non-limiting examples of RNA viral infection include Paramyxoviruses, Hendra and Nipah viruses, Measles, Severe acute respiratory syndrome coronavirus (SARS), COVID-19, Middle east respiratory syndrome coronavirus (MERS), Picornaviruses, Poliomyelitis ('Polio'), Hepatitis A virus (HAV), Rotavirus, Human immunodeficiency virus (HIV), Human T-cell lymphotropic virus (HTLV), Hepatitis C virus (HCV), Hepatitis E virus (HEV), Rabies, Ebola virus disease (EVD), Marburg virus, Lassa fever, Lymphocytic choriomeningitis virus (LCMV), Arboviruses ('ARthropod-BOrne viruses'), Japanese encephalitis (JE), West Nile fever, Yellow fever, Dengue fever, Zika virus, Equine encephalitis viruses, Chikungunya, O'nyong-nyong, Bunyaviruses, Rift valley fever and Crimean-Congo haemorrhagic fever, Hantavirus, and a combination thereof. The RNA viral infection can also be a complication due to a bacterial or parasitic infection.

In some embodiments, a compound of Formula (I), Formula (II), or Formula (III) or the analogue thereof is administered in the form of a pro-drug. a compound of Formula (I), Formula (II), or Formula (III) (or a pharmaceutically acceptable salt, ester, solvate, stereoisomer, tautomer, or prodrug thereof) can be, for example, administered orally. In some embodiments, a compound of Formula (I), Formula (II), or Formula (III) (or a pharmaceutically acceptable salt, ester, solvate, stereoisomer, tautomer, or prodrug thereof) is administered in the form of a pill, a tablet, a microtablet, a pellet, a micropellet, a capsule, a capsule containing microtablets, or a liquid formulation. In some embodiments, a compound of Formula (I), Formula (II), or Formula (III) (or a pharmaceutically acceptable salt, ester, solvate, stereoisomer, tautomer, or prodrug thereof) is administered in the form of a capsule containing enteric coated microtablets.

A compound of Formula (I), Formula (II), or Formula (III) (or a pharmaceutically acceptable salt, ester, solvate, stereoisomer, tautomer, or prodrug thereof) can be administered in various frequency, for example, once, twice, or three times a day. In some embodiments, a compound of Formula (I), Formula (II), or Formula (III) (or a pharmaceutically acceptable salt, ester, solvate, stereoisomer, tautomer, or prodrug thereof) can be administered no more than once, twice, or three times a day. In some embodiments, a compound of Formula (I), Formula (II), or Formula (III) (or a pharmaceutically acceptable salt, ester, solvate, stereoisomer, tautomer, or prodrug thereof) can be administered at least once, twice, or three times a day. In some embodiments, a compound of Formula (I), Formula (II), or Formula (III) (or a pharmaceutically acceptable salt, ester, solvate, stereoisomer, tautomer, or prodrug thereof) is administered once every day, every two days, every three days, every four days, or every five days. The duration for the treatment can vary. For example, a compound of Formula (I), Formula (II), or Formula (III) (or a pharmaceutically acceptable salt, ester, solvate, stereoisomer, tautomer, or prodrug thereof) can be administered over the course of at least one week, at least two weeks, at least three weeks, at least four weeks, at least five weeks, at least ten weeks, at least twenty weeks, at least twenty-six weeks, at least a year, or longer. In some embodiments, a compound of Formula (I), Formula (II), or Formula (III) (or a pharmaceutically acceptable salt, ester, solvate, stereoisomer, tautomer, or prodrug thereof) can be administered over the course of no more than five weeks, no more than ten weeks, no more than twenty weeks, no more than twenty-six weeks, or no more than a year.

Disclosed herein include kits, comprising a compound of Formula (I), Formula (II), or Formula (III) (or a pharmaceutically acceptable salt, ester, solvate, stereoisomer, tautomer, or prodrug thereof), and a label indicating that the kit is for the treatment or amelioration of one or more symptoms of an RNA viral infection.

Disclosed herein include kits comprising a compound of Formula (I), Formula (II), or Formula (III), or a pharmaceutically acceptable salt, ester, solvate, stereoisomer, tautomer, or prodrug thereof; and a label indicating that the kit is for preventing, delaying the onset of, or treating an infection or a disease caused by a RNA virus.

Disclosed herein include kits comprising a compound of Formula (I), Formula (II), or Formula (III), or a pharmaceutically acceptable salt, ester, solvate, stereoisomer, tautomer, or prodrug thereof; and a label indicating that the kit is for preventing, delaying the onset of, or treating an inflammatory effect of an infection or a disease caused by a RNA virus.

In some embodiments, the label indicates that the kit is for prophylaxis administration. In some embodiments, the label indicates that the kit is for low-risk patients, optionally low-risk patients exposed to an RNA virus or suspected of being exposed to an RNA virus. In some embodiments, the label indicates that the kit is for high-risk and/or severe disease patients post-infection with a RNA virus. In some embodiments, the label indicates a compound of Formula (I), Formula (II), or Formula (III) (or the pharmaceutically acceptable salt, ester, solvate, stereoisomer, tautomer, or prodrug thereof) is administered at a daily dose of at least about 600 mg, 620 mg, 640 mg, 660 mg, 680 mg, 700 mg, 720 mg, 740 mg, 760 mg, 780 mg, 800 mg, 820 mg, 840 mg, 860 mg, 880 mg, 900 mg, 920 mg, 940 mg, 960 mg, 980 mg, 1000 mg, 1020 mg, 1040 mg, 1060 mg, 1080 mg, 1100 mg, 1120 mg, 1140 mg, 1160 mg, 1180 mg, 1200 mg, 1220 mg, 1240 mg, 1260 mg, 1280 mg, 1300 mg, 1320 mg, 1340 mg, 1360 mg, 1380 mg, 1400 mg, 1420 mg, 1440 mg, 1460 mg, 1480 mg, 1500 mg, 1520 mg, 1540 mg, 1560 mg, 1580 mg, 1600 mg, 1620 mg, 1640 mg, 1660 mg, 1680 mg, 1700 mg, 1720 mg, 1740 mg, 1760 mg, 1780 mg, 1800 mg, 1820 mg, 1840 mg, 1860 mg, 1880 mg, 1900 mg, 1920 mg, 1940 mg, 1960 mg, 1980 mg, 2000 mg, 2020 mg, 2040 mg, 2060 mg, 2080 mg, 2100 mg, 2120 mg, 2140 mg, 2160 mg, 2180 mg, 2200 mg, 2220 mg, 2240 mg, 2260 mg, 2280 mg, 2300 mg, 2320 mg, 2340 mg, 2360 mg, 2380 mg, 2400 mg, 2420 mg, 2440 mg, 2460 mg, 2480 mg, or 2500 mg, optionally the administering comprises once daily or twice daily oral administration.

Also disclosed herein include compositions comprising a compound of Formula (I), Formula (II), or Formula (III) (or a pharmaceutically acceptable salt, ester, solvate, stereoisomer, tautomer, or prodrug thereof) for use in the treatment of an RNA viral infection in a subject. In some embodiments, the treatment comprises administrating one or more additional therapeutic agents to the subject. The one or more additional therapeutic agents can, for example, comprise a binder of a receptor related to RNA viral infection.

Disclosed herein include methods for preventing, delaying the onset of, or treating an infection or a disease caused by a RNA virus. In some embodiments, the method comprises administering to a subject in need thereof a composition comprising a compound of Formula (I), Formula (II), or Formula (III), or a pharmaceutically acceptable salt, ester, solvate, stereoisomer, tautomer, or prodrug thereof, thereby preventing, delaying the onset of, or treating the infection or the disease.

Disclosed herein include methods for preventing, delaying the onset of, or treating an inflammatory effect of an infection or a disease caused by a RNA virus. In some embodiments, the method comprises administering to a subject in need thereof a composition comprising a compound of Formula (I), Formula (II), or Formula (III), or a pharmaceutically acceptable salt, ester, solvate, stereoisomer, tautomer, or prodrug thereof, thereby preventing, delaying the onset of, or treating the inflammatory effect.

Disclosed herein include methods for preventing, delaying the onset of, or treating an infection or a disease caused by a RNA virus. In some embodiments, the method comprises administering to a subject in need thereof a composition comprising a compound of Formula (I), Formula (II), or Formula (III) (or a pharmaceutically acceptable salt, ester, solvate, stereoisomer, tautomer, or prodrug thereof), thereby preventing, delaying the onset of, or treating the infection or the disease.

Disclosed herein include methods for preventing, delaying the onset of, or treating an inflammatory effect of an infection or a disease caused by a RNA virus. In some embodiments, the method comprises administering to a subject in need thereof a composition comprising a compound of Formula (I), Formula (II), or Formula (III) (or a pharmaceutically acceptable salt, ester, solvate, stereoisomer, tautomer, or prodrug thereof), thereby preventing, delaying the onset of, or treating the inflammatory effect.

In some embodiments, a compound of Formula (I), Formula (II), or Formula (III) (or the pharmaceutically acceptable salt, ester, solvate, stereoisomer, tautomer, or prodrug thereof) is administered at a daily dose of at least about 600 mg, 620 mg, 640 mg, 660 mg, 680 mg, 700 mg, 720 mg, 740 mg, 760 mg, 780 mg, 800 mg, 820 mg, 840 mg, 860 mg, 880 mg, 900 mg, 920 mg, 940 mg, 960 mg, 980 mg, 1000 mg, 1020 mg, 1040 mg, 1060 mg, 1080 mg, 1100 mg, 1120 mg, 1140 mg, 1160 mg, 1180 mg, 1200 mg, 1220 mg, 1240 mg, 1260 mg, 1280 mg, 1300 mg, 1320 mg, 1340 mg, 1360 mg, 1380 mg, 1400 mg, 1420 mg, 1440 mg, 1460 mg, 1480 mg, 1500 mg, 1520 mg, 1540 mg, 1560 mg, 1580 mg, 1600 mg, 1620 mg, 1640 mg, 1660 mg, 1680 mg, 1700 mg, 1720 mg, 1740 mg, 1760 mg, 1780 mg, 1800 mg, 1820 mg, 1840 mg, 1860 mg, 1880 mg, 1900 mg, 1920 mg, 1940 mg, 1960 mg, 1980 mg, 2000 mg, 2020 mg, 2040 mg, 2060 mg, 2080 mg, 2100 mg, 2120 mg, 2140 mg, 2160 mg, 2180 mg, 2200 mg, 2220 mg, 2240 mg, 2260 mg, 2280 mg, 2300 mg, 2320 mg, 2340 mg, 2360 mg, 2380 mg, 2400 mg, 2420 mg, 2440 mg, 2460 mg, 2480 mg, or 2500 mg, optionally the administering comprises once daily or twice daily oral administration.

In some embodiments, the administering is prophylaxis administration. In some embodiments, the administration is 3 hours, 6 hours, 12 hours, 18 hours, 24 hours, 36 hours, 47 hours, 72 hours, 96 hours, 4 days, 5 days, 6 days, or 7 days before commencement of the infection or the disease. In some embodiments, the administration is repeated one or more times per day. In some embodiments, the administration is repeated hourly, daily, or weekly. In some embodiments, the administering comprises administering one or more loading doses and one or more maintenance doses of a compound of Formula (I), Formula (II), or Formula (III), or the pharmaceutically acceptable salt, ester, solvate, stereoisomer, tautomer, or prodrug thereof.

The subject can be a low-risk patient, e.g., a low-risk patient exposed to an RNA virus or suspected of being exposed to an RNA virus. In some embodiments, the subject is a high-risk and/or severe disease patient post-infection with a RNA virus. In some embodiments, the administration does not cause an adverse event in the subject. In some embodiments, the administration does not cause any significant drug-drug interactions and/or genotoxicity in the subject. In some embodiments, therapeutic levels of a compound of Formula (I), Formula (II), or Formula (III) (or the pharmaceutically acceptable salt, ester, solvate, stereoisomer, tautomer, or prodrug thereof) are achieved in the subject with a dose at least 1.1-fold, 1.3-fold, 1.5-fold, 1.7-fold, 1.9-fold, 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, or 100-fold, below the LD₅₀.

In some embodiments, the administration of the composition prevents, delays the onset of, and/or treats the infection, the disease and/or inflammatory effect in the subject comparable to or better than administration of a composition comprising Remdesivir, optionally the composition comprising Remdesivir is subcutaneously administered twice a day at a dose of 150 mg. In some embodiments, the administration of the composition produces an improvement in one or more clinical endpoints in the subject equal to or greater than the improvement in said one or more clinical endpoints in a subject administered a composition comprising Remdesivir, optionally the composition comprising Remdesivir is subcutaneously administered twice a day at a dose of 150 mg, further optionally a clinical end point comprises body weight.

In some embodiments, a significant amount of a compound of Formula (I), Formula (II), or Formula (III) (or the pharmaceutically acceptable salt, ester, solvate, stereoisomer, tautomer, or prodrug thereof) accumulates in the plasma and/or lung tissue of the subject following administration, optionally the lung tissue is the primary site of the infection and/or disease. In some embodiments, the administration achieves lung concentrations of a compound of Formula (I), Formula (II), or Formula (III) (or the pharmaceutically acceptable salt, ester, solvate, stereoisomer, tautomer, or prodrug thereof) of greater than 30 ug/g, optionally the administration comprises once daily oral administration. In some embodiments, the administration achieves an at least 1.1-fold, 1.3-fold, 1.5-fold, 1.7-fold, 1.9-fold, 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, or 100-fold, enrichment in lung to plasma concentrations of a compound of Formula (I), Formula (II), or Formula (III), or the pharmaceutically acceptable salt, ester, solvate, stereoisomer, tautomer, or prodrug thereof. In some embodiments, a compound of Formula (I), Formula (II), or Formula (III) (or the pharmaceutically acceptable salt, ester, solvate, stereoisomer, tautomer, or prodrug thereof) achieves an at least 1.1-fold, 1.3-fold, 1.5-fold, 1.7-fold, 1.9-fold, 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, or 100-fold, greater lung tissue concentration than the minimum therapeutic concentration in the lung tissue. In some embodiments, the C_{Lung}/EC₅₀ ratio of a compound of Formula (I), Formula (II), or Formula (III) (or the pharmaceutically acceptable salt, ester, solvate, stereoisomer, tautomer, or prodrug thereof) exceeds about 1, 2, 3, 4, 5, 6, 7, 8, 9, 10, 12, 14, 16, 18, or 20 at a time point of 1, 2, 3, 4, 5, 6, 7, 8, 9, 10, 12, 14, 16, 18, 20, 22, or 24 hours after one or more administrations of the composition. In some embodiments, the C_{Lung}/EC₉₀ ratio of a compound of Formula (I), Formula (II), or Formula (III) (or the pharmaceutically acceptable salt, ester, solvate, stereoisomer, tautomer, or prodrug thereof) exceeds about 1, 2, 3, 4, 5, 6, 7, 8, 9, 10, 12, 14, 16, 18, or 20 at a time point of 1, 2, 3, 4, 5, 6, 7, 8, 9, 10, 12, 14, 16, 18, 20, 22, or 24 hours after one or more administrations of the composition. In some embodiments, the administering provides a C_{Lung}/EC₉₀ of a compound of Formula (I), Formula (II), or Formula (III) (or the pharmaceutically acceptable salt, ester, solvate, stereoisomer, tautomer, or prodrug thereof) greater than 1 coverage for at least about 24 hours, optionally the administering comprises b.i.d. dosing.

In some embodiments, the inflammatory effect comprises respiratory failure, a sequela of respiratory failure, acute lung injury, or acute respiratory distress syndrome. In some embodiments, the sequela of respiratory failure comprises multi-organ failure.

In some embodiments, the composition comprises a therapeutically or prophylactically effective amount of a compound of Formula (I), Formula (II), or Formula (III) (or a pharmaceutically acceptable salt, ester, solvate, stereoisomer, tautomer, or prodrug thereof). In some embodiments, the subject in need is a subject that is suffering from the infection or the disease, or a subject that is at a risk for the infection or the disease. In some embodiments, the infection or the disease is in the respiratory tract of the subject. In some embodiments, the subject has been exposed to the RNA virus, is suspected to have been exposed to the RNA virus, or is at a risk of being exposed to the RNA virus. In some embodiments, the subject is a mammal. In some embodiments, the subject is a human.

In some embodiments, the RNA virus is a double-stranded RNA virus. In some embodiments, the RNA virus is a positive-sense single-stranded RNA virus. In some embodiments, the positive-sense single-stranded RNA virus is a coronavirus. In some embodiments, the coronavirus is an alpha coronavirus, a beta coronavirus, a gamma coronavirus, or a delta coronavirus. In some embodiments, the coronavirus is Middle East respiratory coronavirus (MERS-CoV), severe acute respiratory syndrome coronavirus (SARS-CoV), or SARS-CoV-2. The coronavirus can be a SARS-CoV-2 variant selected from the group comprising B.1.1.7 (Alpha), B.1.351 (Beta), B.1.525 (Eta), B.1.427/B.1.429 (Epsilon), B.1.526 (Iota), B.1.617.1 (Kappa), B.1.617.2 (Delta), C.37 (Lambda), P.1 (Gamma), P.2 (Zeta), P.3 (Theta), B.1.1.529 (Omicron), derivatives thereof, of any combination thereof. In some embodiments, the infection or disease caused by the RNA virus is common cold, influenza, SARS, coronaviruses, COVID-19, hepatitis C, hepatitis E, West Nile fever, Ebola virus disease, rabies, polio, or measles.

In some embodiments, the composition is a pharmaceutical composition comprising the compound and one or more pharmaceutically acceptable excipients. The method can comprise administering to the subject one or more additional antiviral agents. In some embodiments, at least one of the one or more additional antiviral agents is co-administered to the subject with the composition. In some embodiments, at least one of the one or more additional antiviral agents is administered to the subject before the administration of the composition, after the administration of the composition, or both. In some embodiments, the composition comprises one or more additional therapeutic agents. In some embodiments, the one or more additional therapeutic agents comprise one or more antiviral agents. In some embodiments, the antiviral agent is selected from the group consisting of a nucleoside or a non-nucleoside analogue reverse-transcriptase inhibitor, a nucleotide analogue reverse-transcriptase inhibitor, a NS3/4A serine protease inhibitor, a NS5B polymerase inhibitor, and interferon alpha.

In some embodiments, the composition is administered to the subject by intravenous administration, nasal administration, pulmonary administration, oral administration, parenteral administration, or nebulization. In some embodiments, the composition is aspirated into at least one lung of the subject. In some embodiments, the composition is in the form of powder, pill, tablet, microtablet, pellet, micropellet, capsule, capsule containing microtablets, liquid, aerosols, or nanoparticles. In some embodiments, the composition is in a formulation for administration to the lungs.

In some embodiments, the composition is administered to the subject once, twice, or three times a day. In some embodiments, the composition is administered to the subject once every day, every two days, or every three days. In some embodiments, the composition is administered to the subject over the course of at least two weeks, at least three weeks, at least four weeks, or at least five weeks.

The method can comprise measuring the viral titer of the RNA virus in the subject before administering the composition to the subject, after administering the composition to the subject, or both. In some embodiments, the viral titer is lung bulk virus titer. In some embodiments, administrating the composition results in reduction of the viral titer of the RNA virus in the subject as compared to that in the subject before administration of the composition. In some embodiments, the administration of the composition achieves an at least 1.1-fold, 1.3-fold, 1.5-fold, 1.7-fold, 1.9-fold, 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, or 100-fold, reduction in viral titer in the subject as compared to a subject administered a vehicle control, optionally the viral titer is viral lung titer, optionally viral lung titer is measured from whole lung homogenates. In some embodiments, the viral titer is measured 3 hours, 6 hours, 12 hours, 18 hours, 24 hours, 36 hours, 47 hours, 72 hours, 96 hours, 4 days, 5 days, 6 days, 7 days, 8 days, 9 days, 10 days, 11 days, 12 days, 13 days, or 14 days post-infection. In some embodiments, the method further comprises measuring the body weight of the subject, optionally administering the composition ameliorates disease-associated and/or infection-associated weight loss, optionally in a dose-dependent manner, further optionally the disease-associated and/or infection-associated loss in body weight is less than about 20%, 15%, 14%, 13%, 12%, 11%, 10%, 9%, 8%, 7%, 6%, 5%, 4%, 3%, 2%, 1%, 0.9%, 0.8%, 0.7%, 0.6%, 0.5%, 0.4%, 0.3%. The method can comprise determining global virus distribution in the lungs of the subject. The method can comprise measuring a neutrophil density within the lungs of the subject. In some embodiments, administering the composition results in reduction of the neutrophil density within the lungs of the subject as compared to that in the subject before administration of the composition. The method can comprise measuring a total necrotized cell count within the lungs of the subject. In some embodiments, administering the composition results in reduction of the total necrotized cell count in the subject as compared to that in the subject before administration of the composition. The method can comprise measuring a total protein level within the lungs of the subject. In some embodiments, administering the composition results in reduction of the total protein level within the lungs of the subject as compared to that in the subject before administration of the composition.

In some embodiments, the RNA virus is a coronavirus. In some embodiments, the coronavirus is Middle East respiratory coronavirus (MERS-CoV), severe acute respiratory syndrome coronavirus (SARS-CoV), or SARS-CoV-2. The coronavirus can be a SARS-CoV-2 variant selected from the group comprising B.1.1.7 (Alpha), B.1.351 (Beta), B.1.525 (Eta), B.1.427/B.1.429 (Epsilon), B.1.526 (Iota), B.1.617.1 (Kappa), B.1.617.2 (Delta), C.37 (Lambda), P.1 (Gamma), P.2 (Zeta), P.3 (Theta), B.1.1.529 (Omicron), derivatives thereof, of any combination thereof.

Disclosed herein include compositions comprising a compound of Formula (I), Formula (II), or Formula (III) or a pharmaceutically acceptable salt, ester, solvate, stereoisomer, tautomer, or prodrug thereof, for use in preventing, delaying the onset of, or treating an infection or a disease caused by a RNA virus.

Disclosed herein include compositions comprising a compound of Formula (I), Formula (II), or Formula (III), or a pharmaceutically acceptable salt, ester, solvate, stereoisomer, tautomer, or prodrug thereof, for use in preventing, delaying the onset of, or treating an inflammatory effect of an infection or a disease caused by a RNA virus.

In some embodiments, the inflammatory effect comprises respiratory failure, a sequela of respiratory failure, acute lung injury, or acute respiratory distress syndrome. In some embodiments, the sequela of respiratory failure comprises multi-organ failure. In some embodiments, the composition comprises a therapeutically or prophylactically effective amount of a compound of Formula (I), Formula (II), or Formula (III) (or a pharmaceutically acceptable salt, ester, solvate, stereoisomer, tautomer, or prodrug thereof).

In some embodiments, the RNA virus is a double-stranded RNA virus. In some embodiments, the RNA virus is a positive-sense single-stranded RNA virus. In some embodiments, the positive-sense single-stranded RNA virus is a coronavirus. In some embodiments, the coronavirus is an alpha coronavirus, a beta coronavirus, a gamma coronavirus, or a delta coronavirus. In some embodiments, the coronavirus is Middle East respiratory coronavirus (MERS-CoV), severe acute respiratory syndrome coronavirus (SARS-CoV), or SARS-CoV-2. The coronavirus can be a SARS-CoV-2 variant selected from the group comprising B.1.1.7 (Alpha), B.1.351 (Beta), B.1.525 (Eta), B.1.427/B.1.429 (Epsilon), B.1.526 (Iota), B.1.617.1 (Kappa), B.1.617.2 (Delta), C.37 (Lambda), P.1 (Gamma), P.2 (Zeta), P.3 (Theta), B.1.1.529 (Omicron), derivatives thereof, of any combination thereof.

In some embodiments, the composition is a pharmaceutical composition comprising the a compound of Formula (I), Formula (II), or Formula (III) (or a pharmaceutically acceptable salt, ester, solvate, stereoisomer, tautomer, or prodrug thereof) and one or more pharmaceutically acceptable excipients. In some embodiments, the composition comprises one or more additional therapeutic agents. In some embodiments, the one or more additional therapeutic agents comprise one or more antiviral agents. In some embodiments, the one or more antiviral agents is selected from the group consisting of a nucleoside or a non-nucleoside analogue reverse-transcriptase inhibitor, a nucleotide analogue reverse-transcriptase inhibitor, a NS3/4A serine protease inhibitor, a NS5B polymerase inhibitor, and interferon alpha. In some embodiments, the composition is in the form of powder, pill, tablet, microtablet, pellet, micropellet, capsule, capsule containing microtablets, liquid, aerosols, or nanoparticles. In some embodiments, the composition is in a formulation for administration to the lungs.

In some embodiments of the compound of Formula (I): each of R₁ and R₂ is independently a hydrogen, a halogen atom, a hydroxyl group, a sulfhydryl group, an amino group, an amide group, a carboxyl group, a carbonyl group, a alkoxyalkyl group, or a C1-C3 alkyl group which may be substituted with a halogen atom, a hydroxyl group, a sulfhydryl group, an amino group, an amide group, a carboxyl group, a carbonyl group, or a alkoxyalkyl group; R₃ is a C1-C4 alkyl group which may be substituted with a terminal R₅ group; R₄ is a hydrogen atom or a C1-C5 alkyl or cycloalkyl group, which may be substituted with a halide, hydroxyl, carboxyl, carbonyl, amino, or thiol groups; and/or R₅ is a C1-C10 alkyl, cycloalkylaminoalkyl, aminodialkyl or aminocycloalkyl group, which may be substituted with an amino group, a thiol group, a hydroxyl group, or a carbonyl group.

In some embodiments of the compound of Formula (II): each of R₁ and R₂ is independently a hydrogen, a halogen atom, a hydroxyl group, a sulfhydryl group, an amino group, an amide group, a carboxyl group, a carbonyl group, a alkoxyalkyl group, or a C1-C3 alkyl group which may be substituted with a halogen atom, a hydroxyl group, a sulfhydryl group, an amino group, an amide group, a carboxyl group, a carbonyl group, or a alkoxyalkyl group; R₃ is a C1-C4 alkyl group which may be substituted with a terminal R₅ group; R₄ is a hydrogen atom or a C1-C5 alkyl or cycloalkyl group, which may be substituted with a halide, hydroxyl, carboxyl, carbonyl, amino, or thiol groups; and/or R₅ is a C1-C10 alkyl or cycloalkyl group which may be substituted with an amino group, a thiol group, a hydroxyl group, or a carbonyl group.

In some embodiments of the compound of Formula (III): each of R₁ and R₂ is independently a hydrogen, a halogen atom, a hydroxyl group, a sulfhydryl group, an amino group, an amide group, a carboxyl group, a carbonyl group, a alkoxyalkyl group, or a C1-C3 alkyl group which may be substituted with a halogen atom, a hydroxyl group, a sulfhydryl group, an amino group, an amide group, a carboxyl group, a carbonyl group, or a alkoxyalkyl group; R₃ is a C1-C4 alkyl group which may be substituted with a terminal R₄ group; and/or R₄ is a C1-C10 alkyl or cycloalkyl group which may be substituted with an amino group, a thiol group, a hydroxyl group, or a carbonyl group.

In some embodiments, the compound of Formula I, Formula II, or Formula III is or comprises Pipendoxifene.

### BRIEF DESCRIPTION OF THE DRAWINGS

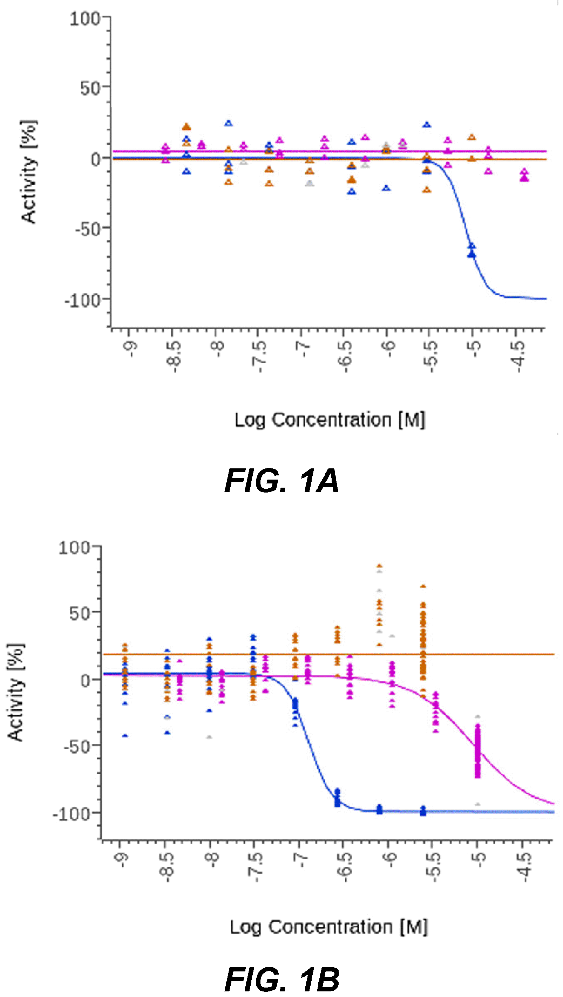

FIG. 1A-FIG. 1B. depicts non-limiting exemplary data related to Pipendoxifene and Remdesivir (control). FIG. 1A depicts non-limiting exemplary data related to Pipendoxifene. EC₅₀ = 8.24µM (CoV-2 cells); CC₅₀= >39.80µM (in uninfected cells); SI: >4.83*. FIG. 1B depicts non-limiting exemplary data related to Remdesivir (control). EC₅₀ = 0.130 µM (CoV-2 cells); CC₅₀=8.87µM (in uninfected cells); SI: 68.23. SI*: The reported selectivity value above is not representative of the true selectivity index of pipendoxifene. SI = CC₅₀/EC₅₀, The top of the equation is "infinite" based on the study reported data.
FIG. 2 depicts a non-limiting exemplary indole synthesis scheme, reproduced from Miller, Chris P., et al. ("Design, synthesis, and preclinical characterization of novel, highly selective indole estrogens." Journal of medicinal chemistry 44.11 (2001): 1654-1657), the content of which is incorporated herein by reference in its entirety.
FIG. 3 shows a non-limiting exemplary pre-clinical study group overview. R=remdesivir, B=berzosertib, P=pipendoxifene, Vehicle Control 1 and 2 = methylcellulose and vitamin E TPGS, LD=low dose, HD=high dose, time of treatment unit=hours
FIG. 4 depicts non-limiting exemplary data related to average plasma concentration for Pipendoxifene after single and multiple oral administration at 50 mg/kg in female 129S1 mice (Group 1).
FIG. 5 depicts non-limiting exemplary data related to average lung tissue concentration for Pipendoxifene after single and multiple oral administration at 50 mg/kg in female 129S1 mice (Group 1).
FIG. 6 depicts non-limiting exemplary data related to average plasma concentration for Pipendoxifene after single and multiple oral administration at 250 mg/kg in female 129S1 mice (Group 2).
FIG. 7 depicts non-limiting exemplary data related to average lung tissue concentration for Pipendoxifene after single and multiple oral administration at 250 mg/kg in female 129S1 mice (Group 2).
FIG. 8 depicts non-limiting exemplary data showing weight of mice over time in an *in vivo* model of SARS-CoV-2 infection. 129/S mice were intranasally infected with 2.5x10⁴ PFU of MA-SARS-CoV-2 and treated orally with 66.67 mg/kg pipendoxifene once daily or subcutaneously with 50 mg/kg remdesivir twice daily for 3 days. Animal weights were monitored daily. N = 6 per group. Data was analyzed by two-way ANOVA (*P < 0.05, **P < 0.01, ***P < 0.001, and ****P < 0.0001).

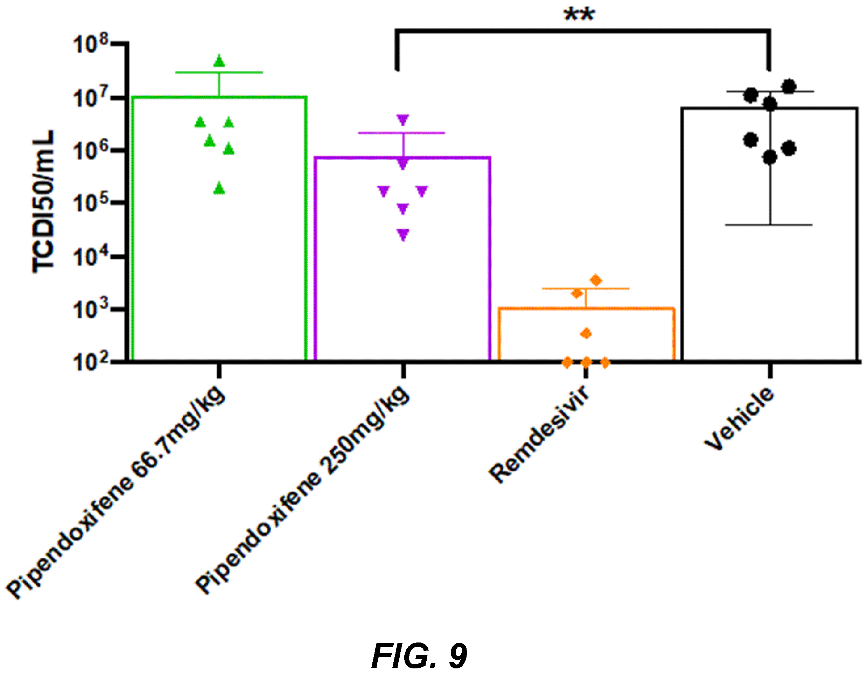

FIG. 9 shows non-limiting exemplary data related to the effect of pipendoxifene administration on lung viral titers in an *in vivo* model of SARS-CoV-2 infection. 129/S mice were intranasally infected with 2.5x10⁴ PFU of MA-SARS-CoV-2 and treated orally with 66.67 or 250 mg/kg pipendoxifene once daily, or subcutaneously 50 mg/kg remdesivir twice daily for 3 days. MA-SARS-CoV-2 lung titers in the pipendoxifene treated group relative to vehicle and remdesivir controls. Virus titers were determined in whole right lung homogenates by TCID₅₀ at day 3 post-infection. N = 6 per group. Data was log transformed and analyzed by two-way ANOVA (*P < 0.05, **P < 0.01, ***P < 0.001, and ****P < 0.0001).
FIG. 10 shows non-limiting exemplary data related to the effect of pipendoxifene treatment on SARS-CoV-2 infection-related weight loss. 129/S mice were intranasally infected with 2.5x10⁴ PFU of MA-SARS-CoV-2 and treated orally with 66.67 or 250 mg/kg pipendoxifene once daily, or subcutaneously with 50 mg/kg remdesivir twice daily for 3 days. Animal weights were monitored daily. N = 11 per group for days -3 to +3, and N=5 after day 4. Data was analyzed by two-way ANOVA (*P < 0.05, **P < 0.01, ***P < 0.001, and ****P < 0.0001).
FIG. 11 depicts non-limiting exemplary docking study data related to the interactions of MDL-001 with the target protein RdRp. The top docking pose of MDL-001 (yellow) in the SARS-CoV-2 RdRp (PDB-ID: 7L1F) active site binding pocket is depicted. Protein is represented by a colored surface, where C atoms are white, O atoms are red, N atoms are blue, and S atoms are yellow.
FIG. 12 depicts the structures of MDL-001, Remdesivir, Nirmatralvir, Molnupiravir, Remdesivir Monophosphate (RMP) and Ritonavir.
FIGS. 13A-13B depict data related to docking scores. FIG. 13A depicts data related to a comparison of docking scores for the 49 compounds against the 5 different RdRp PDBs. FIG. 13B depicts data related to a comparison of docking scores for RMP, Nirmatrelvir, Remdesivir, Molnupiravir and MDL-001 against the five RdRp PDBs; Blue line indicates the average binding score for each compound.
FIGS. 14A-14B depict data related to docking scores. FIG. 14A depicts data related to a comparison of docking scores for the 49 compounds against the 4 different positions in PDB 7l1f. FIG. 14B depicts data related to a comparison of docking scores for RMP, Nirmatrelvir, Remdesivir, Molnupiravir and MDL-001 against the 4 positions in PDB 7l1f; Blue line indicates the average binding score for each compound.
FIG. 15 depicts data related to MA-SARS-CoV-2 lung titers. 129/S mice were intranasally infected with 2.5x10⁴ PFU of MA-SARS-CoV-2 and treated orally with indicated doses of pipendoxifene, or subcutaneously with 100 mg/kg remdesivir twice daily, for 3 days. SARS-CoV-2 titers in the lungs were determined on day 3 post infection. N = 9. Data was log-transformed and analyzed by two-way ANOVA (*P < 0.05, **P < 0.01, ***P < 0.001, and ****P < 0.0001).
FIG. 16 depicts data related to MA-SARS-CoV-2-associated weight loss. 129/S mice were intranasally infected with 2.5x10⁴ PFU of MA-SARS-CoV-2 and treated orally with pipendoxifene, or subcutaneously with 100 mg/kg remdesivir twice daily, for 3 days. Animal weights were monitored daily. N = 9 per group. Data was analyzed by two-way ANOVA (*P < 0.05, **P < 0.01, ***P < 0.001, and ****P < 0.0001).
FIG. 17 depicts data related to pathological severity scores in infected mice. Lungs were harvested on day 3 post-infection, paraffin embedded and 5µm sections stained for H&E. To evaluate comprehensive histological changes, lung tissue sections were scored based on pathological changes outlined in the material and methods.
FIG. 18 depicts data related to IF-based live-virus antiviral (solid lines) and MTT-based cytotoxicity (dashed lines) dose response curves for pipendoxifene, berzosertib, and nirmatrelvir against a panel of SARS-CoV-2 variants in HeLa-ACE2 cells (Replicate 1). The IC₅₀ and CC₅₀ of each compound against each variant is indicated. No loss of activity was observed for tested inhibitors against the Omicron variant. Data are means ± SD of replicate 1 performed in biological triplicate.
FIG. 19 depicts data related to IF-based live-virus antiviral (solid lines) and MTT-based cytotoxicity (dashed lines) dose response curves for pipendoxifene, berzosertib, and nirmatrelvir against a panel of SARS-CoV-2 variants in HeLa-ACE2 cells (Replicate 2). The IC₅₀ and CC₅₀ of each compound against each variant is indicated. No loss of activity was observed for tested inhibitors against the Omicron variant. Data are means ± SD of replicate 2 performed in biological triplicate.
FIG. 20 depicts data related to antiviral IC50 calculated from 6-point dose-response curves in IF-based live-virus antiviral assays for pipendoxifene, berzosertib, and nirmatrelvir against a panel of SARS-CoV-2 variants in HeLa-ACE2 cells. The IC₅₀ was calculated and graphed using GraphPad Prism version 8.0.2. No loss of activity was observed for tested inhibitors against the Omicron variant. Data are means ± SD of two independent replicates performed in biological triplicate.
FIG. 21 depicts data related to IF-based live-virus antiviral (solid lines) and MTT-based cytotoxicity (dashed lines) dose response curves for pipendoxifene and pimodivir against a A/WSN/33 in A549 cells. The IC₅₀ and CC₅₀ of each compound against each variant is indicated.. Data are means ± SD performed in biological triplicate.
FIG. 22 depicts data related to individual plasma concentration for MDL-001 after single oral administration at 250 mg/kg in male SD rat (Group 1).
FIG. 23 depicts data related to average plasma concentration for MDL-001 after single oral administration at 250 mg/kg in male SD rat (Group 1).
FIG. 24 depicts data related to individual plasma concentration for MDL-001 after single oral administration at 500 mg/kg in male SD rat (Group 2).
FIG. 25 depicts data related to average plasma concentration for MDL-001 after single oral administration at 500 mg/kg in male SD rat (Group 2).
FIG. 26 depicts data related to individual plasma concentration for MDL-001 after single oral administration at 750 mg/kg in male SD rat (Group 3).
FIG. 27 depicts data related to average plasma concentration for MDL-001 after single oral administration at 750 mg/kg in male SD rat (Group 3).
FIG. 28 depicts data related to individual plasma concentration for MDL-001 after single oral administration at 1000 mg/kg in male SD rat (Group 4).
FIG. 29 depicts data related to average plasma concentration for MDL-001 after single oral administration at 1000 mg/kg in male SD rat (Group 4).
FIG. 30 depicts data related to a comparison of average plasma concentration for MDL-001 after single oral administration at 250 mg/kg (Group 1), 500 mg/kg (Group 2), 750 mg/kg (Group 3), 1000 mg/kg in male SD rat (Group 4).

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented herein. It will be readily understood that the aspects of the present disclosure, as generally described herein, and illustrated in the Figures, can be arranged, substituted, combined, separated, and designed in a wide variety of different configurations, all of which are explicitly contemplated herein and made part of the disclosure herein.

All patents, published patent applications, other publications, and sequences from GenBank, and other databases referred to herein are incorporated by reference in their entirety with respect to the related technology.

The methods, compounds, pharmaceutical compositions and articles of manufacture provided herein are characterized by a variety of component ingredients, steps of preparation, and steps of execution and associated biophysical, physical, biochemical or chemical parameters. As would be apparent to those of skill in the art, the methods provided herein can include any and all permutations and combinations of the compounds, compositions, articles of manufacture and associated ingredients, steps and/or parameters as described below.

Disclosed herein include methods for preventing, delaying the onset of, or treating an infection or a disease caused by a RNA virus. In some embodiments, the method comprises administering to a subject in need thereof a composition comprising a compound of Formula (I), Formula (II), or Formula (III), or a pharmaceutically acceptable salt, ester, solvate, stereoisomer, tautomer, or prodrug thereof, thereby preventing, delaying the onset of, or treating the infection or the disease.

Disclosed herein include methods for preventing, delaying the onset of, or treating an inflammatory effect of an infection or a disease caused by a RNA virus. In some embodiments, the method comprises administering to a subject in need thereof a composition comprising a compound of Formula (I), Formula (II), or Formula (III), or a pharmaceutically acceptable salt, ester, solvate, stereoisomer, tautomer, or prodrug thereof, thereby preventing, delaying the onset of, or treating the inflammatory effect.

Disclosed herein include kits comprising a compound of Formula (I), Formula (II), or Formula (III), or a pharmaceutically acceptable salt, ester, solvate, stereoisomer, tautomer, or prodrug thereof; and a label indicating that the kit is for preventing, delaying the onset of, or treating an infection or a disease caused by a RNA virus.

Disclosed herein include kits comprising a compound of Formula (I), Formula (II), or Formula (III), or a pharmaceutically acceptable salt, ester, solvate, stereoisomer, tautomer, or prodrug thereof; and a label indicating that the kit is for preventing, delaying the onset of, or treating an inflammatory effect of an infection or a disease caused by a RNA virus.

Disclosed herein include compositions comprising a compound of Formula (I), Formula (II), or Formula (III) or a pharmaceutically acceptable salt, ester, solvate, stereoisomer, tautomer, or prodrug thereof, for use in preventing, delaying the onset of, or treating an infection or a disease caused by a RNA virus.

Disclosed herein include compositions comprising a compound of Formula (I), Formula (II), or Formula (III), or a pharmaceutically acceptable salt, ester, solvate, stereoisomer, tautomer, or prodrug thereof, for use in preventing, delaying the onset of, or treating an inflammatory effect of an infection or a disease caused by a RNA virus.

### Definitions

Unless defined otherwise, technical and scientific terms used herein have the same meaning as commonly understood by one of ordinary skill in the art to which the present disclosure belongs. *See, e.g*. Singleton et al., Dictionary of Microbiology and Molecular Biology 2nd ed., J. Wiley & Sons (New York, NY 1994); Sambrook et al., Molecular Cloning, A Laboratory Manual, Cold Spring Harbor Press (Cold Spring Harbor, NY 1989). For purposes of the present disclosure, the following terms are defined below.

As used herein, a "subject" refers to an animal that is the object of treatment, observation or experiment. "Animals" include cold- and warm-blooded vertebrates and invertebrates such as fish, shellfish, reptiles and, in particular, mammals. "Mammal" includes, without limitation, mice; rats; rabbits; guinea pigs; dogs; cats; sheep; goats; cows; horses; primates, such as monkeys, chimpanzees, and apes, and, in particular, humans.

As used herein, a "patient" refers to a subject that is being treated by a medical professional, such as a Medical Doctor (i.e. Doctor of Allopathic medicine or Doctor of Osteopathic medicine) or a Doctor of Veterinary Medicine, to attempt to cure, or at least ameliorate the effects of, a particular disease or disorder or to prevent the disease or disorder from occurring in the first place.

As used herein, "administration" or "administering" refers to a method of giving a dosage of a pharmaceutically active ingredient to a vertebrate.

As used herein, a "dosage" refers to the combined amount of the active ingredients (e.g., pipendoxifene).

As used herein, "therapeutically effective amount" or "pharmaceutically effective amount" is meant an amount of therapeutic agent, which has a therapeutic effect. The dosages of a pharmaceutically active ingredient which are useful in treatment are therapeutically effective amounts. Thus, as used herein, a therapeutically effective amount means an amount of therapeutic agent which produces the desired therapeutic effect as judged by clinical trial results and/or model animal studies.

As used herein, a "therapeutic effect" relieves, to some extent, one or more of the symptoms of a disease or disorder. For example, a therapeutic effect may be observed by a reduction of the subjective discomfort that is communicated by a subject (e.g., reduced discomfort noted in self-administered patient questionnaire). "Treat," "treatment," or "treating," as used herein refers to administering a therapeutic agent or pharmaceutical composition to a subject for prophylactic and/or therapeutic purposes. The term "prophylactic treatment" refers to treating a subject who does not yet exhibit symptoms of a disease or condition, but who is susceptible to, or otherwise at risk of, a particular disease or condition, whereby the treatment reduces the likelihood that the patient will develop the disease or condition. The term "therapeutic treatment" refers to administering treatment to a subject already suffering from a disease or condition.

As used herein, EC₅₀ is the value of a graded dose response curve that represents the concentration of a compound where 50% of its maximal effect is observed.

As used herein, CC₅₀ is the 50% cytotoxic concentration defined as the compound's concentration (µg/mL) required for the reduction of cell viability by 50%.

As used herein, SI = CC₅₀/EC₅₀. The selectivity index (SI) is a ratio that measures the window between cytotoxicity and antiviral activity by dividing the CC₅₀ value into the EC₅₀ value. The higher the SI ratio, the theoretically more effective and safe a drug would be during in *vivo* treatment for a given viral infection.

"Individual" as used herein refers to a; person, human adult or child, mammal, or non-human primate.

"IC50" as used herein refers to the molar concentration of a compound which binds 50% of receptor related to RNA viral infection in vitro.

"Ki" as used herein refers to the kinetic inhibition constant in molar concentration units which denotes the affinity of the compound for the receptor related to RNA viral infection as measured by a binding assay or as calculated from the IC50 value using the Cheng-Prusoff equation.

"Patient" as used herein refers to a mammal, e.g., a human, mouse, rat, guinea pig, dog, cat, horse, cow, pig, or non-human primate, such as a monkey, chimpanzee, baboon or rhesus.

"Pharmaceutically acceptable" as used herein means approved by a regulatory agency of the Federal or a state government or listed in the U.S. Pharmacopeia or other generally recognized pharmacopeia for use in animals, and more particularly in humans.

"Pharmaceutically acceptable salt" refers to a salt of a compound that is pharmaceutically acceptable and that possesses the desired pharmacological activity of the parent compound. Such salts include but are not limited to: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2- hydroxyethanesulfonic acid, benzenesulfonic acid, 4-chlorobenzenesulfonic acid, 2- naphthalenesulfonic acid, 4-toluenesulfonic acid, camphorsulfonic acid, 4- methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, N- methylglucamine and the like.

"Pharmaceutically acceptable vehicle" refers to a diluent, adjuvant, excipient or carrier with which a compound disclosed herein is administered.

"Preventing" or "prevention" refers to a reduction in risk of acquiring a disease or disorder (i.e., causing at least one of the clinical symptoms of the disease not to develop in a patient that may be exposed to or predisposed to the disease but does not yet experience or display symptoms of the disease).

"Prodrug" refers to a derivative of a drug molecule that requires a transformation within the body to release the active drug. Prodrugs are frequently (though not necessarily) pharmacologically inactive until converted to the parent drug. Typically, prodrugs are designed to overcome pharmaceutical and/or pharmacokinetically based problems associated with the parent drug molecule that would otherwise limit the clinical usefulness of the drug.

"Promoiety" refers to a form of protecting group that when used to mask a functional group within a drug molecule converts the drug into a prodrug. Typically, the promoiety will be attached to the drug via bond(s) that are cleaved by enzymatic or non- enzymatic means in vivo. Ideally, the promoiety is rapidly cleared from the body upon cleavage from the prodrug.

"Protecting group" refers to a grouping of atoms that when attached to a reactive group in a molecule masks, reduces or prevents that reactivity. Examples of protecting groups can be found in Green et al., "Protective Groups in Organic Chemistry", (Wiley, 2.sup.nd ed. 1991) and Harrison et al., "Compendium of Synthetic Organic Methods", Vols. 1 8 (John Wiley and Sons, 1971 1996). Representative amino protecting groups include, but are not limited to, formyl, acetyl, trifluoroacetyl, benzyl, benzyloxycarbonyl ("CBZ"), tert-butoxycarbonyl ("Boc"), trimethylsilyl ("TMS"), 2- trimethylsilyl-ethanesulfonyl ("SES"), trityl and substituted trityl groups, allyloxycarbonyl, 9-fluorenylmethyloxycarbonyl ("FMOC"), nitro-veratryloxycarbonyl ("NVOC") and the like. Representative hydroxy protecting groups include, but are not limited to, those where the hydroxy group is either acylated or alkylated such as benzyl, and trityl ethers as well as alkyl ethers, tetrahydropyranyl ethers, trialkylsilyl ethers and allyl ethers.

"Treating" or "treatment" of any disease or disorder as used herein, refers, in one embodiment, to ameliorating the disease or disorder (i.e., arresting or reducing the development of the disease or at least one of the clinical symptoms thereof). In another embodiment "treating" or "treatment" refers to ameliorating at least one physical parameter, which may not be discernible by the patient. In some embodiments, "treating" or "treatment" refers to inhibiting the disease or disorder, either physically, (e.g., stabilization of a discernible symptom), physiologically, (e.g., stabilization of a physical parameter), or both. In some embodiments, "treating" or "treatment" refers to delaying the onset of the disease or disorder.

"Therapeutically effective amount" as used herein, means the amount of a compound that, when administered to an individual for treating a disease, is sufficient to effect such treatment for the disease or to achieve the desired clinical response. The "therapeutically effective amount" will vary depending on the compound, the disease and its severity and the age, weight, etc., of the patient to be treated.

As used herein, a "subject" refers to an animal that is the object of treatment, observation or experiment. "Animal" includes cold- and warm-blooded vertebrates and invertebrates such as fish, shellfish, reptiles and, in particular, mammals. "Mammal" includes, without limitation, mice; rats; rabbits; guinea pigs; dogs; cats; sheep; goats; cows; horses; primates, such as monkeys, chimpanzees, and apes, and, in particular, humans.

As used herein, a "dosage" refers to an amount of therapeutic agent administered to a patient.

As used herein, a "daily dosage" refers to the total amount of therapeutic agent administered to a patient in a day.

As used herein, the term "therapeutic agent" means a substance that is effective in the treatment of a disease or condition.

The term "alkyl" refers to a straight or branched chain hydrocarbon wherein each of the substituents on the carbons may be independently selected from hydrogen or deuterium.

The term "cycloalkyl" refers to a C₃-C₈ alkyl ring wherein each of the substituents on the carbons may be independently selected from hydrogen or deuterium.

The term "halogen" refers to atoms of the group VIIA elements fluorine, chlorine, bromine, or iodine.

The term "hydroxyl" refers to the functional group of the radical form -OH.

The term "sulfhydryl" refers to the functional group of the radical form -SH.

The term "carbonyl" refers to the functional group consisting of a carbon atom double-bonded to an oxygen atom.

The term "amino" refers to the functional group of the radical form -N(R1)R2, wherein R1 and R2 may independently be either an alkyl or hydrogen atom OR a quaternary ammonium cation, with four total alkyl substituents.

The term "carboxyl" refers to the functional group that comprises a double-bonded carbon atom linked to an oxygen group and a hydroxyl group through a single bond.

The term "*alkoxyalkyl*" refers to the functional group of the radical form -OR, wherein R is an alkyl.

The term "amide" refers to the functional group consisting of a carbonyl group linked to a nitrogen atom. This nitrogen atom may in turn be either unsubstituted or may have up to two alkyl substituents.

The term "aminoalkyl" refers to an amino group unbroken non-cyclic chain of carbon atoms that may be substituted with other chemical groups. It may also be branched or unbranched, substituted or unsubstituted.

The term "aminodialkyl" refers to an amino group substituted with two unbroken non-cyclic chain of carbon atoms that may be substituted with other chemical groups. They may also be branched or unbranched, substituted or unsubstituted.

The term "aminocycloalkyl" refers to a disubstituted amino group wherein the two substituents are connected to form a cyclic ring of 3-8 atoms.

### Pipendoxifene

There is an urgent need for medicines that can be identified quickly as treatments for patients in health crises. A large number of patients with rare, neglected, and challenging diseases have little or no therapeutic options available because the cost:benefit ratio to pharmaceutical companies do not incentivize them to tackle these diseases. An approach to addressing this problem is to use computational technology that is able to predict novel mechanism of action of existing drugs, thereby creating an opportunity to pursue rapid therapeutic development at a much lower cost than traditional paths. The use of software and advanced computing in pharmaceutical research has shown to be an effective approach to discovering new drugs. Many of these computational methods have been adopted from other fields, including mathematics and physics. One of the most fascinating examples of this interdisciplinary approach is the successful application of data science and artificial intelligence to biomedical research. Of particular interest is the use of machine learning and its more sophisticated variant, artificial neural network (ANN) deep learning. ANNs resemble the neural connections of the human brain and work by solving problems - running queries through different hierarchies of concepts and related questions to find answers. These algorithms are trained on existing data in order to make predictions. For example, large experimental datasets of small molecules and their known protein binding targets are curated and processed; an expansive set of chemical descriptors, structural moieties, and biophysical features are then extracted from the small molecules and captured in machine-readable formats. This robust representation of each small molecule structure can then serve as the training set for a probabilistic neural network model, which has no bias towards known pharmacophores *a priori*. The deep learning approach is capable of discovering attributes relevant to binding potency, intrinsic to the chemical composition of a query molecule, that are not readily discernable using empirical methods. The algorithm priority-ranks the predictions using a probability score to identify the compounds disclosed in the application. Thus chemical information can be used in neural networks to predict protein binding interactions for any drug compound, and therefore new therapeutic uses for existing drugs, which alleviates much of the time, cost, and burden of advancing a therapeutic treatment to patients suffering from rare or challenging illnesses, including emerging pathogenic epidemics. The compositions, methods, and kits provided herein can be employed in concert with those described in International Patent Application No. PCT/US2021/035282, entitled "METHODS AND COMPOSITIONS FOR TREATING RNA VIRAL INFECTIONS" filed on June 1, 2021, the content of which is incorporated herein by reference in its entirety. In some embodiments, Pipendoxifene (or an analogue thereof), which was computationally predicted as an antiviral pharmaceutical candidate in said reference, is administered in combination with one or more compounds described in said reference (e.g., those shown in Tables 1-6). The identification of Pipendoxifene as an effective antiviral therapeutic agent as described herein (as well as the dosing regimens and pharmacokinetics described herein) is both novel and unexpected, as others (e.g., groups performing HTS screening) have actively and specifically reported that pipendoxifene is not active against COVID in vitro, let alone in vivo. For example, Pipendoxifene was found to be INACTIVE in various high-throughput screening (HTS) assays, including ReFRAME study A00473 (COVID-19 Cytopathic Effect (CPE) Assay; https://reframedb.org/assays/A00473), ReFRAME study A00511 ("SARS CoV-2 CPE Cytotoxicity Assay"; https://reframedb.org/assays/A00511), and ReFRAME study A00466 ("SARS-CoV-2/HeLa-ACE2 HCI assay - % infected cells"; https://reframedb.org/assays/A00466) in the Scripps ReFrame database (pipendoxifene compound data at https://reframedb.org/compound_data/JICOGKJOQXTAIP-UHFFFAOYSA-N;qid=Q27095593). This illustrates the limitations of currently available HTS screening methods (e.g., drugs that look alike are not necessarily chemically/biologically alike) and the unexpected nature of the present disclosures.

Provided herein is an oral antiviral therapeutic, MDL-001 (Pipendoxifene), which can be used for viral load reduction and/or symptom reduction with activity against SARS-CoV-2 variants and/or pan antiviral activity (*See*, e.g., Examples). As described herein, the antiviral activity of Pipendoxifene (MDL-001) was discovered using artificial intelligence (AI) and machine learning (ML) by the CHEMprint^{™} AI-drug discovery platform. As disclosed herein, this therapeutic can demonstrate broad antiviral activity and can be used to treat SARS-CoV-2/COVID-19.

As shown in the Examples, MDL-001 (Pipendoxifene) demonstrates a combination of potential best-in-class antiviral characteristics. First, in a SARS-CoV-2 adapted mouse model of disease, MDL-001 demonstrated SARS-CoV-2 viral load reduction of log₁₀ 2.7 PFU/mL at 375 mg/kg BID dosing on Day 3 post infection. For comparison, Owen et al, in a November 2, 2021 Science article, reported that PF-07321332 (Nirmatrelvir) demonstrated a log₁₀ 1.91 CCID₅₀/mL reduction in viral load at 1000 mg/kg BID on Day 4, in a separate SARS-CoV-2 adapted mouse model of disease. Second, MDL-001 demonstrated a statistically significant reduction of preclinical symptoms of COVID-19 at the earliest time point observable in the model. Third, in vitro studies found that MDL-001 inhibited SARS-CoV-2 Variants of Concern (VoC's) with nanomolar level activity in a cell based assay of disease. Fourth, MDL-001 was found to inhibit the H1N1 Influenza virus in cell based assays as well.

As shown in the Examples, MDL-001 (Pipendoxifene) can demonstrate a rare combination of nanomolar in vitro activity across VoC's, preclinical symptomatic reduction at the limit of detection of the model, preclinical viral load reduction greater than 2.5 logs, broad-spectrum antiviral activity, and clinical safety and tolerability, which make it a favorable next generation oral SARS-CoV-2 and pan-antiviral therapeutic candidate. There is a need for safe and effective antiviral therapies with broader activity than what is currently available, such as for treatment of patients with various respiratory infections from RNA viruses (e.g., influenza and SARS-CoV-2). These respiratory viral infections often look the same clinically and it takes crucial time to obtain test results and choose the right antiviral for the infection. Similar to how currently available antibiotics can treat many types of bacterial infections, there is a need for a next generation of antivirals having broader activity, allowing effective therapy to be started as quickly as possible. MDL-001 (Pipendoxifene) can fill this therapeutic void and create a new treatment paradigm for respiratory viral illnesses.

As disclosed herein, Pipendoxifene or an analogue thereof comprises to individual stereoisomers, diasteteromers, conformational isomers as well as the racemates and pro-drugs thereof. Analogues of Pipendoxifene can include any compound of Formula (I), Formula (II), or Formula (III) as disclosed herein. In some embodiments, the compound of Formula (I), Formula (II), or Formula (III) is Pipendoxifene. A compound of Formula (I), Formula (II), or Formula (III) (or a pharmaceutically acceptable salt, ester, solvate, stereoisomer, tautomer, or prodrug thereof) can be used, for example, to treat RNA viral infections. For example, a compound of Formula (I), Formula (II), or Formula (III) (or a pharmaceutically acceptable salt, ester, solvate, stereoisomer, tautomer, or prodrug thereof) can be administered to a patient in need (for example, a patient suffering from, or at a risk of developing, one or more of the RNA viral infections disclosed herein) at a daily dosage in the range of about 0.01 to 9000 mg administered orally, for an average adult human. It is recognized by those of skill in the art that the exact dosage may be adjusted depending on the severity of symptoms, body weight of the individual and/or other clinical circumstances existing in a given individual. Moreover, it is also recognized that dosage may be adjusted when a compound of Formula (I), Formula (II), or Formula (III) (or a pharmaceutically acceptable salt, ester, solvate, stereoisomer, tautomer, or prodrug thereof) is used in combination with other pharmacologically active substances.

To prepare the pharmaceutical compositions of the present disclosure, a compound of Formula (I), Formula (II), or Formula (III) (or a pharmaceutically acceptable salt, ester, solvate, stereoisomer, tautomer, or prodrug thereof) can be intimately admixed with a pharmaceutically acceptable vehicle carrier according to conventional pharmaceutical compounding techniques, which may take a wide variety of forms depending on the form of preparation desired for administration (e.g., oral, transdermal, transmucosal, buccal, sublingual, transdermal, inhalation, nasal, rectal, vaginal, parenteral). In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules and tablets, suitable carriers and additives include starches, sugars, diluents, granulating agents, lubricants, binders, disintegrating agents and the like. Because of their ease in administration, tablets and capsules represent an advantageous oral dosage unit form, in which case solid pharmaceutical carriers are obviously employed. If desired, tablets may be sugar coated or enteric coated by standard techniques.

In addition, various controlled- release delivery methods, well known to those skilled in the art may be employed to improve bioavailability, reduce side effects, or transdermal delivery may be facilitated by various permeability enhancers or devises. Suppositories may be prepared, in which case cocoa butter could be used as the carrier. For parenterals, the carrier usually comprise sterile water, though other ingredients, for example, for purposes such as aiding solubility or for preservation, may be included. Injectable suspensions may also be prepared in which case appropriate liquid carriers, suspending agents and the like may be employed. Inhalable formulations and aerosols, topical formulations, nanoparticle and microparticle formulations and bioerodible and non-bioerodible formulations may also be prepared.

Included within the scope of the present disclosure are the various individual anomers, diastereomers and enantiomers as well as mixtures thereof, of a compound of Formula (I), Formula (II), or Formula (III). For example, the selective use of a particular enantiomer (e.g. R or S) of a compound of Formula (I), Formula (II), or Formula (III) (or a pharmaceutically acceptable salt, ester, solvate, stereoisomer, tautomer, or prodrug thereof) to achieve a desired therapeutic effect is contemplated within the scope of the present disclosure since various enantiomers may have differential affinities for the receptor related to RNA viral infection. Also contemplated herein is the selective combination of various individual isomers, such as enantiomers in specific ratios (e.g. 3R:1S) to achieve a therapeutic effect. In addition, the compounds disclosed herein (e.g., Pipendoxifene, a compound of Formula (I), Formula (II), or Formula (III), or a pharmaceutically acceptable salt, ester, solvate, stereoisomer, tautomer, or prodrug thereof) also include any pharmaceutically acceptable salts, for example: alkali metal salts, such as sodium and potassium; ammonium salts; monoalkylammonium salts; dialkylammonium salts; trialkylammonium salts; tetraalkylammonium salts; and tromethamine salts. Hydrates and other solvates of a compound of Formula (I), Formula (II), or Formula (III) (or a pharmaceutically acceptable salt, ester, solvate, stereoisomer, tautomer, or prodrug thereof) are included within the scope of the present disclosure.

Pharmaceutically acceptable salts of Pipendoxifene or analogues thereof can be prepared by reacting Pipendoxifene with the appropriate base and recovering the salt. In some embodiments, Pipendoxifene or an analogues thereof is administered to the subject in a dosage of about 5-25mg twice daily, or about 50mg two or three times daily, or 100mg once, twice or three times daily.

Also included within the scope of the present disclosure are various pro-drugs that may be converted by various physiologic processes into the active drug substance or which otherwise improves the bioavailability and/or pharmacological characteristics of a compound of Formula (I), Formula (II), or Formula (III). It is known to those of skill in the art that such pro-dugs may be created by creating derivatives of a compound of Formula (I), Formula (II), or Formula (III) which may be changed by normal physiologic and/or metabolic processes occurring with the individual into the pharmacologically active molecules of a compound of Formula (I), Formula (II), or Formula (III) by combining the a compound of Formula (I), Formula (II), or Formula (III) with another molecule or promoiety so as to enhance or control for example, absorption, distribution, metabolism and/or excretion in an individual.

The present disclosure also encompasses prodrugs of a compound of Formula (I), Formula (II), or Formula (III), which on administration undergo chemical conversion by metabolic processes before becoming active pharmacological substances. In general, such prodrugs are functional derivatives of a compound of Formula (I), Formula (II), or Formula (III), which are readily convertible in vivo into a compound of Formula (I), Formula (II), or Formula (III). Prodrugs can be any covalently bonded compounds, which release the active parent drug (e.g., Pipendoxifene) in vivo. In cases in which compounds have unsaturated carbon-carbon double bonds, both the cis (Z) and trans (E) isomers are within the scope of the present disclosure. In cases wherein compounds may exist in tautomeric forms, such as ketoenol tautomers, each tautomeric form is contemplated as being included within the present disclosure whether existing in equilibrium or predominantly in one form. Conventional procedures for the selection and preparation of suitable prodrug derivatives are described, for example, in "Design of Prodrugs", ed. H. Bundgaard, Elsevier, 1985. Prodrug designs are generally discussed in Hardma et al. (eds.), Goodman and Gilman's The Pharmacological Basis of Therapeutics, 9th ed., pages 11-16 (1996). A further thorough study of prodrug design is presented in Higuchi et al., Prodrugs as Novel Delivery Systems, vol. 14, ASCD Symposium Series, and in Roche (ed.), Bioreversible Carriers in Drug Design, American Pharmaceutical Association and Pergamon Press (1987).

A compound of Formula (I), Formula (II), or Formula (III) (or a pharmaceutically acceptable salt, ester, solvate, stereoisomer, tautomer, or prodrug thereof) can be linked, coupled or otherwise attached to another molecule which would facilitate the transport of the compounds or derivatives across cellular or tissue barriers. For example, gastrointestinal absorption can be enhanced by coupling, linking or attaching to another molecule such as a bile acid derivative or analogues to exploit the intestinal bile acid uptake pathway so as to enhance the intestinal absorption. Examples of such conjugations of a specific drug molecule with a carrier molecule, for example a bile acid, are well known to those familiar with the art. For example, Kramer (Biochim. Biophys. Acta. 1227: 137-154, 1994b) describes the conjugation of bile acids with cholesterol lowering drugs (i.e. HMG-CoA reductase inhibitors) for example lovastatin to improve gastrointestinal absorption and to facilitate more specific target organ drug delivery.

In addition, a compound of Formula (I), Formula (II), or Formula (III) (or a pharmaceutically acceptable salt, ester, solvate, stereoisomer, tautomer, or prodrug thereof) can be linked, coupled or otherwise attached to molecules which improve penetration of the blood brain barrier. For example, coupling, linking or attaching a compound of Formula (I), Formula (II), or Formula (III) (or a pharmaceutically acceptable salt, ester, solvate, stereoisomer, tautomer, or prodrug thereof) to an essential fatty acid or vitamin to improve penetration into the central nervous system. Such techniques and a large range of molecules and promoieties which can achieve these effects are well known to those skilled in the art of pharmaceutical science. Methods to produce prodrugs using choline derivatives are described in US Patent Application published as US2001007865. The specific examples noted in the foregoing examples are provided for illustrative purposes and are not meant in any way to limit the scope contemplated herein.

A compound of Formula (I), Formula (II), or Formula (III) (or a pharmaceutically acceptable salt, ester, solvate, stereoisomer, tautomer, or prodrug thereof) may be used in conjunction with one or more other therapeutic agents (e.g., drug compounds) and used according to the methods of the present disclosure, for example the therapeutic agents have a use that is also effective in treating RNA viral infection and/or co-morbid conditions.

When administered, the pharmaceutical composition comprising a compound of Formula (I), Formula (II), or Formula (III) (or a pharmaceutically acceptable salt, ester, solvate, stereoisomer, tautomer, or prodrug thereof) is applied in pharmaceutically acceptable amounts and in pharmaceutically acceptable compositions. Such preparations may routinely contain salts, buffering agents, preservatives, compatible carriers, and optionally other therapeutic ingredients. When used in medicine the salts should be pharmaceutically acceptable, but non-pharmaceutically acceptable salts may conveniently be used to prepare pharmaceutically acceptable salts thereof and are not excluded herein. Such pharmacologically and pharmaceutically acceptable salts include, but are not limited to, those prepared from the following acids: hydrochloric, hydrobromic, sulfuric, nitric, phosphoric, maleic, acetic, salicylic, p-toluene sulfonic, tartaric, citric, methane sulfonic, formic, malonic, succinic, naphthalene-2-sulfonic, and benzene sulfonic. Also, pharmaceutically acceptable salts can be prepared as alkaline metal or alkaline earth salts, such as sodium, potassium or calcium salts. Suitable buffering agents include: acetic acid and a salt (1-2% W/V); citric acid and a salt (1-3% W/V); boric acid and a salt (0.5-2.5% W/V); and phosphoric acid and a salt (0.8-2% W/V). Suitable preservatives include benzalkonium chloride (0.003-0.03% W/V); chlorobutanol (0.3-0.9% W/V); parabens (0.01-0.25% W/V) and thimerosal (0.004- 0.02% W/V).

A compound of Formula (I), Formula (II), or Formula (III) (or a pharmaceutically acceptable salt, ester, solvate, stereoisomer, tautomer, or prodrug thereof) is preferred to be administered in safe and effective amounts. An effective amount means that amount necessary to delay; the onset, inhibit the progression, halt altogether the onset or progression of, or to reduce the clinical manifestations or symptoms of the particular condition being treated. In general, an effective amount for treating an RNA viral infection are an amount necessary to inhibit the symptoms of the particular RNA viral infection in situ in a particular individual. When administered to an individual, effective amounts depends on the particular condition being treated; the severity of the condition; individual patient parameters including age, physical condition, size and weight; concurrent treatment; frequency of treatment; and the mode of administration. These factors are well known to those of ordinary skill in the art and can be addressed with no more than routine experimentation. It is preferred generally that a minimum dose be used, that is, the lowest safe dosage that provides appropriate relief of symptoms.

Dosage may be adjusted appropriately to achieve desired drug levels, locally or systemically. Daily doses of active compounds (e.g., Pipendoxifene, a compound of Formula (I), Formula (II), or Formula (III), or a pharmaceutically acceptable salt, ester, solvate, stereoisomer, tautomer, or prodrug thereof) can be from about 0.001 mg/kg per day to 200 mg/kg per day. However, it is recognized that these are general ranges and the actual dose used as contemplated in a given individual may less or greater than this dosage range. In the event that the response in an individual subject is insufficient at such doses, even higher doses (or effective higher doses by a different, more localized delivery route) may be employed to the extent that patient tolerance permits.

A variety of administration routes can be suitable to the methods and compositions disclosed herein. The particular administration route selected can depend upon the particular drug selected, the severity of the disease state(s) being treated and the dosage required for therapeutic efficacy. The methods may be practiced using any mode of administration that is medically acceptable, meaning any mode that produces effective levels of the active compounds (e.g., Pipendoxifene, a compound of Formula (I), Formula (II), or Formula (III), or a pharmaceutically acceptable salt, ester, solvate, stereoisomer, tautomer, or prodrug thereof) without causing clinically unacceptable adverse effects and multiple doses over a given period of time are also contemplated. Such modes of administration include oral, rectal, sublingual, transmucosal, buccal, inhalation, rectal, vaginal, parenteral topical, nasal, transdermal or parenteral routes. The term "parenteral" includes subcutaneous, intravenous, intramuscular, or infusion. Depot intramuscular injections suitably prepared may also be used for administration within the scope of the present disclosure.

The compositions may be conveniently presented in unit dosage form and may be prepared by any of the methods well known in the art of pharmacy. In general, the compositions are prepared by uniformly and intimately bringing the compounds into association with a liquid carrier, a finely divided solid carrier, or both, and then, if necessary, shaping the product.

Compositions suitable for oral administration may be presented as discrete units such as capsules, cachets, tablets, or lozenges, each containing a predetermined amount of the active compound (e.g., Pipendoxifene, a compound of Formula (I), Formula (II), or Formula (III), or a pharmaceutically acceptable salt, ester, solvate, stereoisomer, tautomer, or prodrug thereof). Other compositions include suspensions in aqueous liquors or non- aqueous liquids such as a syrup, an elixir, or an emulsion.

Other delivery systems can include time-release, delayed release, sustained release or targeted release delivery systems. Such systems can avoid repeated administrations of the active compounds (e.g., a compound of Formula (I), Formula (II), or Formula (III), Pipendoxifene, or a pharmaceutically acceptable salt, ester, solvate, stereoisomer, tautomer, or prodrug thereof), increasing convenience to the subject and the physician or target release of the active compound to the tissue of interest. Many types of release delivery systems are available and known to those of ordinary skill in the art. They include polymer based systems such as polylactic and polyglycolic acid, polyanhydrides and polycaprolactone; nonpolymer systems that are lipids including sterols such as cholesterol, cholesterol esters and fatty acids or neutral fats such as mono-, di and triglycerides; hydrogel release systems; silastic systems; peptide based systems; wax coatings, compressed tablets using conventional binders and excipients, partially fused implants and the like. In addition, a pump-based hardware delivery system can be used, some of which are adapted for implantation, others of which are adapted for inhalation administration by nose or mouth.

Long-term sustained release devices, pharmaceutical compositions or molecular derivatives also may be used with a compound of Formula (I), Formula (II), or Formula (III) (or a pharmaceutically acceptable salt, ester, solvate, stereoisomer, tautomer, or prodrug thereof). "Long-term" release, as used herein, means that the drug delivery devise is constructed and arranged to deliver therapeutic levels of the active ingredient for at least 2 days, and preferably as long as 60 days. Long-term sustained release devices such as patches, implants and suppositories are well known to those of ordinary skill in the art and include some of the release systems described above. It is also contemplated by the inventors that a compound of Formula (I), Formula (II), or Formula (III) (or a pharmaceutically acceptable salt, ester, solvate, stereoisomer, tautomer, or prodrug thereof) may be formulated in such ways as to achieve various plasma profiles of the compounds in given individuals so as to maintain certain effective profiles of given plasma levels over a period of time. Such formulation strategies are well known to those skilled in the art and may for example include special coatings on tablets or granules containing a compound of Formula (I), Formula (II), or Formula (III) (or a pharmaceutically acceptable salt, ester, solvate, stereoisomer, tautomer, or prodrug thereof) either alone or in combination with other pharmacologically active substances. All such formulations are contemplated with the scope of the present disclosure.

### Methods for treating viral infection

Disclosed herein include methods for preventing, delaying the onset of, or treating an infection or a disease caused by a RNA virus. In some embodiments, the method comprises administering to a subject in need thereof a composition comprising a compound of Formula (I), Formula (II), or Formula (III), or a pharmaceutically acceptable salt, ester, solvate, stereoisomer, tautomer, or prodrug thereof, thereby preventing, delaying the onset of, or treating the infection or the disease.

Disclosed herein include methods for preventing, delaying the onset of, or treating an inflammatory effect of an infection or a disease caused by a RNA virus. In some embodiments, the method comprises administering to a subject in need thereof a composition comprising a compound of Formula (I), Formula (II), or Formula (III), or a pharmaceutically acceptable salt, ester, solvate, stereoisomer, tautomer, or prodrug thereof, thereby preventing, delaying the onset of, or treating the inflammatory effect.

The inflammatory effect can comprise respiratory failure, a sequela of respiratory failure, acute lung injury, or acute respiratory distress syndrome. The sequela of respiratory failure can comprise multi-organ failure. As used herein, the terms "inflammation" and "inflammatory response" shall be given their ordinary meaning, and also include immune-related responses and/or allergic reactions to a physical, chemical, or biological stimulus. Measuring inflammation (e.g. lung inflammation) can comprise measuring the level of a pro-inflammatory cytokine, an anti-inflammatory cytokine, or a combination of pro-inflammatory cytokines and anti-inflammatory cytokines. Inflammation (e.g. lung inflammation) can comprise mast cell degranulation, plasma extravasation, and bronchoconstriction. Administering the composition can result in an at least, or at least about, 2% (e.g., 2%, 3%, 4%, 5%, 6%, 7%, 8%, 9%, 10%, 11%, 12%, 13%, 14%, 15%, 16%, 17%, 18%, 19%, 20%, 25%, 30%, 40%, 50%, 75%, 100%, 150%, 200%, 250%, 500%, 1000%, or higher and overlapping ranges therein) reduction of one or more of mast cell degranulation, plasma extravasation, and bronchoconstriction. In some embodiments of the methods and compositions provided herein, lymphopenia and/or mononuclear cell infiltration in the lungs is reduced by at least, or at least about, 2% (e.g., 2%, 3%, 4%, 5%, 6%, 7%, 8%, 9%, 10%, 11%, 12%, 13%, 14%, 15%, 16%, 17%, 18%, 19%, 20%, 25%, 30%, 40%, 50%, 75%, 100%, 150%, 200%, 250%, 500%, 1000%, or higher and overlapping ranges therein).

A pro-inflammatory cytokine or a pro-inflammatory mediator can be an immuno-regulatory cytokine that favor inflammation. Pro-inflammatory cytokines that are generally responsible for early immune responses include IL-1, IL-6, and TNF-α. IL-1, IL-6, and TNF-α are also considered endogenous pyrogens as they contribute to increasing body temperature. Other examples of pro-inflammatory cytokines or pro-inflammatory mediators include IL-8, IL-11, IL-12, IL-18, GM-CSF, IFN-γ, TGF-β, leukemia inhibitory factors (LIF), oncostatin M (OSM), and a variety of chemokines that attract inflammatory cells. A pro-inflammatory cytokine generally up-regulates or increases the synthesis of secondary pro-inflammatory mediators and other pro-inflammatory cytokines by immune cells. In addition, pro-inflammatory cytokines can stimulate production of acute phase proteins that mediate inflammation and attract inflammatory cells. The method can comprise an at least, or at least about, 2-fold (e.g., 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, 100-fold, or a number or a range between any of these values) reduction in the level of one or more of interferon-γ (IFNγ), IL-1, IL-6, transforming growth factor-α (TGFα), transforming growth factor-β (TGFβ), CCL2, CXCL10, IL-11, IL-12, IL-18, GM-CSF, CXCL9 and IL-8 in the subject. The compositions and methods provided herein can reduce the production and/or amount of a pro-inflammatory cytokine and/or a pro-inflammatory mediator in the lung and/or serum by at least, or at least about, 2% (e.g., 2%, 3%, 4%, 5%, 6%, 7%, 8%, 9%, 10%, 11%, 12%, 13%, 14%, 15%, 16%, 17%, 18%, 19%, 20%, 25%, 30%, 40%, 50%, 75%, 100%, 150%, 200%, 250%, 500%, 1000%, or higher and overlapping ranges therein) compared to if the methods and compositions are not used.

The composition can comprise a therapeutically or prophylactically effective amount of a compound of Formula (I), Formula (II), or Formula (III) (or a pharmaceutically acceptable salt, ester, solvate, stereoisomer, tautomer, or prodrug thereof). The subject in need can be a subject that can be suffering from the infection or the disease, or a subject that can be at a risk for the infection or the disease. The infection or the disease can be in the respiratory tract of the subject. The subject can have been exposed to the RNA virus, can be suspected to have been exposed to the RNA virus, or can be at a risk of being exposed to the RNA virus. The subject can be a mammal. The subject can be a human.

In some embodiments, the RNA virus can be a double-stranded RNA virus. The RNA virus can be a positive-sense single-stranded RNA virus. The positive-sense single-stranded RNA virus can be a coronavirus. The coronavirus can be an alpha coronavirus, a beta coronavirus, a gamma coronavirus, or a delta coronavirus. The coronavirus can be Middle East respiratory coronavirus (MERS-CoV), severe acute respiratory syndrome coronavirus (SARS-CoV), or SARS-CoV-2. The infection or a disease caused by the RNA virus can be common cold, influenza, SARS, coronaviruses, COVID-19, hepatitis C, hepatitis E, West Nile fever, Ebola virus disease, rabies, polio, or measles.

The method can comprise administering to the subject one or more additional antiviral agents. At least one of the one or more additional antiviral agents can be co-administered to the subject with the composition. At least one of the one or more additional antiviral agents can be administered to the subject before the administration of the composition, after the administration of the composition, or both. The composition can comprise one or more additional therapeutic agents. The one or more additional therapeutic agents comprise one or more antiviral agents. The antiviral agent can be selected from the group consisting of a nucleoside or a non-nucleoside analogue reverse-transcriptase inhibitor, a nucleotide analogue reverse-transcriptase inhibitor, a NS3/4A serine protease inhibitor, a NS5B polymerase inhibitor, and interferon alpha.

The composition can be a pharmaceutical composition comprising a compound of Formula (I), Formula (II), or Formula (III) (or a pharmaceutically acceptable salt, ester, solvate, stereoisomer, tautomer, or prodrug thereof) and one or more pharmaceutically acceptable excipients. The composition can be administered to the subject by intravenous administration, nasal administration, pulmonary administration, oral administration, parenteral administration, or nebulization. The composition can be aspirated into at least one lung of the subject. The composition can be in the form of powder, pill, tablet, microtablet, pellet, micropellet, capsule, capsule containing microtablets, liquid, aerosols, or nanoparticles. The composition can be in a formulation for administration to the lungs. A compound of Formula (I), Formula (II), or Formula (III) (or a pharmaceutically acceptable salt, ester, solvate, stereoisomer, tautomer, or prodrug thereof) can also be used prophylactically for preventing, delaying the onset of, or treating an infection or a disease or inflammation caused by a RNA virus. The prophylactically effective amount of a compound of Formula (I), Formula (II), or Formula (III) (or a pharmaceutically acceptable salt, ester, solvate, stereoisomer, tautomer, or prodrug thereof) can be any therapeutically effective amount described herein.

A compound of Formula (I), Formula (II), or Formula (III) (or a pharmaceutically acceptable salt, ester, solvate, stereoisomer, tautomer, or prodrug thereof) can be administered via any suitable route. Potential routes of administration of a compound of Formula (I), Formula (II), or Formula (III) (or a pharmaceutically acceptable salt, ester, solvate, stereoisomer, tautomer, or prodrug thereof) include without limitation oral, parenteral (including intramuscular, subcutaneous, intradermal, intravascular, intravenous, intraarterial, intramedullary and intrathecal), intracavitary, intraperitoneal, and topical (including dermal/epicutaneous, transdermal, mucosal, transmucosal, intranasal [e.g., by nasal spray or drop], intraocular [e.g., by eye drop], pulmonary [e.g., by oral or nasal inhalation], buccal, sublingual, rectal and vaginal). In certain embodiments, a compound of Formula (I), Formula (II), or Formula (III) (or a pharmaceutically acceptable salt, ester, solvate, stereoisomer, tautomer, or prodrug thereof) is administered orally (e.g., as a capsule or tablet, optionally with an enteric coating). In other embodiments, a compound of Formula (I), Formula (II), or Formula (III) (or a pharmaceutically acceptable salt, ester, solvate, stereoisomer, tautomer, or prodrug thereof) is administered parenterally (e.g., intravenously, subcutaneously or intradermally). In further embodiments, a compound of Formula (I), Formula (II), or Formula (III) (or a pharmaceutically acceptable salt, ester, solvate, stereoisomer, tautomer, or prodrug thereof) is administered topically (e.g., dermally/epicutaneously, transdermally, mucosally, transmucosally, buccally or sublingually).

In additional embodiments, a compound of Formula (I), Formula (II), or Formula (III) (or a pharmaceutically acceptable salt, ester, solvate, stereoisomer, tautomer, or prodrug thereof) is administered without food. In some embodiments, a compound of Formula (I), Formula (II), or Formula (III) (or a pharmaceutically acceptable salt, ester, solvate, stereoisomer, tautomer, or prodrug thereof) is administered at least about 1 or 2 hours before or after a meal. In certain embodiments, a compound of Formula (I), Formula (II), or Formula (III) (or a pharmaceutically acceptable salt, ester, solvate, stereoisomer, tautomer, or prodrug thereof) is administered at least about 2 hours after an evening meal. A compound of Formula (I), Formula (II), or Formula (III) (or a pharmaceutically acceptable salt, ester, solvate, stereoisomer, tautomer, or prodrug thereof) can also be taken substantially concurrently with food (e.g., within about 0.5, 1 or 2 hours before or after a meal, or with a meal).

The composition can be administered to the subject once, twice, or three times a day. The composition can be administered to the subject once every day, every two days, or every three days. The composition can be administered to the subject over the course of at least two weeks, at least three weeks, at least four weeks, or at least five weeks. The therapeutically effective amount and the frequency of administration of, and the length of treatment with a compound of Formula (I), Formula (II), or Formula (III) (or a pharmaceutically acceptable salt, ester, solvate, stereoisomer, tautomer, or prodrug thereof) may depend on various factors, including the nature and the severity of the lung inflammation and/or infection/disease, the potency of the compound, the mode of administration, the age, the body weight, the general health, the gender and the diet of the subject, and the response of the subject to the treatment, and can be determined by the treating physician. In some embodiments, a compound of Formula (I), Formula (II), or Formula (III) (or a pharmaceutically acceptable salt, ester, solvate, stereoisomer, tautomer, or prodrug thereof) is administered under a chronic dosing regimen. In certain embodiments, a therapeutically effective amount of a compound of Formula (I), Formula (II), or Formula (III) (or a pharmaceutically acceptable salt, ester, solvate, stereoisomer, tautomer, or prodrug thereof) is administered over a period of at least about 6 weeks, 2 months, 10 weeks, 3 months, 4 months, 5 months, 6 months, 1 year, 1.5 years, 2 years, 3 years or longer (e.g., at least about 6 weeks, 2 months, 3 months or 6 months).

A compound of Formula (I), Formula (II), or Formula (III) (or a pharmaceutically acceptable salt, ester, solvate, stereoisomer, tautomer, or prodrug thereof) can also be used prophylactically to for preventing, delaying the onset of, or treating an infection or a disease or inflammation caused by a RNA virus. The prophylactically effective amount of a compound of Formula (I), Formula (II), or Formula (III) (or a pharmaceutically acceptable salt, ester, solvate, stereoisomer, tautomer, or prodrug thereof) can be any therapeutically effective amount described herein.

Administrating the composition can result in reduction of the viral titer of the RNA virus in the subject as compared to that in the subject before administration of the composition. The method can comprise determining global virus distribution in the lungs of the subject. The method can comprise measuring the viral titer of the RNA virus in the subject before administering the composition to the subject, after administering the composition to the subject, or both. The viral titer can be lung bulk virus titer.

The method can comprise measuring a neutrophil density within the lungs of the subject. Administering the composition can result in reduction of the neutrophil density within the lungs of the subject as compared to that in the subject before administration of the composition. Administering the composition can result in an at least, or at least about, 2% (e.g., 2%, 3%, 4%, 5%, 6%, 7%, 8%, 9%, 10%, 11%, 12%, 13%, 14%, 15%, 16%, 17%, 18%, 19%, 20%, 25%, 30%, 40%, 50%, 75%, 100%, 150%, 200%, 250%, 500%, 1000%, or higher and overlapping ranges therein) reduction of the neutrophil density within the lungs of the subject as compared to that in the subject before administration of the composition.

The method can comprise measuring a total necrotized cell count within the lungs of the subject. Administering the composition can result in reduction of the total necrotized cell count in the subject as compared to that in the subject before administration of the composition. The method can comprise measuring a total protein level within the lungs of the subject. Administering the composition can result in reduction of the total protein level within the lungs of the subject as compared to that in the subject before administration of the composition. In some embodiments, administering the composition results in an at least, or at least about, 2% (e.g., 2%, 3%, 4%, 5%, 6%, 7%, 8%, 9%, 10%, 11%, 12%, 13%, 14%, 15%, 16%, 17%, 18%, 19%, 20%, 25%, 30%, 40%, 50%, 75%, 100%, 150%, 200%, 250%, 500%, 1000%, or higher and overlapping ranges therein) reduction of the total protein level within the lungs of the subject as compared to that in the subject before administration of the composition.

### Kits and Compositions

Disclosed herein include kits comprising a compound of Formula (I), Formula (II), or Formula (III), or a pharmaceutically acceptable salt, ester, solvate, stereoisomer, tautomer, or prodrug thereof; and a label indicating that the kit is for preventing, delaying the onset of, or treating an infection or a disease caused by a RNA virus.

Disclosed herein include kits comprising a compound of Formula (I), Formula (II), or Formula (III), or a pharmaceutically acceptable salt, ester, solvate, stereoisomer, tautomer, or prodrug thereof; and a label indicating that the kit is for preventing, delaying the onset of, or treating an inflammatory effect of an infection or a disease caused by a RNA virus.

In some embodiments, the label indicates that the kit is for prophylaxis administration. In some embodiments, the label indicates that the kit is for low-risk patients, optionally low-risk patients exposed to an RNA virus or suspected of being exposed to an RNA virus. In some embodiments, the label indicates that the kit is for high-risk and/or severe disease patients post-infection with a RNA virus. In some embodiments, the label indicates a compound of Formula (I), Formula (II), or Formula (III) (or the pharmaceutically acceptable salt, ester, solvate, stereoisomer, tautomer, or prodrug thereof) is administered at a daily dose of at least about 600 mg, 620 mg, 640 mg, 660 mg, 680 mg, 700 mg, 720 mg, 740 mg, 760 mg, 780 mg, 800 mg, 820 mg, 840 mg, 860 mg, 880 mg, 900 mg, 920 mg, 940 mg, 960 mg, 980 mg, 1000 mg, 1020 mg, 1040 mg, 1060 mg, 1080 mg, 1100 mg, 1120 mg, 1140 mg, 1160 mg, 1180 mg, 1200 mg, 1220 mg, 1240 mg, 1260 mg, 1280 mg, 1300 mg, 1320 mg, 1340 mg, 1360 mg, 1380 mg, 1400 mg, 1420 mg, 1440 mg, 1460 mg, 1480 mg, 1500 mg, 1520 mg, 1540 mg, 1560 mg, 1580 mg, 1600 mg, 1620 mg, 1640 mg, 1660 mg, 1680 mg, 1700 mg, 1720 mg, 1740 mg, 1760 mg, 1780 mg, 1800 mg, 1820 mg, 1840 mg, 1860 mg, 1880 mg, 1900 mg, 1920 mg, 1940 mg, 1960 mg, 1980 mg, 2000 mg, 2020 mg, 2040 mg, 2060 mg, 2080 mg, 2100 mg, 2120 mg, 2140 mg, 2160 mg, 2180 mg, 2200 mg, 2220 mg, 2240 mg, 2260 mg, 2280 mg, 2300 mg, 2320 mg, 2340 mg, 2360 mg, 2380 mg, 2400 mg, 2420 mg, 2440 mg, 2460 mg, 2480 mg, or 2500 mg, optionally the administering comprises once daily or twice daily oral administration.

The RNA virus can be a coronavirus. The coronavirus can be Middle East respiratory coronavirus (MERS-CoV), severe acute respiratory syndrome coronavirus (SARS-CoV), or SARS-CoV-2.

Disclosed herein include compositions comprising a compound of Formula (I), Formula (II), or Formula (III), or a pharmaceutically acceptable salt, ester, solvate, stereoisomer, tautomer, or prodrug thereof, for use in preventing, delaying the onset of, or treating an infection or a disease caused by a RNA virus.

Disclosed herein include compositions comprising a compound of Formula (I), Formula (II), or Formula (III), or a pharmaceutically acceptable salt, ester, solvate, stereoisomer, tautomer, or prodrug thereof, for use in preventing, delaying the onset of, or treating an inflammatory effect of an infection or a disease caused by a RNA virus.

The inflammatory effect can comprise respiratory failure, a sequela of respiratory failure, acute lung injury, or acute respiratory distress syndrome. The sequela of respiratory failure can comprise multi-organ failure. The composition can comprise a therapeutically or prophylactically effective amount of a compound of Formula (I), Formula (II), or Formula (III) (or a pharmaceutically acceptable salt, ester, solvate, stereoisomer, tautomer, or prodrug thereof).

The therapeutically effective amount and the frequency of administration of, and the length of treatment with a compound of Formula (I), Formula (II), or Formula (III) (or a pharmaceutically acceptable salt, ester, solvate, stereoisomer, tautomer, or prodrug thereof) may depend on various factors, including the nature and the severity of the lung inflammation and/or infection/disease, the potency of a compound of Formula (I), Formula (II), or Formula (III) (or a pharmaceutically acceptable salt, ester, solvate, stereoisomer, tautomer, or prodrug thereof), the mode of administration, the age, the body weight, the general health, the gender and the diet of the subject, and the response of the subject to the treatment, and can be determined by the treating physician. In some embodiments, a therapeutically effective amount of a compound of Formula (I), Formula (II), or Formula (III) (or a pharmaceutically acceptable salt, ester, solvate, stereoisomer, tautomer, or prodrug thereof) for treating or preventing lung inflammation, an infection, and/or a disease as described herein is about 0.1-200 mg, 0.1-150 mg, 0.1-100 mg, 0.1-50 mg, 0.1-30 mg, 0.5-20 mg, 0.5-10 mg or 1-10 mg (e.g., per day or per dose), or as deemed appropriate by the treating physician, which can be administered in a single dose or in divided doses. In certain embodiments, the therapeutically effective dose (e.g., per day or per dose) of a compound of Formula (I), Formula (II), or Formula (III) (or a pharmaceutically acceptable salt, ester, solvate, stereoisomer, tautomer, or prodrug thereof) for treating or preventing lung inflammation, an infection, and/or a disease as described herein is about 0.1-1 mg (e.g., about 0.1 mg, 0.5 mg or 1 mg), about 1-5 mg (e.g., about 1 mg, 2 mg, 3 mg, 4 mg or 5 mg), about 5-10 mg (e.g., about 5 mg, 6 mg, 7 mg, 8 mg, 9 mg or 10 mg), about 10-20 mg (e.g., about 10 mg, 15 mg or 20 mg), about 20-30 mg (e.g., about 20 mg, 25 mg or 30 mg), about 30-40 mg (e.g., about 30 mg, 35 mg or 40 mg), about 40-50 mg (e.g., about 40 mg, 45 mg or 50 mg), about 50-100 mg (e.g., about 50 mg, 60 mg, 70 mg, 80 mg, 90 mg or 100 mg), about 100-150 mg (e.g., about 100 mg, 125 mg or 150 mg), about 150-200 mg (e.g., about 150 mg, 175 mg or 200 mg), about 200-300 mg (e.g., about 200 mg, 220 mg, 240 mg, 260 mg, 280 mg, or 300 mg), about 300-400 mg (e.g., about 300 mg, 320 mg, 340 mg, 360 mg, 380 mg, or 400 mg), about 400-500 mg (e.g., about 400 mg, 420 mg, 440 mg, 460 mg, 480 mg, or 500 mg), about 500-600 mg (e.g., about 500 mg, 520 mg, 540 mg, 560 mg, 580 mg, or 600 mg), or about 600-700 mg (e.g., about 600 mg, 620 mg, 640 mg, 660 mg, 680 mg, or 700 mg). In certain embodiments, the therapeutically effective dose (e.g., per day or per dose) of a compound of Formula (I), Formula (II), or Formula (III) (or a pharmaceutically acceptable salt, ester, solvate, stereoisomer, tautomer, or prodrug thereof) for treating or preventing lung inflammation, an infection, and/or a disease as described herein is about 600 mg, 620 mg, 640 mg, 660 mg, 680 mg, 700 mg, 720 mg, 740 mg, 760 mg, 780 mg, 800 mg, 820 mg, 840 mg, 860 mg, 880 mg, 900 mg, 920 mg, 940 mg, 960 mg, 980 mg, 1000 mg, 1020 mg, 1040 mg, 1060 mg, 1080 mg, 1100 mg, 1120 mg, 1140 mg, 1160 mg, 1180 mg, 1200 mg, 1220 mg, 1240 mg, 1260 mg, 1280 mg, 1300 mg, 1320 mg, 1340 mg, 1360 mg, 1380 mg, 1400 mg, 1420 mg, 1440 mg, 1460 mg, 1480 mg, 1500 mg, 1520 mg, 1540 mg, 1560 mg, 1580 mg, 1600 mg, 1620 mg, 1640 mg, 1660 mg, 1680 mg, 1700 mg, 1720 mg, 1740 mg, 1760 mg, 1780 mg, 1800 mg, 1820 mg, 1840 mg, 1860 mg, 1880 mg, 1900 mg, 1920 mg, 1940 mg, 1960 mg, 1980 mg, 2000 mg, 2020 mg, 2040 mg, 2060 mg, 2080 mg, 2100 mg, 2120 mg, 2140 mg, 2160 mg, 2180 mg, 2200 mg, 2220 mg, 2240 mg, 2260 mg, 2280 mg, 2300 mg, 2320 mg, 2340 mg, 2360 mg, 2380 mg, 2400 mg, 2420 mg, 2440 mg, 2460 mg, 2480 mg, 2500 mg, or greater. In some embodiments, a compound of Formula (I), Formula (II), or Formula (III) (or a pharmaceutically acceptable salt, ester, solvate, stereoisomer, tautomer, or prodrug thereof) is administered for treating or preventing lung inflammation, an infection, and/or a disease as described herein at a daily dose, weekly dose, and/or monthly dose of about 0.1-1 mg (e.g., about 0.1 mg, 0.5 mg or 1 mg), about 1-5 mg (e.g., about 1 mg, 2 mg, 3 mg, 4 mg or 5 mg), about 5-10 mg (e.g., about 5 mg, 6 mg, 7 mg, 8 mg, 9 mg or 10 mg), about 10-20 mg (e.g., about 10 mg, 15 mg or 20 mg), about 20-30 mg (e.g., about 20 mg, 25 mg or 30 mg), about 30-40 mg (e.g., about 30 mg, 35 mg or 40 mg), about 40-50 mg (e.g., about 40 mg, 45 mg or 50 mg), about 50-100 mg (e.g., about 50 mg, 60 mg, 70 mg, 80 mg, 90 mg or 100 mg), about 100-150 mg (e.g., about 100 mg, 125 mg or 150 mg), about 150-200 mg (e.g., about 150 mg, 175 mg or 200 mg), about 200-300 mg (e.g., about 200 mg, 220 mg, 240 mg, 260 mg, 280 mg, or 300 mg), about 300-400 mg (e.g., about 300 mg, 320 mg, 340 mg, 360 mg, 380 mg, or 400 mg), about 400-500 mg (e.g., about 400 mg, 420 mg, 440 mg, 460 mg, 480 mg, or 500 mg), about 500-600 mg (e.g., about 500 mg, 520 mg, 540 mg, 560 mg, 580 mg, or 600 mg), or about 600-700 mg (e.g., about 600 mg, 620 mg, 640 mg, 660 mg, 680 mg, or 700 mg). In some embodiments, a compound of Formula (I), Formula (II), or Formula (III) (or a pharmaceutically acceptable salt, ester, solvate, stereoisomer, tautomer, or prodrug thereof) is administered for treating or preventing lung inflammation, an infection, and/or a disease as described herein at a daily dose, weekly dose, and/or monthly dose of about 600 mg, 620 mg, 640 mg, 660 mg, 680 mg, 700 mg, 720 mg, 740 mg, 760 mg, 780 mg, 800 mg, 820 mg, 840 mg, 860 mg, 880 mg, 900 mg, 920 mg, 940 mg, 960 mg, 980 mg, 1000 mg, 1020 mg, 1040 mg, 1060 mg, 1080 mg, 1100 mg, 1120 mg, 1140 mg, 1160 mg, 1180 mg, 1200 mg, 1220 mg, 1240 mg, 1260 mg, 1280 mg, 1300 mg, 1320 mg, 1340 mg, 1360 mg, 1380 mg, 1400 mg, 1420 mg, 1440 mg, 1460 mg, 1480 mg, 1500 mg, 1520 mg, 1540 mg, 1560 mg, 1580 mg, 1600 mg, 1620 mg, 1640 mg, 1660 mg, 1680 mg, 1700 mg, 1720 mg, 1740 mg, 1760 mg, 1780 mg, 1800 mg, 1820 mg, 1840 mg, 1860 mg, 1880 mg, 1900 mg, 1920 mg, 1940 mg, 1960 mg, 1980 mg, 2000 mg, 2020 mg, 2040 mg, 2060 mg, 2080 mg, 2100 mg, 2120 mg, 2140 mg, 2160 mg, 2180 mg, 2200 mg, 2220 mg, 2240 mg, 2260 mg, 2280 mg, 2300 mg, 2320 mg, 2340 mg, 2360 mg, 2380 mg, 2400 mg, 2420 mg, 2440 mg, 2460 mg, 2480 mg, 2500 mg, or greater. The daily dose, weekly dose, and/or monthly dose of a compound of Formula (I), Formula (II), or Formula (III) (or a pharmaceutically acceptable salt, ester, solvate, stereoisomer, tautomer, or prodrug thereof) can comprise a single administration (e.g., a weekly dose can administered once per week) or multiple administrations. In some embodiments, the dosing regimen comprises administering one or more loading doses and one or more maintenance doses. The term "loading dose" shall be given its ordinary meaning, and shall also refer to a single dose or short duration regimen of a multiple doses having a dosage higher than one or more maintenance doses. A loading dose can, for example, rapidly increase the blood concentration level of a compound of Formula (I), Formula (II), or Formula (III) (or a pharmaceutically acceptable salt, ester, solvate, stereoisomer, tautomer, or prodrug thereof). In some embodiments, the loading dose can increase the blood concentration of a compound of Formula (I), Formula (II), or Formula (III) (or a pharmaceutically acceptable salt, ester, solvate, stereoisomer, tautomer, or prodrug thereof) to a therapeutically effective level in conjunction with a maintenance dose of the compound. The loading dose can be administered once per day, or more than once per day (e.g., up to 4 times per day). The term "maintenance dose" as used herein shall be given its ordinary meaning, and shall also refer to a dose that is serially administered (e.g., at least twice) which is intended to either slowly raise blood concentration levels of a compound of Formula (I), Formula (II), or Formula (III) (or a pharmaceutically acceptable salt, ester, solvate, stereoisomer, tautomer, or prodrug thereof) to a therapeutically effective level, or to maintain such a therapeutically effective level. The daily dose of the maintenance dose can lower than the total daily dose of the loading dose.

The RNA virus can be a double-stranded RNA virus. The RNA virus can be a positive-sense single-stranded RNA virus. The positive-sense single-stranded RNA virus can be a coronavirus. The coronavirus can be an alpha coronavirus, a beta coronavirus, a gamma coronavirus, or a delta coronavirus. The coronavirus can be Middle East respiratory coronavirus (MERS-CoV), severe acute respiratory syndrome coronavirus (SARS-CoV), or SARS-CoV-2.

The composition can be a pharmaceutical composition comprising a compound of Formula (I), Formula (II), or Formula (III) (or a pharmaceutically acceptable salt, ester, solvate, stereoisomer, tautomer, or prodrug thereof) and one or more pharmaceutically acceptable excipients. The composition can comprise one or more additional therapeutic agents. The one or more additional therapeutic agents comprise one or more antiviral agents. The one or more antiviral agents can be selected from a nucleoside or a non-nucleoside analogue reverse-transcriptase inhibitor, a nucleotide analogue reverse-transcriptase inhibitor, a NS3/4A serine protease inhibitor, a NS5B polymerase inhibitor, and interferon alpha.

The composition can be in the form of powder, pill, tablet, microtablet, pellet, micropellet, capsule, capsule containing microtablets, liquid, aerosols, or nanoparticles. The composition can be in a formulation for administration to the lungs. As disclosed herein, a compound of Formula (I), Formula (II), or Formula (III) (or a pharmaceutically acceptable salt, ester, solvate, stereoisomer, tautomer, or prodrug thereof) can be formulated for administration in a pharmaceutical composition comprising a physiologically acceptable surface active agents, carriers, diluents, excipients, smoothing agents, suspension agents, film forming substances, coating assistants, or a combination thereof. In some embodiments, a compound of Formula (I), Formula (II), or Formula (III) (or a pharmaceutically acceptable salt, ester, solvate, stereoisomer, tautomer, or prodrug thereof) is formulated for administration with a pharmaceutically acceptable carrier or diluent. A compound of Formula (I), Formula (II), or Formula (III) (or a pharmaceutically acceptable salt, ester, solvate, stereoisomer, tautomer, or prodrug thereof) can be formulated as a medicament with a standard pharmaceutically acceptable carrier(s) and/or excipient(s) as is routine in the pharmaceutical art. The exact nature of the formulation will depend upon several factors including the desired route of administration. A compound of Formula (I), Formula (II), or Formula (III) (or a pharmaceutically acceptable salt, ester, solvate, stereoisomer, tautomer, or prodrug thereof) can be formulated for oral, intravenous, intragastric, intravascular or intraperitoneal administration. Standard pharmaceutical formulation techniques may be used, such as those disclosed in Remington's The Science and Practice of Pharmacy, 21st Ed., Lippincott Williams & Wilkins (2005), incorporated herein by reference in its entirety.

The term "pharmaceutically acceptable carrier" or "pharmaceutically acceptable excipient" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. In addition, various adjuvants such as are commonly used in the art may be included. Considerations for the inclusion of various components in pharmaceutical compositions are described, e.g., in Gilman et al. (Eds.) (1990); Goodman and Gilman' s: The Pharmacological Basis of Therapeutics, 8th Ed., Pergamon Press, which is incorporated herein by reference in its entirety.

Some examples of substances, which can serve as pharmaceutically-acceptable carriers or components thereof, are sugars, such as lactose, glucose and sucrose: starches, such as corn starch and potato starch; cellulose and its derivatives, such as sodium carboxymethyi cellulose, powdered tragacanth; malt; gelatin; talc; solid lubricants, such as stearic acid and magnesium stearate; calcium sulfate; vegetable oils, such as peanut oil, cottonseed oil, sesame oil, olive oil, com oil and oil of theobroraa; polyols such as propylene glycol, glycerine, sorbitol, mannitol, and polyethylene glycol; aiginic acid; emulsifiers, such as the TWEENS; wetting agents, such sodium lauryl sulfate; coloring agents; flavoring agents; tableting agents, stabilizers; antioxidants; preservatives; pyrogen-free water; isotonic saline; and phosphate buffer solutions.

The choice of a pharmaceutically-acceptable carrier to be used in conjunction with the subject therapeutic agent is basically determined by the way the composition is to be administered.

The compositions described herein are preferably provided in unit dosage form. As used herein, a "unit dosage form" is a composition containing an amount of a compound of Formula (I), Formula (II), or Formula (III) (or a pharmaceutically acceptable salt, ester, solvate, stereoisomer, tautomer, or prodrug thereof) that is suitable for administration to an animal, preferably mammal subject, in a single dose, according to good medical practice. The preparation of a single or unit dosage form however, does not imply that the dosage form is administered once per day or once per course of therapy. Such dosage forms are contemplated to be administered once, twice, thrice or more per day and may be administered as infusion over a period of time (e.g., from about 30 minutes to about 2-6 hours), or administered as a continuous infusion, and may be given more than once during a course of therapy, though a single administration is not specifically excluded. The skilled artisan will recognize that the formulation does not specifically contemplate the entire course of therapy and such decisions are left for those skilled in the art of treatment rather than formulation.

The compositions useful as described above may be in any of a variety of suitable forms for a variety of routes for administration, for example, for oral, nasal, rectal, topical (including transdermal), ocular, intracerebral, intracranial, intrathecal, intra-arterial, intravenous, intramuscular, or other parental routes of administration. The skilled artisan will appreciate that oral and nasal compositions include compositions that are administered by inhalation, and made using available methodologies. Depending upon the particular route of administration desired, a variety of pharmaceutically-acceptable carriers well-known in the art may be used. Pharmaceutically-acceptable carriers include, for example, solid or liquid fillers, diluents, hydrotropies, surface-active agents, and encapsulating substances. Optional pharmaceutically-active materials may be included, which do not substantially interfere with the activity of a compound of Formula (I), Formula (II), or Formula (III) (or a pharmaceutically acceptable salt, ester, solvate, stereoisomer, tautomer, or prodrug thereof). The amount of carrier employed in conjunction with a compound of Formula (I), Formula (II), or Formula (III) (or a pharmaceutically acceptable salt, ester, solvate, stereoisomer, tautomer, or prodrug thereof) is sufficient to provide a practical quantity of material for administration per unit dose of the disclosed compositions. Techniques and compositions for making dosage forms useful in the methods described herein are described in the following references, ail incorporated by reference herein: Modern Pharmaceutics, 4th Ed., Chapters 9 and 10 (Banker & Rhodes, editors, 2002); Lieberman et αi,, Pharmaceutical Dosage Forms: Tablets (1989), and Ansel, Introduction to Pharmaceutical Dosage Forms 8th Edition (2004).

Various oral dosage forms can be used, including such solid forms as tablets, capsules, and granules. Tablets can be compressed, tablet triturates, enteric-coated, sugar-coated, film-coated, or multiple-compressed, containing suitable binders, lubricants, diluents, disintegrating agents, coloring agents, flavoring agents, flow-inducing agents, and melting agents. Liquid oral dosage forms include aqueous solutions, emulsions, suspensions, solutions and/or suspensions reconstituted from non-effervescent granules, and effervescent preparations reconstituted from effervescent granules, containing suitable solvents, preservatives, emulsifying agents, suspending agents, diluents, sweeteners, melting agents, coloring agents and flavoring agents.

The pharmaceutically-acceptable carriers suitable for the preparation of unit dosage forms for peroral administration is well-known in the art. Tablets typically comprise conventional pharmaceutically -compatible adjuvants as inert diluents, such as calcium carbonate, sodium carbonate, mannitol, lactose and cellulose; binders such as starch, gelatin and sucrose; disintegrants such as starch, alginic acid and croscarmelose; lubricants such as magnesium stearate, stearic acid and talc. Glidants such as silicon dioxide can be used to improve flow characteristics of the powder mixture. Coloring agents, such as the FD&C dyes, can be added for appearance. Sweeteners and flavoring agents, such as aspartame, saccharin, menthol, peppermint, and fruit flavors, are useful adjuvants for chewable tablets. Capsules typically comprise one or more solid diluents disclosed above. The selection of carrier components depends on secondary considerations like taste, cost, and shelf stability, which are not critical, and can be readily made by a person skilled in the art.

Peroral compositions also include liquid solutions, emulsions, suspensions, and the like. The pharmaceutically-acceptable carriers suitable for preparation of such compositions are well known in the art. Typical components of carriers for syrups, elixirs, emulsions and suspensions include ethanol, glycerol, propylene glycol, polyethylene glycol, liquid sucrose, sorbitol and water. For a suspension, typical suspending agents include sodium carboxymethyl cellulose, AVICEL RC-591, tragacanth and sodium alginate; typical wetting agents include lecithin and polvsorbate 80; and typical preservatives include methyl paraben and sodium benzoate. Peroral liquid compositions may also contain one or more components such as sweeteners, flavoring agents and colorants disclosed above.

Other compositions useful for attaining systemic delivery of the subject therapeutic agents include sublingual, buccal and nasal dosage forms. Such compositions typically comprise one or more of soluble filler substances such as sucrose, sorbitol and mannitol; and binders such as acacia, microcrystalline cellulose, carboxymethyl cellulose and hydroxypropyi methyl cellulose. Glidants, lubricants, sweeteners, colorants, antioxidants and flavoring agents disclosed above may also be included.

For topical use, creams, ointments, gels, solutions or suspensions, etc., containing a compound of Formula (I), Formula (II), or Formula (III) (or a pharmaceutically acceptable salt, ester, solvate, stereoisomer, tautomer, or prodrug thereof) are employed. Topical formulations may generally be comprised of a pharmaceutical carrier, co-solvent, emulsifier, penetration enhancer, preservative system, and emollient.

For intravenous administration, a compound of Formula (I), Formula (II), or Formula (III) (or a pharmaceutically acceptable salt, ester, solvate, stereoisomer, tautomer, or prodrug thereof) and compositions described herein may be dissolved or dispersed in a pharmaceutically acceptable diluent, such as a saline or dextrose solution. Suitable excipients may be included to achieve the desired pH, including but not limited to NaOH, sodium carbonate, sodium acetate, HC1, and citric acid. In various embodiments, the pH of the final composition ranges from 2 to 8, or preferably from 4 to 7. Antioxidant excipients may include sodium bisulfite, acetone sodium bisulfite, sodium formaldehyde, suifoxylate, thiourea, and EDTA. Other non-limiting examples of suitable excipients found in the final intravenous composition may include sodium or potassium phosphates, citric acid, tartaric acid, gelatin, and carbohydrates such as dextrose, mannitol, and dextran. Further acceptable excipients are described in Powell, et al., Compendium of Excipients for Parenteral Formulations, PDA J Pharm Sci and Tech 1998, 52 238-31 1 and Nema et al., Excipients and Their Role in Approved Injectable Products: Current Usage and Future Directions, PDA J Pharm Sci and Tech 2011, 65 287-332, both of which are incorporated herein by reference in their entirety. Antimicrobial agents may also be included to achieve a bacteriostatic or fungistatic solution, including but not limited to phenyl mercuric nitrate, thimerosal, benzethonium chloride, benzalkonium chloride, phenol, cresol, and chlorobutanol.

The compositions for intravenous administration may be provided to caregivers in the form of one more solids that are reconstituted with a suitable diluent such as sterile water, saline or dextrose in water shortly prior to administration. In other embodiments, the compositions are provided in solution ready to administer parenterally. In still other embodiments, the compositions are provided in a solution that is further diluted prior to administration. In embodiments that include administering a combination of a compound of Formula (I), Formula (II), or Formula (III) (or a pharmaceutically acceptable salt, ester, solvate, stereoisomer, tautomer, or prodrug thereof) and another agent, the combination may be provided to caregivers as a mixture, or the caregivers may mix the two agents prior to administration, or the two agents may be administered separately.

In non-human animal studies, applications of potential products are commenced at higher dosage levels, with dosage being decreased until the desired effect is no longer achieved or adverse side effects disappear. The dosage may range broadly, depending upon the desired effects and the therapeutic indication. Typically, dosages may be between about 0.1 mg/kg and 4000 mg/kg body weight, preferably between about 80 mg/kg and 1600 mg/kg body weight. Alternatively dosages may be based and calculated upon the surface area of the patient, as understood by those of skill in the art.

Depending on the severity and responsiveness of the condition to be treated, dosing can also be a single administration of a slow release composition, with course of treatment lasting from several days to several weeks or until cure is effected or diminution of the disease state is achieved. The amount of a composition to be administered will depend on many factors including the subject being treated, the severity of the affliction, the manner of administration, the judgment of the prescribing physician. A compound of Formula (I), Formula (II), or Formula (III) (or a pharmaceutically acceptable salt, ester, solvate, stereoisomer, tautomer, or prodrug thereof) or combination of therapeutic agents (e.g., an antiviral agent provided herein in combination with a compound of Formula (I), Formula (II), or Formula (III) (or a pharmaceutically acceptable salt, ester, solvate, stereoisomer, tautomer, or prodrug thereof)) may be administered orally or via injection at a dose from 0, 1 mg/kg to 4000 mg/kg of the patient's body weight per day. The dose range for adult humans is generally from 1 g to 100 g/day. Tablets or other forms of presentation provided in discrete units may conveniently contain an amount of a compound of Formula (I), Formula (II), or Formula (III) (or a pharmaceutically acceptable salt, ester, solvate, stereoisomer, tautomer, or prodrug thereof) or combination of compounds disclosed herein (e.g., an antiviral agent provided herein in combination with a compound of Formula (I), Formula (II), or Formula (III) (or a pharmaceutically acceptable salt, ester, solvate, stereoisomer, tautomer, or prodrug thereof)) which is effective at such dosage or as a multiple of the same, for instance, units containing 1 g to 60 g (for example, from about 5 g to 20 g, from about 10 g to 50 g, from about 20 g to 40 g, or from about 25 g to 35 g). The precise amount of therapeutic agent administered to a patient is the responsibility of the attendant physician. However, the dose employed can depend on a number of factors, including the age and sex of the patient, the precise disorder being treated, and its severity. Additionally, the route of administration may vary depending on the condition and its severity. A typical dose of a compound of Formula (I), Formula (II), or Formula (III) (or a pharmaceutically acceptable salt, ester, solvate, stereoisomer, tautomer, or prodrug thereof) can be from 0,02 g to 1.25 g per kg of body weight, for example from 0.1 g to 0.5 g per kg of body weight, depending on such parameters. In some embodiments, the dosage of a compound of Formula (I), Formula (II), or Formula (III) (or a pharmaceutically acceptable salt, ester, solvate, stereoisomer, tautomer, or prodrug thereof) can be from 1 g to 100 g, for example, from 10 g to 80 g, from 15 g to 60 g, from 20 g to 40 g, or from 25 g to 35 g. A physician will be able to determine the required dosage of a compound of Formula (I), Formula (II), or Formula (III) (or a pharmaceutically acceptable salt, ester, solvate, stereoisomer, tautomer, or prodrug thereof) for any particular subject.

The exact formulation, route of administration and dosage for the pharmaceutical compositions comprising a compound of Formula (I), Formula (II), or Formula (III) (or a pharmaceutically acceptable salt, ester, solvate, stereoisomer, tautomer, or prodrug thereof) or combination of therapeutic agents disclosed herein can be chosen by the individual physician in view of the patient's condition. (See, e.g., Fingl et al. 1975, in "The Pharmacological Basis of Therapeutics," which is hereby incorporated herein by reference, with particular reference to Ch. 1). Typically, the dose range of the composition administered to the patient can be from about 0.1 to about 4000 mg/kg of the patient's body weight. The dosage may be a single one or a series of two or more given in the course of one or more days, as is needed by the patient. In instances where human dosages for therapeutic agents have been established for at least some condition, the present disclosure will use those same dosages, or dosages that are between about 0.1 % and about 5000%, more preferably between about 25% and about 1000% of the established human dosage. Where no human dosage is established, as will be the case for newly-discovered pharmaceutical compounds, a suitable human dosage can be inferred from ED₅₀ or ID₅₀ values, or other appropriate values derived from in vitro or in vivo studies, as qualified by toxicity studies and efficacy studies in animals.

The attending physician would know how to and when to terminate, interrupt, or adjust administration due to toxicity or organ dysfunctions. Conversely, the attending physician would also know to adjust treatment to higher levels if the clinical response were not adequate (precluding toxicity). The magnitude of an administrated dose in the management of the disorder of interest will vary with the severity of the condition to be treated and to the route of administration. The severity of the condition may, for example, be evaluated, in part, by standard prognostic evaluation methods. Further, the dose and perhaps dose frequency, will also vary according to the age, body weight, and response of the individual patient. A program comparable to that discussed above may be used in veterinary medicine.

Although the exact dosage will be determined on a drug-by-drug basis, in most cases, some generalizations regarding the dosage can be made. In cases of administration of a pharmaceutically acceptable salt, dosages may be calculated as the free base. In some embodiments, the composition is administered 1 to 4 times per day. Alternatively the compositions disclosed herein may be administered by continuous intravenous infusion, e.g., at a dose of each active ingredient up to 100 g per day. As will be understood by those of skill in the art, in certain situations it may be necessary to administer the compositions disclosed herein in amounts that exceed, or even far exceed, the above-stated, preferred dosage range in order to effectively and aggressively treat particularly aggressive diseases or infections. In some embodiments, a compound of Formula (I), Formula (II), or Formula (III) (or a pharmaceutically acceptable salt, ester, solvate, stereoisomer, tautomer, or prodrug thereof) or combination of therapeutic agents disclosed herein will be administered for a period of continuous therapy, for example for a week or more, or for months or years.

In some embodiments, the dosing regimen of a compound of Formula (I), Formula (II), or Formula (III) (or a pharmaceutically acceptable salt, ester, solvate, stereoisomer, tautomer, or prodrug thereof) or combination of therapeutic agents disclosed herein is administered for a period of time, which time period can be, for example, from at least about 1 week to at least about 4 weeks, from at least about 4 weeks to at least about 8 weeks, from at least about 4 weeks to at least about 12 weeks, from at least about 4 weeks to at least about 16 weeks, or longer. The dosing regimen of a compound of Formula (I), Formula (II), or Formula (III) (or a pharmaceutically acceptable salt, ester, solvate, stereoisomer, tautomer, or prodrug thereof) or combination of therapeutic agents disclosed herein can be administered three times a day, twice a day, daily, every other day, three times a week, every other week, three times per month, once monthly, substantially continuously or continuously.

A compound disclosed herein (e.g., Pipendoxifene, a compound of Formula (I), Formula (II), or Formula (III), or a pharmaceutically acceptable salt, ester, solvate, stereoisomer, tautomer, or prodrug thereof) can be administered alone or in the form of a composition (e.g., a pharmaceutical composition). In some embodiments, a pharmaceutical composition comprises a compound of Formula (I), Formula (II), or Formula (III) (or a pharmaceutically acceptable salt, ester, solvate, stereoisomer, tautomer, or prodrug thereof), and one or more pharmaceutically acceptable carriers or excipients. The composition can optionally contain one or more additional therapeutic agents as described herein. A pharmaceutical composition contains a therapeutically effective amount of a compound of Formula (I), Formula (II), or Formula (III) (or a pharmaceutically acceptable salt, ester, solvate, stereoisomer, tautomer, or prodrug thereof) and one or more pharmaceutically acceptable carriers or excipients, and is formulated for administration to a subject for therapeutic use. For purposes of the content of a pharmaceutical composition, the terms "therapeutic agent", "active ingredient", "active agent" and "drug" encompass prodrugs.

A pharmaceutical composition can contain a compound of Formula (I), Formula (II), or Formula (III) (or a pharmaceutically acceptable salt, ester, solvate, stereoisomer, tautomer, or prodrug thereof) in substantially pure form. In some embodiments, the purity of the therapeutic agent is at least about 95%, 96%, 97%, 98% or 99%. In certain embodiments, the purity of the therapeutic agent is at least about 98% or 99%. In addition, a pharmaceutical composition is substantially free of contaminants or impurities. In some embodiments, the level of contaminants or impurities other than residual solvent in a pharmaceutical composition is no more than about 5%, 4%, 3%, 2% or 1% relative to the combined weight of the intended active and inactive ingredients. In certain embodiments, the level of contaminants or impurities other than residual solvent in a pharmaceutical composition is no more than about 2% or 1% relative to the combined weight of the intended active and inactive ingredients. Pharmaceutical compositions generally are prepared according to current good manufacturing practice (GMP), as recommended or required by, e.g., the Federal Food, Drug, and Cosmetic Act §501(a)(2)(B) and the International Conference on Harmonisation Q7 Guideline.

Pharmaceutically acceptable carriers and excipients include pharmaceutically acceptable materials, vehicles and substances. Non-limiting examples of excipients include liquid and solid fillers, diluents, binders, lubricants, glidants, solubilizers, surfactants, dispersing agents, disintegration agents, emulsifying agents, wetting agents, suspending agents, thickeners, solvents, isotonic agents, buffers, pH adjusters, stabilizers, preservatives, antioxidants, antimicrobial agents, antibacterial agents, antifungal agents, absorption- delaying agents, sweetening agents, flavoring agents, coloring agents, adjuvants, encapsulating materials and coating materials. The use of such excipients in pharmaceutical formulations is known in the art. For example, conventional vehicles and carriers include without limitation oils (e.g., vegetable oils, such as sesame oil), aqueous solvents (e.g., saline, phosphate-buffered saline [PBS] and isotonic solutions [e.g., Ringer's solution]), and solvents (e.g., dimethyl sulfoxide [DMSO] and alcohols [e.g., ethanol, glycerol and propylene glycol]). Except insofar as any conventional carrier or excipient is incompatible with the active ingredient, the disclosure encompasses the use of conventional carriers and excipients in formulations containing a therapeutic agent (e.g., Pipendoxifene, a compound of Formula (I), Formula (II), or Formula (III), or a pharmaceutically acceptable salt, ester, solvate, stereoisomer, tautomer, or prodrug thereof). See, e.g., Remington: The Science and Practice of Pharmacy, 21st Ed., Lippincott Williams & Wilkins (Philadelphia, Pennsylvania [2005]); Handbook of Pharmaceutical Excipients, 5th Ed., Rowe et al., Eds., The Pharmaceutical Press and the American Pharmaceutical Association (2005); Handbook of Pharmaceutical Additives, 3rd Ed., Ash and Ash, Eds., Gower Publishing Co. (2007); and Pharmaceutical Preformulation and Formulation, Gibson, Ed., CRC Press (Boca Raton, Florida, 2004).

Proper formulation can depend on various factors, such as the mode of administration chosen. Potential modes of administration of pharmaceutical compositions comprising a compound of Formula (I), Formula (II), or Formula (III) (or a pharmaceutically acceptable salt, ester, solvate, stereoisomer, tautomer, or prodrug thereof) include without limitation oral, parenteral (including intramuscular, subcutaneous, intradermal, intravascular, intravenous, intraarterial, intraperitoneal, intramedullary, intrathecal and topical), intracavitary, and topical (including dermal/epicutaneous, transdermal, mucosal, transmucosal, intranasal [e.g., by nasal spray or drop], pulmonary [e.g., by oral or nasal inhalation], buccal, sublingual, rectal [e.g., by suppository], and vaginal [e.g., by suppository]).

As an example, formulations of a compound of Formula (I), Formula (II), or Formula (III) (or a pharmaceutically acceptable salt, ester, solvate, stereoisomer, tautomer, or prodrug thereof) suitable for oral administration can be presented as, e.g., boluses; tablets, capsules, pills, cachets or lozenges; as powders or granules; as semisolids, electuaries, pastes or gels; as solutions or suspensions in an aqueous liquid or/and a non-aqueous liquid; or as oil-in-water liquid emulsions or water- in-oil liquid emulsions.

Tablets can contain a compound of Formula (I), Formula (II), or Formula (III) (or a pharmaceutically acceptable salt, ester, solvate, stereoisomer, tautomer, or prodrug thereof) in admixture with, e.g., a filler or inert diluent (e.g., calcium carbonate, calcium phosphate, lactose, mannitol or microcrystalline cellulose), a binding agent (e.g., a starch, gelatin, acacia, alginic acid or a salt thereof, or microcrystalline cellulose), a lubricating agent (e.g., stearic acid, magnesium stearate, talc or silicon dioxide), and a disintegrating agent (e.g., crospovidone, croscarmellose sodium or colloidal silica), and optionally a surfactant (e.g., sodium lauryl sulfate). The tablets can be uncoated or can be coated with, e.g., an enteric coating that protects the active ingredient from the acidic environment of the stomach, or with a material that delays disintegration and absorption of the active ingredient in the gastrointestinal tract and thereby provides a sustained action over a longer time period. In certain embodiments, a tablet comprises a compound of Formula (I), Formula (II), or Formula (III) (or a pharmaceutically acceptable salt, ester, solvate, stereoisomer, tautomer, or prodrug thereof), mannitol, microcrystalline cellulose, magnesium stearate, silicon dioxide, croscarmellose sodium and sodium lauryl sulfate, and optionally lactose monohydrate, and the tablet is optionally film-coated (e.g., with Opadry^{®}).

Push-fit capsules or two-piece hard gelatin capsules can contain a compound of Formula (I), Formula (II), or Formula (III) (or a pharmaceutically acceptable salt, ester, solvate, stereoisomer, tautomer, or prodrug thereof) in admixture with, e.g., a filler or inert solid diluent (e.g., calcium carbonate, calcium phosphate, kaolin or lactose), a binder (e.g., a starch), a glidant or lubricant (e.g., talc or magnesium stearate), and a disintegrant (e.g., crospovidone), and optionally a stabilizer or/and a preservative. For soft capsules or single-piece gelatin capsules, a compound of Formula (I), Formula (II), or Formula (III) (or a pharmaceutically acceptable salt, ester, solvate, stereoisomer, tautomer, or prodrug thereof) can be dissolved or suspended in a suitable liquid (e.g., liquid polyethylene glycol or an oil medium, such as a fatty oil, peanut oil, olive oil or liquid paraffin), and the liquid-filled capsules can contain one or more other liquid excipients or/and semi- solid excipients, such as a stabilizer or/and an amphiphilic agent (e.g., a fatty acid ester of glycerol, propylene glycol or sorbitol).

Compositions for oral administration can also be formulated as solutions or suspensions in an aqueous liquid or/and a non-aqueous liquid, or as oil-in-water liquid emulsions or water-in-oil liquid emulsions. Dispersible powder or granules of a compound of Formula (I), Formula (II), or Formula (III) (or a pharmaceutically acceptable salt, ester, solvate, stereoisomer, tautomer, or prodrug thereof) can be mixed with any suitable combination of an aqueous liquid, an organic solvent or/and an oil and any suitable excipients (e.g., any combination of a dispersing agent, a wetting agent, a suspending agent, an emulsifying agent or/and a preservative) to form a solution, suspension or emulsion.

In some embodiments, a compound of Formula (I), Formula (II), or Formula (III) (or a pharmaceutically acceptable salt, ester, solvate, stereoisomer, tautomer, or prodrug thereof) is contained in an amphiphilic vehicle of a liquid or semi-solid formulation for oral administration which provides improved solubility, stability and bioavailability of the compound, as described in US 2010/0209496. The amphiphilic vehicle contains a solution, suspension, emulsion (e.g., oil-in-water emulsion) or semi-solid mixture of the compound admixed with liquid or/and semi-solid excipients which fills an encapsulated dosage form (e.g., a hard gelatin capsule or a soft gelatin capsule containing a plasticizer [e.g., glycerol or/and sorbitol]). In some embodiments, the amphiphilic vehicle comprises an amphiphilic agent selected from fatty acid esters of glycerol (glycerin), propylene glycol and sorbitol. In certain embodiments, the amphiphilic agent is selected from mono- and di-glycerides of C₈-C₁₂ saturated fatty acids. In further embodiments, the amphiphilic agent is selected from CAPMUL^{®} MCM, CAPMUL^{®} MCM 8, CAPMUL^{®} MCM 10, IMWITOR^{®} 308, IMWITOR^{®} 624, IMWITOR^{®} 742, IMWITOR^{®} 988, CAPRYOL^{™} PGMC, CAPRYOL^{™} 90, LAUROGLYCOL^{™} 90, CAPTEX^{®} 200, CRILL^{™} 1, CRILL^{™} 4, PECEOL^{®} and MAIS INE^{™} 35-1. In some embodiments, the amphiphilic vehicle further comprises propylene glycol, a propylene glycol- sparing agent (e.g., ethanol or/and glycerol), or an antioxidant (e.g., butylated hydroxyanisole, butylated hydroxytoluene, propyl gallate or/and sodium sulfite), or any combination thereof. In additional embodiments, the amphiphilic vehicle contains on a weight basis about 0.1-5% of the compound, about 50-90% of the amphiphilic agent, about 5-40% of propylene glycol, about 5-20% of the propylene glycol- sparing agent, and about 0.01-0.5% of the antioxidant.

A compound of Formula (I), Formula (II), or Formula (III) (or a pharmaceutically acceptable salt, ester, solvate, stereoisomer, tautomer, or prodrug thereof) can also be formulated for parenteral administration by injection or infusion to circumvent gastrointestinal absorption and first-pass metabolism. A representative parenteral route is intravenous.

Additional advantages of intravenous administration include direct administration of a therapeutic agent into systemic circulation to achieve a rapid systemic effect, and the ability to administer the agent continuously or/and in a large volume if desired. Formulations for injection or infusion can be in the form of, e.g., solutions, suspensions or emulsions in oily or aqueous vehicles, and can contain excipients such as suspending agents, dispersing agents or/and stabilizing agents. For example, aqueous or non-aqueous (e.g., oily) sterile injection solutions can contain a compound of Formula (I), Formula (II), or Formula (III) (or a pharmaceutically acceptable salt, ester, solvate, stereoisomer, tautomer, or prodrug thereof) along with excipients such as an antioxidant, a buffer, a bacteriostat and solutes that render the formulation isotonic with the blood of the subject. Aqueous or non-aqueous sterile suspensions can contain a compound of Formula (I), Formula (II), or Formula (III) (or a pharmaceutically acceptable salt, ester, solvate, stereoisomer, tautomer, or prodrug thereof) along with excipients such as a suspending agent and a thickening agent, and optionally a stabilizer and an agent that increases the solubility of the compound to allow for the preparation of a more concentrated solution or suspension. As another example, a sterile aqueous solution for injection or infusion (e.g., subcutaneously or intravenously) can contain a compound of Formula (I), Formula (II), or Formula (III) (or a pharmaceutically acceptable salt, ester, solvate, stereoisomer, tautomer, or prodrug thereof) , NaCl, a buffering agent (e.g., sodium citrate), a preservative (e.g., meta-cresol), and optionally a base (e.g., NaOH) or/and an acid (e.g., HC1) to adjust pH.

For topical administration, a compound of Formula (I), Formula (II), or Formula (III) (or a pharmaceutically acceptable salt, ester, solvate, stereoisomer, tautomer, or prodrug thereof) can be formulated as, e.g., a buccal or sublingual tablet or pill. Advantages of a buccal or sublingual tablet or pill include avoidance of first-pass metabolism and circumvention of gastrointestinal absorption. A buccal or sublingual tablet or pill can also be designed to provide faster release of the compound for more rapid uptake of it into systemic circulation. In addition to a therapeutically effective amount of a compound of Formula (I), Formula (II), or Formula (III) (or a pharmaceutically acceptable salt, ester, solvate, stereoisomer, tautomer, or prodrug thereof), the buccal or sublingual tablet or pill can contain suitable excipients, including without limitation any combination of fillers and diluents (e.g., mannitol and sorbitol), binding agents (e.g., sodium carbonate), wetting agents (e.g., sodium carbonate), disintegrants (e.g., crospovidone and croscarmellose sodium), lubricants (e.g., silicon dioxide [including colloidal silicon dioxide] and sodium stearyl fumarate), stabilizers (e.g., sodium bicarbonate), flavoring agents (e.g., spearmint flavor), sweetening agents (e.g., sucralose), and coloring agents (e.g., yellow iron oxide).

For topical administration, a compound of Formula (I), Formula (II), or Formula (III) (or a pharmaceutically acceptable salt, ester, solvate, stereoisomer, tautomer, or prodrug thereof) can also be formulated for intranasal administration. The nasal mucosa provides a big surface area, a porous endothelium, a highly vascular subepithelial layer and a high absorption rate, and hence allows for high bioavailability. An intranasal solution or suspension formulation can comprise a compound of Formula (I), Formula (II), or Formula (III) (or a pharmaceutically acceptable salt, ester, solvate, stereoisomer, tautomer, or prodrug thereof) along with excipients such as a solubility enhancer (e.g., propylene glycol), a humectant (e.g., mannitol or sorbitol), a buffer and water, and optionally a preservative (e.g., benzalkonium chloride), a mucoadhesive agent (e.g., hydroxyethylcellulose) or/and a penetration enhancer. In certain embodiments, a nasal spray formulation comprises a compound of Formula (I), Formula (II), or Formula (III) (or a pharmaceutically acceptable salt, ester, solvate, stereoisomer, tautomer, or prodrug thereof) , microcrystalline cellulose, sodium carboxymethylcellulose, dextrose and water, and optionally an acid (e.g., HC1) to adjust pH. An intranasal solution or suspension formulation can be administered to the nasal cavity by any suitable means, including but not limited to a dropper, a pipette, or spray using, e.g., a metering atomizing spray pump.

An additional mode of topical administration is pulmonary, including by oral inhalation and nasal inhalation, which is described in detail below.

Other suitable topical formulations and dosage forms include without limitation ointments, creams, gels, lotions, pastes and the like, as described in Remington: The Science and Practice of Pharmacy, 21st Ed., Lippincott Williams & Wilkins (Philadelphia, Pennsylvania, 2005).

Ointments are semi-solid preparations that are typically based on petrolatum or a petroleum derivative. Creams are viscous liquids or semi-solid emulsions, either oil-in-water or water-in-oil. Cream bases are water-washable, and contain an oil phase, an emulsifier and an aqueous phase. The oil phase, also called the "internal" phase, generally comprises petrolatum and a fatty alcohol (e.g., cetyl or stearyl alcohol). The aqueous phase typically, although not necessarily, exceeds the oil phase in volume, and usually contains a humectant. The emulsifier in a cream formulation is generally a non-ionic, anionic, cationic or amphoteric surfactant. Gels are semi-solid, suspension-type systems. Single-phase gels contain organic macromolecules (polymers) distributed substantially uniformly throughout the carrier liquid, which is typically aqueous but can also contain an alcohol (e.g., ethanol or isopropanol) and optionally an oil. Lotions are preparations to be applied to the skin surface without friction, and are typically liquid or semi-liquid preparations in which solid particles, including the active agent, are present in a water or alcohol base. Lotions are usually suspensions of finely divided solids and typically contain suspending agents to produce better dispersion as well as compounds useful for localizing and holding the active agent in contact with the skin. Pastes are semi-solid dosage forms in which the active agent is suspended in a suitable base. Depending on the nature of the base, pastes are divided between fatty pastes or those made from single-phase aqueous gels.

Various excipients can be included in a topical formulation. For example, solvents, including a suitable amount of an alcohol, can be used to solubilize the active agent. Other optional excipients include without limitation gelling agents, thickening agents, emulsifiers, surfactants, stabilizers, buffers, antioxidants, preservatives, cooling agents (e.g., menthol), opacifiers, fragrances and colorants. For an active agent having a low rate of permeation through the skin or mucosal tissue, a topical formulation can contain a permeation enhancer to increase the permeation of the active agent through the skin or mucosal tissue. A topical formulation can also contain an irritation-mitigating excipient that reduces any irritation to the skin or mucosa caused by the active agent, the permeation enhancer or any other component of the formulation.

In some embodiments, a compound of Formula (I), Formula (II), or Formula (III) (or a pharmaceutically acceptable salt, ester, solvate, stereoisomer, tautomer, or prodrug thereof) is delivered from a sustained-release composition. As used herein, the term "sustained-release composition" encompasses sustained-release, prolonged-release, extended-release, slow-release and controlled-release compositions, systems and devices. Use of a sustained- release composition can have benefits, such as an improved profile of the amount of the drug or an active metabolite thereof delivered to the target site(s) over a time period, including delivery of a therapeutically effective amount of the drug or an active metabolite thereof over a prolonged time period. In certain embodiments, the sustained-release composition delivers the compound over a period of at least about 1 day, 2 days, 3 days, 1 week, 2 weeks, 3 weeks, 1 month, 2 months, 3 months or longer. In some embodiments, the sustained-release composition is a drug-encapsulation system, such as nanoparticles, microparticles or a capsule made of, e.g., a biodegradable polymer or/and a hydrogel. In certain embodiments, the sustained-release composition comprises a hydrogel. Non-limiting examples of polymers of which a hydrogel can be composed include polyvinyl alcohol, acrylate polymers (e.g., sodium poly acrylate), and other homopolymers and copolymers having a relatively large number of hydrophilic groups (e.g., hydroxyl or/and carboxylate groups). In other embodiments, the sustained-release drug-encapsulation system comprises a membrane- enclosed reservoir, wherein the reservoir contains a drug and the membrane is permeable to the drug. Such a drug-delivery system can be in the form of, e.g., a transdermal patch.

In some embodiments, the sustained-release composition is an oral dosage form, such as a tablet or capsule. For example, a drug can be embedded in an insoluble porous matrix such that the dissolving drag must make its way out of the matrix before it can be absorbed through the gastrointestinal tract. Alternatively, a drug can be embedded in a matrix that swells to form a gel through which the drug exits. Sustained release can also be achieved by way of a single-layer or multi-layer osmotic controlled-release oral delivery system (OROS). An OROS is a tablet with a semi-permeable outer membrane and one or more small laser- drilled holes in it. As the tablet passes through the body, water is absorbed through the semipermeable membrane via osmosis, and the resulting osmotic pressure pushes the drug out through the hole(s) in the tablet and into the gastrointestinal tract where it can be absorbed.

In further embodiments, the sustained-release composition is formulated as polymeric nanoparticles or microparticles, wherein the polymeric particles can be delivered, e.g., by inhalation or injection or from an implant. In some embodiments, the polymeric implant or polymeric nanoparticles or microparticles are composed of a biodegradable polymer. In certain embodiments, the biodegradable polymer comprises lactic acid or/and glycolic acid [e.g., an L-lactic acid-based copolymer, such as poly(L-lactide-co-glycolide) or poly(L-lactic acid-co-D,L-2-hydroxyoctanoic acid)]. For example, biodegradable polymeric microspheres composed of polylactic acid or/and polyglycolic acid can serve as sustained-release pulmonary drug-delivery systems. The biodegradable polymer of the polymeric implant or polymeric nanoparticles or microparticles can be selected so that the polymer substantially completely degrades around the time the period of treatment is expected to end, and so that the byproducts of the polymer's degradation, like the polymer, are biocompatible.

For a delayed or sustained release of a compound of Formula (I), Formula (II), or Formula (III) (or a pharmaceutically acceptable salt, ester, solvate, stereoisomer, tautomer, or prodrug thereof), a composition can also be formulated as a depot that can be implanted in or injected into a subject, e.g., intramuscularly or subcutaneously. A depot formulation can be designed to deliver the compound over a longer period of time, e.g., over a period of at least about 1 week, 2 weeks, 3 weeks, 1 month, 6 weeks, 2 months, 3 months or longer. For example, the compound can be formulated with a polymeric material (e.g., polyethylene glycol (PEG), polylactic acid (PLA) or polyglycolic acid (PGA), or a copolymer thereof (e.g., PLGA)), a hydrophobic material (e.g., as an emulsion in an oil) or/and an ion- exchange resin, or as a sparingly soluble derivative (e.g., a sparingly soluble salt). As an illustrative example, a compound of Formula (I), Formula (II), or Formula (III) (or a pharmaceutically acceptable salt, ester, solvate, stereoisomer, tautomer, or prodrug thereof) can be incorporated or embedded in sustained-release microparticles composed of PLGA and formulated as a monthly depot.

A compound of Formula (I), Formula (II), or Formula (III) (or a pharmaceutically acceptable salt, ester, solvate, stereoisomer, tautomer, or prodrug thereof) can also be contained or dispersed in a matrix material. The matrix material can comprise a polymer (e.g., ethylene-vinyl acetate) and controls the release of the compound by controlling dissolution or/and diffusion of the compound from, e.g., a reservoir, and can enhance the stability of the compound while contained in the reservoir. Such a release system can be designed as a sustained-release system, can be configured as, e.g., a transdermal or transmucosal patch, and can contain an excipient that can accelerate the compound's release, such as a water- swellable material (e.g., a hydrogel) that aids in expelling the compound out of the reservoir. For example, U.S. Patent Nos. 4,144,317 and 5,797,898 describe examples of such a release system.

The release system can provide a temporally modulated release profile (e.g., pulsatile release) when time variation in plasma levels is desired, or a more continuous or consistent release profile when a constant plasma level is desired. Pulsatile release can be achieved from an individual reservoir or from a plurality of reservoirs. For example, where each reservoir provides a single pulse, multiple pulses ("pulsatile" release) are achieved by temporally staggering the single pulse release from each of multiple reservoirs.

Alternatively, multiple pulses can be achieved from a single reservoir by incorporating several layers of a release system and other materials into a single reservoir. Continuous release can be achieved by incorporating a release system that degrades, dissolves, or allows diffusion of a compound through it over an extended time period. In addition, continuous release can be approximated by releasing several pulses of a compound in rapid succession ("digital" release). An active release system can be used alone or in conjunction with a passive release system, as described in U.S. Patent No. 5,797,898.

In addition, pharmaceutical compositions comprising a compound of Formula (I), Formula (II), or Formula (III) (or a pharmaceutically acceptable salt, ester, solvate, stereoisomer, tautomer, or prodrug thereof) can be formulated as, e.g., liposomes, micelles (e.g., those composed of biodegradable natural or/and synthetic polymers, such as lactosomes), microspheres, microparticles or nanoparticles, whether or not designed for sustained release. For example, liposomes can be used as sustained release pulmonary drug-delivery systems that deliver drugs to the alveolar surface for treatment of lung diseases and systemic diseases.

The pharmaceutical compositions can be manufactured in any suitable manner known in the art, e.g., by means of conventional mixing, dissolving, suspending, granulating, dragee-making, levigating, emulsifying, encapsulating, entrapping or compressing processes.

A pharmaceutical composition can be presented in unit dosage form as a single dose wherein all active and inactive ingredients are combined in a suitable system, and components do not need to be mixed to form the composition to be administered. The unit dosage form can contain an effective dose, or an appropriate fraction thereof, of a compound of Formula (I), Formula (II), or Formula (III) (or a pharmaceutically acceptable salt, ester, solvate, stereoisomer, tautomer, or prodrug thereof). Representative examples of a unit dosage form include a tablet, capsule or pill for oral administration, and powder in a vial or ampoule for oral or nasal inhalation.

Alternatively, a pharmaceutical composition can be presented as a kit, wherein the active ingredient, excipients and carriers (e.g., solvents) are provided in two or more separate containers (e.g., ampoules, vials, tubes, bottles or syringes) and need to be combined to form the composition to be administered. The kit can contain instructions for storing, preparing and administering the composition (e.g., a solution to be injected intravenously).

A kit can contain all active and inactive ingredients in unit dosage form or the active ingredient and inactive ingredients in two or more separate containers, and can contain instructions for using the pharmaceutical composition.

In some embodiments, a kit contains a compound of Formula (I), Formula (II), or Formula (III) (or a pharmaceutically acceptable salt, ester, solvate, stereoisomer, tautomer, or prodrug thereof) or a pharmaceutically acceptable salt, solvate, hydrate, clathrate, polymorph, prodrug or metabolite thereof, and instructions for administering a compound of Formula (I), Formula (II), or Formula (III) (or a pharmaceutically acceptable salt, ester, solvate, stereoisomer, tautomer, or prodrug thereof). In certain embodiments, a compound of Formula (I), Formula (II), or Formula (III) (or a pharmaceutically acceptable salt, ester, solvate, stereoisomer, tautomer, or prodrug thereof) is contained or incorporated in, or provided by, a device or system configured for pulmonary delivery of the compound by oral inhalation, such as a metered-dose inhaler, a dry powder inhaler or a nebulizer.

### Inhalation Formulations and Devices

Pulmonary administration can be accomplished by, e.g., oral inhalation or nasal inhalation. Advantages of pulmonary drug delivery include, but are not limited to: 1) avoidance of first pass hepatic metabolism; 2) fast drug action; 3) large surface area of the alveolar region for absorption, high permeability of the lungs (thin air-blood barrier), and profuse vasculature of the airways; 4) smaller doses to achieve equivalent therapeutic effect compared to other oral routes; 5) local action within the respiratory tract; 6) reduced systemic side effects; and 7) reduced extracellular enzyme levels compared to the gastrointestinal tract due to the large alveolar surface area. An advantage of oral inhalation over nasal inhalation includes deeper penetration/deposition of the drug into the lungs. Pulmonary administration, whether by oral or nasal inhalation, can be a suitable route of administration for drugs that are intended to act locally in the lungs or/and systemically, for which the lungs serve as a portal to the systemic circulation.

Oral or nasal inhalation can be achieved by means of, e.g., a metered-dose inhaler (MDI), a nebulizer or a dry powder inhaler (DPI). For example, a compound of Formula (I), Formula (II), or Formula (III) (or a pharmaceutically acceptable salt, ester, solvate, stereoisomer, tautomer, or prodrug thereof) can be formulated for aerosol administration to the respiratory tract by oral or nasal inhalation. The drug is delivered in a small particle size (e.g., between about 0.5 micron and about 5 microns), which can be obtained by micronization, to improve, e.g., drug deposition in the lungs and drug suspension stability. The drug can be provided in a pressurized pack with a suitable propellant, such as a hydrofluoroalkane (HFA, e.g., 1,1,1,2-tetrafluoroethane [HFA-134a]), a chlorofluorocarbon (CFC, e.g., dichlorodifluoromethane, trichlorofluoromethane or dichlorotetrafluoroethane), or a suitable gas (e.g., oxygen, compressed air or carbon dioxide). The drug in the aerosol formulation is dissolved, or more often suspended, in the propellant for delivery to the lungs. The aerosol can contain excipients such as a surfactant (which enhances penetration into the lungs by reducing the high surface tension forces at the air-water interface within the alveoli, may also emulsify, solubilize or/and stabilize the drug, and can be, e.g., a phospholipid such as lecithin) or/and a stabilizer. For example, an MDI formulation can comprise a compound of Formula (I), Formula (II), or Formula (III) (or a pharmaceutically acceptable salt, ester, solvate, stereoisomer, tautomer, or prodrug thereof) , a propellant (e.g., an HFA such as 1,1,1,2-tetrafluoroethane), a surfactant (e.g., a fatty acid such as oleic acid), and a co-solvent (e.g., an alcohol such as ethanol). The MDI formulation can optionally contain a dissolved gas (e.g., C0₂). After device actuation, the bursting of C0₂ bubbles within the emitted aerosol droplets breaks up the droplets into smaller droplets, thereby increasing the respirable fraction of drug. As another example, a nebulizer formulation can comprise a compound of Formula (I), Formula (II), or Formula (III) (or a pharmaceutically acceptable salt, ester, solvate, stereoisomer, tautomer, or prodrug thereof) , a surfactant (e.g., a Tween^{®} such as polysorbate 80), a chelator or preservative (e.g., edetate disodium), an isotonicity agent (e.g., sodium chloride), pH buffering agents (e.g., citric acid/sodium citrate), and water. The drug can be delivered by means of, e.g., a nebulizer or an MDI with or without a spacer, and the drug dose delivered can be controlled by a metering chamber (nebulizer) or a metering valve (MDI).

Metered-dose inhalers (also called pressurized metered-dose inhalers [pMDI]) are the most widely used inhalation devices. A metering valve delivers a precise amount of aerosol (e.g., about 20-100 µL) each time the device is actuated. MDIs typically generate aerosol faster than the user can inhale, which can result in deposition of much of the aerosol in the mouth and the throat. The problem of poor coordination between device actuation and inhalation can be addressed by using, e.g., a breath-actuated MDI or a coordination device. A breath-actuated MDI (e.g., Easibreathe^{®}) is activated when the device senses the user's inspiration and discharges a drug dose in response. The inhalation flow rate is coordinated through the actuator and the user has time to actuate the device reliably during inhalation. In a coordination device, a spacer (or valved holding chamber), which is a tube attached to the mouthpiece end of the inhaler, serves as a reservoir or chamber holding the drug that is sprayed by the inhaler and reduces the speed at which the aerosol enters the mouth, thereby allowing for the evaporation of the propellant from larger droplets. The spacer simplifies use of the inhaler and increases the amount of drug deposited in the lungs instead of in the upper airways. The spacer can be made of an anti-static polymer to minimize electrostatic adherence of the emitted drug particles to the inner walls of the spacer.

Nebulizers generate aerosol droplets of about 1-5 microns. They do not require user coordination between device actuation and inhalation, which can significantly affect the amount of drug deposited in the lungs. Compared to MDIs and DPIs, nebulizers can deliver larger doses of drug, albeit over a longer administration time. Examples of nebulizers include without limitation human-powered nebulizers, jet nebulizers (e.g., AeroEclipse^{®} II BAN [breath-actuated], CompAIR^{™} NE-C801 [virtual valve], PARI LC^{®} Plus [breath- enhanced] and SideStream Plus [breath-enhanced]), ultrasonic wave nebulizers, and vibrating mesh nebulizers (e.g., Akita2^{®} Apixneb, I-neb AAD System with metering chambers, Micro Air^{®} NE-U22, Omron U22 and PARI eFlow^{®} rapid). As an example, a pulsed ultrasonic nebulizer can aerosolize a fixed amount of the drug per pulse, and can comprise an opto-acoustical trigger that allows the user to synchronize each breath to each pulse.

Respimat^{®} Soft Mist^{™} inhaler combines advantages of an MDI and a nebulizer. It is a small, hand-held inhaler that does not need a power supply (like an MDI) and slowly aerosolizes a propellant-free drug solution as a soft mist (like a nebulizer), thereby reducing drug deposition in the oropharyngeal region and increasing drug deposition in the central and peripheral lung regions. The Soft Mist^{™} inhaler can create a large fraction of respirable droplets with slow velocity from a metered volume of drug solution. A drug delivered from the Soft Mist^{™} inhaler can potentially achieve the same therapeutic outcome at a significantly lower dose compared to delivery from an MDI.

For oral or nasal inhalation using a dry powder inhaler (DPI), a compound of Formula (I), Formula (II), or Formula (III) (or a pharmaceutically acceptable salt, ester, solvate, stereoisomer, tautomer, or prodrug thereof) can be provided in the form of a dry micronized powder, where the drug particles are of a certain small size (e.g., between about 0.5 micron and about 5 microns) to improve, e.g., aerodynamic properties of the dispersed powder and drug deposition in the lungs. Particles between about 0.5 micron and about 5 microns deposit by sedimentation in the terminal bronchioles and the alveolar regions. By contrast, the majority of larger particles (> 5 microns) do not follow the stream of air into the many bifurcations of the airways, but rather deposit by impaction in the upper airways, including the oropharyngeal region of the throat. A DPI formulation can contain the drug particles alone or blended with a powder of a suitable larger base/carrier, such as lactose, starch, a starch derivative (e.g., hydroxypropylmethyl cellulose) or polyvinylpyrrolidine. The carrier particles enhance flow, reduce aggregation, improve dose uniformity and aid in dispersion of the drug particles. A DPI formulation can optionally contain an excipient such as magnesium stearate or/and leucine that improves the performance of the formulation by interfering with inter-particle bonding (by anti-adherent action). The powder formulation can be provided in unit dose form, such as a capsule (e.g., a gelatin capsule) or a cartridge in a blister pack, which can be manually loaded or preloaded in an inhaler. The drug particles can be drawn into the lungs by placing the mouthpiece or nosepiece of the inhaler into the mouth or nose, taking a sharp, deep inhalation to create turbulent airflow, and holding the breath for a period of time (e.g., about 5-10 seconds) to allow the drug particles to settle down in the bronchioles and the alveolar regions. When the user actuates the DPI and inhales, airflow through the device creates shear and turbulence, inspired air is introduced into the powder bed, and the static powder blend is fluidized and enters the user's airways. There, the drug particles separate from the carrier particles due to turbulence and are carried deep into the lungs, while the larger carrier particles impact on the oropharyngeal surfaces and are cleared. Thus, the user's inspiratory airflow achieves powder de- agglomeration and aeroionisation, and determines drug deposition in the lungs. (While a passive DPI requires rapid inspiratory airflow to de-agglomerate drug particles, rapid inspiration is not recommended with an MDI or nebulizer, since it creates turbulent airflow and fast velocity which increase drug deposition by impaction in the upper airways.) Compared to an MDI, a DPI (including a passive, breath-activated DPI) can potentially deliver larger doses of drug, and larger-size drugs (e.g., macromolecules), to the lungs.

Lactose (e.g., alpha-lactose monohydrate) is the most commonly used carrier in DPI formulations. Examples of grades/types of lactose monohydrate for DPI formulations include without limitation DCL 11, Flowlac^{®} 100, Inhalac^{®} 230, Lactohale^{®} 300, Lactopress^{®} SD 250 (spray-dried lactose), Respitose^{®} SV003 and Sorbolac^{®} 400. A DPI formulation can contain a single lactose grade or a combination of different lactose grades. For example, a fine lactose grade like Lactohale^{®} 300 or Sorbolac^{®} 400 may not be a suitable DPI carrier and may need to be blended with a coarse lactose grade like DCL 11, Flowlac^{®} 100, Inhalac^{®} 230 or Respitose^{®} SV003 (e.g., about a 1:9 ratio of fine lactose to coarse lactose) to improve flow. The distribution of the carrier particle sizes affects the fine particle fraction/dose (FPF or FPD) of the drug, with a high FPF being desired for drug delivery to the lungs. FPF/FPD is the respirable fraction/dose mass out of the DPI device with an aerodynamic particle size < 5 microns in the inspiration air. High FPF, and hence good DPI performance, can be obtained from, e.g., DPI formulations having an approximately 1:9 ratio of fine lactose (e.g., Lactohale^{®} 300) to coarse lactose (e.g., Respitose^{®} SV003) and about 20% w/w overages to avoid deposition of the drug in the capsule shell or the DPI device and to deliver essentially all of the drug to the airways.

Other carriers for DPI formulations include without limitation glucose, mannitol (e.g., crystallized mannitol [Pearlitol 110 C] and spray-dried mannitol [Pearlitol 100 SD]), maltitol (e.g., crystallized maltitol [Maltisorb P90]), sorbitol and xylitol.

To improve the performance of DPI formulations, pulmospheres can be used. These relatively large porous, hollow particles have low particle density and improved dispersibility. Pulmospheres can be prepared using a polymeric or non-polymeric excipient by, e.g., solvent evaporation or spray drying. For example, pulmospheres can be made of phosphatidylcholine, the primary component of human lung surfactant. The relatively large size of pulmospheres allows them to remain in the alveolar region longer than their non- porous counterparts by avoiding phagocytic clearance. Pulmospheres can also be used in aerosol formulations for MDIs as well as for DPIs.

Dry powder inhalers can be classified by dose type into single-unit dose (including disposable and reusable) and multi-dose (including multi-dose reservoirs and multi-unit dose). In a single-unit dose DPI, the formulation can be a powder mix of a micronized drug powder and a carrier and can be supplied in individual capsules, which are inserted into the inhaler for a single dose and are removed and discarded after use. The capsule body containing the dose falls into the device, while the cap is retained in the entry port for subsequent disposal. As the user inhales, the portion of the capsule containing the drug experiences erratic motion in the airstream, causing dislodged particles to be entrained and subsequently inhaled. Particle de-aggregation is caused mainly by turbulence promoted by the grid upstream of the mouthpiece or nosepiece. Examples of single-unit dose DPIs include without limitation Aerolizer^{®}, AIR^{®}, Conix One^{®} (foil seal), Diskhaler^{®}, Diskus^{®}, Handihaler^{®}, Microhaler^{®}, Rotahaler^{®} and Turbo spin^{®}.

A multi-unit dose DPI uses factory-metered and -sealed doses packaged in a manner so that the device can hold multiple doses without the user having to reload. The packaging typically contains replaceable disks or cartridges, or strips of foil-polymer blister packaging that may or may not be reloadable. For example, individual doses can be packaged in blister packs on a disk cassette. Following piercing, inspiratory flow through the packaging depression containing the drug induces dispersion of the powder. The aerosol stream is mixed with a bypass flow entering through holes in the mouthpiece or nosepiece, which gives rise to turbulence and promotes particle de- agglomeration. Advantages of the prepackaging include protection from the environment until use and ensurance of adequate control of dose uniformity. Examples of multi-unit dose DPIs include without limitation Acu-Breath^{®}, Bulkhaler^{®}, Certihaler^{®}, DirectHaler^{®}, Diskhaler^{®}, Diskus^{®}, Dispohaler^{®}, M^{®}, MF-DPI^{®}, Miat-Haler^{®}, NEXT DPI^{®}, Prohaler^{®}, Swinhaler^{®} and Technohaler^{®}.

A multi-dose reservoir DPI stores the formulation in bulk, and has a built-in mechanism to meter individual doses from the bulk upon actuation. It contains multiple doses of small pellets of micronized drug that disintegrate into their primary particles during metering and inhalation. One dose can be dispensed into the dosing chamber by a simple back-and-forth twisting action on the base of the reservoir. Scrapers actively force the drug into conical holes, which causes the pellets to disintegrate. Fluidization of the powder is achieved by shear force as air enters the inhaler, and particle de- agglomeration occurs via turbulence. Advantages of multi-dose reservoir DPIs include their relative ease and low cost of manufacture, and the ease of inclusion of a large number of doses within the device. Examples of multi-dose reservoir DPIs include without limitation Acu-Breath^{®}, Airmax^{®}, Bulkhaler^{®}, Certihaler^{®}, Clickhaler^{®}, Cyclovent^{®}, Dispohaler^{®}, JAGO^{®}, MF-DPI^{®}, Miat-Haler^{®}, NEXT DPI^{®}, Swinhaler^{®} and Turbuhaler^{®}.

Most DPIs are breath-activated ("passive"), relying on the user's inhalation for aerosol generation. Examples of passive DPIs include without limitation Airmax^{®}, Novolizer^{®}, Otsuka DPI (compact cake), and the DPIs mentioned above. The air classifier technology (ACT) is an efficient passive powder dispersion mechanism employed in DPIs. In ACT, multiple supply channels generate a tangential airflow that results in a cyclone within the device during inhalation. There are also power-assisted ("active") DPIs (based on, e.g., pneumatics, impact force or vibration) that use energy to aid, e.g., particle de- agglomeration. For example, the active mechanism of Exubera^{®} inhalers utilizes mechanical energy stored in springs or compressed-air chambers. Examples of active DPIs include without limitation Actispire^{®} (single-unit dose), Aspirair^{®} (multi-dose), Exubera^{®} (single- unit dose), MicroDose^{®} (multi-unit dose and electronically activated), Omnihaler^{®} (single- unit dose), Pfeiffer DPI (single-unit dose), and Spiros^{®} (multi-unit dose).

### RNA viruses

Disclosed herein include methods for preventing, delaying the onset of, or treating an infection, disease, or inflammation caused by a RNA virus. The present disclosure contemplates treating a broad range of viral diseases, including infections of all types, locations, sizes, and characteristics. The RNA virus can be a double-stranded RNA virus. The RNA virus can be a positive-sense single-stranded RNA virus. The positive-sense single-stranded RNA virus can be a coronavirus. The coronavirus can be an alpha coronavirus, a beta coronavirus, a gamma coronavirus, or a delta coronavirus. The coronavirus can be Middle East respiratory coronavirus (MERS-CoV), severe acute respiratory syndrome coronavirus (SARS-CoV), or SARS-CoV-2.

The infection or disease caused by the RNA virus can be common cold, influenza, SARS, coronaviruses, COVID-19, hepatitis C, hepatitis E, West Nile fever, Ebola virus disease, rabies, polio, or measles.

The methods and compositions disclosed herein are useful for preventing, delaying the onset of, or treating an infection, disease, or inflammation caused by a RNA virus. The subject can have been exposed to the RNA virus, can be suspected to have been exposed to the RNA virus, or can be at a risk of being exposed to the RNA virus. The compositions may be used as a prophylactic (to prevent the development of a viral infection) or may be used to treat existing viral infections.

The RNA virus can be an enveloped virus. The RNA virus can a retrovirus. The RNA virus can be a filovirus, arenavirus, bunyavirus, or a rhabdovirus. The RNA virus can be a hepadnavirus, coronavirus, or a flavivirus. The RNA virus can be Respiratory syncytial virus, Parainfluenza virus, Enterovirus 71, Hantavirus, SARS virus, SARS-associated coronavirus, severe acute respiratory syndrome coronavirus (SARS-CoV), or SARS-CoV-2, Sin Nombre virus, Respiratory reovirus. The present disclosure encompasses the treatment of infections with derivatives of any of the viruses disclosed herein. As disclosed herein, the term "derivative of a virus" can refer to a strain of virus that has mutated from an existing viral strain.

The RNA virus can comprise any serotype of human rhinovirus (HRV). HRV may include, without limitation, the species Rhinovirus A (including, but not limited to, serotypes HRV-A1, HRV-A2, HRV-A7, HRV-A8, HRV-A9, HRV-A10, HRV-A11, HRV-A12, HRV-A13, HRV-A15, HRV-A16, HRV-A18, HRV-A19, HRV-A20, HRV-A21, HRV-A22, HRV-A23, HRV-A24, HRV-A25, HRV-A28, HRV-A29, HRV-A30, HRV-A31, HRV-A32, HRV-A33, HRV-A34, HRV-A36, HRV-A38, HRV-A39, HRV-A40, HRV-A41, HRV-A43, HRV-A44, HRV-A45, HRV-A46, HRV-A47, HRV-A49, HRV-A50, HRV-A51, HRV-A53, HRV-A54, HRV-A55, HRV-A56, HRV-A57, HRV-A58, HRV-A59, HRV-A60, HRV-A61, HRV-A62, HRV-A63, HRV-A64, HRV-A65, HRV-A66, HRV-A67, HRV-A68, HRV-A71, HRV-A73, HRV-A74, HRV-A75, HRV-A76, HRV-A77, HRV-A78, HRV-A80, HRV-A81, HRV-A82, HRV-A85,HRV-A88, HRV-A89, HRV-A90, HRV-A94, HRV-A95, HRV-A96, HRV-A98, HRV-A100, HRV-A101, HRV-A102 and HRV-A103), Rhino virus B (including, but not limited to, the serotypes HRV-B3, HRV-B4, HRV-B5, HRV-B6, HRV-B14, HRV-B17, HRV-B26, HRV-B27, HRV-B35, HRV-B37, HRV-B42, HRV-B48, HRV-B52, HRV-B69, HRV-B70, HRV-B72, HRV-B79, HRV-B83, HRV-B84, HRV-B86, HRV-B91, HRV-B92, HRV-B93, HRV-B97, and HRV-B99), and Rhinovirus C (including, but not limited to, serotypes HRV-C1, HRV-C2, HRV-C3, HRV-C4, HRV-C5, HRV-C6, HRV-C7, HRV-C8, HRV-C9, HRV-C10, HRV-C11, HRV-C12, HRV-C13, HRV-C14, HRV-C15, HRV-C16, HRV-C17, HRV-C18, HRV-C19, HRV-C20, HRV-C21, HRV-C22, HRV-C23, HRV-C24, HRV-C25, HRV-C26, HRV-C27, HRV-C28, HRV-C29, HRV-C30, HRV-C31, HRV-C32, HRV-C33, HRV-C34, HRV-C35, HRV-C36, HRV-C37, HRV-C38, HRV-C39, HRV-C40, HRV-C41, HRV-C42, HRV-C43, HRV-C44, HRV-C45, HRV-C46, HRV-C47, HRV-C48, HRV-C49, HRV-C50 and HRV-C51).

In some embodiments the RNA virus is an influenza A virus. Non-limiting examples of influenza A viruses include subtype H10N4, subtype H10N5, subtype H10N7, subtype H10N8, subtype H10N9, subtype H11N1, subtype H11N13, subtype H11N2, subtype H11N4, subtype H11N6, subtype H11N8, subtype H11N9, subtype H12N1, subtype H12N4, subtype H12N5, subtype H12N8, subtype H13N2, subtype H13N3, subtype H13N6, subtype H13N7, subtype H14N5, subtype H14N6, subtype H15N8, subtype H15N9, subtype H16N3, subtype H1N1, subtype H1N2, subtype H1N3, subtype H1N6, subtype H1N9, subtype H2N1, subtype H2N2, subtype H2N3, subtype H2N5, subtype H2N7, subtype H2N8, subtype H2N9, subtype H3N1, subtype H3N2, subtype H3N3, subtype H3N4, subtype H3N5, subtype H3N6, subtype H3N8, subtype H3N9, subtype H4N1, subtype H4N2, subtype H4N3, subtype H4N4, subtype H4N5, subtype H4N6, subtype H4N8, subtype H4N9, subtype H5N1, subtype H5N2, subtype H5N3, subtype H5N4, subtype H5N6, subtype H5N7, subtype H5N8, subtype H5N9, subtype H6N1, subtype H6N2, subtype H6N3, subtype H6N4, subtype H6N5, subtype H6N6, subtype H6N7, subtype H6N8, subtype H6N9, subtype H7N1, subtype H7N2, subtype H7N3, subtype H7N4, subtype H7N5, subtype H7N7, subtype H7N8, subtype H7N9, subtype H8N4, subtype H8N5, subtype H9N1, subtype H9N2, subtype H9N3, subtype H9N5, subtype H9N6, subtype H9N7, subtype H9N8, and subtype H9N9.

Specific examples of strains of influenza A virus include, but are not limited to: A/sw/Iowa/15/30 (H1N1); A/WSN/33 (H1N1); A/eq/Prague/1/56 (H7N7); A/PR/8/34; A/mallard/Potsdam/178-4/83 (H2N2); A/herring gull/DE/712/88 (H16N3); A/sw/Hong Kong/168/1993 (H1N1); A/mallard/Alberta/211/98 (H1N1); A/shorebird/Delaware/168/06 (H16N3); A/sw/Netherlands/25/80 (H1N1); A/sw/Germany/2/81 (H1N1); A/sw/Hannover/1/81 (H1N1); A/sw/Potsdam/1/81 (H1N1); A/sw/Potsdam/15/81 (H1N1); A/sw/Potsdam/268/81 (H1N1); A/sw/Finistere/2899/82 (H1N1); A/sw/Potsdam/35/82 (H3N2); A/sw/Cote d'Armor/3633/84 (H3N2); A/sw/Gent/1/84 (H3N2); A/sw/Netherlands/12/85 (H1N1); A/sw/Karrenzien/2/87 (H3N2); A/sw/Schwerin/103/89 (H1N1); A/turkey/Germany/3/91 (H1N1); A/sw/Germany/8533/91 (H1N1); A/sw/Belgium/220/92 (H3N2); A/sw/GentN230/92 (H1N1); A/sw/Leipzig/145/92 (H3N2); A/sw/Re220/92 hp (H3N2); A/sw/Bakum/909/93 (H3N2); A/sw/Schleswig-Holstein/1/93 (H1N1); A/sw/Scotland/419440/94 (H1N2); A/sw/Bakum/5/95 (H1N1); A/sw/Best/5C/96 (H1N1); A/sw/England/17394/96 (H1N2); A/sw/Jena/5/96 (H3N2); A/sw/Oedenrode/7C/96 (H3N2); A/sw/Lohne/1/97 (H3N2); A/sw/Cote d'Armor/790/97 (H1N2); A/sw/Bakum/1362/98 (H3N2); A/sw/Italy/1521/98 (H1N2); A/sw/Italy/1553-2/98 (H3N2); A/sw/Italy/1566/98 (H1N1); A/sw/Italy/1589/98 (H1N1); A/sw/Bakum/8602/99 (H3N2); A/sw/Cotes d'Armor/604/99 (H1N2); A/sw/Cote d'Armor/1482/99 (H1N1); A/sw/Gent/7625/99 (H1N2); A/Hong Kong/1774/99 (H3N2); A/sw/Hong Kong/5190/99 (H3N2); A/sw/Hong Kong/5200/99 (H3N2); A/sw/Hong Kong/5212/99 (H3N2); A/sw/Ille et Villaine/1455/99 (H1N1); A/sw/Italy/1654-1/99 (H1N2); A/sw/Italy/2034/99 (H1N1); A/sw/Italy/2064/99 (H1N2); A/sw/Berlin/1578/00 (H3N2); A/sw/Bakum/1832/00 (H1N2); A/sw/Bakum/1833/00 (H1N2); A/sw/Cote d'Armor/800/00 (H1N2); A/sw/Hong Kong/7982/00 (H3N2); A/sw/Italy/1081/00 (H1N2); A/sw/Belzig/2/01 (H1N1); A/sw/Belzig/54/01 (H3N2); A/sw/Hong Kong/9296/01 (H3N2); A/sw/Hong Kong/9745/01 (H3N2); A/sw/Spain/33601/01 (H3N2); A/sw/Hong Kong/1144/02 (H3N2); A/sw/Hong Kong/1197/02 (H3N2); A/sw/Spain/39139/02 (H3N2); A/sw/Spain/42386/02 (H3N2); A/Switzerland/8808/2002 (H1N1); A/sw/Bakum/1769/03 (H3N2); A/sw/Bissendorf/IDT1864/03 (H3N2); A/sw/Ehren/IDT2570/03 (H1N2); A/sw/Gescher/IDT2702/03 (H1N2); A/sw/Haselünne/2617/03 hp (H1N1); A/sw/Loningen/IDT2530/03 (H1N2); A/sw/IVD/IDT2674/03 (H1N2); A/sw/Nordkirchen/IDT1993/03 (H3N2); A/sw/Nordwalde/IDT2197/03 (H1N2); A/sw/Norden/IDT2308/03 (H1N2); A/sw/Spain/50047/03 (H1N1); A/sw/Spain/51915/03 (H1N1); A/sw/Vechta/2623/03 (H1N1); A/sw/Visbek/IDT2869/03 (H1N2); A/sw/Waltersdorf/IDT2527/03 (H1N2); A/sw/Damme/IDT2890/04 (H3N2); A/sw/Geldern/IDT2888/04 (H1N1); A/sw/Granstedt/IDT3475/04 (H1N2); A/sw/Greven/IDT2889/04 (H1N1); A/sw/Gudensberg/IDT2930/04 (H1N2); A/sw/Gudensberg/IDT2931/04 (H1N2); A/sw/Lohne/IDT3357/04 (H3N2); A/sw/Nortrup/IDT3685/04 (H1N2); A/sw/Seesen/IDT3055/04 (H3N2); A/sw/Spain/53207/04 (H1N1); A/sw/Spain/54008/04 (H3N2); A/sw/Stolzenau/IDT3296/04 (H1N2); A/sw/Wedel/IDT2965/04 (H1N1); A/sw/Bad Griesbach/IDT4191/05 (H3N2); A/sw/Cloppenburg/IDT4777/05 (H1N2); A/sw/Dotlingen/IDT3780/05 (H1N2); A/sw/Dotlingen/IDT4735/05 (H1N2); A/sw/Egglham/IDT5250/05 (H3N2); A/sw/Harkenblek/IDT4097/05 (H3N2); A/sw/Hertzen/IDT4317/05 (H3N2); A/sw/Krogel/IDT4192/05 (H1N1); A/sw/Laer/IDT3893/05 (H1N1); A/sw/Laer/IDT4126/05 (H3N2); A/sw/Merzen/IDT4114/05 (H3N2); A/sw/Muesleringen-S./IDT4263/05 (H3N2); A/sw/Osterhofen/IDT4004/05 (H3N2); A/sw/Sprenge/IDT3805/05 (H1N2); A/sw/Stadtlohn/IDT3853/05 (H1N2); A/swNoglarn/IDT4096/05 (H1N1); A/sw/Wohlerst/IDT4093/05 (H1N1); A/sw/Bad Griesbach/IDT5604/06 (H1N1); A/sw/Herzlake/IDT5335/06 (H3N2); A/sw/Herzlake/IDT5336/06 (H3N2); A/sw/Herzlake/IDT5337/06 (H3N2); and A/wild boar/Germany/R169/2006 (H3N2).

Other specific examples of strains of influenza A virus include, but are not limited to: A/Toronto/3141/2009 (H1N1); A/Regensburg/D6/2009 (H1N1); A/Bayern/62/2009 (H1N1); A/Bayern/62/2009 (H1N1); A/Bradenburg/19/2009 (H1N1); A/Bradenburg/20/2009 (H1N1); A/Distrito Federal/2611/2009 (H1N1); A/Mato Grosso/2329/2009 (H1N1); A/Sao Paulo/1454/2009 (H1N1); A/Sao Paulo/2233/2009 (H1N1); A/Stockholm/37/2009 (H1N1); A/Stockholm/41/2009 (H1N1); A/Stockholm/45/2009 (H1N1); A/swine/Alberta/OTH-33-1/2009 (H1N1); A/swine/Alberta/OTH-33-14/2009 (H1N1); A/swine/Alberta/OTH-33-2/2009 (H1N1); A/swine/Alberta/OTH-33-21/2009 (H1N1); A/swine/Alberta/OTH-33-22/2009 (H1N1); A/swine/Alberta/OTH-33-23/2009 (H1N1); A/swine/Alberta/OTH-33-24/2009 (H1N1); A/swine/Alberta/OTH-33-25/2009 (H1N1); A/swine/Alberta/OTH-33-3/2009 (H1N1); A/swine/Alberta/OTH-33-7/2009 (H1N1); A/Beijing/502/2009 (H1N1); A/Firenze/10/2009 (H1N1); A/Hong Kong/2369/2009 (H1N1); A/Italy/85/2009 (H1N1); A/Santo Domingo/572N/2009 (H1N1); A/Catalonia/385/2009 (H1N1); A/Catalonia/386/2009 (H1N1); A/Catalonia/387/2009 (H1N1); A/Catalonia/390/2009 (H1N1); A/Catalonia/394/2009 (H1N1); A/Catalonia/397/2009 (H1N1); A/Catalonia/398/2009 (H1N1); A/Catalonia/399/2009 (H1N1); A/Sao Paulo/2303/2009 (H1N1); A/Akita/1/2009 (H1N1); A/Castro/JXP/2009 (H1N1); A/Fukushima/1/2009 (H1N1); A/Israel/276/2009 (H1N1); A/Israel/277/2009 (H1N1); A/Israel/70/2009 (H1N1); A/Iwate/1/2009 (H1N1); A/Iwate/2/2009 (H1N1); A/Kagoshima/1/2009 (H1N1); A/Osaka/180/2009 (H1N1); A/Puerto Montt/Bio87/2009 (H1N1); A/Sao Paulo/2303/2009 (H1N1); A/Sapporo/1/2009 (H1N1); A/Stockholm/30/2009 (H1N1); A/Stockholm/31/2009 (H1N1); A/Stockholm/32/2009 (H1N1); A/Stockholm/33/2009 (H1N1); A/Stockholm/34/2009 (H1N1); A/Stockholm/35/2009 (H1N1); A/Stockholm/36/2009 (H1N1); A/Stockholm/38/2009 (H1N1); A/Stockholm/39/2009 (H1N1); A/Stockholm/40/2009 (H1N1;) A/Stockholm/42/2009 (H1N1); A/Stockholm/43/2009 (H1N1); A/Stockholm/44/2009 (H1N1); A/Utsunomiya/2/2009 (H1N1); A/WRAIR/0573N/2009 (H1N1); and A/Zhejiang/DTID-ZJU01/2009 (H1N1).

In some embodiments the RNA virus is an influenza B virus. Non-limiting examples of influenza B viruses include strain Aichi/5/88, strain Akita/27/2001, strain Akita/5/2001, strain Alaska/16/2000, strain Alaska/1777/2005, strain Argentina/69/2001, strain Arizona/146/2005, strain Arizona/148/2005, strain Bangkok/163/90, strain Bangkok/34/99, strain Bangkok/460/03, strain Bangkok/54/99, strain Barcelona/215/03, strain Beijing/15/84, strain Beijing/184/93, strain Beijing/243/97, strain Beijing/43/75, strain Beijing/5/76, strain Beijing/76/98, strain Belgium/WV106/2002, strain Belgium/WV107/2002, strain Belgium/WV109/2002, strain Belgium/WV114/2002, strain Belgium/WV122/2002, strain Bonn/43, strain Brazil/952/2001, strain Bucharest/795/03, strain Buenos Aires/161/00), strain Buenos Aires/9/95, strain Buenos Aires/SW16/97, strain Buenos AiresNL518/99, strain Canada/464/2001, strain Canada/464/2002, strain Chaco/366/00, strain Chaco/R113/00, strain Cheju/303/03, strain Chiba/447/98, strain Chongqing/3/2000, strain clinical isolate SA1 Thailand/2002, strain clinical isolate SA10 Thailand/2002, strain clinical isolate SA100 Philippines/2002, strain clinical isolate SA101 Philippines/2002, strain clinical isolate SA1 10 Philippines/2002), strain clinical isolate SA112 Philippines/2002, strain clinical isolate SA113 Philippines/2002, strain clinical isolate SA114 Philippines/2002, strain clinical isolate SA2 Thailand/2002, strain clinical isolate SA20 Thailand/2002, strain clinical isolate SA38 Philippines/2002, strain clinical isolate SA39 Thailand/2002, strain clinical isolate SA99 Philippines/2002, strain CNIC/27/2001, strain Colorado/2597/2004, strain CordobaNA418/99, strain Czechoslovakia/16/89, strain Czechoslovakia/69/90, strain Daeku/10/97, strain Daeku/45/97, strain Daeku/47/97, strain Daeku/9/97, strain B/Du/4/78, strain B/Durban/39/98, strain Durban/43/98, strain Durban/44/98, strain B/Durban/52/98, strain Durban/55/98, strain Durban/56/98, strain England/1716/2005, strain England/2054/2005), strain England/23/04, strain Finland/154/2002, strain Finland/159/2002, strain Finland/160/2002, strain Finland/161/2002, strain Finland/162/03, strain Finland/162/2002, strain Finland/162/91, strain Finland/164/2003, strain Finland/172/91, strain Finland/173/2003, strain Finland/176/2003, strain Finland/184/91, strain Finland/188/2003, strain Finland/190/2003, strain Finland/220/2003, strain Finland/WV5/2002, strain Fujian/36/82, strain Geneva/5079/03, strain Genoa/11/02, strain Genoa/2/02, strain Genoa/21/02, strain Genova/54/02, strain Genova/55/02, strain Guangdong/05/94, strain Guangdong/08/93, strain Guangdong/5/94, strain Guangdong/55/89, strain Guangdong/8/93, strain Guangzhou/7/97, strain Guangzhou/86/92, strain Guangzhou/87/92, strain Gyeonggi/592/2005, strain Hannover/2/90, strain Harbin/07/94, strain Hawaii/10/2001, strain Hawaii/1990/2004, strain Hawaii/38/2001, strain Hawaii/9/2001, strain Hebei/19/94, strain Hebei/3/94), strain Henan/22/97, strain Hiroshima/23/2001, strain Hong Kong/110/99, strain Hong Kong/1115/2002, strain Hong Kong/112/2001, strain Hong Kong/123/2001, strain Hong Kong/1351/2002, strain Hong Kong/1434/2002, strain Hong Kong/147/99, strain Hong Kong/156/99, strain Hong Kong/157/99, strain Hong Kong/22/2001, strain Hong Kong/22/89, strain Hong Kong/336/2001, strain Hong Kong/666/2001, strain Hong Kong/9/89, strain Houston/1/91, strain Houston/1/96, strain Houston/2/96, strain Hunan/4/72, strain Ibaraki/2/85, strain ncheon/297/2005, strain India/3/89, strain India/77276/2001, strain Israel/95/03, strain Israel/WV187/2002, strain Japan/1224/2005, strain Jiangsu/10/03, strain Johannesburg/1/99, strain Johannesburg/96/01, strain Kadoma/1076/99, strain Kadoma/122/99, strain Kagoshima/15/94, strain Kansas/22992/99, strain Khazkov/224/91, strain Kobe/1/2002, strain, strain Kouchi/193/99, strain Lazio/1/02, strain Lee/40, strain Leningrad/129/91, strain Lissabon/2/90), strain Los Angeles/1/02, strain Lusaka/270/99, strain Lyon/1271/96, strain Malaysia/83077/2001, strain Maputo/1/99, strain Mar del Plata/595/99, strain Maryland/1/01, strain Memphis/1/01, strain Memphis/12/97-MA, strain Michigan/22572/99, strain Mie/1/93, strain Milano/1/01, strain Minsk/318/90, strain Moscow/3/03, strain Nagoya/20/99, strain Nanchang/1/00, strain Nashville/107/93, strain Nashville/45/91, strain Nebraska/2/01, strain Netherland/801/90, strain Netherlands/429/98, strain New York/1/2002, strain NIB/48/90, strain Ningxia/45/83, strain Norway/1/84, strain Oman/16299/2001, strain Osaka/1059/97, strain Osaka/983/97-V2, strain Oslo/1329/2002, strain Oslo/1846/2002, strain Panama/45/90, strain Paris/329/90, strain Parma/23/02, strain Perth/211/2001, strain Peru/1364/2004, strain Philippines/5072/2001, strain Pusan/270/99, strain Quebec/173/98, strain Quebec/465/98, strain Quebec/7/01, strain Roma/1/03, strain Saga/S172/99, strain Seoul/13/95, strain Seoul/37/91, strain Shangdong/7/97, strain Shanghai/361/2002), strain Shiga/T30/98, strain Sichuan/379/99, strain Singapore/222/79, strain Spain/WV27/2002, strain Stockholm/10/90, strain Switzerland/5441/90, strain Taiwan/0409/00, strain Taiwan/0722/02, strain Taiwan/97271/2001, strain Tehran/80/02, strain Tokyo/6/98, strain Trieste/28/02, strain Ulan Ude/4/02, strain United Kingdom/34304/99, strain USSR/100/83, strain Victoria/103/89, strain Vienna/1/99, strain Wuhan/356/2000, strain WV194/2002, strain Xuanwu/23/82, strain Yamagata/1311/2003, strain Yamagata/K500/2001, strain Alaska/12/96, strain GA/86, strain NAGASAKI/1/87, strain Tokyo/942/96, and strain Rochester/02/2001.

In some embodiments the RNA virus is an influenza C virus. Non-limiting examples of influenza C viruses include strain Aichi/1/81, strain Ann Arbor/1/50, strain Aomori/74, strain California/78, strain England/83, strain Greece/79, strain Hiroshima/246/2000, strain Hiroshima/252/2000, strain Hyogo/1/83, strain Johannesburg/66, strain Kanagawa/1/76, strain Kyoto/1/79, strain Mississippi/80, strain Miyagi/1/97, strain Miyagi/5/2000, strain Miyagi/9/96, strain Nara/2/85, strain NewJersey/76, strain pig/Beijing/115/81, strain Saitama/3/2000), strain Shizuoka/79, strain Yamagata/2/98, strain Yamagata/6/2000, strain Yamagata/9/96, strain BERLIN/1/85, strain ENGLAND/892/8, strain GREAT LAKES/1167/54, strain JJ/50, strain PIG/BEIJING/10/81, strain PIG/BEIJING/439/82), strain TAYLOR/1233/47, and strain C/YAMAGATA/10/81.

### Additional therapeutic agents

In some embodiments, the method can comprise administering to the subject in need thereof one or more additional therapeutic agents (e.g., antiviral agents). The additional therapeutic agents (e.g., antiviral agents) can be co-administered to the subject with the composition. The additional therapeutic agents (e.g., antiviral agents) can be administered to the subject before the administration of the composition, after the administration of the composition, or both. The composition can comprise one or more additional therapeutic agents (e.g., antiviral agents).

The antiviral agent can be selected from the group consisting of a nucleoside or a non-nucleoside analogue reverse-transcriptase inhibitor, a nucleotide analogue reverse-transcriptase inhibitor, a NS3/4A serine protease inhibitor, a NS5B polymerase inhibitor, and interferon alpha.

As disclosed herein, co-administration of particular ratios and/or amounts of a compound of Formula (I), Formula (II), or Formula (III) (or a pharmaceutically acceptable salt, ester, solvate, stereoisomer, tautomer, or prodrug thereof, e.g., a therapeutic agent) and one or more additional therapeutic agents (e.g., antiviral agents) can result in synergistic effects in preventing, delaying the onset of, or treating an infection, disease, or inflammatory effect caused by a RNA virus. These synergistic effects can be such that the one or more effects of the combination compositions are greater than the one or more effects of each component alone at a comparable dosing level, or they can be greater than the predicted sum of the effects of all of the components at a comparable dosing level, assuming that each component acts independently. The synergistic effect can be, be about, be greater than, or be greater than about, 5, 10, 20, 30, 50, 75, 100, 110, 120, 150, 200, 250, 350, or 500% better than the effect of treating a subject with one of the components alone, or the additive effects of each of the components when administered individually. The effect can be any of the measurable effects described herein. The composition comprising a plurality of components can be such that the synergistic effect is, for example, a reduction in lung inflammation and that lung inflammation is reduced to a greater degree as compared to the sum of the effects of administering each component, determined as if each component exerted its effect independently, also referred to as the predicted additive effect herein. For example, if a composition comprising component (a) yields an effect of a 20% reduction in lung inflammation and a composition comprising component (b) yields an effect of 50% reduction in lung inflammation, then a composition comprising both component (a) and component (b) would have a synergistic effect if the combination composition's effect on lung inflammation was greater than 70%.

A synergistic combination composition can have an effect that is greater than the predicted additive effect of administering each component of the combination composition alone as if each component exerted its effect independently. For example, if the predicted additive effect is 70%, an actual effect of 140% is 70% greater than the predicted additive effect or is 1 fold greater than the predicted additive effect. The synergistic effect can be at least, or at least about, 20, 50, 75, 90, 100, 150, 200 or 300% greater than the predicted additive effect. In some embodiments, the synergistic effect can be at least, or at least about, 0.2, 0.5, 0.9, 1.1, 1.5, 1.7, 2, or 3 fold greater than the predicted additive effect.

In some embodiments, the synergistic effect of the combination compositions can also allow for reduced dosing amounts, leading to reduced side effects to the subject and reduced cost of treatment. Furthermore, the synergistic effect can allow for results that are not achievable through any other treatments. Therefore, proper identification, specification, and use of combination compositions can allow for significant improvements in the reduction and prevention of lung inflammation.

The additional therapeutic agents provided herein can include antagonists of transient receptor potential cation channels, including but not limited to transient receptor potential ankyrin A1 (TRPA1) antagonists {e.g., camphor, isopentenyl pyrophosphate, A967079, GRC-17536, (4R)-1,2,3,4-tetrahydro-4-[3-(3-methoxypropoxy)phenyl]-2-thioxo-5H-indeno[1,2-d]pyrimidin-5-one, 2-amino-4-arylthiazole compounds disclosed in WO 2012/085662 A1, and specialized pro-resolving mediators (SPMs) (e.g., metabolites of polyunsaturated fatty acids [PUFAs])}, transient receptor potential vanilloid (TRPV) antagonists (e.g., TRPV1 antagonists [e.g., capsazepine, iodo-resiniferatoxin, AMG-517, GRC-6211, NGD-8243, SB-705498 and SPMs {e.g., PUFA metabolites}] and TRPV3 antagonists [e.g., SPMs {e.g., PUFA metabolites}]), and analogs, derivatives and salts thereof.

The additional therapeutic agents provided herein can include TRPV1 agonists that cause decrease in TRPV1 activity (desensitization) upon prolonged exposure of TRPV1 to the stimuli, including but not limited to capsaicin, camphor, carvacrol, menthol, methyl salicylate, resiniferatoxin, tinyatoxin, and analogs, derivatives and salts thereof.

The additional therapeutic agents provided herein can include antagonists of protease-activated receptors (PARs) and inhibitors of activating proteases, including but not limited to PAR1 antagonists (e.g., SCH-530,348), PAR2 antagonists {e.g., AY-117, ENMD-1068, ENMD-106836, GB-83, tetracyclines (e.g., doxycycline, minocycline and tetracycline), FSLLRY-NH₂ (PAR-3888-PD, Ac-FSLLRY-NH₂ and anti-PAR2 antibodies (e.g., SAM-11 [SC-13504], P2pa1-21 and P2pa1-2135}, PAR4 antagonists {e.g, ethanol, YD-3, statins atorvastatin, cerivastatin, fluvastatin, lovastatin, pitavastatin, pravastatin, rosuvastatin and simvastatin), pepducin P4 pal-10, pepducin P4 pal-15, trans-cinnamoyl-APGKF-NH₂, trans-cinnamoyl-YPGKF-NH₂, and anti-PAR4 antibodies (e.g., C-19 and SC-1249)}, inhibitors of serine proteases {e.g., benzamidine hydrochloride, 4-iodo-1-benzothiophene-2-carboximidamide hydrochloride (inhibits trypsin and tryptase), inhibitors of kallikreins (e.g., camostat, nafamostat, gabexate, ecallantide and α₁-inhibitor), trypsin inhibitors tosyllysine chloromethyl ketone [TLCK] hydrochloride, α₁-antitrypsin, aprotinin, ovomucin and soybean trypsin inhibitor), and tryptase inhibitors (e.g., camostat, nafamostat, gabexate, AMG-126737 and APC-366)}, inhibitors of cysteine proteases {e.g., E-64 (non-specific inhibitor), JNJ-10329670, RWJ-445380, cystatin C, leupeptin, stefin A, stefin B, testican-1, chloroquine, fluoromethyl ketone, naphthalene endoperoxide (inhibits cathepsin B, L and S), CA-074 (inhibits cathepsin B), odanacatib (MK-0822, inhibits cathepsin K), CLIK-148 and CLIK-195 (inhibit cathepsin L), and CLIK-60 and E-6438 (inhibit cathepsin S)}, and analogs, derivatives, fragments and salts thereof;

The additional therapeutic agents provided herein can include antagonists of endothelin receptors, including but not limited to selective endothelin A receptor (ETAR) antagonists {e.g., ambrisentan, atrasentan, sitaxentan, zibotentan, BQ-123, 4-amino-N-(3,4-dimethylisoxazol-5-yl)benzenesulfonamide; (2R)-2-[[(2R)-2-[[(2 S)-2-(azepane-1-carbonylamino)-4-methylpentanoyl]amino]-3-(1-formylindol-3-yl)propanoyl]amino]-3-(1H-indol-3-yl)propanoic acid; 3-benzodioxol-5-yl)-1-[2-(dibutylamino)-2-oxoethyl]-2-(4-methoxyphenyl)pyrrolidine-3-carboxylic acid; (2R,3R,4S)-4-(1,3-benzodioxol-5-yl)-1-[2-(dibutylamino)-2-oxoethyl]-2-(4-methoxyphenyl)pyrrolidine-3-carboxylic acid; (2R,3R,4S)-4-(1,3-benzodioxol-5-yl)-1[2-(dibutylamino)-2-oxoethyl]-2-(2-methoxyphenyl)pyrrolidine-3-carboxylic acid; 3-(1,3-benzodioxol-5-yl)-5-hydroxy-5-(4-methoxyphenyl)-4-[(3,4,5-trimethoxyphenyl)methyl]furan-2-one; 2-(1,3-benzodioxol-5-yl)-4-(4-methoxyphenyl)-4-oxo-3-[(3,4,5-trimethoxyphenyl)methyl]but-2-enoate; 5-(4-bromophenyl)-6-[2-(5-bromopyrimidin-2-yl)oxyethoxy]-N-(propylsulfamoyl)pyrimidin-4-amine; 4-tert-butyl-N-[6-(2-hydroxyethoxy)-5-(2-methoxyphenoxy)-2-(pyrimidin-2-yl)pyrimidin-4-yl]benzenesulfonamide; [(7R)-5-chloro-3-[(1E,3E,5S)-3,5-dimethylhepta-1,3-dienyl]-7-methyl-6,8-dioxoisochromen-7-yl]acetate; N-(4-chloro-3-methyl-1,2-oxazol-5-yl)-2-[2-(6-methyl-2H-1,3-benzodioxol-5-yl)acetyl]thiophene-3-sulfonamide; (2S)-2-(4,6-dimethoxypyrimidin-2-yl)oxy-3-methoxy-3,3-diphenylpropanoic acid; (2S)-2-[(4,6-dimethylpyrimidin-2-yl)oxyl-3-methoxy-3,3-diphenylpropanoic acid; N-[6-(2-hydroxyethoxy)-5-(2-methoxyphenoxy)-2-[2-(2H-tetrazol-5-yl)pyridin-4-yl]pyrimidin-4-yl]-5-methylpyridine-2-sulfonamide; N-[6-(2-hydroxyethoxy)-5-(2-methoxyphenoxy)-2-[2-(2H-tetrazol-5-yl)pyridin-4-yl]pyrimidin-4-yl]-5-propan-2-ylpyridine-2-sulfonamide; 6-(2-hydroxy-ethoxy)-5-(2-methoxyphenoxy)-2-[2-(1,2,3-triaza-4-azanidacyclopenta-2,5-dien-5-yl)pyridin-4-yl]pyrimidin-4-yl]-(5-methylpyridin-2-yl)sulfonylazanide; 2-[(3R,6R,9S,12R,15S)-6-(1H-indol-3-ylmethyl)-9-(2-methylpropyl)-2,5,8,11,14-pentaoxo-12-propan-2-yl-1,4,7,10,13-pentazabicyclo[13.3.0]octadecan-3-yl]acetic acid; N-[6-methoxy-5-(2-methoxyphenoxy)-2-pyridin-4-ylpyrimidin-4-yl]-5-methylpyridisulfonamide; N-(3-methoxy-5-methylpyrazin-2-yl)-2-[4-(1,3,4-oxadiazol-2-yl)phenyl]pyridine-3-sulfonamide; and N-[5-(2-methoxyphenoxy)-2-pyridin-4-yl-6-(trideuteriomethoxy)pyrimidine-4-yl]-5-methylpyridine-2-sulfonamide}, selective endothelin B receptor (ETBR) antagonists (e.g., A-192621 and BQ-788), dual ETAR/ETBR antagonists (e.g., bosentan, macitentan and tezosentan), and analogs, derivatives and salts thereof.

The additional therapeutic agents provided herein can include inhibitors of Toll-like receptors (TLRs), including, but not limited to TIR7/non-TLR9 inhibitors (e.g., ODN 2087, ODN 20958 and ODN 20959), dual TLR7/TLR9 inhibitors (e.g., chloroquine, hydroxychloroquine, quinacrine, AT791, DV056, E6446, IMO-3100, IMO-8400 and ODN 2088), and analogs, derivatives, fragments and salts thereof.

The additional therapeutic agents provided herein can include inhibitors of mitogen-activated protein (MAP) kinases, including but not limited to p38 MAP kinase inhibitors {e.g., BMS-582949, CPSI-2364, 4-(4-fluorophenyl)-2-(4-hydroxyphenyl)-5-(4-pyridyl)-1H-imidazole, trans-4-[4-(4-fluorophenyl)-5-(2-methoxy-4-pyrimidinyl)-1H-imidazole-1-yl-]cyclohexanol, and 4-(4-fluorophenyl)-2-(4-methylsulfinylphenyl)-5-(4-pyridyl)-1H-imidazole}, and analogs, derivatives and salts thereof.

The additional therapeutic agents provided herein can include inhibitors of mitogen-activated protein kinase kinases (MEKs), including but not limited to MEK 1 inhibitors {e.g., N-[3-[5-(2-aminopyrimidin-4-yl)-2-tert-butyl-1,3-thiazol-4-yl]-2-fluorophenyl]-2,6-difluorobenzenesulfonamide; N-[3-[5-(2-aminopyrimidin-4-yl)-2-tert-butyl-1,3-thiazol-4-yl]-2-fluorophenyl]-2,6-difluorobenzenesulfonamide, methanesulfonic acid; 6-(4-bromo-2-chloroanilino)-7-fluoro-N-(2-hydroxyethoxy)-3-methylbenzimidazole-5-carboxamide; 5-bromo-N-(2,3-dihydroxypropoxy)-3,4-difluoro-2-(2-fluoro-4-iodoanilino)benzamide; 6-(4-bromo-2-fluoroanilino)-7-fluoro-N-(2-hydroxyethoxy)-3-methylbenzimidazole-5-carboxamide; 2-[4-[(2-butyl-4-oxo-1,3-diazaspiro[4.4]non-1-en-3-yl)methyl]-2-(ethoxymethyl)phenyl]-N-(3,4-dimethyl-1,2-oxazol-5-yl)benzenesulfonamide; 2-[4-[(2-butyl-4-oxo-1,3-diazaspiro[4.4]non-1-en-3-yl)methyl]-2-(ethoxymethyl)phenyl]-N-(4,5-dimethyl-1,2-oxazol-3-yl)benzenesulfonamide; 2-[4-[(2-butyl-4-oxo-1,3-diazaspiro [4.4]non-1-en-3-yl)methyl]-2-propylphenyl]-N-(4,5-dimethyl-1,2-oxazol-3-yl)benzenesulfonamide; 2-(2-chloro-4-iodoanilino)-N-(cyclopropylmethoxy)-3,4-difluorobenzamide; N-[3-[3-cyclopropyl-5-(2-fluoro-4-iodoanilino)-6,8-dimethyl-2,4,7-trioxopyrido[4,3-d]pyrimidin-1-yl]phenyl]acetamide; 3,4-difluoro-2-(2-fluoro-4-iodoanilino)-N-(2-hydroxyethoxy)-5-[(3-oxooxazinan-2-yl)methyl]benzamide; N-[3,4-difluoro-2-(2-fluoro-4-iodoanilino)-6-methoxyphenyl]-[(2S)-2,3-dihydroxypropyl]cyclopropane-1-sulfonamide; [3,4-difluoro-2-(2-fluoro-4-iodoanilino)phenyl]-[3-hydroxy-3-[(2S)-piperidin-2-yl]azetidin-1-yl]methanone; N-[(2R)-2,3-dihydroxypropoxy]-3,4-difluoro-2-(2-fluoro-4-iodoanilino)benzamide; (2S,3S)-2-[(4R)-4-[4-[(2R)-2,3-dihydroxypropoxy]phenyl]-2,5-dioxoimidazolidin-1-yl]-N-(2-fluoro-4-iodophenyl)-3-phenylbutanamide; 3-[(2R)-2,3-dihydroxypropyl]-6-fluoro-5-(2-fluoro-4-iodoanilino)-8-methylpyrido[2,3-(1]pyrimidine-4,7-dione; N-[(2S)-2,3-dihydroxypropyl]-3-(2-fluoro-4-iodoanilino)pyridine-4-carboxamide, and 2-(2-fluoro-4-iodoanilino)-N-(2-hydroxyethoxy)-1,5-dimethyl-6-oxopyridine-3-carboxamide}, and analogs, derivatives and salts thereof.

The additional therapeutic agents provided herein can include inhibitors of calcitonin gene-related peptide (CGRP) or receptor therefor or the production thereof, including but not limited to CORP receptor antagonists (e.g., olcegepant, telcagepant, ubrogepant, eptinezumab [ALD-403], AMG-334, LY-2951742 and TEV-48125), and analogs, derivatives, fragments and salts thereof.

The additional therapeutic agents provided herein can include inhibitors of gastrin-releasing peptide (GRP) or the receptor therefor (GRPR, aka bombesin receptor 2 [BBR2]) or the production thereof, including but not limited to CRPR antagonists (e.g.; RC-3095), and analogs, derivatives and salts thereof.

The additional therapeutic agents provided herein can include inhibitors of nerve growth factor (NGF) or receptors therefor tropomyosin kinase receptor A [TrkA]) or the production thereof, including but not limited to NGF inhibitors (e.g., fulranumab and tanezumab), NGF receptor inhibitors (e.g., TrkA inhibitors such as A0879, CT327 and K252a), and analogs, derivatives, fragments and salts thereof.

The additional therapeutic agents provided herein can include inhibitors of neurotensin or receptors therefor (e.g., neurotensin receptor 1 [NTSR1], NTSR2 and so 1) or the production thereof, including but not limited to selective NTSR1 antagonists (e.g., SR-48,692), selective NTSR2 antagonists (e.g., levocabastine), unselective receptor antagonists (e.g., SR-142,948), and analogs, derivatives and salts thereof.

The additional therapeutic agents provided herein can include inhibitors of somatostatin or receptors therefor (e.g., somatostatin receptors [SSTRs] 1 to 5) or the production thereof, including but not limited to selective SSTR2 antagonists (e.g., CYN 154806), selective SSTRS antagonists (e.g., BIM 23056), unselective SSTR antagonists (e.g., cyclosomatostatin), and analogs, derivatives, fragments and salts thereof.

The additional therapeutic agents provided herein can include inhibitors of vasoactive intestinal peptide (VIP) or receptors therefor (e.g., VIPR1 and VIPR2) or the production thereof, including but not limited to VIP receptor antagonists {e.g., PG 97-269, ViPhyb, VIP(6-28)-NH₂, [p-Cl-D-Phe⁶, Leu¹⁷]VIP-NH₂, [Ac-His¹, D-Phe², Lys¹⁵, Arg¹⁶]VIP(3-7)GRF(8-27)-NH₂, and [Ac-Tyr¹, D-Phe²]GRF(1-29)-NH₂}, and analogs, derivatives, fragments and salts thereof.

The additional therapeutic agents provided herein can include inhibitors of bradykinin or receptors therefor (e.g., B1 and B2) or the production thereof, including but not limited to bradykinin inhibitors (e.g., aloe, bromelain and polyphenols), bradykinin receptor B2 antagonists (e.g., icatibant and FR-173657), inhibitors of kallikreins (e.g., ecallantide, camostat, nafamostat, gabexate and C1-inhibitor), and analogs, derivatives and salts thereof.

The additional therapeutic agents provided herein can include inhibitors of corticotropin-releasing hormone (CRH, aka corticoliberin) or receptors therefor (e.g., CRHR1 and CRHR2) or the production thereof, including but not limited to CRHR1 antagonists (e.g., antalarmin, pexacerfont, CP-154,526 LWH-234, NBI-27914 and R-121,919), CRHR2 antagonists (e.g., astressin-B), and analogs, derivatives and salts thereof.

The additional therapeutic agents provided herein can include antihistamines, including but not limited to antihistamines that inhibit action at the histamine H₁ receptor (e.g., acrivastine, antazoline, astemizole, azatadine, azelastine, bepotasiine, bilastine, bromodiphenhydramine, brompheniramine, buclizine, carbinoxamine, cetirizine, chlorcyclizine, chlorodiphenhydramine, chlorpheniramine, chlorpromazine, chloropyramine, cidoxepin, clemastine, cyclizine, cyproheptadine, desloratadine, dexbrompheniramine, dexchlorpheniramine, dimenhydrinate, dimetindene, diphenhydramine, doxepin, doxylamine, ebastine, embramine, esmirtazapine [(S)-(+)-enantiomer of mirtazapine], fexofenadine, hydroxyzine, ketotifen, levocabastine, levocetirizine, loratadine, meclozine mepyramine, mirtazapine, mizolastine, olopatadine, orphenadrine, phenindamine, pheniramine, phenyltoloxamine, promethazine, pyrilamine, quetiapine, quifenadine, rupatadine, terfenadine, trimeprazine tripelennamine and triprolidine), antihistamines that inhibit action at the histamine H₃ receptor (e.g., betahistine, burimamide, ciproxifan, clobenpropit, conessine, failproxifan, impentamine, iodophenpropit, irdabisant, pitolisant, thioperamide, A-349,821, ABT-239 and VUF-568), antihistamines that inhibit action at the histamine H₄ receptor (e.g., clobenpropit, thioperamide, A943931, A987306, JNJ-7777120, VUF-6002 and ZPL-389), and analogs, derivatives and salts thereof.

The additional therapeutic agents provided herein can include inhibitors of phospholipase A2 (e.g., secreted and cytosolic PLA2), including but not limited to arachidonyl trifluoromethyl ketone, bromoenol lactone, chloroquine, cytidine 5-diphosphoamines, darapladib, quinacrine, vitamin E, RO-061606, ZPL-521, lipocortins (annexins), and analogs, derivatives, fragments and salts thereof.

The additional therapeutic agents provided herein can include inhibitors of pro-inflammatory prostaglandins (e.g., prostaglandin E2) or receptors therefor or the production thereof, including but not limited to non-steroidal anti-inflammatory drugs (NSAIDs) (e.g., non-selective COX-1/COX-2 inhibitors such as aspirin and selective COX-2 inhibitors such as coxibs), glucocorticoids, cyclopentenone prostaglandins (e.g., prostaglandin J2 [PGJ2], Δ12-PGJ2 and 15-deoxy-Δ12,14-PGJ2), and analogs, derivatives and salts thereof, inhibitors of leukotrienes or receptors therefor or the production thereof, including but not limited to leukotriene receptor antagonists (e.g., cinalukast, gemilukast, iralukast, montelukast, pranlukast, tomelukast, verlukast, zafirlukast, CP-199330, HAMI-3379, ICI-198615 and MK-571), 5-lipoxygenase inhibitors (e.g., baicalein, caffeic acid, curcumin, hyperforin, meclofenamic acid, meclofenamate sodium, zileuton and MK-886), and analogs, derivatives and salts thereof.

The additional therapeutic agents provided herein can include mast cell stabilizers, including but not limited to cromoglicic acid (cromolyn), ketotifen, methylxanthines, nedocromil, olopatadine, omalizumab, pemirolast, quercetin. β₂-adrenoreceptor agonists {including short-acting β₂-adrenergic agonists (e.g., bitolterol, fenoterol, isoprenaline [isoproterenol], levosalbutamol [levalbuterol], orciprenaline [metaproterenol], pirbuterol, procaterol, ritodrine, salbutamol [albuterol] and terbutaline), long-acting β₂-adrenergic agonists arformoterol, bambuterol, clenbuterol, formoterol and salmeterol), and ultralong-acting β₂-adrenergic agonists (e.g., carmoterol, indacaterol, milveterol, olodaterol and vilanterol)}, and analogs, derivatives and salts thereof.

The additional therapeutic agents provided herein can include Janus kinase (JAX) inhibitors, including, but not limited to JAK1 inhibitors (e.g., GLPG0634 and GSK2586184). JAK2 inhibitors (e.g., lestaurtinib, pacritinib, CYT387 and TG101348), JAK3 inhibitors (e.g., ASP-015K, 8348 and VX-509), dual JAK1/JAK2 inhibitors (e.g., baricitinib and ruxolitinib), dual JAK1/JAK3 inhibitors (e.g., tofacitinib), and analogs, derivatives and salts thereof.

The additional therapeutic agents provided herein can include immunomodulators, including but not limited to imides (e.g., thalidomide, lenalidomide, pomalidomide and apremilast), xanthine derivatives (e.g., lisofylline, pentoxifylline and propentofylline), and analogs, derivatives and salts thereof.

The additional therapeutic agents provided herein can include immunosuppressants, including but not limited to glucocorticoids, antimetabolites (e.g., hydroxyurea [hydroxycarbamide], antifolates [e.g., methotrexate], and purine analogs [e.g., azathioprine, mercaptopurine and thioguanine]), calcineurin inhibitors (e.g, ciclosporin [cyclosporine A], pimecrolimus and tacrolimus), inosine-5'-monophosphate dehydrogenase (IMPDH) inhibitors (e.g., mycophenolic acid and derivatives thereof [e.g., mycophenolate sodium and mycophenolate mofetil]), mechanistic/mammalian target of rapamycin (mTOR) inhibitors (e.g., rapamycin [sirolimus], deforolimus [ridaforolimus], everolimus, temsirolimus, umirolimus [biolimus A9], zotarolimus and RTP-801), modulators of sphingosine-1-phosphate receptors (e.g., SIPR1) (e.g., fingolimod), serine C-palmitoyltransferase inhibitors (e.g., myriocin), and analogs, derivatives and salts thereof.

The additional therapeutic agents provided herein can include corticosteroids/glucocorticoids, including but not limited to hydrocortisone types (e.g., cortisone and derivatives thereof [e.g., cortisone acetate], hydrocortisone and derivatives thereof [e.g., hydrocortisone acetate, hydrocortisone-17-aceponate, hydrocortisone-17-buteprate, hydrocortisone-17-butyrate and hydrocortisone-17-valerate], prednisolone, methylprednisolone and derivatives thereof [e.g., methylprednisolone aceponate], prednisone, and tixocortol and derivatives thereof [e.g., tixocortol pivalate]), betamethasone types (e.g., betamethasone and derivatives thereof [e.g., betamethasone dipropionate, betamethasone sodium phosphate and betamethasone valerate], dexamethasone and derivatives thereof [e.g., dexamethasone sodium phosphate], and fluocortolone and derivatives thereof [e.g., fluocortolone caproate and fluocortolone pivalate]), halogenated steroids (e.g., alclometasone and derivatives thereof [e.g., alclometasone dipropionate], beclometasone and derivatives thereof [e.g., beclometasone dipropionate], clobetasol and derivatives thereof [e.g., clobetasol-17-propionate], clobetasone and derivatives thereof [e.g., clobetasone-17-butyrate], desoximetasone and derivatives thereof [e.g., desoximetasone acetate], diflorasone and derivatives thereof [e.g., diflorasone diacetate], diflucortolone and derivatives thereof [e.g., diflucortolone valerate], fluprednidene and derivatives thereof [e.g., fluprednidene acetate], fluticasone and derivatives thereof [e.g., fluticasone propionate], halobetasol [ulobetasol] and derivatives thereof [e.g., halobetasol proprionate], halometasone and derivatives thereof [e.g., halometasone acetate], and mometasone and derivatives thereof [e.g., mometasone furoate]), acetonides and related substances (e.g., amcinonide, budesonide, ciclesonide, desonide, fluocinonide, fluocinolone acetonide, flurandrenolide [flurandrenolone or fludroxycortide], halcinonide, triamcinolone acetonide and triamcinolone alcohol), carbonates (e.g., prednicarbate), and analogs, derivatives and salts thereof.

The additional therapeutic agents provided herein can include inhibitors of pro-inflammatory cytokines or receptors therefor, including but not limited to inhibitors of (e.g., antibodies to) tumor necrosis factor-alpha (TNF-α) (e.g, adalimumab, certolizumab pegol, golimumab, infliximab, etanercept, bupropion and ART-621), inhibitors of (e.g., antibodies to) pro-inflammatory interferons (e.g., interferon-alpha [IFN-α]) or receptors therefor, inhibitors of (e.g., antibodies to) pro-inflammatory interleukins or receptors therefor (e.g., IL-1 [e.g., IL-1α and IL-1β] or IL-1R [e.g., EBI-005 {isunakinra}], IL-2 or IL-2R [e.g., basiliximab and daclizumab], IL-4 or IL-4R [e.g., dupilumab], IL-5 [e.g., mepolizumab] or IL-5R, IL-6 [e.g., clazakizumab, elsilimomab, olokizumab, siltuximab and sirukumab] or IL-6R [e.g., sarilumab and tocilizumab], IL-8 or IL-8R, IL-12 [e.g., briakinumab and ustekinumab] or IL-12R, IL-13 or IL-13R, IL-15 or IL-15R, IL-17 [e.g., ixekizumab and secukinumab] or IL-17R [e.g., brodalumab], IL-18 or IL-18R, IL-20 [e.g., the antibody 7E] or IL-20R, IL-22 [e.g., fezakinumab] or IL-22R, IL-23 [e.g., briakinumab, guselkumab, risankizumab, tildrakizumab SCH-9002221, ustekinumab and BI-655066] or IL-23R, IL-31 or IL-31R [e.g., anti-IL-31 receptor A antibodies such as nemolizumab], IL-33 or IL-33R, and IL-36 or IL-36R), and analogs, derivatives, fragments and salts thereof.

The additional therapeutic agents provided herein can include inhibitors of the production of pro-inflammatory cytokines or receptors therefor, including but not limited to inhibitors of the production of TNF-α (e.g., myxoma virus M013 protein, *Yersinia* YopM, protein, glucocorticoids, immunomodulatory imides, PDE4 inhibitors, p38 MAP kinase inhibitors, inhibitors of TLRs such as TLR7 and TLR9, scrim protease inhibitors [e.g., gabexate and nafamostat], and prostacyclin, carbacyclin and analogs and derivatives thereof [e.g., beraprost, cicaprost, ciprosten, eptaloprost, iloprost and treprostinil]), IFN-α (e.g., alefacept and inhibitors of TLRs such as TLR7 and TLR9), IL-1 (e.g., IL-1α, and IL-1β) (e.g., M013 protein, YopM protein, nafamostat, prostacyclin, glucocorticoids, TNF-α inhibitors, inhibitors of TLRs such as TLR7 and TLR9, and PAR1 antagonists), IL-2 (e.g., glucocorticoids, calcineurin inhibitors and PDE4 inhibitors), IL-4 (e.g., glucocorticoids and serine protease inhibitors [e.g., gabexate and nafamostat]), IL-5 (e.g., glucocorticoids), IL-6 M013 protein, nafamostat, prostacyclin, tranilast, glucocorticoids, immunomodulatory imides, TNF-α inhibitors, and inhibitors of TLRs such as TLR7 and TLR9), IL-8 alefacept, glucocorticoids and PAR2 antagonists [e.g., tetracyclines]), IL-12 (e.g., apilimod, YopM protein, PDE4 inhibitors, and inhibitors of TLRs such as TLR7 and TLR9), IL-15 (e.g., YopM protein), IL-17 (e.g., protein kinase C [PKC] inhibitors such as sotrastaurin), IL-18 (e.g., MOD protein and YopM protein), and IL-23 (e.g., apilimod, alefacept and PDE4 inhibitors), and analogs, derivatives, fragments and salts thereof.

The additional therapeutic agents provided herein can include other kinds of anti-inflammatory agents, including but not limited to inhibitors of pro-inflammatory transcription factors e.g., inhibitors of NE-κB [e.g., nafamostat, M013 protein, penetranin, (-)-DHMEQ, IT-603, IT-901 and PBS-1086] and inhibitors of STAT [signal transducer and activator of transcription] proteins [e.g., JAK1, JAK2 and JAK3 inhibitors]), antagonists of the prostaglandin D₂ receptor (DP₁) or/and the chemoattractant receptor homologous molecule expressed on TH₂ cells (CRTH2) (e.g., TS-022), phosphodiesterase (PDE) inhibitors (e.g., PDE4 inhibitors such as apremilast, cilomilast, ibudilast, piclamilast, roflumilast, crisaborole, diazepam, luteolin, mesembrenone, rolipram, AN2728 and E6005), IgE inhibitors (e.g., anti-IgE antibodies such as omalizumab), myeloperoxidase inhibitors (e.g., dapsone), specialized pro-resolving mediators (SPMs) (e.g., metabolites of polyunsaturated fatty acids such as lipoxins, resolvins [including resolvins derived from 5Z,8Z,11Z,14Z,17Z-eicosapentaenoic acid {EPA}, resolvins derived from 4Z,7Z,10Z,13Z,16Z,19Z-docosahexaenoic acid {DHA}, and resolvins derived from 7Z,10Z,13Z,16Z,19Z-docosahexaenoic acid {n-3 DPA}], protectins/neuroprotectins [including DHA-derived protectins/neuroprotectins and n-3 DPA-derived protectins/neuroprotectins], maresins [including DHA-derived maresins and n-3 DPA-derived maresins], n-3 DPA metabolites, n-6 DPA {4Z,7Z,10Z,13Z,16Z-docosapentaenoic acid} metabolites, oxo-DHA metabolites, oxo-DPA metabolites, docosahexaenoyl ethanolamide metabolites, cyclopentenone prostaglandins [e.g., Δ12-PGJ2 and 15-deoxy-Δ12,14-PGJ2], and cyclopentenone isoprostanes [e.g., 5,6-epoxyisoprostane A2 and 5,6-epoxyisoprostane E2]), disease-modifying antirheumatic drugs (DMARDs, e.g., sulfasalazine and mesalazine [5-aminosalicylic acid]), anti-allergic agents (e.g., antihistamines, inhibitors of leukotrienes or receptors therefor or the production thereof, mast cell stabilizers, glucocorticoids, epinephrine [adrenaline] and tranilast), ultraviolet radiation (e.g., ultraviolet A and B), and analogs, derivatives, fragments and salts thereof.

The additional therapeutic agents provided herein can include antagonists of serotonin receptors, including but not limited to 5-HT₂ antagonists (e.g., clozapine, cyproheptadine ketanserin, pizotifen [pizotyline] and quetiapine), 5-HT₃ antagonists (e.g., alosetron, bemesetron, cilansetron, dolasetron, granisetron, ondansetron, palonosetron, ricasetron, tropanserin, tropisetron, zatosetron, mirtazapine, esmirtazapine and substances present in ginger [e.g., galanolactone, gingerols and shogaols]), and analogs, derivatives and salts thereof.

The additional therapeutic agents provided herein can include antagonists of muscarinic acetylcholine receptors (e.g., M1 to M5), including but not limited to aclidinium, atropine, benzatropine, biperiden, chlorpheniramine, cyclopentolate, darifenacin, dicyclomine, dimenhydrinate, diphenhydramine, doxepin, doxylamine, flavoxate, glycopyrrolate, hyoscyamine, ipratropium, orphenadrine, oxitropium, oxybutynin, pirenzepine, procyclidine, scopolamine (hyoscine), solifenacin, tolterodine, tiotropium, trihexyphenidyl, tropicamide, tricyclic antidepressants, and analogs, derivatives and salts thereof.

Examples of non-steroidal anti-inflammatory drugs (NSAIDs) the can be employed with the compounds provided herein include, but are not limited to: acetic acid derivatives, such as aceclofenac, bromfenac, diclofenac, etodolac, indomethacin, ketorolac, nabumetone, sulindac, sulindac sulfide, sulindac sulfone and tolmetin; anthranilic acid derivatives (fenamates), such as flufenamic acid, meclofenamic acid, mefenamic acid and tolfenamic acid; enolic acid derivatives (oxicams), such as droxicam, isoxicam, lornoxicam, meloxicam, piroxicam and tenoxicam; propionic acid derivatives, such as fenoprofen, flurbiprofen, ibuprofen, dexibuprofen, ketoprofen, dexketoprofen, loxoprofen, naproxen and oxaprozin; salicylates, such as diflunisal, salicylic acid, acetylsalicylic acid (aspirin), choline magnesium trisalicylate, and salsalate; COX-2-selective inhibitors, such as apricoxib, celecoxib, etoricoxib, firocoxib, fluorocoxibs (e.g., fluorocoxibs A-C), lumiracoxib, mavacoxib, parecoxib, rofecoxib, tilmacoxib (JTE-522), valdecoxib, 4-O-methylhonokiol, niflumic acid, DuP-697, CG100649, GW406381, NS-398, SC-58125, benzothieno[3,2-d]pyrimidin-4-one sulfonamide thio-derivatives, and COX-2 inhibitors derived from *Tribulus terrestris*; other kinds of NSAIDs, such as monoterpenoids (e.g., eucalyptol and phenols [e.g., carvacrol]), anilinopyridinecarboxylic acids (e.g., clonixin), sulfonanilides (e.g., nimesulide), and dual inhibitors of lipooxygenase (e.g., 5-LOX) and cyclooxygenase (e.g., COX-2) [e.g., chebulagic acid, licofelone, 2-(3,4,5-trimethoxyphenyl)-4-(N-methylindol-3-yl)thiophene, and di-tert-butylphenol-based compounds (e.g., DTPBHZ, DTPINH, DTPNHZ and DTPSAL)]; and analogs, derivatives and salts thereof.

The one or more antiviral agents and/or the one or more additional therapeutic agents can one or more of the following: Gimsilumab, an anti-granulocyte-macrophage colony stimulating factor monoclonal antibody, a non-viral gene therapy producing monoclonal antibodies, EB05, a non-steroidal anti-inflammatory molecule (sPLA2 inhibitor), Opdivo (nivolumab), a PD-1 blocking antibody, IC14, a recombinant chimeric anti-CD14 monoclonal antibody, avastin (bevacizumab), a vascular endothelial growth factor inhibitor, a PD-1 blocking antibody, Thymosin, meplazumab, an anti-CD147 antibody, an antibody combination REGN-COV2 (REGN10933+REGN10987) against the spike protein MEDI3506, a monoclonal antibody targeting interleukin 33, OmniChicken platform antibodies, antibodies from recovered COVID-19 patients, Antibody 47D11, Polyclonal hyperimmune globulin (H-IG), LY-CoV555 antibody, otilimab, an anti-granulocyte macrophase colony-stimulating factor (GM-CSF) antibody, LY3127804, an anti-Angiopoietin 2 (Ang2) antibody, a CXC10 antagonist, polyclonal hyperimmune globulin (H-IG), Octagam, intravenous Immunoglobulin (IVIG), single domain antibodies (sdAbs), an engineered monoclonal antibody derived from camelids, a super-antibody or antibody cocktail to target potential mutations of SARS-CoV-2, AiRuiKa (camrelizumab), an anti-programmed cell death protein (PD-1) antibody, Linked nanobody antibody, antibodies from recovered COVID-19 patients, OmniRat platform antibodies, Soliris (eculizumab), a complement inhibitor, CT-P59, Ultomiris (ravulizumab-cwvz), rCIG (recombinant anti-coronavirus 19 hyperimmune gammaglobulin), VIR-7831, VIR-7832, Gamifant (emapalumab), an anti-interferon gamma antibody, leronlimab (PRO 140), an CCR5 antagonist, polyclonal hyperimmune globulin (H-IG), Sylvant (siltuximab), an interleukin-6 targeted monoclonal antibody, Actemra (tocilizumab), an interleukin-6 receptor antagonist, Kevzara (sarilumab), an interleukin-6 receptor antagonist, purified ovine immunoglobulin from immunized sheep, lenzilumab, an anti-granulocyte-macrophage colony stimulating factor antibody, Ilaris (canakinumab), an interleukin-1beta blocker, JS016 antibody, TJM2 (TJ003234), an anti-granulocyte-macrophage colony stimulating factor antibody, COVI-SHIELD antibody cocktail, an antibody targeting the S protein, COVID-EIG plasma, SAB-185, polyclonal hyperimmune globulin (H-IG), IFX-1, an anti-C5a antibody, CERC-002, an anti-LIGHT monoclonal antibody, Remsima (infliximab), an anti-TNF antibody, TY027, a monoclonal antibody targeting SARS-CoV-2, IgY-110, an anti-CoV-2 antibody (nasal spray application), mavrilimumab, an anti-granulocyte-macrophase colony-stimunlating factor receptor-alpha monoclonal antibody, BDB-100, monocloncal anti-C5a antibody, TZLS-501, an anti-interleukin-6 receptor monoclonal antibody, itolizumab, anti-CD6 IgG1 monoclonal antibody, GC5131A, BTL-tml, galidesivir, emetine hydrochloride, DAS181, recombinant sialidase (nebulized), Favilavir/Favipiravir/T-705/Avigan, Vicromax, ISR-50, Levovir (clevudine), AB001, EIDD-2801, an oral ribonucleoside analog, ASC09, an HIV protease inhibitor, Tamiflu (oseltamivir), a neuraminidase inhibitor, Truvada, emtricitabine, tenofovir, a HIV-1 nucleoside analog reverse transcriptase inhibitor, Virazole, ribavirin for inhalation solution, AT-527, an oral purine nucleotide prodrug, Ganovo (danoprevir), a hepatitis C virus NS3 protease inhibitor, ritonavir, remdesivir, a nucleotide analog, Arbidol (umifenovir), Prezcobix (darunavir, HIV-1 protease inhibitor/cobicistat, CYP3A inhibitor), Kaletra/Aluvia (lopinavir/ritonavir), an HIV-1 protease inhibitor, prophylactic antiviral CRISPR in human cells (PAC-MAN), GC376, AmnioBoost, concentrated allogeneic MSCs and cytokines derived from amniotic fluid, Astrostem-V, allogenic adipose-derived mesenchymal stem cells (HB-adMSCs), bone marrow-derived allogenic mesenchymal stem cells (BM-Allo-MSC), mesenchymal stem cells, allogenic adipose-derived mesenchymal stem cells (HB-adMSCs) haNK, natural killer cells, Ryoncil (remestemcel-L), allogenic mesenchymal stem cells, MultiStem, bone marrow stem cells, allogeneic T-cell therapies, Autologous Adipose-Tissue Derived Mesenchymal Stem Cells (ADMSCs) and allogeneic MSCs, CYNK-001, CAP-1002, allogenic cardiosphere-derived cells, PLX cell product, placenta-based cell therapy, Chimeric antigen receptors (CAR)/T cell receptors (TCR)-T cell therapy, natural killer cell-based therapy, small mobile stem (SMS) cells, IMS001, human embryonic stem cell-derived mesenchymal stem cells (hES-MSC), VIR-2703 (ALN-COV) siRNA, OT-101, a TGF-Beta antisense drug, inhaled mRNA, peptide conjugated antisense oligonucleotides, Ampligen, rintatolimod, BXT-25, glycoprotein, EDP1815, Ivermectin, tradipitant, a neurokinin-1 receptor antagonist, piclidenoson, A3 adenosine receptor agonist, Ryanodex (dantrolene sodium), a skeletal muscle relaxant, Jakafi/jakavi (ruxolitinib), nitazoxanide, antiprotozoal, peptides targeting the NP protein, interferon/peginterferon alpha-2b, PegIntron, Sylatron, IntronA, PegiHep, roscovitine seliciclib, cyclin-dependent kinase (CDK)2/9 inhibitor, ATYR1923, a fusion protein comprising immuno-modulatory domain of histidyl tRNA synthetase fused to the Fc region of a human antibody, a modulator of neuropilin-2, Leukine (sargramostim, rhu-Granulocyte macrophage colony stimulating factor), ADX-1612, HSP 90 inhibitor, DSTAT (dociparstat sodium), glycosaminoglycan derivative of heparin, BIO-11006, Recombinant human interferon alpha-1b, ST-001 nanoFenretinide (fenretinide), Activase (alteplase), tissue plasminogen activator (tPA), camostat mesylate, a transmembrane protease serine 2 (TMPRSS2) inhibitor, nitric oxide, Cozaar (losartan), an angiotensin II receptor blocker (ARB), Otezla (apremilast), an inhibitor of phosphodiesterase 4 (PDE4), IMU-838, a selective oral dihydroorotate dehydrogenase (DHODH) inhibitor, Colchicine, Brilacidin, a defensin mimetic, Metablok (LSALT peptide), a selective dipeptidase-1 antagonist, nafamostat, CD24Fc, an agent comprising nonpolymorphic regions of CD24 attached to the Fc region of human IgG1, Aplidin (plitidepsin), fadraciclib (CYC065), a cyclin-dependent kinase (CDK)2/9 inhibitor, Aviptadil, a synthetic form of Vasoactive Intestinal Polypeptide (RLF-100), solnatide, a synthetic molecule with a structure based on the lectin-like domain of human Tumour Necrosis Factor alpha, PP-001, MRx-4DP0004, a strain of Bifidobacterium breve isolated from the gut microbiome of a healthy human, ARMS-1, BLD-2660, a small molecule inhibitor of calpain (CAPN) 1, a small molecule inhibitor of CAPN2, a small molecule inhibitor of CAPN9, LAU-7b (fenretinide), N-803, an IL-15 "superagonist" (Nogapendekin alfa inbakicept), Rebif, interferon beta-1a, DIBI, an iron-binding polymer, EPAspire, an oral formulation of highly purified eicosapentaenoic acid free fatty acid (EPA-FFA) in gastro-resistant capsules, MN-166 (ibudilast), a small molecule macrophase migration inhibitory factor (MIF) inhibitor, a phosphodiesterase (PDE) 4 inhibitor, a PDE10 inhibitor, ADX-629, an orally available reactive aldehyde species (RASP) inhibitor, Calquence (acalabrutinib), a Bruton's tyrosine kinase (BTK) inhibitor, Auxora (CM4620-IE), a calcium release-activated calcium (CRAC) channel inhibitor Neumifil, a multivalent carbohydrate binding molecule, Diovan (valsartan), an angiotensin II receptor blocker (ARB), Yeliva (opaganib, ABC294640), an oral sphingosine kinase-2 (SK2) selective inhibitor, WP1122, a glucose decoy prodrug, Kineret (anakinra), an interleukin-1 receptor antagonist, a microbiome therapeutic, Coronzot, bemcentinib, a selective AXL kinase inhibitor, a synthesized nanoviricide drug, Chloroquine/Hydroxychloroquine, an antimalarial drug Senicapoc, vazegepant, a CGRP receptor antagonist, APN01, a recombinant soluble human Angiotensin Converting Enzyme 2, GP1681, a small molecule inhibitor of cytokine release, ST266, a cell-free biologic made from anti-inflammatory proteins secreted by placental cells, recombinant human plasma gelsolin (rhupGSN), pacritinib, an oral kinase inhibitor with specificity for JAK2, IRAK1 and CSFIR, Ruconest (recombinant human C1 esterase inhibitor), Cerocal (ifenprodil), NP-120, an NDMA receptor glutamate receptor antagonist targeting Glu2NB, Peginterferon lambda, Pepcid (famotidine), a histamine-2 (H2) receptor antagonist, heparin, a low molecular weight heparin (enoxaparin), an anticoagulant, Xeljanz (tofacitinib), a Janus kinase (JAK) inhibitor, Xpovio (selinexor), a selective inhibitor of nuclear export (SINE) compound, a pH barrier, transepithelial nebulized alkaline treatment, Luvox (fluvoxamine), a selective serotonin reuptake inhibitor, Micardis (telmisartan), brensocatib, a reversible inhibitor of dipeptidyl peptidase 1 (DPP1) Novaferon, RHB-107 (upamostat, WX-671), a serine protease inhibitor, UNI9011, FW-1022, DWRX2003, niclosamide, Lysteda/Cyklokapron/LB1148 (tranexamic acid), an antifibrinolytic PUL-042 inhalation solution, ABX464, Gleevac (imatinib), Traumakine (interferon beta 1-a), Veyonda (idronoxil), Farxiga (dapagliflozin), a sodium-glucose cotransporter 2 (SGLTs) inhibitor, Gilenya (fingolimod), a sphingosine 1-phosphate receptor modulator, sPIF, a synthetic pre implantation factor, SNG001, an inhaled formulation of interferon beta-1a, Methylprednisolone, ciclesonide (Alvesco), hydrocortisone, corticosteroids Olumiant (baricitinib), a Janus kinase (JAK) inhibitor, dipyridamole (Persantine), an anticoagulant, AT-001, an aldose reductase inhibitor, Vascepa (icosapent ethyl), a form of eicosapentaenoic acid, OP-101, a dendrimer-based therapy, apabetalone (RVX-208), a selective BET (bromodomain and extra-terminal) inhibitor, Flarin (lipid ibuprofen), Almitrine, VP01, an Angiotensin II Type 2 receptor activator, leflunomide, a pyrimidine synthesis inhibitor, Pulmozyme (nebulised dornase alfa), a recombinant DNase enzyme, AQCH, MSTT1041A (anti-ST2, the receptor for IL-33), UTTR1147A (IL-22-Fc), CIGB-258, FSD-201, ultramicronized palmitoylethanolamide, PB1046, a long-acting sustained release human vasoactive intestinal peptide (VIP) analogue, PTC299, an oral small molecule inhibitor of dihydroorotate dehydrogenase (DHODH), raloxifene (Evista), an estrogen agonist/antagonist, losmapimod, an oral selective p38 mitogen activated protein kinase inhibitor, dutasteride, an anti-androgen, M5049, small molecule capable of blocking the activation of Toll-like receptor (TLR)7 and TLR8, Eritoran, a TLR-4 antagonist, desidustat, a hypoxia inducible factor prolyl hydroxylase inhibitor, merimepodib, an IMPDH inhibitor, azithromycin, Cenicriviroc, a chemokine receptor 2 and 5 dual antagonist, Firazyr (icatibant), a bradykinin B2 antagonist, Razoprotafib, Tie 2 activating compound (AKB-9778), or any combination thereof.

Antiviral agents provided include, but are not limited to abacavir; acemannan; acyclovir; acyclovir sodium; adefovir; alovudine; alvircept sudotox; amantadine hydrochloride; amprenavir; aranotin; arildone; atevirdine mesylate; avridine; cidofovir; cipamfylline; cytarabine hydrochloride; delavirdine mesylate; desciclovir; didanosine; disoxaril; edoxudine; efavirenz; enviradene; enviroxime; famciclovir; famotine hydrochloride; fiacitabine; fialuridine; fosarilate; trisodium phosphonoformate; fosfonet sodium; ganciclovir; ganciclovir sodium; idoxuridine; indinavir; kethoxal; lamivudine; lobucavir; memotine hydrochloride; methisazone; nelfinavir; nevirapine; palivizumab; penciclovir; pirodavir; ribavirin; rimantadine hydrochloride; ritonavir; saquinavir mesylate; somantadine hydrochloride; sorivudine; statolon; stavudine; tilorone hydrochloride; trifluridine; valacyclovir hydrochloride; vidarabine; vidarabine phosphate; vidarabine sodium phosphate; viroxime; zalcitabine; zidovudine; zinviroxime, interferon, cyclovir, alpha-interferon, and/or beta globulin. In certain aspects, other antibodies against viral proteins or cellular factors may be used in combination with a therapeutic composition described herein.

Antibacterial agents provided herein include, but are not limited to, β-lactam antibiotics, penicillins (such as natural penicillins, aminopenicillins, penicillinase-resistant penicillins, carboxy penicillins, ureido penicillins), cephalosporins (first generation, second generation, and third generation cephalosporins), and other β-lactams (such as imipenem, monobactams,), β-lactamase inhibitors, vancomycin, aminoglycosides and spectinomycin, tetracyclines, chloramphenicol, erythromycin, lincomycin, clindamycin, rifampin, metronidazole, polymyxins, sulfonamides and trimethoprim, and quinolines. Anti-bacterials also include, but are not limited to: Acedapsone, Acetosulfone Sodium, Alamecin, Alexidine, Amdinocillin, Amdinocillin Pivoxil, Amicycline, Amifloxacin, Amifloxacin Mesylate, Amikacin, Amikacin Sulfate, Aminosalicylic acid, Aminosalicylate sodium, Amoxicillin, Amphomycin, Ampicillin, Ampicillin Sodium, Apalcillin Sodium, Apramycin, Aspartocin, Astromicin Sulfate, Avilamycin, Avoparcin, Azithromycin, Azlocillin, Azlocillin Sodium, Bacampicillin Hydrochloride, Bacitracin, Bacitracin Methylene Disalicylate, Bacitracin Zinc, Bambermycins, Benzoylpas Calcium, Berythromycin, Betamicin Sulfate, Biapenem, Biniramycin, Biphenamine Hydrochloride, Bispyrithione Magsulfex, Butikacin, Butirosin Sulfate, Capreomycin Sulfate, Carbadox, Carbenicillin Disodium, Carbenicillin Indanyl Sodium, Carbenicillin Phenyl Sodium, Carbenicillin Potassium, Carumonam Sodium, Cefaclor, Cefadroxil, Cefamandole, Cefamandole Nafate, Cefamandole Sodium, Cefaparole, Cefatrizine, Cefazaflur Sodium, Cefazolin, Cefazolin Sodium, Cefbuperazone, Cefdinir, Cefepime, Cefepime Hydrochloride, Cefetecol, Cefixime, Cefinenoxime Hydrochloride, Cefinetazole, Cefinetazole Sodium, Cefonicid Monosodium, Cefonicid Sodium, Cefoperazone Sodium, Ceforanide, Cefotaxime Sodium, Cefotetan, Cefotetan Disodium, Cefotiam Hydrochloride, Cefoxitin, Cefoxitin Sodium, Cefpimizole, Cefpimizole Sodium, Cefpiramide, Cefpiramide Sodium, Cefpirome Sulfate, Cefpodoxime Proxetil, Cefprozil, Cefroxadine, Cefsulodin Sodium, Ceftazidime, Ceftibuten, Ceftizoxime Sodium, Ceftriaxone Sodium, Cefuroxime, Cefuroxime Axetil, Cefuroxime Pivoxetil, Cefuroxime Sodium, Cephacetrile Sodium, Cephalexin, Cephalexii Hydrochloride, Cephaloglycini, Cephaloridine, Cephalothin Sodium, Cephapirin Sodium, Cephradine, Cetocycline Hydrochloride, Cetophenicol, Chloramphenicol, Cliloramphenicol Palmitate, Chloramphenicol Pantotheniate Complex, Chloramphenicol Sodium Succinate, Chlorhexidine Phosphanilate, Chloroxylenol, Chlortetracycline Bisulfate, Chlortetracycline Hydrochloride, Cinoxacin, Ciprofloxacin, Ciprofloxacin Hydrochloride, Cirolemycin, Clarithromycin, Clinafloxacin Hydrochloride, Clildamycin, Clindamycin Hydrochloride, Clindamycin Palmitate Hydrochloride, Clindamycin Phosphate, Clofazimine, Cloxacillin Benzathine, Cloxacillin Sodium, Cloxyquin, Colistimethate Sodium, Colistin Sulfate, Coumermycin, Coumermycin Sodium, Cyclacillin, Cycloserine, Dalfopristin, Dapsone, Daptomycin, Demeclocycine, Demeclocycine Hydrochloride, Demecycline, Denofungin, Diaveridine, Dicloxacillin, Dicloxacillin Sodium, Dihydrostreptomycin Sulfate, Dipyrithione, Dirithromycin, Doxycycline, Doxycycline Calcium, Doxycycline Fosfatex, Doxycycline Hyclate, Droxacin Sodium, Enoxacin, Epicillin, Epitetracycline Hydrochloride, Erythromycin, Erythromycin Acistrate, Erythromycin Estolate, Erythromycin Ethylsuccinate, Erythromycin Gluceptate, Erythromycin Lactobionate, Erythromycin Propionate, Erythromycin Stearate, Ethambutol Hydrochloride, Ethionamide, Fleroxacin, Floxacillin, Fludalanine, Flumequine, Fosfomycin, Fosfomycin Tromethamine, Fumoxicillin, Furazolium Chloride, Furazolium Tartrate, Fusidate Sodium, Fusidic Acid, Gentamicin Sulfate, Gloximonam, Gramicidin, Haloprogin, Hetacillin, Hetacillin Potassium, Hexedine, Ibafloxacin, Imipenem, Isoconazole, Isepamicin, Isoniazid, Josamycin, Kanamycin Sulfate, Kitasamycin, Levofuraltadone, Levopropylcillin Potassium, Lexithromycin, Lincomycin, Lincomycin Hydrochloride, Lomefloxacin, Lomefloxacin Hydrochloride, Lomefloxacin Mesylate, Loracarbef, Mafenide, Meclocycline, Meclocycline Sulfosalicylate, Megalomicin Potassium Phosphate, Mequidox, Meropenem, Methacycline, Methacycline Hydrochloride, Methenamine, Methenamine Hippurate, Methenamine Mandelate, Methicillin Sodium, Metioprim, Metronidazole Hydrochloride, Metronidazole Phosphate, Mezlocillin, Mezlocillin Sodium, Minocycline, Minocycline Hydrochloride, Mirincamycin Hydrochloride, Monensin, Monensin Sodium, Nafcillin Sodium, Nalidixate Sodium, Nalidixic Acid, Natamycin, Nebramycin, Neomycin Palmitate, Neomycin Sulfate, Neomycin Undecylenate, Netilmicin Sulfate, Neutramycin, Nifuradene, Nifuraldezone, Nifuratel, Nifuratrone, Nifurdazil, Nifurimide, Nifuirpirinol, Nifurquinazol, Nifurthiazole, Nitrocycline, Nitrofurantoin, Nitromide, Norfloxacin, Novobiocin Sodium, Ofloxacin, Ormetoprim, Oxacillin Sodium, Oximonam, Oximonam Sodium, Oxolinic Acid, Oxytetracycline, Oxytetracycline Calcium, Oxytetracycline Hydrochloride, Paldimycin, Parachlorophenol, Paulomycin, Pefloxacin, Pefloxacin Mesylate, Penamecillin, Penicillin G Benzathine, Penicillin G Potassium, Penicillin G Procaine, Penicillin G Sodium, Penicillin V, Penicillin V Benzathine, Penicillin V Hydrabamine, Penicillin V Potassium, Pentizidone Sodium, Phenyl Aminosalicylate, Piperacillin Sodium, Pirbenicillin Sodium, Piridicillin Sodium, Pirlimycin Hydrochloride, Pivampicillin Hydrochloride, Pivampicillin Pamoate, Pivampicillin Probenate, Polymyxin B Sulfate, Porfiromycin, Propikacin, Pyrazinamide, Pyrithione Zinc, Quindecamine Acetate, Quinupristin, Racephenicol, Ramoplanin, Ranimycin, Relomycin, Repromicin, Rifabutin, Rifametane, Rifamexil, Rifamide, Rifampin, Rifapentine, Rifaximin, Rolitetracycline, Rolitetracycline Nitrate, Rosaramicin, Rosaramicin Butyrate, Rosaramicin Propionate, Rosaramicin Sodium Phosphate, Rosaramicin Stearate, Rosoxacin, Roxarsone, Roxithromycin, Sancycline, Sanfetrinem Sodium, Sarmoxicillin, Sarpicillin, Scopafungin, Sisomicin, Sisomicin Sulfate, Sparfloxacin, Spectinomycin Hydrochloride, Spiramycin, Stallimycin Hydrochloride, Steffimycin, Streptomycin Sulfate, Streptonicozid, Sulfabenz, Sulfabenzamide, Sulfacetamide, Sulfacetamide Sodium, Sulfacytine, Sulfadiazine, Sulfadiazine Sodium, Sulfadoxine, Sulfalene, Sulfamerazine, Sulfameter, Sulfamethazine, Sulfamethizole, Sulfamethoxazole, Sulfamonomethoxine, Sulfamoxole, Sulfanilate Zinc, Sulfanitran, Sulfas alazine, Sulfasomizole, Sulfathiazole, Sulfazamet, Sulfisoxazole, Sulfisoxazole Acetyl, Sulfisoxazole Diolamine, Sulfomyxin, Sulopenem, Sultamicillin, Suncillin Sodium, Talampicillin Hydrochloride, Teicoplanin, Temafloxacin Hydrochloride, Temocillin, Tetracycline, Tetracycline Hydrochloride, Tetracycline Phosphate Complex, Tetroxoprim, Thiamphenicol, Thiphencillin Potassium, Ticarcillin Cresyl Sodium, Ticarcillin Disodium, Ticarcillin Monosodium, Ticlatone, Tiodonium Chloride, Tobramycin, Tobramycin Sulfate, Tosufloxacin, Trimethoprim, Trimethoprim Sulfate, Trisulfapyrimidines, Troleandomycin, Trospectomycin Sulfate, Tyrothricin, Vancomycin, Vancomycin Hydrochloride, Virginiamycin, and/or Zorbamycin.

Anti-fungal agents provided herein include, but are not limited to, azoles, imidazoles, polyenes, posaconazole, fluconazole, itraconazole, amphotericin B, 5-fluorocytosine, miconazole, ketoconazole, Myambutol (Ethambutol Hydrochloride), Dapsone (4,4'-diaminodiphenylsulfone), Paser Granules (aminosalicylic acid granules), rifapentine, Pyrazinamide, Isoniazid, Rifadin IV, Rifampin, Pyrazinamide, Streptomycin Sulfate and Trecator-SC (Ethionamide) and/or voriconazole (VfendTM).

### Synthesis, Pro-drugs, Analogues, and Metabolites of Disclosed Compounds Pipendoxifene

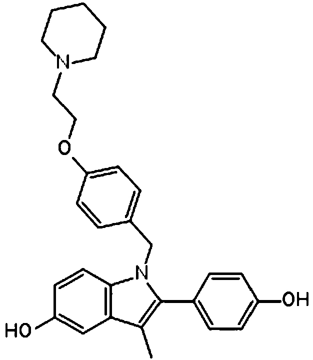

### Pipendoxifene

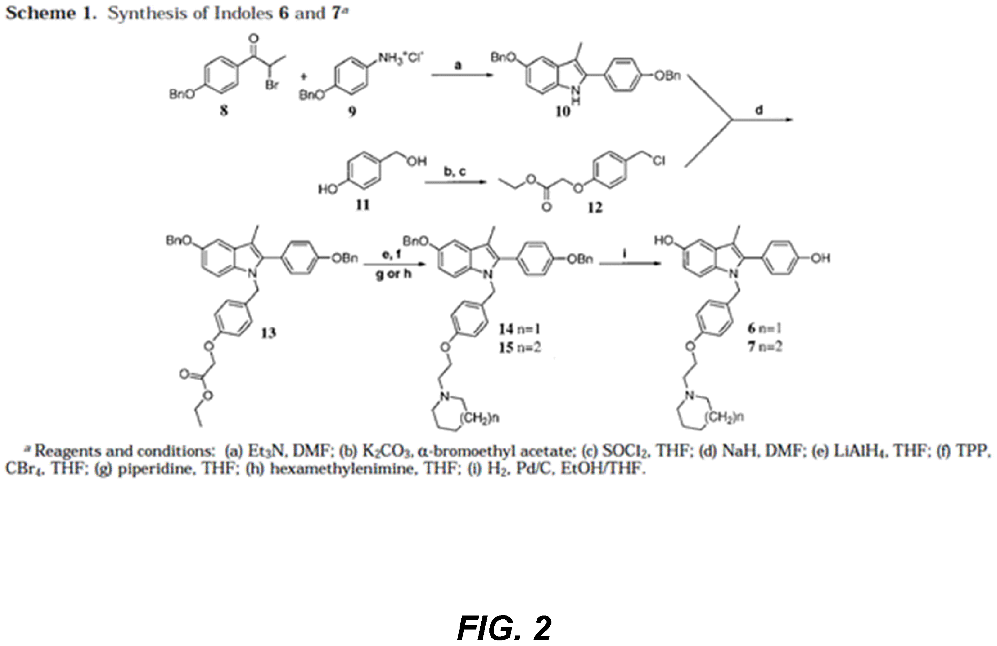

**Kingdom**: Organic compounds
**Super Class**: Organoheterocyclic compounds
**Class**: 2-phenylindoles, Indoles and derivatives
**Sub Class**: Indoles
**Direct Parent**: 2-phenylindoles
**Alternative Parents**: Phenylpyrroles / N-alkylindoles / Hydroxyindoles / 3-methylindoles / Phenoxy compounds / Phenol ethers / Alkyl aryl ethers / 1-hydroxy-2-unsubstituted benzenoids / Piperidines / Heteroaromatic compounds / Trialkylamines / Azacyclic compounds / Organopnictogen compounds / Hydrocarbon derivatives
**Substituents**: 1-hydroxy-2-unsubstituted benzenoid / 2-phenylindole / 2-phenylpyrrole / 3-alkylindole / 3-methylindole / Alkyl aryl ether / Amine / Aromatic heteropolycyclic compound / Azacycle / Benzenoid / Ether / Heteroaromatic compound / Hydrocarbon derivative / Hydroxyindole / Monocyclic benzene moiety / N-alkylindole / Organic nitrogen compound / Organic oxygen compound / Organonitrogen compound / Organooxygen compound / Organopnictogen compound / Phenol / Phenol ether / Phenoxy compound / Piperidine / Pyrrole / Substituted pyrrole / Tertiary aliphatic amine / Tertiary amine
**Molecular Framework**: Aromatic heteropolycyclic compounds
**Discovery** (Miller 2001): After various explorations around the core and side chain, ERA-923 and TSE-424 were discovered, both novel, highly selective estrogens with particularly non-estrogenic profiles on rat uterine tissue**.** **FIG. 2** depicts a non-limiting exemplary indole synthesis scheme.

**Pipendoxifene Metabolism** (Cotreau 2002): In rat and monkey studies, ERA-923 was found to be primarily metabolized to an indole glucuronide, whereas one of the secondary metabolites was a phenyl glucuronide. In vitro studies with human liver slices and microsomes indicate that the primary human metabolite is the same indole glucuronide as found in the preclinical models.

**BZA Metabolism**: Bazedoxifene is highly metabolized in mice, rats and monkeys to the phenyl and indole glucuronides (4'- and 5-glucuronides). The relative levels of these two metabolites were found to be different among species, with both 4'- and 5-glucuronides present in mouse and the 5-glucuronide predominant in rats, monkeys, human. The metabolites are pharmacologically active and antagonized the effect of estrogen in the rat uterus. Comparison of in vitro and in vivo metabolism data suggested that the bazedoxifene-4'-glucuronide is formed in monkeys and humans but preferentially excreted in the bile. Minor metabolites included the diglucuronide (monkey, human) and N-oxide (Tg.Ras mouse urine, human feces). CYP enzymes played a minor role in metabolism. In vitro studies showed that human UGT1A1 and UGT1A10 were active in glucuronidating parent compound. The primary route of excretion was biliary/fecal in mice, rats, monkeys, and humans. Urinary excretion was minor. In mice, >50% of an oral dose was recovered within 24 hours. In rats, recovery of radioactivity was >97% and in monkeys and women it was ca. 85% within 7 days. Less than 1% of dose was recovered in urine. In feces, unchanged drug was the predominant form (>90%). The major urinary metabolite in rats was bazedoxifene-5-glucuronide in rats and bazedoxifene-4'-glucuronide in monkeys.

### MDL-001 (PIPENDOXIFENE)

### Drug Substance and Drug Product

**Exemplary dosage form**: MDL-001 Capsules or Tablets (e.g., oral tablets)
**Exemplary route of administration**: Once daily administered orally
**Chemical Name**: 3-Methyl-4-hydroxy-2-(4-hydroxyphenyl)-1-[4-(piperidinoethoxy)phenylmethyl]indole
**IUPAC Name**: 2-(4-hydroxyphenyl)-3-methyl-1-[[4-(2-piperidin-1-ylethoxy)phenyl]methyl]indol-5-ol
**Chemical Abstracts Service (**CAS) Number / UNII Number: 198480-55-6 / TPC5Q8496G
**Other Names**: ERA-923
**WHO ATC Code**: G03X-C (Selective estrogen receptor modulators); L02A (Hormones and Related Agents)
**Class**: Antineoplastics; Cytostatics; Indoles; Piperidines
**Molecular Formula**: C₂₉ H₃₂ N₂ O₃
**Molecular Weight**: 456.6
**Exemplary Indication:** Pipendoxifene can be used to treat mild-to-moderate coronavirus disease 2019 (COVID-19) in adults and pediatric patients (12 years of age and older weighing at least 40 kg) with positive results of direct severe acute respiratory syndrome coronavirus 2 (SARS-CoV-2) viral testing, and who are at high risk for progression to severe COVID-19, including hospitalization or death.
**Other Exemplary Indications**: Given the pan antiviral activity demonstrated in vitro and supported by in silico docking studies, MDL-001 can be used for the treatment of other RNA viruses and influenza-like illnesses.

### Overview

The novel coronavirus, SARS-CoV-2, has caused a global pandemic infecting more than 483 million and killing over 6.1 million people worldwide. The USA has shared in this burden with 81 million people infected and over one million deaths at this writing (Worldometers.info/coronavirus, 2022). SARS-CoV-2 is a highly infectious and transmissible novel coronavirus that infiltrates bronchial epithelial cells, pneumocytes and upper respiratory tract cells in humans (Vkovski et al, 2022). This infectious process and subsequent immune response can lead to lung injury and the hallmark symptoms of acute respiratory disease syndrome (ARDS) and even death (Torres Acosta et al, 2020).

SARS-CoV-2 infection occurs when the host cell receptor ACE2 facilitates cellular entry into the host cell. The viral proteins, like viral proteases and polymerases, are essential for replication of the SARS-CoV-2. Pertinent to this application, the RNA-dependent RNA polymerase (RdRp) is vital to the viral replication process of SARS-CoV-2. Importantly, this target is necessary for all replicative processes of RNA viruses. Thus, an agent, such as MDL-001 that has broad antiviral activity against RdRp, especially RNA viruses that cause respiratory tract infections with similar symptoms (e.g., COVID-19, influenza A+B), can allow starting treatment to patients presenting with these symptoms immediately while patients are waiting for their diagnostic test results. The earlier access to therapy potentially means control of disease and abatement of symptoms before escalation to a more serious condition.

### Unmet Medical Need

Currently approved and authorized treatments for COVID-19 have an acceptable risk/benefit profile for the clinical settings for which they have been authorized or approved. In the outpatient setting, the ideal Target Product Profile (TPP) of an efficacious treatment that can be taken orally, once a day without significant safety risks (drug-drug interaction, genotoxicity, liver/hepatic toxicity etc.) has yet to be realized. There is high unmet medical need for a therapeutic with this TPP. Thus, new treatments that fit this favorable TPP need to be expedited through development as the next generation of effective therapies for COVID-19. Additionally, agents that potentially have broader antiviral activity and meet this TPP, should be further expedited as there are currently no authorized or approved treatments for COVID-19 that are also indicated for broader antiviral treatment.

### Antiviral Drugs That Are Approved, Authorized, or Under Evaluation for the Treatment of COVID-19

Because SARS-CoV-2 replication leads to many of the clinical manifestations of COVID-19, antiviral therapies are being investigated for the treatment of COVID-19. These drugs prevent viral replication through various mechanisms, including blocking SARS-CoV-2 entry, inhibiting the activity of SARS-CoV-2 3-chymotrypsin-like protease (3CLpro) and RdRp, and causing lethal viral mutagenesis. Because viral replication may be particularly active early in the course of COVID-19, antiviral therapy may have the greatest impact before the illness progresses to the hyperinflammatory state that can characterize the later stages of disease, including critical illness. For this reason, it is necessary to understand the role of antiviral medications, such as MDL-001, in treating mild, moderate, severe, and critical illness in order to optimize treatment for people with COVID-19. (COVID-19 Treatment Guidelines)

### Conclusion

The expedited development of therapies for COVID-19 in the outpatient setting has successfully saved lives, but issues remain for these first-developed drugs including effectiveness, route of administration, burden of medications, toxicity and drug-drug interactions etc. The ideal TPP of an efficacious treatment for COVID-19 includes a) oral administration, b) once a day dosing, and c) no significant safety risks, which has yet to be realized. Thus, there is high unmet medical need for a therapeutic with this profile.

Further, while some of these agents may have broad antiviral activity, like molnupiravir and remdesivir, they have not been evaluated for patients with presenting respiratory syndromes, which may be a paradigm shift in how outpatients with viral respiratory infections are treated. Such a model is analogous to using broad spectrum antibiotics prior to specific bacterial pathogens being identified via culture or other tests.

MDL-001 can realize the TPP described and can have broad spectrum antiviral activity.

### Rationale for MDL-001 (pipendoxifene) Development in COVID-19 and Other Respiratory Viruses

### Pipendoxifene Development History

Pipendoxifene was previously under development by Ligand Pharmaceuticals and Wyeth-Ayerst Laboratories (now Pfizer) for breast cancer and it is believed that pipendoxifene underwent the required IND enabling nonclinical testing prior to entering into first in-human clinical trials in 1999.

### Summary of Pipendoxifene Clinical Data

The safety and pharmacokinetics of pipendoxifene were evaluated in 2 randomized, double-blind, placebo-controlled trials involving healthy postmenopausal women described below.

### Study 1: Single Dose Safety and Pharmacokinetics of Pipendoxifene (N = 46)

Trial: Multiple-Dose, Safety, Pharmacokinetics, and Pharmacodynamics of a New Selective Estrogen Receptor Modulator, ERA-923, in Healthy Postmenopausal Women.

The single dose study enrolled 46 subjects who were given a single pipendoxifene dose or placebo followed by a single higher dose after a 20-day washout period (1/5, 5/25, 25/50, 50/75, 75/100, 150/200 and 200 mg oral).

Pipendoxifene was well tolerated with only mild transient adverse events observed at all doses. In addition, there were no clinically significant changes in laboratory values or laboratory toxicities at any pipendoxifene dose compared to placebo. No vaginal discharge or bleeding or changes in clotting parameters were observed with treatment. The t_{1/2} value was comparable for all doses (30 ± 16 h). Apparent clearance increased with dose. (Sorbera et al, 2002)

### Study 2: Multiple Dose Safety and Pharmacokinetics of Pipendoxifene (N = 50)

Trial: Multiple-Dose, Safety, Pharmacokinetics, and Pharmacodynamics of a New Selective Estrogen Receptor Modulator, ERA-923, in Healthy Postmenopausal Women.

This multiple dose study enrolled 50 subjects who were orally administered pipendoxifene (10, 50, 100, 150 or 200 mg) once daily after an overnight fast except on day 14 when it was taken 10 minutes after a standard high fat breakfast and on days 1 to 28 when subjects were fasted for 4 hours post dosing for 28-days.

Pipendoxifene was also safe and well tolerated. As with the first study, adverse events were mild, reversible and unrelated to dose. The most common adverse events associated with the agent included headache (28% vs. 30% in placebo), pain (23% vs. 10% in placebo) and hot flashes (20% vs. 20% in placebo). No clinically relevant changes in laboratory parameters or vaginal bleeding or discharge were observed. In addition, there was no increase in the incidence of ovarian cysts or significant changes in endometrial thickness as compared to placebo.

Examination of markers for bone metabolism showed no differences in serum bone alkaline phosphatase, serum osteocalcin and urine free deoxypyridinoline between the pipendoxifene groups and placebo. In addition, total cholesterol, HDL, LDL and triglycerides were similar between placebo and treatment groups on days 14 and 28. Dosing with pipendoxifene for 28 days was concluded to be safe and well tolerated in postmenopausal women. (Corteau et al, 2002).

Pharmacokinetic analysis revealed that the agent underwent extensive metabolism and enterohepatic recirculation. Clearance values increased with dose (~4-10.5 l/h/kg) but were similar on days 14 and 28 for each dose. Mean AUC _{(0-24 h)} for unconjugated pipendoxifene increased less than proportional with increasing dose in fasted and fed states. The AUC_{(0-24 h)} and Cₘₐₓ values for unconjugated pipendoxifene on day 14 (fed state) were significantly greater than those obtained on day 28 in groups given doses of 50 mg or higher. The mean terminal t_{1/2} ranged from 15.8-27.3 h for all doses and steady-state plasma levels were achieved after about 4-5 days of dosing. The mean t_{1/2} values for the 10, 50 and 100 mg were similar. However, mean t_{1/2} values for the 150 and 200 mg doses were significantly different (15.8 and 26 h, respectively). A second peak in plasma concentrations was observed suggesting enterohepatic circulation. These secondary peaks were more marked in the fasted state as compared to the fed state. From these results it appears that a high-fat breakfast may increase absorption of the agent.

In summary Pipendoxifene was well tolerated in healthy postmenopausal women (5 sequential cohorts of 10 subjects) when doses of up to 200 mg/day were administered for 28 days. Adverse events were mild and transient (Gandhi et al , 2000) (Gandhi et al - 2, 2000). Among healthy postmenopausal women, the incidence of adverse events was similar for pipendoxifene and placebo recipients, with only grade 1 events reported. (Gandhi et al 2000).

### Other Studies with Pipendoxifene

It appears that Phase II trials among persons with metastatic breast cancer in the US were conducted in mid 2000 (NCT00006369, 2021).

### Assessment of Pipendoxifine Clinical Data as it Relates to MDL-001 Administration

All safety related observations discussed and summarized above are similar to that of the placebo and are found after dosing regimens far beyond than the 10-14 day dosing regimen proposed for MDL-001 as a treatment for COVID-19. As noted below, the starting dose can be selected based upon studies conducted (e.g., those described herein), published data and FDA guidance documents for the selection of a suitable starting dose.

### Selection of MDL-001 as an Antiviral Candidate

The use of MDL-001 as an antiviral drug candidate and a COVID-19 therapeutic was discovered as described herein. The known human safety and tolerability discussed above (no observed Grade 3 or Grade 4 AEs), associated pharmacokinetics (long half-life) and oral route of administration for pipendoxifene, provides an ideal profile for the next generation COVID-19, which, in some embodiments, also is a broader spectrum antiviral therapeutic.

### CHEMprint^{™} Drug Discovery Platform: In silico Discovery and Confirmatory In silico

### Docking of MDL-001 (Examples)

The CHEMprint^{™} platform was employed to discover drugs for disease(s) for which the drugs are predicted to generate disease modifying activity. CHEMprint^{™} combines multiple disciplines including cheminformatics, quantitative structure-activity relationships (QSAR), in silico drug-protein binding and artificial intelligence (AI) and machine learning (ML) in a single platform, to accomplish this task. This proprietary AI/ML-driven drug discovery platform can leverage the existing compendium of known compounds to discover novel applications associated with targets and indications previously unknown and non-obvious to the literature. As discussed below, this process identified MDL-001 as the antiviral agent discussed herein.

### MDL-001 In silico Discovery

The CHEMPrint^{™} platform was used to discover novel drug(s) that may be effective for the treatment of ambulatory persons with COVID-19 with mild, moderate or severe symptoms. Specifically, 4 targets (ACE2, M^{pro}, PLpro and RdRp) were selected and developed target specific iterations of the CHEMprint^{™} platform to discover small molecule inhibitors of Sars-CoV-2. The platform virtually screened over 16,000 compounds and discovered a priority ranked list of compounds it predicted would create disease modifying activity. It was predicted that MDL-001 would interact with the SARS-CoV-2 protein RdRp, which is vital to the viral replication process of SARS-CoV-2 and other RNA viruses and generate disease modifying activity.

### MDL-001 Confirmatory In silico Docking Study (Example 9)

In silico docking studies were conducted to confirm CHEMprint^{™}'s findings. Results demonstrate that MDL-001 has a strong affinity towards Sars-CoV2 RdRp, with a comparable docking score to those of remdesivir and other known Sars-CoV-2 active drugs, which mediate their activity via RdRp. These studies support both CHEMprint^{™}'s predicted MOA for MDL-001 and MDL-001's potential as a therapeutic to treat Covid-19. Interestingly, docking studies also indicated that MDL-001 has a similarly strong affinity to a variety of viral RdRps. These results support a potential mechanism of action for MDL-001's broad spectrum antiviral activity.

### MDL-001 In vitro Activity (Examples)

MDL-001 has demonstrated consistent broad in vitro antiviral activity. MDL-001's in silico SARS-CoV-2 antiviral activity predicted by CHEMprint^{™} and supported by docking studies, reported above were confirmed in a series of live virus SARS-CoV-2 assays. In vitro testing has demonstrated MDL-001 has activity across all SARS-CoV-2 variants and H1N1 (influenza). This indicates that the omicron variant has not gained any resistance to MDL-001, and potency should be maintained in the clinic during the ongoing omicron wave of SARS-CoV-2 infection. Furthermore, the antiviral activity of MDL-001 against an influenza A virus indicates that it has potential broad-spectrum antiviral activity across viral families. This is consistent with the in silico supported hypothesis of an RdRp target, which is partially conserved across all RNA viruses.

### SARS-CoV-2 Screening Assay

CHEMprint^{™} predicted in silico that MDL-001 would bind to the viral protein RdRp and elicit disease modifying activity. These results were confirmed via in silico binding studies. In silico findings were then confirmed in an in vitro screening study conducted using a validated live virus inhibition cell assay of disease in HeLa-ACE2/SARS-CoV-2 (Bakowski et al, 2021). In this study, compounds were defined as hits if they were found to be potent and selective (EC₅₀<10 uM, CC₅₀>40 uM and/or SI>10) in the assay. This screening study revealed that of all identified hits, MDL-001 had the strongest antiviral TPP out of the compounds tested.

### SARS-CoV-2 Variant Panel

The in vitro efficacy of MDL-001 against a panel of SARS-CoV-2 variants was assessed. Antiviral activity of compounds against SARS-CoV-2/WA1, (mouse-adapted) MA-SARS-CoV-2/WA1, the Alpha variant (B.1.1.7), the Beta variant (B.1.351), the Delta variant (B.1.617.2), and the Omicron variant (B.1.1.529) was assessed in HeLa-ACE2 cells. Full 6-point SARS-CoV-2 antiviral curves using an immunostaining-based protocol with concurrent cytotoxicity curves (MTT Assay) were generated for all compounds and IC₅₀/IC₉₀/CC₁₀/CC₅₀ were calculated. Experiments were performed twice in triplicate. Nirmatrelvir and DMSO controls were included with all experiments.

MDL-001 was calculated to have an IC₅₀ of 0.72uM against SARS-CoV-2/WA1 (WT) across two replicates performed in biological triplicate. MDL-001 maintained a similar IC₅₀/IC₉₀ against all variants compared to the parental SARS-CoV-2/WA1 in HeLa-ACE2 cells. Full details of this study can be found in Example 11. Note that the study methodology is same as that used by Varona et al, 2022.

### Influenza Panel

The broad-spectrum antiviral activity of MDL-001 was assessed against an influenza virus based on predictions made by the CHEMprint^{™} platform and in silico docking studies of MDL-001 relative to modeling various RdRp's. Full 6-point influenza A/WSN/33 antiviral curves using an immunostaining-based protocol with concurrent cytotoxicity curves (MTT Assay) were generated for all compounds and IC₅₀/IC₉₀/CC₁₀/CC₅₀ were calculated. Experiments were performed in triplicate. Nirmatrelvir and DMSO controls were included with all experiments.

MDL-001 was observed to have an IC₅₀ of 7.49uM against A/WSN/33 indicating it has detectable antiviral activity against an H1N1 influenza A virus. Full details of this study can be found in Example 11.

### In vivo Nonclinical Pharmacology Study Data Supporting COVID-19 Activity (Examples)

In three nonclinical studies conducted MDL-001 progressively demonstrated significant relief of the primary symptomatic endpoint of COVID-19 (weight loss) in a murine model of disease and reduction in the primary biomarker endpoint for disease (SARS-CoV-2 viral load) in the lungs of the same murine model without any demonstration of toxicity. This model has been reported in the literature previously (Rathnasinghe et al, 2021). MDL-001 demonstrated significant potency against COVID-19 and SARS-CoV-2, including reduction of the primary symptomatic endpoint at Day 2 - the earliest time point where statistical significance has been measured for any potential therapeutic in the disease model as confirmed by the researchers - and at least a 2.7 log reduction in viral load at Day 3, as compared to 1.4 and 1.9 log reductions at Day 4 reported for PF-07321332, an oral protease inhibitor from Pfizer, dosed at 300 and 1000 mg/kg, respectively, in a murine model of disease (Owen et al, 2021).

In the first preclinical efficacy Proof-of-concept (POC) study, treatment with MDL-001 protected mice from MA-SARS-CoV-2 associated weight loss in a statistically significant fashion on day 2 and day 3 post infection (p<0.05), similar to the remdesivir group. MDL-001 did not have an impact on viral titers (Example 5).

In the second preclinical study exploring preclinical efficacy with increased doses, treatment with MDL-001 protected mice from MA-SARS-CoV-2 associated weight loss, similar to the remdesivir group. This effect was dose-dependent, with the 250 mg/kg MDL-001 group achieving a statistically significant improvement on day 6 post infection and significantly reducing viral load viral load by approximately 1 Log10 (p<0.01) on day 3 post infection. (Example 6).

In the third study assessing preclinical efficacy with dose optimization, MDL-001 treatment at 250 mg/kg once a day (QD) and 125 mg/kg twice a day (BID) reduced viral lung titers on day 3, in agreement with previous results, but the reductions did not achieve statistical significance. However, the 250 and 375 mg/kg BID MDL-001 groups demonstrated an over two log reduction in viral lung titers on day 3, which was statistically significant (2.39 reduction, p<0.001 and 2.70 reduction, p<0.0001, respectively). Additionally, treatment with 250 and 375 mg/kg BID MDL-001 protected mice from MA-SARS-CoV-2 associated weight loss with statistical significance at day 2 and 3, similar to the remdesivir group (D2, p<0.01 and D3, p<0.0001) (Example 10).

Note, as reported in the pharmacokinetics sections below, t_{1/2} was found to be approximately an order of magnitude less in in the murine model of disease as compared to reported clinical t_{1/2} in healthy volunteers. Thus, BID dosing was evaluated in the murine model, in an effort to more closely model a QD dosing regimen in humans.

In conclusion, nonclinical studies provided herein have demonstrated MDL-001 progressively elicited significant relief of the primary symptomatic endpoint of COVID-19 (weight loss) in a murine model of disease (Rathnasinghe et al, 2021) and reduction in the primary biomarker for disease (SARS-CoV-2 viral load) in the lungs of the same murine model without any demonstration of toxicity. MDL-001 demonstrated significant potency against COVID-19 and SARS-CoV-2, including reduction of the primary symptomatic endpoint at Day 2 - the earliest time point where statistical significance has been measured for any potential therapeutic in the disease model as confirmed by researchers - and at least a 2.7 log reduction in viral load at Day 3, as compared to 1.4 and 1.9 log reductions at Day 4 reported for PF-07321332, an oral protease inhibitor from Pfizer (Owen et al, 2021).

### Plasma Pharmacokinetics and Lung Concentrations (See Examples)

### MDL-001 Mouse and Rat PK Summary

Plasma and lung exposure to MDL-001 was investigated in the female mouse (50 and 250 mg/kg for 5 days) and male rat (250, 500, 750 and 1000 mg/kg, single dose). In both studies, MDL-001 was well tolerated with no adverse clinical signs. Relevant pharmacokinetic and tissue distribution findings are presented below.

### Mouse - Examples 4 and A3

MDL-001 was rapidly absorbed and distributed to the target tissue after oral administration in the mouse, with concentrations in both plasma and lung quantifiable at the first time point (0.5 h). Plasma Cmax increased dose proportionally from 50 to 250 mg/kg. Plasma AUClast increased slightly greater than dose-proportionally. Mean Tmax values ranged from 2.0 - 6.0 h. Following Tmax, plasma concentrations declined, with t1/2 ranging from 2.36 - 3.96 h.

MDL-001 lung exposure was remarkably high, with Day 1 Lung/Plasma AUC ratios of 65 and 36 at 50 and 250 mg/kg, respectively. On Day 5, Lung/Plasma AUC ratios were 56 and 44. Tmax ranged from 4.0 - 8.0 h, slightly later than plasma Tmax. Similar to plasma, no significant accumulation was observed following repeat administration.

MDL-001 Lung Cmax concentrations exceeded its SARS-CoV-2 IC50 value (0.72 uM, 328 ng/mL) by 17- and 121-fold at 50 and 250 mg/kg, respectively. Coverage extended through 24 h at the higher dose, with Lung C24h exceeding the IC50 value by 5.4-fold.

In addition, MDL-001's highly favorable Lung/Plasma distribution should translate to high therapeutic windows in the target tissue relative to any potential systemic toxicity.

### Rat - Example 12

MDL-001 was rapidly absorbed and distributed to the target tissue after oral administration in the rat, with concentrations in both plasma and lung quantifiable at the first time point (0.25 h). Plasma Cmax increased about 50 % from 250 to 1000 mg/kg. Plasma AUClast increased about 2-fold for the 4-fold increase in dose level. Mean Tmax values ranged from 5.3 - 6.0 h. Following Tmax, plasma concentrations declined, with t1/2 ranging from 4.71 - 6.06 h.

MDL-001 lung exposure was remarkably high, with Day 1 Lung/Plasma C24h ratios ranging from 97 - 122 across the dose range.

MDL-001 Lung C24h concentrations exceeded its SARS-CoV-2 IC50 value (0.72 uM, 328 ng/mL) by 9.7- , 13.7-, 13.3-, and 40.9-fold at 250, 500, 6750 and 1000 mg/kg, respectively. Coverage extended through 24 h at the higher dose, with Lung C24h exceeding the IC50 value by 5.4-fold.

As was observed in the mouse, MDL-001's highly favorable Lung/Plasma distribution should translate to high therapeutic windows in the target tissue relative to any potential systemic toxicity.

### Relevance to clinical use

In addition, Pipendoxifene was well tolerated in Phase I SAD (n=46, up to 200 mg) and MAD (n=50, 28 days, up to 200 mg) trials. In addition, the much longer plasma t1/2 in humans (15.8 - 27.3 h) compared to mouse and rat should translate to longer coverage of the IC50 in humans in both plasma and lung, beyond the very positive results outlined above. As noted above, MDL-001 exhibits a remarkably high Lung/Plasma partitioning, which is further expected to provide high separation between target tissue efficacy and any potential systemic toxicity.

### MDL-001 Target Product Profile (TPP)

MDL-001 can have an ideal TPP for outpatient persons with mild to moderate COVID-19. The drug has been demonstrated to significantly reduce both the symptoms of COVID-19 and the viral load of SARS-CoV-2 in preclinical animal models, comparable with highest performing oral medications available to patients. The therapeutic is known to be orally available, has once a day dosing potential via its extended human half-life and is not known to have any significant safety risks (drug-drug interaction, genotoxicity, liver/hepatic toxicity etc.) in human clinical trials.

### Approach to Calculation of Starting Clinical Dose

Available preclinical and clinical data from the literature demonstrate that MDL-001 can be safely tolerated up to at least 20 mg/kg/day in mice and up to 200 mg/daily in humans. (Sorbera et al, 2002). Preclinical data provided herein demonstrate MDL-001 dosing up to 750mg/kg/day without any observation of safety or tolerability issues.

The maximum safe starting dose determination (MSSD) can be calculated based on the FDA Guidance for Industry Estimating the Maximum Safe Starting Dose in Initial Clinical Trials for Therapeutics in Adult Healthy Volunteers (FDA Guidance on MSSD, 2005). It takes into consideration, the GLP toxicity studies' No Observed Adverse Effect Dose Level (NOAEL), a Human Equivalent Dose (HED) calculation, and then a 1/10th adjustment on that dose, as well as other dosing and safety data available on pipendoxifene.

In conclusion, as noted previously, the clinical dose can be determined taking into consideration nonclinical and clinical dosing information known about MDL-001, the results from the proposed PK/ADME studies in Example 13, and NOAEL determination from the GLP toxicology study outlined in Example 13 to propose a safe starting dose for the clinical studies of MDL-001.

### EXAMPLES

Some aspects of the embodiments discussed above are disclosed in further detail in the following examples, which are not in any way intended to limit the scope of the present disclosure.

### Example 1

### RNA-dependent RNA polymerase (RdRp)

Identification: UniProtKB: A0A5H2X758, A0A5H2WYC2, A0A5H2WTX4, A0A5H2WUC6, A0A5H2WYC7

### Alternative names/synonyms

RdRp NSP12, Coronaviral RdRp NSP12, SARS NSP12, SARS-CoV NSP12, SARS coronavirus nonstructural protein NSP12, Coronavirus nonstructural protein NSP12, SARS nonstructural protein NSP12, SARS coronavirus nonstructural protein 12, Coronavirus nonstructural protein 12, Nonstructural protein 12, RNA polymerase NSP12, NSP12, Proteins, NSP12, SARS-CoV-2 NSP12, Severe acute respiratory syndrome coronavirus NSP12, Coronavirus RNA-dependent RNA polymerase NSP12, Viral nonstructural protein NS12 (coronavirus), RNA-dependent RNA polymerase NSP12, Coronavirus NSP12 Coronaviral RdRp, SARS RdRp, CoV RdRp, SARS-CoV-2 RdRp, SARS-CoV RdRp, Coronavirus RNA-dependent RNA polymerase, SARS RNA-dependent RNA polymerase, SARS RNA-directed RNA polymerase, Coronavirus RNA-directed RNA polymerase, SARS-CoV RNA-directed RNA polymerase, SARS-CoV-2 RNA-directed RNA polymerase, SARS-CoV-2 RNA replicase, SARS-CoV RNA replicase, SARS RNA replicase, CoV RNA replicase, Coronavirus RNA replicase, Severe acute respiratory syndrome coronavirus RNA replicase, CoV replicase, SARS-CoV replicase, SARS-CoV-2 replicase, RNA replicase NSP12

### Structure and sequence

The RNA-dependent RNA polymerase (RdRp), also known as Nsp12, of SARS-CoV-2 is composed of 6 sections: nsp7, nsp8-1, nsp-8-2, "thumb", "palm" and "fingers". The replication/transcription complex includes small proteins nsp7 and nsp8 as accessory factors that increase the template binding and enzymatic activity of Nsp12. The sequence of Nsp12 is highly conserved across coronaviruses such as SARS-CoV, MERS-CoV and SARS-CoV-2. The SARS-CoV-2 RdRp sequence shares 97% similarity to SARS-CoV. The highly conserved active site has two successive and surface accessible aspartates in a beta-turn structure. The detailed structure of the novel coronavirus' RdRp complex and role of each domain is described in Gao et al, 2020, Yin et al 2020, and Venkataraman et al, 2018.

### Physiology and Disease

RdRP, also known as Nsp12, is a polymerase that catalyzes the replication of RNA from an RNA template and is a vital enzyme for RNA viruses' replication/transcription complex. This enzyme synthesizes a full-length negative-strand RNA template that can subsequently be used to replicate and transcribe the viral genome. RdRp is a core replication/transcriptional element of all RNA viruses.

### Known Inhibitors

RdRp's crucial role in the life cycle of RNA viruses has led to its targeted inhibition for a number of viral infections such as hepatitis C virus, Zika virus, and coronaviruses. Current experimental drugs for this target include remdesivir, galidesivir, sofosbuvir, ribavirin, and favipiravir. Remdesivir is an antiviral inhibitor of RdRp that has shown to be effective against RNA viruses such as SARS-CoV, MERS-CoV and Ebola virus. The antiviral activity of Remdesivir is proposed due to its resemblance to an ATP used by RdRp. Remdesivir may be adequate to bind to the polymerase and hinder the enzyme's ability to incorporate additional RNA subunits, resulting in a failed genome replication. A recently discovered potent inhibitor known as ID-184 has been shown to bind RdRp more tightly than other experimental inhibitors.

Various RdRP inhibitors and information thereof are provided herein and include Pipendoxifene (or a pharmaceutically acceptable salt, ester, solvate, stereoisomer, tautomer, or prodrug thereof). As disclosed herein, these compounds can be used alone, or in combination with other therapeutic agent(s) for preventing, delaying the onset of, or treating an infection or a disease caused by a RNA virus (e.g., SARS-CoV-2), and/or for preventing, delaying the onset of, or treating an inflammatory effect of an infection or a disease caused by a RNA virus (e.g., SARS-CoV-2).

### Target Relevance to RNA Viral Infection

The RNA-dependent RNA polymerase of SARS-CoV-2 is essential for replication and transcription of the virus. This polymerase is identified as a main viral target for COVID-19 drug therapies. Given the highly conserved sequence of RdRp across all RNA viruses, there is a number of RdRp inhibitors that have been tested against SARS CoV-2 (Howes 2020). Without being bound by any particular theory, targeted inhibition of Nsp12-RdRp would presumably not cause toxicity and side-effects on host cells. A drug therapy that could act to stop or disrupt viral replication could attenuate viral propagation.

### Example 2

### Pipendoxifene, Berzosertib, AZD-5991, PRX-07034 and Tocladesine: Discovery and Validation of Antivirals with Activity Against SARS-CoV2 Using the ActivPred Digital Chemistry Platform and a HeLa-ACE2 Cell-based High-content Imaging Assay

### Summary

In December 2019, a novel coronavirus (known as COVID-19 or SARS-CoV-2) broke out in Wuhan, China. Since the outbreak, this global pandemic has infected more than 100M people and killed more than 2.4M as of February 1st, 2021. While there has been a rush towards vaccine development, a vast amount of research efforts have also focused their efforts towards discovering SARS-CoV-2 therapeutics. Traditional drug development methods began this process from scratch, but as of recently, researchers have grown accustomed to a faster and more affordable drug discovery approach known as drug repurposing. The ActivPred Digital Chemistry AI Drug Discovery Platform discovered hundreds of drugs that were identified to bind four targets including one host and three viral targets (ACE2, 3CLpro, PLpro and RdRp) necessary for infection and infection propagation of COVID-19. Those drugs were priority ranked using several factors and 35 small molecule drugs that ActivPred predicted would have activity against COVID-19 were selected for experimental testing. These 35 compounds were tested in a CoV-2/HeLa-ACE2 cell-based phenotypic assay. A total of 5 small molecule drugs demonstrated measurable antiviral activity in the CoV-2/HeLa-ACE2 cellular assay; yielding a 14.3% hit rate for ActivPred's discoveries. A total of 2 drugs (pipendoxifene and berzosertib) were found to be potent (EC₅₀ <9.6uM), nontoxic and selective (CC₅₀ >39.8uM and/or SI >10); yielding a hit rate of 5.7% for this higher threshold. These hit rates are encouraging as compared to the hit rates obtained by a best in class COVID-19 High Throughput Screen (HTS) of the ReFrame library, which yielded hit rates of 2.75% and 0.56%, respectively, and were significantly higher than typical hit rates reported elsewhere in the literature for HTS. These results demonstrate that ActivPred was 5x and 10x more efficient in identifying potent, non-toxic and selective drugs active against COVID-19 as compared to the best in class COVID-19 HTS approach, which was one to two orders of magnitude more efficient than typical HTS studies. The results and subsequent analysis demonstrated that pipendoxifene was potent (EC₅₀ =8.24uM), nontoxic (CC₅₀ >39.8uM) and selective (SI = 17-35+) and that berzosertib was potent (EC₅₀=0.114uM), non-toxic (CC₅₀=3.14uM) and selective (SI=27.56). Furthermore, as compared to the remdesivir control, the only current FDA approved COVID-19 therapeutic, pipendoxifene has a larger Hill coefficient, a preferred clinical cytotoxicity profile, a significantly longer half-life and a preferred oral administration route. Berzosertib is more potent than remdesivir while exhibiting an acceptable safety profile, a significantly longer half-life and a preferred oral administration potential. According to the experimental results and analysis, pipendoxifene and berzosertib are the most suitable compounds to be considered for further clinical development out of those discovered by the ActivPred platform and experimentally validated. With additional pre-clinical and clinical validation, pipendoxifene and berzosertib may represent an alternative to remdesivir in the treatment of COVID-19 and other RNA virus infections.

### ActivPred Discovery and Selection Protocol:

The ActivPred platform was trained using relevant small molecules from the public database PubChem. ActivPred then discovered compounds, using the compound libraries PubChem and DrugBank, that bind targets relevant to SARS-CoV-2 infection, replication and propagation. Targets of interest for this study included the host target ACE2 (UniProtKB - Q9BYF1 (ACE2_HUMAN)) and the viral targets 3CLpro (UniProtKB - P0DTD1 (R1AB_SARS2), P0C6X7, P0C6U8), PLpro (UniProtKB - P0DTC1 (R1A_SARS2), K4LC41) and RdRp (UniProtKB: A0A5H2X758, A0A5H2WYC2, A0A5H2WTX4, A0A5H2WUC6, A0A5H2WYC7). ActivPred discovered several hundred compounds that could exhibit antiviral activity against SARS-CoV-2 and potentially other viruses and indications via the targets indicated above. The inventions were then downselected to a list of 35 compounds based upon a selection criteria. Specifically, the highest priority ranked in silico discoveries that had never previously been found to exhibit any antiviral activity and had not yet been mentioned with the target of interest were selected to move forward into in vitro testing.

### CoV-2/HeLa-ACE2 Protocol

A total of 35 small molecule discoveries made by the ActivPred platform were subjected to a cell-based phenotypic assay involving a HeLa host cell line that was engineered to constitutively express human ACE2. ACE2 is a host cell receptor that is utilized by SARS-CoV-2 for cellular entry. This assay was used to identify compounds that could inhibit SARS-CoV-2 viral entry or replication. The 38 small molecules that were tested in this assay included 3 controls (remdesivir, apilimod, and puromycin) and the 35 molecules discovered by the ActivPred Digital Discovery Platform and predicted to have antiviral activity. 25nL of the dry compounds and controls (remdesivir, apilimod, and puromycin) were spotted in 384-well plates at final concentrations of 1.9 µM or 9.6 µM dependent on the library stock. Remdesivir was used as the positive control whereas apilimod and puromycin were for toxicity control. The assay plates were then seeded with HeLa-ACE2 cells and infected with SARS-CoV-2 an hour later. After a 24-hour incubation period, the plates were fixed and stained with detection reagents such as anti-SARS-CoV-2 antibodies paired with an Alexa488 to visualize the virus and DAPI to visualize host cell nuclei. The stained plates were then observed using ImageXpress Micro Confocal (IXMC) imaging with immunofluorescence. Lastly, an algorithm quantified the number of infected cells present after the incubation period. The details of this procedure and findings are listed below. The protocol used was published previously in Bakowski et al (2020).

### Overview

To identify compounds that could inhibit entry or replication of SARS-CoV-2 in human cells, a high-content imaging (HCI) 384-well format assay was used, involving HeLa cells expressing the human SARS-CoV-2 receptor, the angiotensin-converting enzyme 2, or ACE2 (HeLa-ACE2). In this assay HeLa-ACE2 cells are infected with SARS-CoV-2 virus in the presence of compounds of interest, and viral infection is quantified 24 hours later. The assay relies on immunofluorescent detection of SARS-CoV-2 proteins with sera purified from patients exposed to the virus, which together with host cell nuclear staining allows for quantification of the percent infected cells in each well.

### Virus generation

Vero E6 cells (ATCC CRL-1586) were plated in a T225 flask with complete DMEM (Corning 15-013- CV) containing 10% FBS, 1×PenStrep (Corning 20-002-CL), 2 mM L-Glutamine (Corning 25-005-CL) overnight at 37 5% CO2. The media in the flask was removed and 2 mL of SARS-CoV-2 strain USAWA1/2020 (BEI Resources NR-52281) in complete DMEM was added to the flask at a multiplicity of infection (MOI) of 0.5 and was allowed to incubate for 30 minutes at 34°C 5% CO2. After incubation, 30 mL of complete DMEM was added to the flask. The flask was then placed in a 34°C incubator at 5% CO2 for 5 days. On day 5 post infection the supernatant was harvested and centrifuged at 1,000×g for 5 minutes. The supernatant was filtered through a 0.22 µM filter and stored at -80°C.

### Compound Management

High-purity compounds (>95%) dissolved in high-quality dimethyl sulfoxide (DMSO) were used in the study. Compound quality control was performed by liquid chromatography-mass spectrometry and/or 1 H-NMR when required. The library was prepared at two concentrations, 2 and 10 mM, to support low-concentration (2-10 µM) and high-concentration (10-50 µM) screening formats. Echo-qualified 384-well low dead volume plus microplates (LP-0200-BC; Labcyte Inc.) were used as the library source plates to support acoustic transfer with an Echo 555 Liquid Handler (Labcyte Inc.). Compounds not soluble in DMSO were plated in water (129 compounds); compounds lacking long-term solubility in DMSO were suspended just before dispensing to avoid precipitation (71 compounds). Additional details available at https://reframedb.org/about.

### Controls

Remdesivir at elevated concentrations was able to eliminate infected cells almost completely and it was used at a concentration of 2.5 µM as a positive control, with data normalized to it and neutral DMSO control wells. While apilimod was more potent than remdesivir, it had a fractionally lower maximal efficacy (85-90% of uninfected cells at the highest effective concentrations) compared to remdesivir. Additionally, compound toxicity in the context of infection was assessed by quantifying the total cell numbers per well, with cytotoxic protein synthesis inhibitor puromycin as a positive control (average EC50 = 547 ± 27 nM, average ± sem of 5 independent experiments; HeLa-ACE2 CC50 = 2.45 ± 0.23 µM, average ± sem of 5 independent experiments). Notably, a concomitant increase in cell numbers coincided with the antiviral activity of remdesivir and apilimod, likely due to reduction in proliferation of infected cells. Altering the multiplicity of infection had modest effects on the potency of control compounds in the same experiment, with a 2.7-fold increase in remdesivir's EC50 from MOI=1 to MOI=26, and a 3.7-fold increase in apilimod's EC50, but not that of puromycin.

### HeLa-ACE2 stable cell line

HeLa-ACE2 cells were generated through transduction of human ACE2 lentivirus. The lentivirus was created by co-transfection of HEK293T cells with pBOB-hACE2 construct and lentiviral packaging plasmids pMDL, pREV, and pVSV-G (Addgene) using Lipofectamine 2000 (Thermo Fisher Scientific, 11668019). Supernatant was collected 48 h after transfection then used to transduce pre-seeded HeLa cells. 12 h after transduction stable cell lines were collected, scaled up and stored. Cells were maintained in DMEM (Gibco, 11965-092) with 10% FBS (Gibco, 10438026) and 1× sodium pyruvate (Gibco, 11360070) at 37 5% CO2.

### SARS-CoV-2/HeLa-ACE2 high-content screening assay

Compounds were acoustically transferred into 384-well µclear-bottom plates (Greiner, Part. No. 781090-2B). HeLa-ACE2 cells were seeded in 13 µL DMEM with 2% FBS at a density of 1.0×103 cells per well. Plated cells were transported to the BSL3 facility where 13 µL of SARS-CoV-2 diluted in assay media was added per well at a concentration of 2.0×106 PFU/mL (assay multiplicity of infection (MOI) = 2.2). Plates were incubated for 24 h at 34 5% CO2, and then fixed with 25 µL of 8% formaldehyde for 1 h at 34 5% CO2. Plates were washed with 1xPBS 0.05% Tween 20 in between fixation and subsequent primary and secondary antibody staining. Human polyclonal sera diluted 1:500 in Perm/Wash buffer (BD Biosciences 554723) was added to the plate and incubated at RT for 2 h. Six µg/mL of goat anti-human H+L conjugated Alexa 488 (Thermo Fisher Scientific A11013) together with 8 µM of antifade-46-diamidino-2-phenylindole (DAPI; Thermo Fisher Scientific D1306) in SuperBlock T20 (PBS) buffer (Thermo Fisher Scientific 37515) was added to the plate and incubated at RT for 1 h in the dark. Plates were imaged using the ImageXpress Micro Confocal High-Content Imaging System (Molecular Devices) with a 10× objective, with 4 fields imaged per well. Images were analyzed using the Multi-Wavelength Cell Scoring Application Module (MetaXpress), with DAPI staining identifying the host-cell nuclei (the total number of cells in the images) and the SARS-CoV-2 immunofluorescence signal leading to identification of infected cells.

### Uninfected host cell cytotoxicity counter screen

Compounds were acoustically transferred into 1,536-well µclear plates (Greiner Part. No. 789091). HeLa-ACE2 cells were maintained as described for the infection assay and seeded in the assay-ready plates at 400 cells/well in DMEM with 2% FBS and plates were incubated for 24 hours at 37 5% CO2. To assess cell viability, the Image-iT DEAD green reagent (Thermo Fisher) was used according to manufacturer instructions. Cells were fixed with 4% paraformaldehyde, and counterstained with DAPI. Fixed cells were imaged using the ImageXpress Micro Confocal High-Content Imaging System (Molecular Devices) with a 10× objective, and total live cells per well quantified in the acquired images using the Live Dead Application Module (MetaXpress).

### Data analysis

Image analysis was carried out with MetaXpress (version 6.5.4.532). Primary in vitro screen and the host cell cytotoxicity counter screen data were uploaded to Genedata Screener, Version 16.0.3-Standard. Data were normalized to neutral (DMSO) minus inhibitor controls (2.5 µM remdesivir for antiviral effect and 10 µM puromycin for infected host cell toxicity). For the uninfected host cell cytotoxicity counter screen 40 µM puromycin (Sigma) was used as the positive control. For dose response experiments compounds were tested in technical triplicates on different assay plates and dose curves were fitted with the four parameter Hill Equation. Technical replicate data were analyzed using median condensing.

### Results/Discussion

### ActivPred Platform Predictive Metrics versus Traditional Drug Discovery Methods

Using the ActivPred Digital Chemistry AI Drug Discovery Platform, 35 small molecule drugs were discovered that ActivPred predicted would have activity against COVID-19 via 4 targets that are relevant to SARS-CoV-2 infection, replication and propagation. This included the host target ACE2 (UniProtKB - Q9BYF1 (ACE2_HUMAN)), as well as the viral targets 3CLpro (UniProtKB - P0DTD1 (R1AB_SARS2), P0C6X7, P0C6U8), PLpro (UniProtKB - P0DTC1 (R1A_SARS2), K4LC41) and RdRp(UniProtKB: A0A5H2X758, A0A5H2WYC2, A0A5H2WTX4, A0A5H2WUC6, A0A5H2WYC7). Five compounds from that group of 35 demonstrated activity in the HeLa-ACE2 cell-based SARS-CoV-2 assay: PRX-07034, AZD-5991, VE-822/berzosertib, tocladesine and pipendoxifene (ERA-923) - yielding a 14.3% hit rate for these discoveries. For comparison, a best-in-class high-throughput screen (HTS) of the nearly 12,000 compound ReFrame library for activity against COVID-19 demonstrated a hit rate of 2.75%. Thus, the ActivPred platform was 5x more efficient at predicting COVID-19 active drug compounds than the gold-standard COVID-19 HTS method from similar drug libraries. It has been reported elsewhere that HTS methods typically yield hit rates between 0.01% and 0.14%. Thus, the "gold-standard" HTS method was 10-100x (one to two orders of magnitude) better than traditional HTS approaches and the ActivPred platform was 100-1000x (two to three orders of magnitude) more efficient at predicting active COVID-19 compounds than typical HTS methods.

Compounds identified by a "gold-standard" HTS as active against COVID-19 were considered to be potent, safe and selective (i.e. candidates for further development) if they were found to have EC50's <9.6 µM, CC50's >39.8 µM and/or SI's >10 in a 10 point dose response assay. A hit rate of 0.56% (66/11,861) was reported for this more selective screen. In the present disclosure, 2 of 35 drugs discovered by the ActivPred platform (pipendoxifene and berzosertib) met this threshold, revealing a 5.7% hit rate for this more selective threshold and demonstrating that the ActivPred platform was 10.2x (an order of magnitude) more efficient in identifying potent, safe and selective drugs against COVID-19 than high performing HTS discovery methods.

The efficiency of ActivPred as compared to traditional HTS drug development can also be measured in terms of capital required to obtain a "hit". The cost to use ActivPred and validate 35 drug compounds is a fraction of the cost required to screen nearly 12,000 compounds in a primary screen and over 300 drugs in a secondary screen.

### Drugs Discovered by ActivPred and Experimentally Validated

### Pipendoxifene results

Pipendoxifene reported a EC₅₀ higher than the remdesivir control (8.24 µM vs 0.13 µM), indicating that remdisivir is more potent. However, pipendoxifene reported a CC₅₀ of >39.80 µM because no cell death was observed within the concentrations tested in this study, as compared to a CC₅₀ of 8.87 µM for remdesivir. Without being bound by any particular theory, this can means that pipendoxifene is disrupting viral replication at a concentration far lower than the concentration that leads to any cell death. The selectivity index of pipendoxifene to remdesivir was calculated to be >4.83 vs 68, respectively, based on experimental data. However, this calculation is a misnomer, as it is impossible to calculate a true selectivity index of pipendoxifene based on this assay, as pipendoxifene's CC₅₀ is greater than 39.80 µM, but otherwise unknown, making the SI infinite without additional information. Additional cytotoxicity testing could be performed to assess the selectivity index of the compound at some point in the future. However, mathematical approximations of selectivity index based upon using the concentration of pipendoxifene safely administered in FDA approved clinical trials as an underapproximation of CC₅₀, suggest that pipendoxifene has a SI of at least 10-20+, but more likely at least 35+ (see Table 2 below). Pipendoxifene has a larger Nₕ (Hill) coefficient (4), than remdesivir (3.3) and berzosertib (1.485). Without being bound by any particular theory, this suggests that once an initial pipendoxifene molecule binds its target, the drug exerts its full effect on viral replication within a smaller concentration range than remdesivir, which is a significant finding given pipendoxifene's CC₅₀. (See FIGS. 1A-1B).

### Conclusions

Taken together, pipendoxifene and berzosertib demonstrate the strongest profile out of the 5 compounds that showed measurable antiviral activity when taking EC₅₀, CC₅₀, Hill Coefficient and SI into account relative to the remdesivir control in this study. A separate published study using the same experimental protocol and analysis classified potent drugs as those having an EC₅₀ < 9.6uM and safe and selective drugs as those having a CC₅₀ >39.8 uM and/or CC50/EC50 >10. Based on the above-described analysis paradigm, pipendoxifene and Benzosertib are both potent, non-toxic and selective.

### Pipendoxifene analysis

Another important factor to consider besides the EC₅₀, CC₅₀, Hill Coefficient and SI of a compound when priority ranking drugs to advance from in vitro to in vivo studies is their half-life (t ½). The t ½ of a species is the time it takes for the concentration of that substance to fall to half of its initial value. Pipendoxifene's t ½ is 15.8-27.3 hours and berzosertib's is about 17 hours, whereas remdesivir's t ½ is 1 hour. Half-lives are vital for dosing regimens and peak-to-trough ratios at the steady state. Without being bound by any particular theory, it is possible that because pipendoxifene's and berzosertib's t ½ is significantly longer than remdesivir's t ½, that both compounds could serve as longer acting antivirals that inhibit more viral replication cycles per dose. Based on the consideration of half-life, remdesivir may be expected to effectively inhibit viral replication for only a few hours each day (i.e. per dose), while pipendoxifene and berzosertib may be expected to effectively inhibit viral replication for essentially the entirety of each day (i.e. per dose). The long half-life and strong potency of these drugs makes them potentially ideal once a day antiviral therapeutics. In contrast, based on its half life, remdesivir would have to be taken several times a day in order to exert its maximal effect (note - it is approved for once daily use only). Thus, in some embodiments, pipendoxifene and berzosertib could potentially be more effective than remdesivir in a clinical setting.

The current standard of care for SARS-CoV-2 infection, remdesivir, can only be administered intravenously. This route of administration (r.o.a.) is a limitation for many patients that are either not able to obtain a bed in a hospital to receive remdesivir due to ICUs being at capacity and/or for those that cannot bear the financial burdens of occupying hospital beds in order to receive medication. If SARS-CoV-2 infected individuals had the option to be prescribed an oral medication that can easily be self-administered at home, then patients/payers would be saving substantial amounts of money as well as putting less people at risk by further exposing health-care professionals and other non-covid related patients in hospitals.

Pipendoxifene was predicted to bind to the viral protein RdRp. RdRp is vital to the viral replication process of SARS-CoV-2. Importantly, it is vital to the replication process of all RNA viruses. Remdesivir binds the same target. Without being bound by any particular theory, pipendoxifene offers an expansive clinical and market opportunity and a direct alternative to remdesivir. There is a possibility that the EC₅₀ (8.42 uM) of pipendoxifene reported in this study may be artificially high due to the assay's design. COVID-19 targets such as ACE2 are "early acting targets'' because they are involved in initial infection of the cell by the virus. Therefore, drugs that bind targets such as ACE2, are enriched in 24 hour screens of activity. RdRp is a "late acting target" of COVID-19. Drugs that bind RdRp are exhibit maximal effectiveness after initial infection and multiple viral replication cycles, i.e. at later time points. Consequently, in some embodiments, a 72 hour study of pipendoxifene against COVID-19 would likely enhance its effectiveness, relative to other potential drugs.

Based on the analysis above and below, pipendoxifene is recommended for in vivo validation based on the results of this study.

### Pipendoxifene - Background, Novelty and Clinical/Preclinical Dosing Justification

Pipendoxifene was originally developed as a selective estrogen receptor modulator by Ligand and Wyeth (now Pfizer). Two other drugs, bazedoxifene and raloxifene, were previously identified as active against COVID-19 that were also originally developed as estrogen receptor modulators in a ReFRAME screen for SARS-CoV-2 activity of 11,861 compounds using the same methods used in this study (Bakowski et al, 2020) published on June 16th, 2020. These two selective estrogen receptor modulators (bazedoxifene, EC₅₀ = 3.47 µM and raloxifene EC₅₀ = 4.13 µM) have been previously found to inhibit Ebola virus infection. There has been speculation, without a definitive conclusion, on the exact mechanism of action for Ebola virus inhibition by raloxifene and bazedoxifene. One proposed mechanism of action for inhibition of Ebola virus by raloxifene is by downregulating a viral protein necessary for progeny assembly (VP40) in a dose-dependent manner. Bazedoxifene and raloxifene are speculated to be active compounds that block Ebola VLP entry in vitro at an IC50<10 µM and SI >10 and it is hypothesized that viral entry into host cells is blocked by interfering with Ebola virus surface glycoprotein. Finally, Raloxifene and bazedoxifene have been hypothesized to inhibit Ebola virus-like particle entry into cells by blocking TPC ion channels that are utilized as a host factor for cellular entry by the Ebola virus. Notably, the ActivPred Platform discovered that pipendoxifene inhibited COVID-19 via RdRp and RdRp is absent from these hypothesized MOAs for bazedoxifene and raloxifene Ebola inhibition demonstrating the surprising nature of the discoveries provided herein and the strength of the ActivPred platform. Interestingly, pipendoxifene was not identified in the High Throughput Screen (HTS) of the ReFrame library that identified bazedoxifene and raloxifene, even though pipendoxifene is part of the ReFrame library, nor was it identified previously to inhibit Ebola virus, further indicating the surprising nature of the discoveries provided herein and the strength of the ActivPred platform.

Pipendoxifene has reached phase 2 in terms of clinical trial development and was developed as a back-up drug to bazedoxifene. Pipendoxifene was discontinued when bazedoxifene demonstrated success in clinical trials. Available preclinical and clinical data show that pipendoxifene can be safely tolerated up to at least 20mg/kg/day in mice and up to 200mg/daily in humans. However, based on FDA accepted mathematics, which allow clinical researchers to convert between human and various animal dosing using various Km values (for instance 3 for mice and 5 for hamsters), a study was conducted in mice and submitted to the FDA with an apparent dosing of at least 66.67mg/kg/day to justify the 200mg/day dosing in human clinical trials. Furthermore, the mouse dosing was likely significantly higher than 66.67mg/kg/day because a significant safety factor is typically applied when moving from mouse to human studies and applying the FDA Km factor of 3. Thus, mice may have been dosed at 133.33 mg/kg/day or greater without AE's (adverse events) to arrive at a FDA accepted clinical dose of 200 mg/day.

Since bazedoxifene was successful (FDA approved late 2013), it has more publicly available preclinical and clinical data than its backup drug, pipendoxifene. Therefore, it may be reasonable to use publicly available data for bazedoxifene to estimate or model pipendoxifene dosing that could be tolerated in a mouse in *vivo* model. Bazedoxifene is known to be safely tolerated at 100mg/kg/day in rats and 20mg/day in humans (FDA approved dosing). As can be seen, the highest concentration tested in rats for Bazedoxifene (100mg/kg/day) is 14.9x greater than the highest concentration justified by using the clinical dose and the FDA Km value (20/3=6.7mg/kg/day), indicating that the 2x rat dose concentration number assumed in the previous paragraph for pipendoxifene (133.33mg/kg/day) clinical to non-clinical dosing is reasonable.

Thus, pipendoxifene mouse dosing of 66.67mg/kg/day is justified by the clinically tolerated dose of pipendoxifene (200mg/day) and dosing of up to at least 133.33mg/kg/day is justified in mouse testing after applying the dosing logic above.

### Comparison of EC₅₀ and EC₉₀ in vitro concentrations to clinical concentrations of Pipendoxifene

Pipendoxifene's known clinical doses and derived preclinical doses that do not elicit Grade 2 or higher adverse events (AEs) were used to calculate approximate equivalent in vitro concentrations. These values were then compared to the EC₅₀ (given) and EC₉₀ (calculated) values from the in vitro HeLa-ACE2 cell-based SARS-CoV-2 assay.

The EC₉₀ of pipendoxifene was calculated using the Hill equation (Equation I below), given the nₕ coefficient of 4 and EC₁₀ (5.01187 µM) derived from the graph in the experimental data described herein. ${n}_{H = \frac{{log}_{10} 81}{{log}_{10} \left({EC}_{90}/{EC}_{10}\right)}}$

The Hill equation calculated the EC₉₀ to be 15.03 µM, meaning that there is 90% response from the drug at this concentration. $4 = \frac{{log}_{10} 81}{{log}_{10} \left(\frac{{EC}_{90}}{5.01187}\right)}$ $log {EC}_{90} - log \left(5.01184\right) = \frac{log 81}{4}$ $log {EC}_{90} - 0.6999 = 0.47712$ $log {EC}_{90} = 1.1771$ ${EC}_{90} = {10}^{1.1771}$ ${EC}_{90} = 15.03 μ M$

The EC₉₀ was also calculated using Equation II where the variables F equals the percent response, H equals the Hill slope coefficient and EC₅₀ equals 8.24 µM from the in vitro data of pipendoxifene. This equation calculated the EC₉₀ to be 14.27 µM, meaning that there is 90% response from the drug at this concentration. ${EC}_{F} = {\left(\frac{F}{100 - F}\right)}^{\frac{1}{H}} ∗ {EC}_{50}$ ${EC}_{F} = {\left(\frac{F}{100 - F}\right)}^{\frac{1}{H}} ∗ {EC}_{50}$ ${EC}_{90} = {\left(\frac{90}{100 - 90}\right)}^{\frac{1}{4}} ∗ 8.24$ ${EC}_{90} = {9}^{\left(0.25\right)} ∗ 8.24$ ${EC}_{90} = 14.27 μ M$

These equations result in an EC₉₀ range of 14.27-15.02 µM, and are used for comparison to the approximated equivalent in vitro concentrations of pipendoxifene calculated below.

Two methods were used to calculate pipendoxifene equivalent in vitro concentrations beginning with either 1) the known, tolerated Phase 1 clinical dosage or 2) a mouse and hamster dosage derived from the known tolerated Phase 1 clinical dosage (see above). Both methods can be found here: https://www.researchgate.net/post/How-to-extrapolate-result-from-in-vitro-ug-mL-to-in-vivo and http://microbiology.ucdavis.edu/privalsky/tc-growth-area.

### Method 1.a

The first method was used to calculate an approximate equivalent in vitro concentration beginning with the highest known safely tolerated clinical dose of pipendoxifene (200 mg/day, oral administration). When calculating mouse dosages, 2x the derived pipendoxifene animal dosage (133.33 mg/kg, justified above), and the mid-point of those two dosages (100mg/kg/day) were used as well. The clinical dosage is then converted to a mouse dosage using the Km value of 3 (FDA). Next, the average mass of a mouse is multiplied by the mouse dosage in equal metrics to find the amount administered to each mouse in grams. The molar mass is then divided from the mouse dosage and this value is then multiplied by 1 over the volume of mouse equivalent; this method uses the molarity formula (M=(m/MW)*(1/V)). The same method of calculation was also used for a hamster example by converting the clinical dose to an equivalent hamster dose using the FDA Km value of 5. The result is a pipendoxifene equivalent in vitro concentration derived from the highest known safely tolerated clinical dose of the drug.
*mice dosage* → *amount adminstered to each mouse* → *concentration of drug in vitro* (*uM*) $\frac{C}{Km} = A \to \frac{A}{1000} ∗ \frac{MM \left(g\right)}{1} ∗ \frac{1}{1000} = B \to \frac{B}{MW} ∗ \frac{1}{V} ∗ \frac{1000000}{1} = D$

Method 1.a Formula (Equation III). C=clinical dosage (mg/m²), Km=FDA conversion factor, A=Animal dosage (mg/kg), MW= molecular weight (g/mol), MM= average animal mass (g), B= animal dosage per subject (g), V= volume of animal equivalent (L), D= concentration of drug in vitro (uM).

**TABLE 1A: CONVERSION OF A CLINICAL DOSAGE OF PIPENDOXIFENE TO AN EQUIVALENT IN VITRO CONCENTRATION**

| clinical dosage of pipendoxifene (mg/m2) | molar mass (g/mol) | mice dosage (mg/kg) | average mouse mass (g) | amount administered to each mouse (mg) | amount administered to each mouse (g) |
|---|---|---|---|---|---|
| 200 | 456.6 | 66.6666667 | 25 | 1.66666667 | 0.00166667 |
| 200 * 1.5 | 456.6 | 100 | 25 | 2.5 | 0.0025 |
| 200 * 2 | 456.6 | 133.3 | 25 | 3.325 | 0.003325 |
| clinical dosage of pipendoxifene (mg/m2) | molar mass (g/mol) | hamster dosage (mg/kg) | average hamster mass (g) | amount administered to each hamster (mg) | amount administered to each hamster (g) |
| 200 | 456.6 | 40 | 125 | 5 | 0.005 |
| *The selectivity index here was calculated using the EC₅₀ (8.23 µM) from the in vitro experiment and the approximate equivalent in vitro concentration as the CC₅₀. By definition, this calculation underestimates the CC₅₀ and the SI because no toxicity was observed at the human dose of 200 mg/day that these numbers were derived from. Compounds with an SI >10 are considered to be "selective". | | | | | |

**TABLE 1B: CONVERSION OF A CLINICAL DOSAGE OF PIPENDOXIFENE TO AN EQUIVALENT IN VITRO CONCENTRATION**

| | | | | |
|---|---|---|---|---|
| clinical dosage of pipendoxifene (mg/m2) | volume of mouse equivalent (L) | concentration of drug in vitro (M) | concentration of drug in vitro (µM) | Selectivity Index (CC50/EC50) |
| 200 | 0.025 | 0.000146007 | 146.006716 | 17.719* |
| 200 * 1.5 | 0.025 | 0.00021901 | 219.010074 | 26.58 |
| 200 * 2 | 0.025 | 0.00029128 | 291.94043 | 35.43 |
| clinical dosage of pipendoxifene (mg/m2) | volume of hamster equivalent (L) | concentration of drug in vitro (M) | concentration of drug in vitro (µM) | Selectivity Index (CC50/EC50) |
| 200 | 0.125 | 8.7604E-05 | 87.6040298 | 10.631557* |
| *The selectivity index here was calculated using the EC₅₀ (8.23 µM) from the in vitro experiment and the approximate equivalent in vitro concentration as the CC₅₀. By definition, this calculation underestimates the CC₅₀ and the SI because no toxicity was observed at the human dose of 200 mg/day that these numbers were derived from. Compounds with an SI >10 are considered to be "selective". | | | | |

As seen in Table 1, the equivalent in vitro drug concentrations (146-291 µM, via mouse, and 87.6 µM, via hamster, calculations) of pipendoxifene derived from the known safe and well tolerated human clinical dosing are well above the EC₅₀ (8.24 µM) and EC₉₀ of pipendoxifene (15.03 µM) observed in the HeLa-ACE2 SARS-CoV-2 assay. This difference between the observed in vitro EC₅₀ and EC₉₀ of pipendoxifene and the calculated in vitro concentrations derived from the clinically justified safe and tolerated dosing allows for a significant buffer that might be expected to account for any error in the calculation due to factors such as absorption and metabolism, which the calculation is unable to take into account. Since the 200 mg/m² clinical dosage of pipendoxifene was well tolerated in a Phase 1 clinical trial, it is also logical to use the equivalent in vitro concentration as a minimum CC₅₀ to calculate a new selectivity index. By definition, this calculation underestimates the CC₅₀ and the SI because no toxicity was observed at the human dose of 200 mg/day that these numbers were derived from, but it does provide a more precise understanding of SI than the currently undefined experimental value. This approximation allows for calculating a minimum potential selectivity of pipendoxifene of 10.63-35.43, versus the experimentally observed value of ">4.83", when the CC₅₀ is 87.6-146 µM. Taken together, this analysis leads to the conclusion that there is evidence to support that pipendoxifene may be effective against COVID-19 in hamster or mouse models of disease at the clinically supported safe and well tolerated dose.

Based on this analysis, there appears to be definitive justification for dosing pipendoxifene in mice at, at least 66.67mg/kg/day and justification at 100 and/or 133.33 mg/kg/day, and in hamsters at 40 mg/kg and justification at 60 and/or 80 mg/kg/day, based on a lack of toxicity at the equivalent clinical doses. It also appears that those concentrations are predicted to elicit a measurable antiviral response, based on the in vitro results and the mathematics above, in a mouse or hamster model.

### Method 1.b

The first method beginning with the clinical dosage of the drug can also be converted to an equivalent in vitro dose per well by using the growth area and working assay volume of a single well in a 384w plate. The growth area (0.1cm²) of a single well in a 384w plate and assay volume used in the experiment is provided in Table 2. Next, the calculated in vitro dose per well was then converted to a resultant concentration using the molarity formula (M= (m/MW) * (1/V)). See Equation IV below. Without being bound by any particular theory, this method of conversion is potentially more accurate as the variables used are representative of the same values used in the in vitro experiment and the method converts from human to in vitro directly, rather than via an animal model. Method 1.b formula is shown in Equation IV. D=clinical dosage (mg/m²), MW=molecular weight (g/mol), G= growth area of each well in greiner uclear 384w plates (m²),V=volume for in vitro (L), C= concentration in vitro (µM), I=equivalent in vitro dose (g).
*equivalent in vitro dose (g)* → *concentration in vitro (uM)* $\frac{D}{1} ∗ \frac{G}{1} ∗ \frac{1}{1000} = I \to \frac{I}{MW} ∗ \frac{1}{V} ∗ \frac{1 ∗ {10}^{6}}{1} = C$

**TABLE 2: CONVERSION OF CLINICAL DOSAGE (MG/M²) OF PIPENDOXIFENE TO AN APPROXIMATE IN VITRO CONCENTRATION (µM)**

| clinical dosage of pipendoxifene (mg/m2) | molar mass (g/mol) | growth area of each well in Greiner uclear | growth area of each well in Greiner uclear | equiv. in vitro dose in each well(mg) | equiv. in vitro dose (g) | volume for in vitro (L) | concentration in vitro (M) | concentration in vitro (µM) | Selectivity Index (CC50/EC50) |
|---|---|---|---|---|---|---|---|---|---|
| | | 384-well plates (cm²) | 384-well plates (m²) | | | | | | |
| 200 | 456.6 | 0.1 | 0.00001 | 0.002 | 0.0000 02 | 2.60E-05 | 0.000168469 | 168.469288 | 20.4453019 |

As seen in Table 2, Method 1.b arrives at a resultant in vitro concentration of 168.47 µM that was calculated from the highest known safe and well tolerated clinical dosage of pipendoxifene (200mg). The concentration is higher than the EC₅₀ (8.24 µM) and EC₉₀ (15.03 µM) values of pipendoxifene observed in the HeLa-ACE2 cell-based SARS-CoV-2 assay reported here. This difference between the observed in vitro EC₅₀ and EC₉₀ of pipendoxifene and the calculated in vitro concentrations derived from the clinically justified safe and tolerated dosing allows for a significant buffer that might be expected to account for any error in the calculation due to factors such as absorption and metabolism, which the calculation is unable to take into account. Since the 200mg/m² clinical dosage of pipendoxifene was well tolerated in a Phase 1 clinical trial, it is therefore also logical to use the equivalent in vitro concentration as a minimum CC₅₀ to calculate a new selectivity index. By definition, this calculation underestimates the CC₅₀ and the SI because no toxicity was observed at the human dose of 200 mg/day that these numbers were derived from, but it does give us a more precise understanding of SI than the currently undefined experimental value. This approximation allows for calculation of a minimum potential selectivity of pipendoxifene of 20.45, (versus the experimentally observed value of ">4.83"), when the CC₅₀ is 168.47 µM. Taken together, this analysis suggests that there is evidence to support that pipendoxifene may be effective against COVID-19 clinically at the clinically supported safe and well tolerated dose.

### Conclusions

The ActivPred platform discovered 5 active compounds that exhibited antiviral activity against COVID-19. The ActivPred platform discovered 2 active antiviral compounds against COVID-19 that were found to be potent (EC₅₀ < 9 µM) and selective (CC₅₀/EC₅₀ > 10 or CC₅₀ > 39.8 µM). The ActivPred platform yielded a success rate of 14.3% for identifying novel and active antiviral compounds from the prioritized list of 35, which is higher than a "gold-standard" HTS success rate of 2.75%. Furthermore, ActivPred's success rate for discovering novel potent and selective antivirals is 5.7% in comparison to a "gold-standard" HTS rate of 0.56%. ActivPred was 5x and 10x more efficient in identifying active or potent and selective drugs against COVID-19, respectively, compared to a "gold-standard" HTS approach. ActivPred is significantly more successful, affordable and faster than the traditional HTS approach to drug discovery that is utilized by the majority of scientific researchers.

Both pipendoxifene and berzosertib are defined as potent and selective antiviral agents against SARS-CoV-2. Based on the in vitro data presented herein and in *vivo* dosage to in vitro drug concentration conversions, there is scientific justification to support that pipendoxifene may demonstrate antiviral activity at safely tolerated doses in *vivo*. Based on the in vitro data from this study and in *vivo* dosage to in vitro drug concentration conversions, there is scientific justification to support that berzosertib may demonstrate antiviral activity at safely tolerated doses in *vivo*.

When considering the ability of the ActivPred platform, the in vitro data from this experiment and the equivalent dosage to concentration conversions, it appears that in *vivo* development of berzosertib and pipendoxifene is scientifically justified. The significantly longer half-life of berzosertib and pipendoxifene as compared to remdesivir means that these drugs may potentially be preferred to remdesivir, assuming in vivo and clinical success relative to efficacy. It is expected that the compound concentrations predicted herein will elicit a measurable antiviral response (e.g., in a mouse or hamster model).

### Example 3

### In Vivo Validation of Berzosertib and Pipendoxifene

### Definitions

***Viral load*/*burden*/*titre*/*titer***. Viral load, also known as viral burden, viral titre or viral titer, is a numerical expression of the quantity of virus in a given volume of fluid; sputum and blood plasma being two bodily fluids.

***Virus Shedding***. Viral shedding refers to the expulsion and release of virus progeny following successful reproduction during a host-cell infection. Once replication has been completed and the host cell is exhausted of all resources in making viral progeny, the viruses may begin to leave the cell by several methods.

***Inoculum***. A small amount of material containing bacteria, viruses, or other microorganisms that is used to start a culture.

***PFU***. A plaque-forming unit (PFU) is a measure used in virology to describe the number of virus particles capable of forming plaques per unit volume.

***qRT-PCR***. Quantitative reverse transcription polymerase chain reaction (qRT-PCR) technology can detect viral SARS-CoV-2 RNA in the upper respiratory tract for a mean of 17 days (maximum 83 days) after symptom onset.

**sgRT-qPCR** (subgenomic RT-qPCR).

***TCID50*** (Median Tissue Culture Infectious Dose) assay is one method used to verify the viral titer of a testing virus. Host tissue cells are cultured on a well plate titer, and then varying dilutions of the testing viral fluid are added to the wells.

***Relation of viral titer to pfu***. The viral titer is a quantitative measurement of the biological activity of your virus and is expressed as plaque forming units (pfu) per ml.

***PBS*** (phosphate buffered saline) is a balanced salt solution used for a variety of cell culture applications, such as washing cells before dissociation, transporting cells or tissue, diluting cells for counting, and preparing reagents.

***Control groups*** can comprise positive, negative, test, vehicle groups. Positive control group: This group is also called a "disease control group" meaning, animals belonging to this group will be induced with the disease. Negative control group: Animals belonging to this group will not be induced with the disease and will not receive any treatment as well. Test group: This group is also called a "treatment group". Animals belonging to this group will be first induced with the disease and after induction of the disease, the animals are treated with a pre-decided dose of drug "X" based on the literature review and its efficacy is evaluated in the disease, by different evaluation parameters. The drug "X" either is given orally, intraperitoneally, or intramuscularly after dissolution into a particular solvent based on its oil or water solubility. Vehicle control group: Animals belonging to this group are induced with the disease and after induction of the disease, a similar quantity of the vehicle to that of test group which is used to dissolve the drug "X" is administered by the same route and for the same duration to the animals, in order to evaluate the effect of vehicle on the disease, whether it also has some impact on the disease activity or any side effect.

### Introduction

Berzosertib (VX-970, VE-822) and pipendoxifene (ERA-923) bind the viral proteins 3C-like protease (3CLpro) and RNA-dependent RNA polymerase(RdRp), respectively. By binding these viral proteins that are necessary for proper viral replication, berzosertib and pipendoxifene are able to inhibit SARS-CoV-2 replication and subsequent propagation. These *in silico* discoveries were validated as having potent (<9.6 µM) and selective (SI >10 or CC₅₀ <30 µM) antiviral activity in *an in vitro* HeLa-ACE2/SARS-CoV-2 high-content imaging (HCI) assay as described herein. Accordingly, pipendoxifene and berzosertib are advanced into *in vivo* efficacy studies. Antiviral efficacy is first evaluated in an Ad-hACE2/SARS-CoV-2 mouse model and subsequently in a more complex *in vivo* study involving Golden Syrian hamsters.

### In Vivo SARS-CoV-2 Infection Animal Models

**TABLE 3A: COMPARATIVE SARS-COV-2 IN VIVO MODEL CHARACTERISTICS**

| Animal | ACE2 (present naturally that can bind SARS-CoV-2 spike protein) | Susceptibility | BSL3 adaptable | Evidence of viral shedding |
|---|---|---|---|---|
| Transgenic mice (k18-hACE2) | | ✔ | ✔ | ✔ |
| Syrian hamster | ✔ | ✔✔ | ✔ | ✔✔ |
| ferrets | ✔ | ✔ | ✔ | ✔ |
| rhesus macaques | ✔ | ✔ | ✔ | ✔ |
| pigs | ✔ | | ✔ | |
| fruit bats | ✔ | ✔ | ✔ | |
| cats | ✔ | ✔ | | |
| chickens and ducks | ✔ | | ✔ | |
| dogs | ✔ | | ✔ | |
| minks | ✔ | ✔ | | ✔ |

**TABLE 3B: COMPARATIVE SARS-COV-2 IN VIVO MODEL CHARACTERISTICS**

| Animal | Histopathological basis of disease | Transmission | Clinical signs of disease | SARS-CoV-2 survival |
|---|---|---|---|---|
| Transgenic mice (k18-hACE2) | ✔(mild to lethal dependent on hACE2 expression) | | ✔ | |
| Syrian hamster | ✔✔ | ✔ | ✔✔ | ✔✔ |
| ferrets | ✔ | ✔✔ | ✔ | ✔ |
| rhesus macaques | ✔ | | | |
| pigs | | | | |
| fruit bats | | | | |
| cats | ✔ | ✔ | | ✔ |
| chickens and ducks | | | | |
| dogs | | | | |
| minks | | | ✔✔ | |

Selection of an in vivo model is important when designing an efficacy study. The chosen animal model is representative of the disease in humans while also providing sufficient data and measurements to achieve significant results. There are numerous animal models that have been developed and explored to assess antiviral efficacy of drugs such as transgenic mice, syrian hamsters (*Mesocricetus auratus*), ferrets (*Mustela putorius furo*), non-human-primates such as rhesus macaques *(Macaca mulatta*), pigs (*Sus* s*crofa domesticus),* fruit bats (Rousettus aegyptiacus), chickens and ducks, dogs (Canis lupus familiaris), and minks (Neovison vison). When selecting an animal model, one should consider a number of factors including clinical signs of disease, laboratory handling, susceptibility, transmission, evidence of viral shedding, histopathological evidence of disease, microscopic findings, disease timeline and cost-effectiveness to name a few.

Without being bound by any particular theory, the susceptibility of an animal to SARS-CoV-2 infection is primarily based on the ability of the SARS-CoV-2 spike protein being able to perform cellular entry by interacting its receptor binding domain (RBD) with the Angiotensin-converting Enzyme 2 (ACE2). The level of susceptibility to SARS-CoV-2 varies across organisms given that the DNA sequence of the ACE2 receptor differs as well. In terms of susceptibility, cats, non-human-primates, minks, ferrets, hamsters, transgenic mice and fruit bats have demonstrated susceptibility to SARS-CoV-2 whereas dogs (very mild), pigs, chicken and ducks have not. Mice have to be genetically engineered to express hACE2 or sensitized to hACE2 in order to be susceptible to the virus. These transgenic mice face a limitation on the length of experimentation because the mutation has lethal effects due to neuroinvasion by SARS-CoV-2. Nonetheless, they serve as a useful model to study SARS-CoV-2 replication in the lungs in shorter periods. Contrastingly, syrian hamsters express an ACE2 receptor that has high functional interaction with the receptor binding domain of SARS-CoV-2.

Biosafety level 3 (BSL-3) is applicable to clinical, diagnostic, teaching, research, or production facilities where work is performed with agents that may cause serious or potentially lethal disease through inhalation, to the personnel, and may contaminate the environment. The conditions in an environment of a biosafety level-3 (BSL-3) lab can influence the magnitude of difficulty researchers will experience when handling certain animals. A BSL3 lab is necessary for SARS-CoV-2 isolation according to the CDC guidelines. Cats and minks are not suitable for the conditions of a BSL3 lab and have been reported to be difficult to handle in this type of setting. Despite being susceptible to SARS-CoV-2 and demonstrating some basis of disease, these two models cannot be considered as a well-suited animal model to study therapeutics because the difficulty of handling could pose complications for data collection.

Evidence and the degree of virus replication and viral shedding is another important aspect of in vivo model selection. These measurable aspects of SARS-CoV-2 are especially important given that pipendoxifene and berzosertib inhibit targets that are vital to viral replication; RdRp and 3CLpro, respectively. Syrian golden hamsters, non-human-primates, transgenic mice and ferrets exhibit detectable virus replication in the respiratory tract. Notably, syrian hamsters have shown to have high levels of viral RNA present in oral swabs, high viral loads, and extensive virus shedding. It is valuable that the hamster/SARS-CoV-2 model exhibits these characteristics as they allow for multiple measurements of viral replication and propagation to be taken and to evaluate the potential attenuation that a therapeutic may have.

The clinical signs of disease and the ability to observe changes in haematological parameters, like body weight and lung inflammation, can be invaluable for analysis of a therapeutics effect on not only the level of virus replication and shedding but also for the overall immune response of an animal to SARS-CoV-2 infection. The transgenic mice models can develop severe clinical signs of disease and have high detectable levels of viral replication in response to SARS-CoV-2 infection. Ferrets, as a mild model of disease, have been shown to display little to no clinical signs of disease when infected with SARS-CoV-2. The same mild findings in ferrets are applicable to the haematological parameters, body weight and lung inflammation. Mild disease and clinical signs are observed in non-human-primates, but they do exhibit haematological changes. Syrian hamsters demonstrate clinical signs of disease such as ruffed hair, hunched posture, laboured breathing, and dramatic weight loss. They also are shown to model mild-to-moderate disease in response to SARS-CoV-2 infection. It has been shown that syrian hamster lungs experience significant inflammation and edema that is valuable when evaluating a therapeutic attenuation of these disease effects inflicted by SARS-CoV-2.

Transmission of SARS-CoV-2 is a valuable action to study when concerned with the epidemiology of a virus and/or for vaccination efficacy. Both syrian hamsters, nonhuman primates and ferrets are able to transmit the virus to uninfected individuals as they all are susceptible to the virus in a BSL3 setting. However, ferrets are a favorable model to study SARS-CoV-2 transmission given that they are able to very efficiently transmit the virus. In regards to the study design proposed in this report, disease transmission is not accounted for nor is it feasible given the experimental design not requiring an uninfected control. Given this, ferrets are a valuable preclinical model to study disease transmission (i.e. for vaccine development) but not the best-suited for therapeutic attenuation of moderate SARS-CoV-2 disease effects.

The timeline of an experiment can be important when considering a drug with a longer half-life that may require a few days to reach its steady-state necessary to exert its full effects. When considering an experiment longer than 3 days and one to study the long-term effects of a drug, transgenic mice that are genetically engineered to express human ACE2 or are sensitized to hACE2 are not well-suited for this. Mice develop mild to lethal disease dependent on the expression of human ACE2 and can die from neurological infection caused by SARS-CoV-2 after 3 days. Hamster, ferrets, and non-human primates are better suited for longer complex studies. Transgenic mice can still be useful for shorter study periods that have less complex endpoints. On the other hand, hamsters are able to resolve the infection on their own within approximately 2 weeks. This timeline allows for a longer assessment of a therapeutic effect without requiring concern for the possibility of the subjects having a lethal response to the infection and compromising the experimental results.

An *in vivo* study is conducted that aims to assess the antiviral efficacy of berzosertib and pipendoxifene. For a study that is solely evaluating a therapeutic ability to reduce viral load, a transgenic mice model is well suited and cost-effective before advancing into a more complex study. In order for an animal to be considered as a well-suited model for a complex SARS-CoV-2 efficacy study, they should be highly susceptible to SARS-CoV-2, able to be handled with relative ease in a BSL3 setting, exhibit measurable clinical signs of disease, and have a high likelihood of surviving the virus. If the mouse efficacy study is successful, the literature suggests that the syrian golden hamster encapsulates the necessary complex experimental features and is the best-suited animal model to advance with when evaluating therapeutic efficacy against SARS-CoV-2.

### IND-Enabling COVID-19 Drugs that Used Syrian Hamster Design

As of February 18, 2021, there are a total of at least 7 drugs in human clinical trials that used Syrian Hamsters as part of their preclinical data to support the efficacy of each drug against SARS-CoV-2. The success demonstrated by these drugs in their ability to begin clinical trials supports the preclinical acceptance of Syrian hamsters serving as a proper model to study therapeutic efficacy against SARS-CoV-2. This further supports that syrian hamsters are not only the best-suited in vivo model for the experimental design that can be used to test the antiviral efficacy of pipendoxifene and berzosertib, but that they also serve as an FDA-accepted representative model of SARS-CoV-2 disease.

**TABLE 4: IND-ENABLING DRUGS THAT USED SYRIAN HAMSTERS AS PRECLINICAL DATA**

| Drug | Hamster age (weeks) | Quantity of COVID-19 Clinical Trials | References |
|---|---|---|---|
| Favipiravir | Syrian hamsters (6-10 weeks old | 43 COVID clinical trials listed | https://pubmed.ncbi.nlm.nih.gov/33037151/ |
| | | | https://clinicaltrials.gov/ct2/results?cond=Covid1 9&term=Favipiravir&cntry=&state=&city=&dist = |
| Ivermectin | 5-6 weeks old | 59 COVID clinical trials listed | https://www.biorxiv.org/content/10.1101/2020.11 .21.392639v1 |
| | | | https://clinicaltrials.gov/ct2/results?recrs=&cond =Covid19&term=Ivermectin&cntry=&state=&cit y=&dist= |
| STI-2020 | 6 weeks old | 3 COVID Clinical Trials | https://www.biorxiv.org/content/10.1101/2020.10 .28.359836v1 |
| | | | https://clinicaltrials.gov/ct2/results?recrs=&cond =Covid19&term=STI-2020&cntry=&state=&city=&dist= |
| Hydroxychloroquine | ? | 218 COVID studies | https://insight.jci.org/articles/view/143174 |
| | | | https://clinicaltrials.gov/ct2/results?term=hydrox ychloroquine&cond=Covid19&Search=Apply&r ecrs=b&recrs=a&recrs=f&recrs=d&recrs=e&age _v=&gndr=&type=&rslt= |
| MK-4482 | ? | 2 COVID studies | https://www.ncbi.nlm.nih.gov/pmc/articles/PMC 75531531 |
| | | | https://clinicaltrials.gov/ct2/results?recrs=&cond =Covid19&term=MK-4482&cntrv=&state=&citv=&dist= |
| RITONAVIR | | 87 COVID studies | https://www.biorxiv.ore/content/10.1101/2021.02 .14.431129v1.full |
| | | | https://clinicaltrials.gov/ct2/results?term=Ritonav ir&cond=Covid19&Search=Apply&recrs=b&rec rs=a&recrs=f&recrs=d&recrs=e&age_v=&gndr= &type=&rslt= |
| EIDD-2801 | | 4 COVID Studies | https://www.biorxiv.ore/content/10.1101/2021.02 .14.431129v1.full |
| | | | https://clinicaltrials.gov/ct2/results?term=EIDD-2801&cond=Covid19&Search=Apply&recrs=b& recrs=a&recrs=f&recrs=d&recrs=e&age_v=&gn dr=&type=&rslt= |

### IND-Enabling COVID-19 Drugs that used Syrian Hamster Design

To assess the antiviral activity of berzosertib and pipendoxifene in vivo, they are first tested in an Ad-hACE2 model of SARS-CoV-2 infection in mice. The purpose of conducting this experiment first is to evaluate the in vivo translation of the drug's antiviral efficacy before launching a more complex study in syrian hamsters. The sole endpoint measures viral titers in lungs on day 3 post SARS-CoV-2 infection. This assesses if the drugs are able to reduce viral load in the mice's lungs, and therefore confirming antiviral activity that was observed in vitro. The experiment consists of 4 infected groups with 6 mice each: pipendoxifene (PO QD), berzosertib (PO QD), remdesivir (positive control, SC BID) and vehicle control (IP QD). The dose selection for pipendoxifene and berzosertib is 66.67 mg/kg and 60 mg/kg, respectively. These doses are chosen as they were given in previous preclinical experiments to the same species (berzosertib) or it is a derived dose from a clinically safe dose in humans using FDA accepted mathematics (pipendoxifene).

Although it is likely that berzosertib and pipendoxifene have been dosed in mice at higher concentrations PO, there is no publicly available evidence of these potential doses. In the clinic, and in hamster animal studies, a loading dose as commonly seen for antivirals is administered. However, due to the lack of published data demonstrating doses at higher concentrations than mentioned above that are nontoxic, a loading dose may not be administered to the mice in this study to prevent compromising the antiviral efficacy study due to possible toxicity issues.

On day 0/hour 0, the mice are inoculated intranasally with 1x10⁴ PFU SARS-CoV-2, a standard inoculation concentration used by the Garcia-Sastre Laboratory at the Icahn School of Medicine at Mount Sinai. Groups 1-4 are administered the designated dose of each drug or vehicle assigned to a given group starting 1-2 hours prior to inoculation on Day 0 and continues daily to Day 2. All mice are euthanized on Day 3 post SARS-CoV-2 infection. After euthanization, lungs are collected for viral titers to measure viral load. This study outline was proposed by Dr. Adolfo Garcia-Sastre and Dr. Kris White from Mt. Sinai and this method has been published previously^{.}

The primary goal of this study is to demonstrate whether the two experimental drugs inhibit viral replication by reducing viral load. It is expected that the two experimental drugs inhibit viral replication by reducing viral load. The treated groups are expected to have a significant reduction in viral titers and both pipendoxifene and berzosertib is to advance into phase 1 and 2 preclinical testing in syrian golden hamsters as this would confirm the compounds' antiviral activity.

### Phase 1 In vivo PK study (naive syrian hamster)

Prior to assessing the antiviral efficacy of berzosertib and pipendoxifene in Syrian Gold hamsters (Mesocricetus auratus), a pharmacokinetic (PK) and biodistribution study are conducted, as there are currently no publicly available references that have evaluated the absorption, distribution, metabolism, and excretion (ADME) effects on berzosertib and pipendoxifene in hamsters. Scientific literature suggests that hamsters metabolism differs from mouse significantly enough that a PK and biodistribution study is useful to perform prior to dose selection for an efficacy study. The resultant concentration in the lungs is important to know before beginning the antiviral efficacy study because SARS-CoV-2 is a respiratory virus that infiltrates host cells via ACE2 receptors in lung tissue. This PK study can assure that the proposed doses will reach sufficient concentration levels to potentially be efficacious. The low dose selected for berzosertib (60 mg/kg) has been safely administered in mice before with no overt toxicity effects and the low dose selected for pipendoxifene (40 mg/kg) was derived from the clinical dose using FDA accepted mathematics. The high doses for these compounds are a multiple of the low dose that is justified by the clinical dosage and follows dose-selection suggestions for preclinical models.

Each test article are evaluated in both 72M and 72F hamsters. Two cohorts are assembled per drug to assess both a low and high dose level (mg/kg). The 10+ weeks old hamsters are quarantined and acclimated to the lab environment for five days. There is a single dose on day 1 of the study by oral administration. Body weights and detailed observations are taken before the first dose as a baseline and daily throughout the study. Cage-side observations are taken twice daily for mortality and morbidity. Pharmacokinetic blood collections are taken at 1, 2, 4, 6, 8, 12, 24, 36 and 48 hours post dose. 4 animals/sex/group have blood collected (as much as possible prior to euthanasia). Animals are subject to terminal blood collections beginning at 6 hours post dose and terminal lung tissue collections are performed. Pharmacokinetic analysis of plasma samples are done by a qualified LC-MS method applicable to the test materials. The lung tissue are homogenized and analyzed by a qualified LC-MS method. Concentration analysis are completed using WinNonlin Data Analysis software.

**TABLE 5: EXPERIMENTAL OUTLINE FOR PHASE 1 PK TESTING**

| Group | Treatment | Dose Level (mg/kg) | Dosing Route | Animals No. |
|---|---|---|---|---|
| 1 | Test Article 1 | Low | Oral | 36 M/36 F |
| 2 | Test Article 1 | High | Oral | 36 M/36 F |
| 3 | Test Article 2 | Low | Oral | 36 M/36 F |
| 4 | Test Article 2 | High | Oral | 36 M/36 F |

The results of this study can demonstrate whether or not the proposed doses for the subsequent antiviral efficacy study is high enough to exhibit sufficient concentration in the lungs and other essential organs. Suitable dosing regimens for the disclosed compounds are expected to be identified. After the concentration found sufficient is identified, the doses are used in the following study.

### Bioanalytical Assay Development

### REGULATORY COMPLIANCE: Non-GLP

**OBJECTIVE:** Develop method(s) for quantification of Test Article in hamster lung tissue for use in the efficacy and PK studies outlined below.

**TABLE 6: BIOANALYTICAL ASSAY DEVELOPMENT DETAIL**

| METHOD | LC-MS/MS |
|---|---|
| METHOD DEVELOPMENT | Development of methods to quantify Test Article in hamster lung tissue. |
| MATERIAL REQUIREMENT | Estimated to be 200 mg API (reference standard quality with CoA), 10-20 mg stable label IS. |

### Non-GLP Oral Pharmacokinetic/ Biodistribution Study in Hamster

### REGULATORY COMPLIANCE: Non-GLP

**OBJECTIVE:** To understand Test Article PK in hamsters. This study is expected to enable the assessment of local concentrations and inform dose selection for the efficacy study.

**TABLE 7: NON-GLP ORAL PHARMACOKINETIC/ BIODISTRIBUTION STUDY IN HAMSTER STUDY DESIGN**

| Group | Treatment | Dose | Route | Animals | Time points |
|---|---|---|---|---|---|
| 1 | Control Vehicle | Single Dose | | 4 | Body Weights at 48 hr |
| 2 | Berzosertib | Single dose | PO | 32 | 8 terminal time points out to 48 hours (0.5, 1, 2, 4, 8, 12, 24 and 48 hours) |
| 3 | Pipendoxifene | Single dose | | 32 | |
| | | | | | n = 4 animals at each terminal time point |

**TABLE 8: NON-GLP ORAL PHARMACOKINETIC/ BIODISTRIBUTION STUDY IN HAMSTER STUDY DETAIL**

| | |
|---|---|
| ANIMALS | 68 Syrian Hamster, plus 6 spare animals |
| TEST ARTICLE | Berzosertib, Pipendoxifene |
| DOSE ROUTE / FREQUENCY | Single dose via PO Oral on Day 0 |
| CLINICAL OBSERVATIONS | Twice daily |
| BODY WEIGHT | Prior to treatment |
| NECROPSY / TISSUE HARVEST | Euthanize n = 4 animals at each of 8 time points out to 48 hours. Collect terminal body weight and lung tissue only for bioanalytical analysis. Timepoints (0.5, 1, 2, 4, 8, 12, 24 and 48 hours) |
| BIOANALYSIS | Analysis of 64 lung tissue samples. |
| TOXICOKINETIC DATA ANALYSIS | Non-compartmental analysis performed to focus on Cₘₐₓ, Tₘₐₓ, AUC and apparent terminal half-life in each lung tissue. |
| TIMELINES | Pre- Study Activities: 2-3 Weeks |
| | In-Life: 1 Week |
| | Bioanalysis: 3-4 weeks |
| | Provision of Data: 3-4 weeks |

### Pre-clinical In vivo antiviral efficacy (Syrian hamster/SARS-CoV-2 model):

### REGULATORY COMPLIANCE: Non-GLP

**OBJECTIVE:** To assess the pharmacology of a repeat dosing of test article via IP delivery in a SARS-CoV-2 challenged hamster model.

**TABLE 9: SARS-COV-2 EFFICACY MODEL IN SYRIAN HAMSTER STUDY DESIGN**

| **Group** | **Challenge** | **Compound** | **Route** | **Animals** | **End Points** |
|---|---|---|---|---|---|
| 1 | | PBS (Vehicle) Control | PO | 6 | |
| 2 | SARS-CoV-2 | Remdesivir Control (7.5mg/kg SID) | IP | 6 | Daily Body weights, Behavior, oral swabs (Day 1, 3, and 5), Collect lung, respiratory tract and (to be fixed, held. Processed to slides by amendment only) |
| 3 | | TA 1 High | PO | 12 | |
| 4 | | TA 1 Low | | 12 | |
| 5 | | TA 2 High | | 12 | |
| 6 | | TA 2 Low | | 12 | |

**TABLE 10: SARS-COV-2 EFFICACY MODEL IN SYRIAN HAMSTER STUDY DETAIL**

| | |
|---|---|
| ANIMALS | 60 Hamsters plus 5 spares; (males); approximately 110-150g |
| TEST ARTICLE | Test Article |
| CHALLENGE | SARS-CoV-2 |
| DOSE ROUTE / FREQUENCY | Viral challenge: Intranasal instillation; single dose; Day 0 |
| | Group 1: BID PO Starting on Day 0 |
| | Group 2: BID IP Starting on Day 0 |
| | Groups 3-6 BID PO, 6/12 animals are treated at time of infection and the remaining 6 animals are treated starting 6 hr. post infection. |
| DETAILED OBSERVATIONS | Twice daily starting at Day -3 |
| BODY WEIGHT | Daily starting at day -3 |
| ORAL SWABS | Day 1, 3 and 5 post infection |
| NECROPSY / TISSUE HARVEST (Table 11) | Day 5 (all animals) |
| | Terminal Body weights, lung, Lung weight, oral swabs, respiratory tract and (to be fixed, held, and processed to histopathology by amendment only) |
| ORGAN WEIGHTS | Lung |
| HISTOPATHOLOGY (Table 11) | Fix respiratory tract to include nasal, trachea and lungs. Preserved in 10% neutral buffered formalin. |
| | Added by Amendment Only: embedded in paraffin and sectioned and stained with hematoxylin and eosin for subsequent histological analysis by board certified pathologist. |
| VIROLOGY (Table 12) | Oral Swab: gRT-qPCR |
| | Lung: RT-qPCR and TCID₅₀ |
| GENERAL TIMELINE | Pre-study activities: 4 weeks (quarantine & IACUC) |
| | In-life: 2 weeks (includes B3 acclimation) |
| | Sample Sterilization: 1 week |
| | PCR: 3 weeks from sample sterilization |
| | TCID50: TBD |
| | Report (no histopathology): 4-6 weeks |
| | Optional Histopathology: ~10 weeks |
| | Report: ~12-14 weeks (concurrent with histopathology) |

**TABLE 11: TERMINAL PROCEDURES- ORGAN OR TISSUES TO BE WEIGHED, PRESERVED, AND MICROSCOPICALLY EXAMINED**

| Tissue | Organ Weight Taken | Collected and Preserved |
|---|---|---|
| Animal Identification | | X |
| Lung | X | X |
| Gross lesions | | X |

**TABLE 12: TISSUE COLLECTION**

| Tissues | Number of animals | Number of samples per Animal | | Total Number of Samples/animal |
|---|---|---|---|---|
| | | TCID50² | RT-qPCR | |
| Oral Swab | 60 | 0 | 3 | 180 |
| Lungs² | 60 | 1 | 1 | 120 |
| TOTAL | | 1 | 4 | 300 |
| ¹Number of samples per tissue varies based target tissues of interest | | | | |
| ²To be analyzed by amendment only | | | | |
| ³Assumes 1 sample from 2 locations in the lungs | | | | |

It is expected that berzosertib and pipendoxifene are successful in phase 0 and phase 1, and berzosertib and pipendoxifene are then evaluated in a more complex, IND-enabling antiviral efficacy study performed in Syrian Golden hamsters (Mesocricetus auratus). This animal model was chosen because it has commonly been used in the past for respiratory virus infections. This species of hamster also has a similar ACE2 sequence to that of humans, allowing for proper SARS-CoV-2 infection and the development of mild to moderate disease. Unlike transgenic mice that can die after 3 days of infection from a lethal mutation, hamsters are able to recover from SARS-CoV-2 in approximately 2 weeks, allowing for a longer evaluation of a therapeutic effect on recovery. As elaborated above, this animal model provides the essential qualities of a SARS-CoV-2 model necessary to evaluate if an antiviral is able to reduce viral load, virus shedding, clinical signs of disease, weight loss, and lung inflammation.

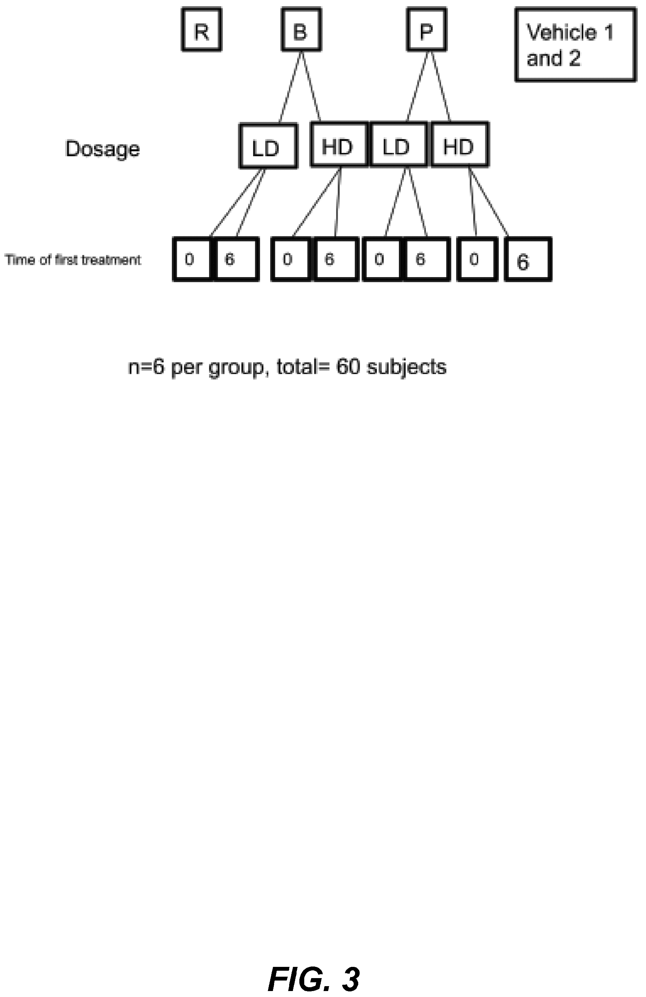

The main study have about a total of 60 hamsters plus 10 spare animals divided into 4 groups: remdesivir (positive control, n=6), PBS (vehicle control, n=6), berzosertib (low n=12), berzosertib (high n=12) and pipendoxifene (low n=12), pipendoxifene (high, n=12). This study design assesses the dose-ranging antiviral effects of berzosertib and pipendoxifene with two different time points (0 and 6hr) of initial treatment post challenge. By administering at two different time-points, the results allow for an assessment of when the drugs are best-acting. Pipendoxifene is delivered using 4% methylcellulose as the vehicle whereas berzosertib is delivered using 10% Vitamin E d-alpha tocopheryl polyethylene glycol 1000 succinate (TPGS). These vehicles were previously used for oral administration of each corresponding drug. The endpoints assess antiviral efficacy by measuring daily body weights, behavior, oral swabs with sgRT-qPCR, viral titers with TCID₅₀ in the lung, and lung weight for edema and inflammation. The results are quantitatively compared to the positive control and current standard of care for SARS-CoV-2 infection, remdesivir. See FIG. 3 and Tables 9-13 for details of exemplary study conditions.

Hamsters are challenged with SARS-CoV-2 by intranasal inoculation at a chosen PFU on Day 0. The intranasal route of inoculation was selected as it has been shown to result in high viral load and severe inflammation in the lungs, body weight loss, clinical signs of disease, and virus shedding from the oral cavity whereas oral inoculation results in little to none of these disease characteristics. Group 1 (vehicle control) begins dosing BID PO on Day 0 at time of infection, group 2 (positive control) begins dosing at 7.5 mg/kg SID IP on Day 0 at time of infection, and half of groups 3-6 begins dosing either (60mg/kg or TBD mg/kg) berzosertib or (40 mg/kg or TBD mg/kg) pipendoxifene BID PO at time of infection while the remaining half begins treatment 6 hours post challenge. Oral administration (PO) of the experimental drugs is chosen as they have both been studied in animal models (mice) via this route of administration before and it is advantageous to IP or IV administration in terms of ease, discomfort and accessibility in the clinic.

The initial dose of each drug is a loading dose (amount TBD); a dose that is higher than subsequent doses. The loading dose can be equal to the highest safely tested dose from the PK Phase 1 study. Typically, drugs with a longer half-life are able to reach steady-state relative to half-life when they are first administered with a loading dose. Given that berzosertib (t ½ = 17h) and pipendoxifene (t ½ = 30h) have relatively long half-lives, a loading dose would be ideal to achieve the necessary therapeutic level on the first dose. It has been supported in a number of different viral cases that a long half-life is a key characteristic to suppressing viral infection and reactivation; some infections resolved with only one dose_{.} The loading dose can be determined by consulting a PK expert and relying on the logic inferred from the in vitro study results. Additional doses may be implemented into the Phase 1 PK study design.

Hamsters lose weight in respiratory distress and so, a baseline body weight is taken and daily body weights starting Day 3. Detailed observations are performed twice daily starting from Day 3. Oral and pharyngeal swabs are taken on day 1, 3 and 5 pi and viral load present in these samples are quantified by sgRT-qPCR. Animals are euthanized on day 5pi and terminal body weights, lung, lung weight, oral and pharyngeal swabs, and respiratory tract tissue is collected. The held tissues are processed for histopathology analysis if necessary.

In conclusion, these studies (phase 0-2) can provide data involving the compound's biodistribution in hamster lungs, and both simple and complex SARS-CoV-2 antiviral efficacy data. While both compounds are being administered orally in the preclinical studies, pipendoxifene has been administered orally in humans whereas berzosertib has only been studied via intravenous administration. The proposed doses are expected to reach sufficient concentration levels in the lungs to be efficacious, including when administered orally. The compounds are expected to demonstrate efficacy versus SARS-CoV-2.

### Overall Pre-clinical in vivo Experimental design

**Animal model**: Syrian Hamster.

**Inoculation PFU or TCID₅₀**: Existing models can be inoculated intranasally with 5x10² (25uL)- 2x10⁶(50uL) TCID₅₀ or 1x10⁴-1x10⁵ PFU of SARS-CoV-2 in 100uL PBS.

**Sample size and groups**: n=6 per group, 2 experimental treatment groups, (2 groups per treatment for dose-range and 2 different time points for first dose), 1 positive control (remdesivir) and 1 vehicle control group (PBS, TPGS or methylcellulose). Total of 10 groups. Total of 60 hamsters.

**Route of Admin, time and frequency of Dosing**: Compounds are administered to both treatment groups orally via oral gavage. Remdesivir needs to be administered Via i.p. injection. Time of 1st is either at the time of infection or 0-6hr pi. In some embodiments, frequency of dosing is every day. It is expected the disclosed compounds show an advantage over remdesivir's required dosing regimen. In a dose-ranging study, one low and one high dose are administered. In some embodiments, a loading dose is given at the beginning of the course of treatment before dropping down to a lower maintenance dose. A loading dose can be most useful for drugs that are eliminated from the body relatively slowly, e.g. have a long systemic half-life

### Length of Experiment 4-7 days

**Measurements** Weight, behavior, appearance (clinical signs of disease) are monitored twice daily. Oral and nasal swabs (or pharyngeal) are collected on days 1, 3, and 5 post-infection to measure viral shedding, determined by RT-PCR (genomic). It is expected treatment with the disclosed compounds reduces viral shedding. Lung, respiratory tract and nasal turbinate tissue samples are collected after euthanization for viral and histopathology analysis (tissue samples are collected, held and processed if needed). Viral load is detected by TCID₅₀ and RT-qPCR methods in lungs and respiratory tract only. It is expected treatment with the disclosed compounds reduces viral load. Treated lungs are weighed in comparison to uninfected and other controls to assess for lung damage attributed by edema and inflammation caused by SARS-CoV-2. Treatment with the disclosed compounds is expected to reduce, delay, or prevent lung damage attributed by edema and inflammation caused by SARS-CoV-2.

**TABLE 13: ANIMAL MODELS USED FOR STUDY CLINICAL MANIFESTATION/PATHOGENESIS, DRUG AND VACCINE EFFICACY**

| Animal models | Strains | Dose and route used | Clinical/pathological Lesions | Uses/limitations |
|---|---|---|---|---|
| Mice | KI8-hACE2 transgenic | Intranasal inoculation with SARS-CoV-2 stock virus at a dosage of 10⁵ TCID₅₀ SARS-CoV-2 | Bodyweight loss from 3-5 days, lethargic with laboured breathing, and all mice died within 7 days | Animal model for COVID-19 pathogenesis for evaluating vaccines and therapeutics |
| | | | Interstitial pneumonia, neural damage of CNS was also seen | Limitation: |
| | | | | Lethal encephalitis |
| Mice | HFH4-hACE2 transgenic mice | Intranasal inoculation of 3 x 10⁴ TCID₅₀ SARS-CoV-2 | Bodyweight loss from day 4 to 6, respiratory distress and neurological symptoms | Animal model for COVID-19 pathogenesis for evaluating vaccines and therapeutics |
| | | | Pathological changes include interstitial pneumonia | |
| | | | | Limitation: |
| | | | | Lethal encephalitis, HFH4-hACE2 mice showed different susceptibility with the infection of SARS-CoV-2 based on gender and age |
| Mice | Ad5-hACE2-transduced mice using inbred mice | Intranasal inoculation with 1 x 10⁵ PFU of SARS-CoV-2 | Bodyweight loss at 4 to-6 days, labored breathing, interstitial pneumonia | Useful to study the efficacy of vaccines and therapies such as convalescent plasma therapy |
| | | | | Limitation: Don't develop severe disease and no extrapulmonary manifestations of diseases |
| Mice | SARS-CoV-2 MA model (Reverse genetics Using BALB/c mice) | Intranasal inoculation with 10⁵ PFU SARS-CoV-2 | Bodyweight loss at 3 -4 days, Pathological changes in lungs varies depending on mice age | Used for antiviral and vaccine development |
| | | | | Inbred mice model, age-related COVID-19 infections studies |
| Mice | C57BL/6 hDPP4 | 1 x 10⁴ PFU mouse-adapted SARS-MA15 in 50 µl | The antiviral drug had improved pulmonary function and reduced virus titer and body weight loss in C57BL/6 hDPP4 mice | This model can be used to study antivirals drugs for COVID-19 |
| Non-Human Primates | *Macaca mulatta* Rhesus monkey) *acaca fascicularis* Cynomolgus monkey) | 4.75 ml of 10⁵ pfu/ml SARS- CoV-2 intratracheally (4 ml), intranasally (0.5 ml) and on the conjunctiva (0.25 ml) | *M. mulatta* showed a good response to SARS-CoV-2, with decreased bodyweight, pulmonary abnormality, viral replication. increased inflammatory cytokine expression and pathological changes in the lungs | Good animal model for COVID-19 pathogenesis for evaluating drugs and vaccines. A good model for mild to moderate illness studies |
| | | | | Limitation: NHPs do not develop the acute lung injury that is observed in mouse models |
| Ferrets | *Mustela putorius furo* | Intranasal inoculation with 10^{5.5} TCID₅₀ of NMC-nCoV02 | Weight loss increased body temperatures | Useful for the studies related to disease transmission, antiviral, and new vaccine development |
| Hamsters | *Mesocricetus auratus* | Intranasal inoculation of 10⁵ plaque-forming units in 100µl of SARS-CoV-2 | Increased respiratory rate, decreasing activity, progressive weight loss with pathological lesions in the lower tract | Limitations: Mortality was not observed in hamsters |

| *Animal models used for disease transmission and neutralization antibodies production studies* | | | | |
|---|---|---|---|---|
| Hamsters | *Mesocricetus auratus* | Intranasal inoculation with 8 x 10⁴ TCID₅₀ SARS-CoV-2 | Disease transmitted from donor to naive contact hamsters by direct contact or via aerosols | Model to study on mild COVID-19 cases that occur in humans and for disease transmission studies |
| Ferrets | *Mustela putorius furo* | Intranasal dose with 6 x 10⁵ TCID₅₀ of SARS-CoV-2 virus | Virus shedding was found during direct contact and indirect recipient ferrets | A good model for the study transmission between individuals |
| Mice and Rats | BALB/c mice and Wistar rats | Inactivated Vaccine administered at a various dose to see whether neutralizing antibodies are produced | PicoVacc inactivated vaccine | An inactivated virus vaccine (PiCoVacc) generated neutralizing antibodies in this strain |
| Mice | Knockout mice ACE2*⁻¹⁻* | Intranasal inoculation with SARS -CoV virus 10^{5.23} TCID₅₀ | ACE2 receptor is needed for acute lung injury | Acute lung injury studies |
| | STAT-1^{-*1*-} | Intranasal inoculation with 10⁵ pfu/50 µl rMAI5 or the recombinant or biological epidemic virus, icSARS or Urhani. | Increased susceptibility, prolonged virus shedding, and mortality following infection with either virus | Useful in SARS CoV studies but not in SARS-CoV 2 |
| | TMPRSS2^{-*1*-} | Intranasal inoculation with SARS-CoV10⁵ TCID50 | TMPRSS2 Is needed for virus entry for pathogenesis | Use to understand virus entry and the development of inhibitors |

### Example 4

### Determination of Pharmacokinetics of Pipendoxifene Following Oral Repeated Dosing In Female 129S1 Mice

In this example, the plasma and lung tissue concentrations and pharmacokinetics of pipendoxifene were evaluated in female 129S1 mice following single and 5-day repeat oral (PO) administrations at 50 mg/kg and 250 mg/kg. Blood samples were collected via submandibular (non-terminal) or by cardiac puncture (terminal) route. The generated mouse plasma and lung tissue were sent for bioanalysis. The measured plasma and lung tissue concentrations of pipendoxifene were measured for pharmacokinetic (PK) analysis. PK parameters were determined using Phoenix WinNonlin (v8.3) software. The PK summary data are presented in Table 14 and Table 15. The average plasma and lung tissue concentrations are provided in Table 16.

**TABLE 14: PK SUMMARY DATA FOR PLASMA**

| **PK Parameter** | **Group 1, SD** | **Group 1, MD** | **Group 2, SD** | **Group 2, MD** |
|---|---|---|---|---|
| | **Dose:50mg/kg Pipendoxifene** | | **Dose: 250mg/kg Pipendoxifene** | |
| Cₘₐₓ (ng/mL) ± SE | 179±21 | 178±8 | 1020±693 | 1050±230 |
| tₘₐₓ (hr) | 2.0 | 6.0 | 6.0 | 6.0 |
| t_{1/2} (hr) | 2.36 | 2.58 | 3.96 | 2.52 |
| MRTₗₐₛₜ (hr) | 6.95 | 7.10 | 8.86 | 8.32 |
| AUCₗₐₛₜ (hr·ng/mL) ± SE | 1600 ±115 | 1391 ±55 | 10269 ±2101 | 11628 ±1191 |
| AUCₗₐₛₜ_D (hr·kg·ng/mL/mg) | 32.0 | 27.8 | 41.1 | 46.5 |
| SD: Single dose; MD: Multiple, 5-day, dose; ND: Not determined; SE: Standard error | | | | |

**TABLE 15: PK SUMMARY DATA FOR LUNG**

| **PK Parameter** | **Group 1, SD** | **Group 1, MD** | **Group 2, SD** | **Group 2, MD** |
|---|---|---|---|---|
| | **Dose: 50mg/kg Pipendoxifene** | | **Dose: 250mg/kg Pipendoxifene** | |
| Cₘₐₓ (ng/g) ± SE | 8310 ± 364 | 5613 ± 925 | 24733 ± 10000 | 39000 ± 8845 |
| tₘₐₓ (hr) | 4.0 | 8.0 | 8.0 | 4.0 |
| t_{1/2} (hr) | ND | ND | ND | ND |
| MRTₗₐₛₜ (hr) | 6.79 | 7.35 | 8.27 | 7.22 |
| AUCₗₐₛₜ (hr·ng/g)± SE | 105253± 8124 | 78138± 9521 | 369615 ±102095 | 515080 ±50723 |
| AUCₗₐₛₜ_D (hr·kg·ng/g/mg) | 2105 | 1563 | 1478 | 2060 |
| SD: Single dose; MD: Multiple, 5-day, dose; ND: Not determined; SE: Standard error | | | | |

**TABLE 16: AVERAGE PLASMA AND LUNG TISSUE CONCENTRATION OF PIPENDOXIFENE VS TIME**

| Time (hr) | **Group 1, SD (50 mg/kg Pipendoxifene)** | | **Group 1, MD (50 mg/kg Pipendoxifene)** | | **Group 2, SD (250 mg/kg Pipendoxifene)** | | **Group 2, MD (250 mg/kg Pipendoxifene)** | |
|---|---|---|---|---|---|---|---|---|
| | **Plasma (ng/mL)** | **Lung (ng/g)** | **Plasma (ng/mL)** | **Lung (ng/g)** | **Plasma (ng/mL)** | **Lung (ng/g)** | **Plasma (ng/mL)** | **Lung (ng/g)** |
| 0.5 | 63.9±28.2 | NA | 51.8±6.7 | NA | 88.8±14.1 | NA | 147±49 | NA |
| 1.0 | 89.1±71.2 | 2673± 1463 | 56.0±20.7 | 1443±454 | 195±90 | 5223±2028 | 225±106 | 5877±2051 |
| 2.0 | 179±37 | NA | 110±11 | NA | 662±114 | NA | 739±214 | NA |
| 4.0 | 107±23 | 8310±630 | 86.2±23.0 | 4803±1040 | 393±182 | 23067±7722 | 635±303 | 39000±15321 |
| 6.0 | 142±30 | NA | 178±15 | NA | 1020±1200 | NA | 1050±399 | NA |
| 8.0 | 78.5±31.0 | 6920±1358 | 82.1±18.1 | 5613±1602 | 344±259 | 24733±17321 | 575±194 | 35267±6926 |
| 12 | 61.9±12.0 | NA | 47.0±7.0 | NA | 628±182 | NA | 640±166 | NA |
| 24 | 1.12 | 203±35 | 1.95 | 288± 144 | 33.6±46.4 | 3888± 2853 | 9.89±3.94 | 1770± 684 |
| SD: Single dose; MD: Multiple, 5-day, dose; NA: Not applicable | | | | | | | | |

A study was performed to determine the pharmacokinetics of MDL-001 following oral repeated dosing in female 129S1 mice (Example 4). Plasma and lung tissue concentrations and pharmacokinetics of MDL-001 were evaluated following single and 5-day repeat oral (PO) administrations at 50 mg/kg and 250 mg/kg. Blood samples were collected via submandibular (non-terminal) or by cardiac puncture (terminal). The generated mouse plasma and lung tissue were sent for bioanalysis. The measured plasma and lung tissue concentrations of MDL-001 were employed for pharmacokinetic (PK) analysis. PK parameters were determined using Phoenix WinNonlin (v8.3) software. The PK summary data are presented in Table 14 and Table 15. The average plasma and lung tissue concentrations are provided in Table 16 for MDL-001.

All animals behaved normally during the experiment. No adverse reactions were observed following the PO administrations of MDL-001 in female 129S1 mice in this study. One mouse #153, Group 2, died after the 12-hour time point blood collection, due to stress from sampling. Lungs from Mouse #153 were collected at the 12-hour time point.

MDL-001 was rapidly absorbed and distributed to the target tissue after oral administration, with concentrations in both plasma and lung quantifiable at the first time point (0.5 h). Plasma Cₘₐₓ increased dose proportionally from 50 to 250 mg/kg. Plasma AUCₗₐₛₜ increased slightly greater than dose-proportionally. Mean Tₘₐₓ values ranged from 2 - 6 h. Following Tₘₐₓ, plasma concentrations declined with an apparent t_{1/2} ranging from 2.36 - 3.96 h. No accumulation was observed comparing Day 1 and Day 5 Cₘₐₓ or AUCₗₐₛₜ values, as expected given the short plasma t_{1/2} in mouse.

MDL-001 lung exposure was remarkably high, with Day 1 Lung/Plasma AUC ratios of 65 and 36 at 50 and 250 mg/kg, respectively. On Day 5, Lung/Plasma AUC ratios were 56 and 44. Tₘₐₓ ranged from 4.0 - 8.0 h, slightly later than plasma Tₘₐₓ. Similar to plasma, no significant accumulation was observed following repeat administration.

In some embodiments, MDL-001's remarkably high Lung/Plasma distribution translates to high therapeutic windows in the target tissue relative to any potential systemic toxicities.

### OBJECTIVE AND INTRODUCTION

The objective of this study was to determine the PK and lung tissue concentrations of pipendoxifene following single and repeat PO administrations in female 129S1 mice at 50 mg/kg and 250 mg/kg.

In this study, the plasma and lung concentrations versus time profile of pipendoxifene were determined in female 129S1 mice following single and repeat PO dose administrations at 50 mg/kg and 250 mg/kg. The measured plasma and lung tissue concentrations of pipendoxifene were used for PK analysis.

### EXPERIMENTAL METHODS

### Test System and Animal Description

The female 129S1 mice, a total of 96 (24 per group) and obtained from Jackson Lab, were utilized for this study. Animals were identified by cage labels and tail marks. A single room was used for all the animals. Animals were healthy at the start of the trial and were randomly assigned to treatment groups. The study was not blinded. Food and water was offered ad libitum*.*

### Dosing Formulations

All dosing formulations were prepared fresh on the day of dosing. The dosing solution preparation methods are presented below (See, section entitled "Dosing Solution Preparation").

### Animal Dosing

Freshly prepared dosing solutions of pipendoxifene were administered to the female 129S1 as a single and repeat PO dose at 50 mg/kg and 250 mg/kg. Blood samples were collected via submandibular (non-terminal) or by cardiac puncture (terminal) route, placed into chilled tubes containing K₂EDTA as anticoagulant, inverted several times, and kept on ice until centrifugation. The blood samples were centrifuged at temperatures of 2°C to 8°C, at 3,000xg, for 5 minutes. The plasma samples were transferred to labeled clear polypropylene tubes for analysis.

The lung tissue samples were collected at the selected time points, rinsed with saline, patted dry, and weighed to at least 3 significant figures. The tissue samples were placed into chilled tubes for analysis.

### PK Parameters

PK parameters were calculated from the time course of the plasma or lung concentrations with Phoenix WinNonlin (v8.3) software using a non-compartmental model with a sparse approach. The maximum plasma/lung concentration (Cₘₐₓ) and the time to reach maximum plasma/lung drug concentration (tₘₐₓ) after PO dosing were observed from the data. The area under the time concentration curve (AUC) was calculated using the linear trapezoidal rule with calculation to the last quantifiable data point, and with extrapolation to infinity if applicable. Plasma half-life (t_{1/2}) was calculated from 0.693/slope of the terminal elimination phase. Mean residence time (MRT) was calculated by dividing the area under the moment curve (AUMC) by the AUC. Any samples below the limit of quantitation (1 ng/mL) were treated as zero for PK data analysis.

### RESULTS

### Observations and Adverse Reactions

All animals behaved normally during the experiment. No adverse reactions were observed following the PO administrations of pipendoxifene in female 129S1 mice in this study.

### Analytical Data and PK Results

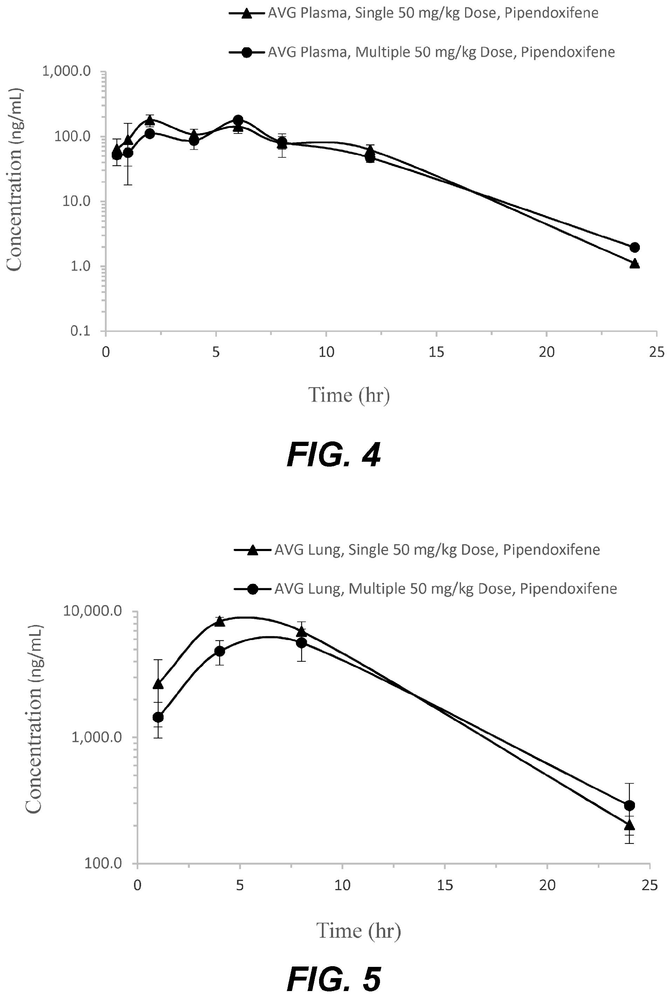

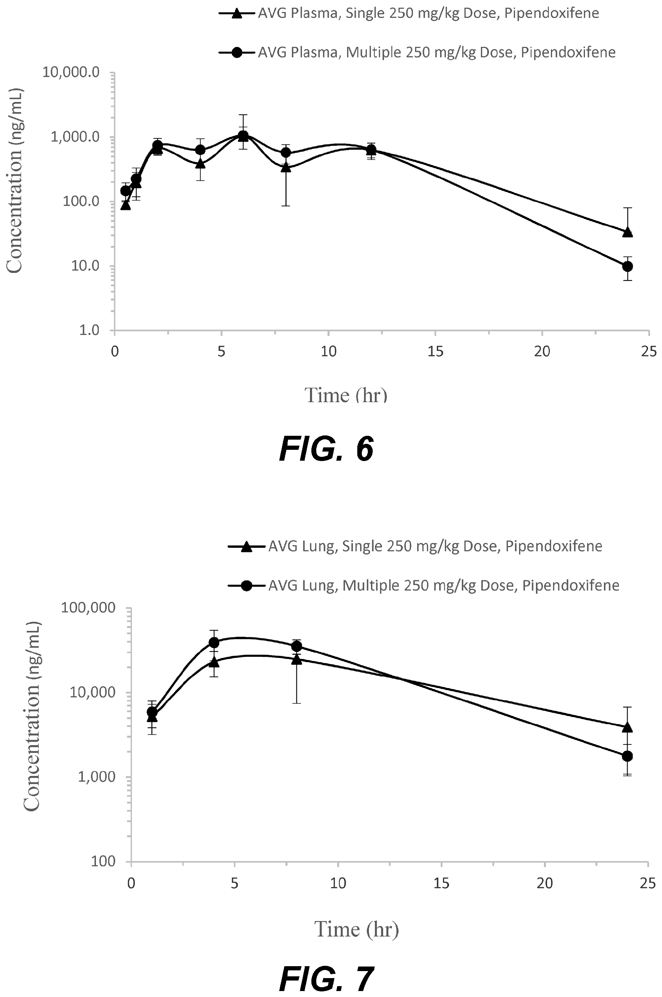

Individual and average plasma concentrations and PK data are shown in Table 17-Table 18 and Table 21-Table 22. Individual and average lung tissue concentrations are shown in Table 19-Table 20 and Table 23-Table 24. Average plasma and lung tissue concentrations are also shown in FIG. 4-FIG. 7. All data are expressed as ng/mL of the free base for plasma or ng/g for lung tissue. Plasma samples that were below the limit of quantification were not used in the calculation of averages. Comparison of average drug concentration in plasma and lung tissue are presented in Table 25.

### Analysis of Results

PK analysis found that a significant amount of the study drug was found to accumulate in plasma, but more importantly, in lung tissue (the primary site of disease). The PK study observed no adverse events at doses up to 250mg/kg in mice. The PK study demonstrates that Pipendoxifene administered once daily, orally could achieve lung concentrations of greater than 30 ug/g in the preclinical model. The PK study was conducted at two doses that bracketed the dose evaluated in a preclinical POC study which demonstrated that once daily, oral Pipendoxifene was non-inferior to twice daily, subcutaneous remdesivir. Lung samples were collected at 1, 4, 8 and 24 hours.

### C_{Lung}/EC_{50/90} Analysis

Single dose, low dose PK analysis demonstrated that the C_{Lung}/EC₉₀ ratio at 4 and 8 hours exceeded 1. The data indicates that it would be reasonable to extrapolate that the C_{Lung}/EC₅₀ ratio exceeds 1 for multiple additional hours beyond the 8 hour time point.

Single dose, high dose PK analysis demonstrated that the C_{Lung}/EC₅₀ ratio exceeded 1 at all time points (1, 4, 8, 24 hours) and exceeded 5 at 4 and 8 hours.

Multi-dose, high dose PK analysis demonstrated that the C_{Lung}/EC₉₀ ratio at 1 hour exceeded 1 and at 4 and 8 hours exceeded 5.

### Plasma concentrations

The PK analysis demonstrated a ~50x enrichment in lung to plasma concentrations.

### Half Life

The PK analysis demonstrated that the t_{1/2, Plasma} in humans is more than 5x greater than the t_{1/2, Plasma} observed in the preclinical model.

### Analysis

The data from this PK study demonstrates that once daily oral dosing of Pipendoxifene achieved therapeutic concentrations in the primary diseased tissue (lung) for sustained windows in high-dose, multi-dose settings as well as in low-dose, single-dose settings. It is important to note that the low-dose, single-dose study was performed at a dose lower than that found to achieve efficacy in the preclinical POC study.

The data demonstrates that a) increasing the dose and b) dosing Pipendoxifene for multiple days, results in c) the drug achieving tissue concentrations multiples greater than the minimum therapeutic concentration in the primary diseased tissue (lung) and d) an extension of the time that the therapeutic concentration is sustained in the diseased tissue. These results provide context as to why Pipendoxifene demonstrated preclinical POC. Furthermore, it is clear from the data that b.i.d. dosing would be expected to provide C_{Lung}/EC₉₀>1 coverage for at least 24 hours in the preclinical model.

Importantly, the plasma half-life of Pipendoxifene was found to be significantly shorter in the preclinical model than in humans. Thus, one may infer that there is a significant possibility that the window within which a therapeutic concentration is sustained in the diseased tissue may be extended in humans as compared to the animal model evaluated here.

No adverse events were observed in the PK study. The favorable lung to plasma ratio of Pipendoxifene is emblematic of a drug with a preferential safety profile and supports this finding. The reported clinical safety and tolerability profile of Pipendoxifene and its drug class generally also support these findings. Furthermore, an analysis of low dose/high dose results demonstrated dose proportional increases in lung concentration of the drug, which conforms to both the observed safety/tolerability results and suggests that the dose of Pipendoxifene can be significantly increased from the doses tested to date. At a minimum, these results appear to demonstrate that a) therapeutic levels have been achieved with doses significantly below the LD₅₀ and b) Pipendoxifene should allow for the administration of a loading dose significantly greater than that evaluated to date.

**TABLE 17: INDIVIDUAL AND AVERAGE PLASMA CONCENTRATIONS (NG/ML) AND PHARMACOKINETIC PARAMETERS FOR PIPENDOXIFENE AFTER SINGLE ORAL ADMINISTRATION AT 50 MG/KG IN FEMALE 129S1 MICE (GROUP 1)**

| **Single Pipendoxifene Oral Dose (50 mg/kg; Plasma)** | | | | | |
|---|---|---|---|---|---|
| **Time (hr)** | **Mouse #** | **Conc. (ng/mL)** | **Mean (ng/mL)** | | **SD (ng/mL)** |
| 0.50 | 156 | 91.2 | 63.9 | | 28.2 |
| | 157 | 65.6 | | | |
| | 158 | 34.8 | | | |
| 1.0 | 156 | 170 | 89.1 | | 71.2 |
| | 157 | 61.3 | | | |
| | 158 | 36.0 | | | |
| 2.0 | 159 | 156 | 179 | | 37 |
| | 160 | 221 | | | |
| | 161 | 159 | | | |
| 4.0 | 159 | 133 | 107 | | 23 |
| | 160 | 95.7 | | | |
| | 161 | 92.4 | | | |
| 6.0 | 162 | 122 | **142** | | 30 |
| | 163 | 127 | | | |
| | 164 | 176 | | | |
| 8.0 | 162 | 52.8 | **78.5** | | 31.0 |
| | 163 | 69.8 | | | |
| | 164 | 113 | | | |
| 12 | 165 | 55.1 | **61.9** | | 12.0 |
| | 166 | 75.8 | | | |
| | 167 | 54.9 | | | |
| 24 | 165 | BLOQ | **1.12** | | NA |
| | 166 | 1.15 | | | |
| | 167 | 1.08 | | | |
| **Cₘₐₓ (ng/mL) ± SE** | | | | 179 ± 21 | |
| **tₘₐₓ (hr)** | | | | 2.0 | |
| **t_{1/2} (hr)** | | | | 2.36 | |
| **MRTₗₐₛₜ (hr)** | | | | 6.95 | |
| **AUCₗₐₛₜ (hr·ng/mL) ± SE** | | | | 1600 ± 115 | |
| **AUC_{∞} (hr·ng/mL)** | | | | 1602 | |
| **Dose-normalized Values¹** | | | | | |
| **AUCₗₐₛₜ (hr·kg·ng/mL/mg) ± SE** | | | | 32.0 | |
| **AUC_{∞} (hr·kg·ng/mL/mg)** | | | | 32.0 | |
| Cₘₐₓ: maximum plasma concentration; tₘₐₓ: time of maximum plasma concentration; t_{1/2}: half-life; time points used are in bold text; MRTₗₐₛₜ: mean residence time, calculated to the last observable time point; BLOQ: below limit of quantification (1 ng/mL); SE: standard error; AUCₗₐₛₜ: area under the curve, calculated to the last observable time point; AUC_{∞}: AUC extrapolated to ∞; ¹Dose-normalized by dividing the parameter by the nominal dose in mg/kg; NA: not applicable | | | | | |

**TABLE 18: INDIVIDUAL AND AVERAGE PLASMA CONCENTRATIONS (NG/ML) AND PHARMACOKINETIC PARAMETERS FOR PIPENDOXIFENE AFTER MULTIPLE ORAL ADMINISTRATION FOR FIVE CONSECUTIVE DAYS AT 50 MG/KG IN FEMALE 129S1 MICE (GROUP 1)**

| **Five Consecutive Days Pipendoxifene Oral Dose (50 mg/kg; Plasma)** | | | | | | |
|---|---|---|---|---|---|---|
| **Time (hr)** | **Mouse #** | **Conc. (ng/mL)** | **Mean (ng/mL)** | | **SD (ng/mL)** | |
| 0.50 | 168 | 48.7 | 51.8 | | | 6.7 |
| | 169 | 59.5 | | | | |
| | 170 | 47.1 | | | | |
| 1.0 | 168 | 38.7 | 56.0 | | | 20.7 |
| | 169 | 78.9 | | | | |
| | 170 | 50.3 | | | | |
| 2.0 | 171 | 120 | 110 | | | 11 |
| | 172 | 113 | | | | |
| | 173 | 97.9 | | | | |
| 4.0 | 171 | 111 | 86.2 | | | 23.0 |
| | 172 | 65.6 | | | | |
| | 173 | 82.1 | | | | |
| 6.0 | 174 | 163 | 178 | | | 15 |
| | 175 | 178 | | | | |
| | 176 | 192 | | | | |
| 8.0 | 174 | 102 | **82.1** | | | 18.1 |
| | 175 | 66.6 | | | | |
| | 176 | 77.6 | | | | |
| 12 | 177 | 44.3 | **47.0** | | | 7.0 |
| | 178 | 54.9 | | | | |
| | 179 | 41.8 | | | | |
| 24 | 177 | BLOQ | **1.95** | | | NA |
| | 178 | 2.81 | | | | |
| | 179 | 1.08 | | | | |
| **Cₘₐₓ (ng/mL) ± SE** | | | | 178 ± 8 | | |
| **tₘₐₓ (hr)** | | | | 6.0 | | |
| **t_{1/2} (hr)** | | | | 2.58 | | |
| **MRTₗₐₛₜ (hr)** | | | | 7.10 | | |
| **AUCₗₐₛₜ (hr·ng/mL) ± SE** | | | | 1391 ± 55 | | |
| **AUC_{∞} (hr·ng/mL)** | | | | 1396 | | |
| **Dose-normalized Values¹** | | | | | | |
| **AUCₗₐₛₜ (hr·kg·ng/mL/mg) ± SE** | | | | 27.8 | | |
| **AUC_{∞} (hr·kg·ng/mL/mg)** | | | | 27.9 | | |
| Cₘₐₓ: maximum plasma concentration; tₘₐₓ: time of maximum plasma concentration; t_{1/2}: half-life; time points used are in bold text; MRTₗₐₛₜ: mean residence time, calculated to the last observable time point; BLOQ: below limit of quantification (1 ng/mL); SE: standard error; AUCₗₐₛₜ: area under the curve, calculated to the last observable time point; AUC_{∞}: AUC extrapolated to ∞; ¹Dose-normalized by dividing the parameter by the nominal dose in mg/kg; NA: not applicable | | | | | | |

**TABLE 19: INDIVIDUAL AND AVERAGE LUNG TISSUE CONCENTRATIONS (NG/G) FOR PIPENDOXIFENE AFTER SINGLE ORAL ADMINISTRATION AT 50 MG/KG IN FEMALE 129S1 MICE (GROUP 1)**

| **Single Oral Pipendoxifene Dose (50 mg/kg; Lung Tissue)** | | | | | |
|---|---|---|---|---|---|
| **Time (hr)** | **Mouse #** | **Conc. (ng/g)** | **Mean (ng/g)** | | **SD (ng/g)** |
| 1.0 | 156 | 4250 | 2673 | | 1463 |
| | 157 | 2410 | | | |
| | 158 | 1360 | | | |
| 4.0 | 159 | 7680 | 8310 | | 630 |
| | 160 | 8940 | | | |
| | 161 | 8310 | | | |
| 8.0 | 162 | 5360 | 6920 | | 1358 |
| | 163 | 7560 | | | |
| | 164 | 7840 | | | |
| 24 | 165 | 241 | 203 | | 35 |
| | 166 | 193 | | | |
| | 167 | 174 | | | |
| **Cₘₐₓ (ng/g) ± SE** | | | | 8310 ± 364 | |
| **tₘₐₓ (hr)** | | | | 4.0 | |
| **t_{1/2} (hr)** | | | | 2ND | |
| **MRTₗₐₛₜ (hr)** | | | | 6.79 | |
| **AUCₗₐₛₜ (hr·ng/g) ± SE** | | | | 105253 ± 8124 | |
| **AUC_{∞} (hr·ng/g)** | | | | 2ND | |
| **Dose-normalized Values¹** | | | | | |
| **AUCₗₐₛₜ (hr·kg·ng/g/mg) ± SE** | | | | 2105 | |
| **AUC_{∞} (hr·kg·ng/g/mg)** | | | | 2ND | |
| Cₘₐₓ: maximum tissue concentration; tₘₐₓ: time of maximum tissue concentration; t_{1/2}: half-life; MRTₗₐₛₜ: mean residence time, calculated to the last observable time point; AUCₗₐₛₜ: area under the curve, calculated to the last observable time point; SE: standard error; ^{2ND}: not determined, not enough data points on terminal elimination phase; ¹Dose-normalized by dividing the parameter by the nominal dose in mg/kg | | | | | |

**TABLE 20: INDIVIDUAL AND AVERAGE LUNG TISSUE CONCENTRATIONS (NG/G) FOR PIPENDOXIFENE AFTER MULTIPLE ORAL ADMINISTRATION FOR FIVE CONSECUTIVE DAYS AT 50 MG/KG IN FEMALE 129S1 MICE (GROUP 1)**

| **Five Consecutive Days Oral Pipendoxifene Dose (50 mg/kg; Lung Tissue)** | | | | | |
|---|---|---|---|---|---|
| **Time (hr)** | **Mouse #** | **Conc. (ng/g)** | **Mean (ng/g)** | | **SD (ng/g)** |
| 1.0 | 168 | 919 | 1443 | | 454 |
| | 169 | 1700 | | | |
| | 170 | 1710 | | | |
| 4.0 | 171 | 5940 | 4803 | | 1040 |
| | 172 | 3900 | | | |
| | 173 | 4570 | | | |
| 8.0 | 174 | 3870 | 5613 | | 1602 |
| | 175 | 5950 | | | |
| | 176 | 7020 | | | |
| 24 | 177 | 220 | 288 | | 144 |
| | 178 | 454 | | | |
| | 179 | 191 | | | |
| **Cₘₐₓ (ng/g) ± SE** | | | | 5613 ± 925 | |
| **tₘₐₓ (hr)** | | | | 8.0 | |
| **t_{1/2} (hr)** | | | | ²ND | |
| **MRTₗₐₛₜ (hr)** | | | | 7.35 | |
| **AUCₗₐₛₜ (hr·ng/g) ± SE** | | | | 78138 ± 9521 | |
| **AUC_{∞} (hr·ng/g)** | | | | ²ND | |
| **Dose-normalized Values¹** | | | | | |
| **AUCₗₐₛₜ (hr·kg·ng/g/mg) ± SE** | | | | 1563 | |
| **AUC_{∞} (hr·kg·ng/g/mg)** | | | | ²ND | |
| Cₘₐₓ: maximum tissue concentration; tₘₐₓ: time of maximum tissue concentration; t_{1/2}: half-life; MRTₗₐₛₜ: mean residence time, calculated to the last observable time point; AUCₗₐₛₜ: area under the curve, calculated to the last observable time point; SE: standard error; ²ND: not determined, not enough data points on terminal elimination phase; ¹Dose-normalized by dividing the parameter by the nominal dose in mg/kg | | | | | |

**TABLE 21: INDIVIDUAL AND AVERAGE PLASMA CONCENTRATIONS (NG/ML) AND PHARMACOKINETIC PARAMETERS FOR PIPENDOXIFENE AFTER SINGLE ORAL ADMINISTRATION AT 250 MG/KG IN FEMALE 129S1 MICE (GROUP 2)**

| **Single Pipendoxifene Oral Dose (250 mg/kg; Plasma)** | | | | | |
|---|---|---|---|---|---|
| **Time (hr)** | **Mouse #** | **Conc. (ng/mL)** | **Mean (ng/mL)** | | **SD (ng/mL)** |
| 0.50 | 180 | 74.8 | 88.8 | | 14.1 |
| | 181 | 103 | | | |
| | 182 | 88.7 | | | |
| 1.0 | 180 | 100 | 195 | | 90 |
| | 181 | 205 | | | |
| | 182 | 279 | | | |
| 2.0 | 183 | 739 | 662 | | 114 |
| | 184 | 716 | | | |
| | 185 | 531 | | | |
| 4.0 | 183 | 380 | 393 | | 182 |
| | 184 | 581 | | | |
| | 185 | 217 | | | |
| 6.0 | 186 | 225 | **1020** | | 1200 |
| | 187 | 435 | | | |
| | 188 | 2400 | | | |
| 8.0 | 186 | 121 | **344** | | 259 |
| | 187 | 283 | | | |
| | 188 | 628 | | | |
| 12 | 189 | 570 | **628** | | 182 |
| | 190 | 832 | | | |
| | 191 | 483 | | | |
| 24 | 189 | 8.62 | **33.6** | | 46.4 |
| | 190 | 87.2 | | | |
| | 191 | 5.05 | | | |
| **Cₘₐₓ (ng/mL) ± SE** | | | | 1020 ± 693 | |
| **tₘₐₓ (hr)** | | | | 6.0 | |
| **t_{1/2} (hr)** | | | | 3.96 | |
| **MRTₗₐₛₜ (hr)** | | | | 8.86 | |
| **AUCₗₐₛₜ (hr·ng/mL) ± SE** | | | | 10269 ± 2101 | |
| **AUC_{∞} (hr·ng/mL)** | | | | 10461 | |
| **Dose-normalized Values¹** | | | | | |
| **AUCₗₐₛₜ (hr·kg·ng/mL/mg) ± SE** | | | | 41.1 | |
| **AUC_{∞} (hr·kg·ng/mL/mg)** | | | | 41.8 | |
| Cₘₐₓ: maximum plasma concentration; tₘₐₓ: time of maximum plasma concentration; t_{1/2}: half-life; time points used are in bold text; MRTₗₐₛₜ: mean residence time, calculated to the last observable time point; BLOQ: below limit of quantification (1 ng/mL); SE: standard error; AUCₗₐₛₜ: area under the curve, calculated to the last observable time point; AUC_{∞}: AUC extrapolated to ∞; ¹Dose-normalized by dividing the parameter by the nominal dose in mg/kg | | | | | |

**TABLE 22: INDIVIDUAL AND AVERAGE PLASMA CONCENTRATIONS (NG/ML) AND PHARMACOKINETIC PARAMETERS FOR PIPENDOXIFENE AFTER MULTIPLE ORAL ADMINISTRATION FOR FIVE CONSECUTIVE DAYS AT 250 MG/KG IN FEMALE 129S1 MICE (GROUP 2)**

| **Five Consecutive Days Pipendoxifene Oral Dose (250 mg/kg; Plasma)** | | | | | |
|---|---|---|---|---|---|
| **Time (hr)** | **Mouse #** | **Conc. (ng/mL)** | **Mean (ng/mL)** | | **SD (ng/mL)** |
| 0.50 | 192 | 199 | 147 | | 49 |
| | 193 | 138 | | | |
| | 194 | 103 | | | |
| 1.0 | 192 | 348 | 225 | | 106 |
| | 193 | 168 | | | |
| | 194 | 160 | | | |
| 2.0 | 195 | 667 | 739 | | 214 |
| | 196 | 570 | | | |
| | 197 | 980 | | | |
| 4.0 | 195 | 497 | 635 | | 303 |
| | 196 | 426 | | | |
| | 197 | 982 | | | |
| 6.0 | 198 | 818 | 1050 | | 399 |
| | 199 | 1510 | | | |
| | 200 | 821 | | | |
| 8.0 | 198 | 656 | **575** | | 194 |
| | 199 | 716 | | | |
| | 200 | 354 | | | |
| 12 | 201 | 801 | **640** | | 166 |
| | 202 | 651 | | | |
| | 203 | 469 | | | |
| 24 | 201 | 13.0 | **9.89** | | 3.94 |
| | 202 | 11.2 | | | |
| | 203 | 5.46 | | | |
| **Cₘₐₓ (ng/mL) ± SE** | | | | 1050 ± 230 | |
| **tₘₐₓ (hr)** | | | | 6.0 | |
| **t_{1/2} (hr)** | | | | 2.52 | |
| **MRTₗₐₛₜ (hr)** | | | | 8.32 | |
| **AUCₗₐₛₜ (hr·ng/mL) ± SE** | | | | 11628 ± 1191 | |
| **AUC_{∞} (hr·ng/mL)** | | | | 11664 | |
| **Dose-normalized Values¹** | | | | | |
| **AUCₗₐₛₜ (hr·kg·ng/mL/mg) ± SE** | | | | 46.5 | |
| **AUC_{∞} (hr·kg·ng/mL/mg)** | | | | 46.7 | |
| Cₘₐₓ: maximum plasma concentration; tₘₐₓ: time of maximum plasma concentration; t_{1/2}: half-life; time points used are in bold text; MRTlast: mean residence time, calculated to the last observable time point; BLOQ: below limit of quantification (1 ng/mL); SE: standard error; AUCₗₐₛₜ: area under the curve, calculated to the last observable time point; AUC_{∞}: AUC extrapolated to ∞; ¹Dose-normalized by dividing the parameter by the nominal dose in mg/kg | | | | | |

**TABLE 23: INDIVIDUAL AND AVERAGE LUNG TISSUE CONCENTRATIONS (NG/G) FOR PIPENDOXIFENE AFTER SINGLE ORAL ADMINISTRATION AT 250 MG/KG IN FEMALE 129S1 MICE (GROUP 2)**

| **Single Oral Pipendoxifene Dose (250 mg/kg; Lung Tissue)** | | | | |
|---|---|---|---|---|
| **Time (hr)** | **Mouse #** | **Conc. (ng/g)** | **Mean (ng/g)** | **SD (ng/g)** |
| 1.0 | 180 | 2900 | 5223 | 2028 |
| | 181 | 6130 | | |
| | 182 | 6640 | | |
| 4.0 | 183 | 22400 | 23067 | 7722 |
| | 184 | 31100 | | |
| | 185 | 15700 | | |
| 8.0 | 186 | 10200 | 24733 | 17321 |
| | 187 | 20100 | | |
| | 188 | 43900 | | |
| 24 | 189 | 4610 | 3888 | 2853 |
| | 190 | 6310 | | |
| | 191 | 743 | | |
| **Cₘₐₓ (ng/g) ± SE** | | | | 24733 ± 10000 |
| **tₘₐₓ (hr)** | | | | 8.0 |
| **t_{1/2} (hr)** | | | | ²ND |
| **MRTₗₐₛₜ (hr)** | | | | 8.27 |
| **AUCₗₐₛₜ (hr·ng/g) ± SE** | | | | 369615 ± 102095 |
| **AUC_{∞} (hr·ng/g)** | | | | ²ND |
| **Dose-normalized Values¹** | | | | |
| **AUCₗₐₛₜ (hr·kg·ng/g/mg) ± SE** | | | | 1478 |
| **AUC_{∞} (hr·kg·ng/g/mg)** | | | | ²ND |
| Cₘₐₓ: maximum tissue concentration; tₘₐₓ: time of maximum tissue concentration; t_{1/2}: half-life; MRTₗₐₛₜ: mean residence time, calculated to the last observable time point; AUCₗₐₛₜ: area under the curve, calculated to the last observable time point; SE: standard error; ²ND: not determined, not enough data points on terminal elimination phase; ¹Dose-normalized by dividing the parameter by the nominal dose in mg/kg | | | | |

**TABLE 24: INDIVIDUAL AND AVERAGE LUNG TISSUE CONCENTRATIONS (NG/G) FOR PIPENDOXIFENE AFTER MULTIPLE ORAL ADMINISTRATION FOR FIVE CONSECUTIVE DAYS AT 250 MG/KG IN FEMALE 129S1 MICE (GROUP 2)**

| **Five Consecutive Days Oral Pipendoxifene Dose (250 mg/kg; Lung Tissue)** | | | | | |
|---|---|---|---|---|---|
| **Time (hr)** | **Mouse #** | **Conc. (ng/g)** | **Mean (ng/g)** | | **SD (ng/g)** |
| 1.0 | 192 | 8240 | 5877 | | 2051 |
| | 193 | 4570 | | | |
| | 194 | 4820 | | | |
| 4.0 | 195 | 42600 | 39000 | | 15321 |
| | 196 | 22200 | | | |
| | 197 | 52200 | | | |
| 8.0 | 198 | 37500 | 35267 | | 6926 |
| | 199 | 40800 | | | |
| | 200 | 27500 | | | |
| 24 | 201 | 2520 | 1770 | | 684 |
| | 202 | 1610 | | | |
| | 203 | 1180 | | | |
| **Cₘₐₓ (ng/g) ± SE** | | | | 39000 ± 8845 | |
| **tₘₐₓ (hr)** | | | | 4.0 | |
| **t_{1/2} (hr)** | | | | ²ND | |
| **MRTₗₐₛₜ (hr)** | | | | 7.22 | |
| **AUCₗₐₛₜ (hr·ng/g) ± SE** | | | | 515080 ± 50723 | |
| **AUC_{∞} (hr·ng/g)** | | | | ²ND | |
| **Dose-normalized Values¹** | | | | | |
| **AUCₗₐₛₜ (hr·kg·ng/g/mg) ± SE** | | | | 2060 | |
| **AUC_{∞} (hr·kg·ng/g/mg)** | | | | ²ND | |
| Cₘₐₓ: maximum tissue concentration; tₘₐₓ: time of maximum tissue concentration; t_{1/2}: half-life; MRTₗₐₛₜ: mean residence time, calculated to the last observable time point; AUCₗₐₛₜ: area under the curve, calculated to the last observable time point; SE: standard error; ²ND: not determined, not enough data points on terminal elimination phase; ¹Dose-normalized by dividing the parameter by the nominal dose in mg/kg | | | | | |

**TABLE 25: AVERAGE PLASMA AND LUNG TISSUE CONCENTRATION OF PIPENDOXIFENE VS TIME (GROUP 1 & 2)**

| Time (hr) | **Group 1, SD (50 mg/kg Pipendoxifene)** | | **Group 1, MD (50 mg/kg Pipendoxifene)** | | **Group 2, SD (250 mg/kg Pipendoxifene)** | | **Group 2, MD (250 mg/kg Pipendoxifene)** | |
|---|---|---|---|---|---|---|---|---|
| | **Plasma (ng/mL)** | **Lung (ng/g)** | **Plasma (ng/mL)** | **Lung (ng/g)** | **Plasma (ng/mL)** | **Lung (ng/g)** | **Plasma (ng/mL)** | **Lung (ng/g)** |
| 0.5 | 63.9±28.2 | NA | 51.8±6.7 | NA | 88.8±14.1 | NA | 147±49 | NA |
| 1.0 | 89.1±71.2 | 2673± 1463 | 56.0±20.7 | 1443±454 | 195±90 | 5223±2028 | 225±106 | 5877±2051 |
| 2.0 | 179±37 | NA | 110±11 | NA | 662±114 | NA | 739±214 | NA |
| 4.0 | 107±23 | 8310±630 | 86.2±23.0 | 4803±1040 | 393±182 | 23067±7722 | 635±303 | 39000±15321 |
| 6.0 | 142±30 | NA | 178±15 | NA | 1020±1200 | NA | 1050±399 | NA |
| 8.0 | 78.5±31.0 | 6920±1358 | 82.1±18.1 | 5613±1602 | 344±259 | 24733±17321 | 575±194 | 35267±6926 |
| 12 | 61.9±12.0 | NA | 47.0±7.0 | NA | 628±182 | NA | 640±166 | NA |
| 24 | 1.12 | 203±35 | 1.95 | 288± 144 | 33.6±46.4 | 3888± 2853 | 9.89±3.94 | 1770± 684 |
| SD: Single dose; MD: Multiple, 5-day, dose; NA: Not applicable | | | | | | | | |

### DOSING SOLUTION PREPARATION

For pipendoxifene, 60 mL of 0.5% Methyl cellulose (MC) in water was prepared three days before use by dissolving 0.3 g of MC in 57 mL of water. Described below are the preparations for dosing solution on each treatment day for each group (Also, see Table 26 and Table 27 below).

### Day 1

### Group 1 (PO): 5 mg/mL Pipendoxifene in 0.5% MC in water

7.81 mL of 0.5% MC in water was added to 40.24 mg of pipendoxifene powder, sonicated for 30 minutes in a bath sonicator to result in suspension.

### Group 2 (PO): 25 mg/mL Pipendoxifene in 0.5% MC in water

7.57 mL of 0.5% MC in water was added to 195.02 mg of pipendoxifene powder, sonicated for 30 minutes in a bath sonicator to result in suspension.

### Day 2

### Group 1 (PO): 5 mg/mL Pipendoxifene in 0.5% MC in water

4.53 mL of 0.5% MC in water was added to 23.37 mg of pipendoxifene powder, sonicated for 30 minutes in a bath sonicator to result in suspension.

### Group 2 (PO): 25 mg/mL Pipendoxifene in 0.5% MC in water

4.06 mL of 0.5% MC in water was added to 104.61 mg of pipendoxifene powder, sonicated for 30 minutes in a bath sonicator to result in suspension.

### Day 3

### Group 1 (PO): 5 mg/mL Pipendoxifene in 0.5% MC in water

4.56 mL of 0.5% MC in water was added to 23.51 mg of pipendoxifene powder, sonicated for 30 minutes in a bath sonicator to result in suspension.

### Group 2 (PO): 25 mg/mL Pipendoxifene in 0.5% MC in water

3.93 mL of 0.5% MC in water was added to 101.38 mg of pipendoxifene powder, sonicated for 30 minutes in a bath sonicator to result in suspension.

### Day 4

### Group 1 (PO): 5 mg/mL Pipendoxifene in 0.5% MC in water

4.60 mL of 0.5% MC in water was added to 23.69 mg of pipendoxifene powder, sonicated for 30 minutes in a bath sonicator to result in suspension.

### Group 2 (PO): 25 mg/mL Pipendoxifene in 0.5% MC in water

3.94 mL of 0.5% MC in water was added to 101.53 mg of pipendoxifene powder, sonicated for 30 minutes in a bath sonicator to result in suspension.

### Day 5

### Group 1 (PO): 5 mg/mL Pipendoxifene in 0.5% MC in water

4.13 mL of 0.5% MC in water was added to 21.30 mg of pipendoxifene powder, sonicated for 30 minutes in a bath sonicator to result in suspension.

### Group 2 (PO): 25 mg/mL Pipendoxifene in 0.5% MC in water

3.97 mL of 0.5% MC in water was added to 102.41 mg of pipendoxifene powder, sonicated for 30 minutes in a bath sonicator to result in suspension.

**TABLE 26: DOSING SOLUTION PREPARATION**

| | |
|---|---|
| *Species (N=)* | Female 129S1 mice, N=24 per group; total N = 96 |
| *Test Article(s)* | Pipendoxifene (97% pure) |
| *Parent*/*Salt* | NA |
| *Molecular weight* | 456.58 |
| *Formula weight* | 456.58 |
| *Administration Route* | All PO, single dose or multiple dose (5 days dose) |
| *Nominal Dose* | Group 1: 50 mg/kg; Group 2: 250 mg/kg |
| *Nominal Dosing Volume* | 10 mL/kg |
| *Nominal Concentration* | Group 1: 5 mg/mL; Group 2: 25 mg/mL |
| *Dosing Vehicle* | 0.5% MC in water |
| *Preparation* | Prepared each day of dosing |
| *Storage Conditions* | Stored remaining dosing solution frozen at -80 °C |

**TABLE 27: MATERIALS LIST**

| *Material Name* | *Supplier* | *Cat#* |
|---|---|---|
| Methyl cellulose, Visc. 4000 cPs (MC) | Sigma | M0512-100G |
| De-ionized water | milli-Q in house | NA |
| *Equipment List* | | |
| Balance | | |
| Bath sonicator | | |
| 10 mL pipette | | |

### Example 5

### Analysis of pipendoxifene in an in vivo model of SARS-CoV-2 infection

Provided in this example is analysis of pipendoxifene in *in vivo* models of SARS-CoV-2. Mice are not normally susceptible to infection by SARS-CoV-2 since the murine ACE2 protein, which is used by the virus as a receptor for cell entry, is different from the human analogue, hACE2. Therefore, SARS-CoV-2 animal models for antiviral efficacy experiments were used.

A variant of virus (termed MA-SARS-CoV-2) was obtained after series of passaging in different backgrounds of laboratory mice as well as mACE-2 expressing VeroE6 cells. Briefly, the virus was serially passaged every 2 days via intranasal inoculation of the virus in 50 µl volume derived from the spun-down supernatants of lung homogenates. The mouse adaptation of the SARS-CoV-2 variant was studied in C57Bl6, BALB/c and 129S1/SVMJ (termed 129 for simplicity) mice models. Viral stocks were sequenced after propagation to verify the integrity of the original viral genome.

### Materials and Methods

All the antiviral studies were performed in animal biosafety level 3 (BSL3) facility at the Icahn School of Medicine in Mount Sinai Hospital, New York City. All work was conducted under protocols approved by the Institutional Animal Care and Use Committee

### (IACUC).

Female 10-week-old specific pathogen-free 129 mice (the Jackson laboratory strain 002448) were utilized. The infected pipendoxifene, vehicle, and remdesivir groups each had 6 mice. Remdesivir were administrated subcutaneously (S.C.), and pipendoxifene and vehicle were administered by oral gavage (P.O.) once per day for 3 days. Dosage levels were as follows; Group 1: 66.67 mg/kg pipendoxifene; Group 2: Vehicle, Group 3: 50 mg/kg remdesivir. The first dose of all treatments was administered 1 hour before (prophylactic) intranasal infection with 2.5 × 10⁴ PFU of MA-SARS-CoV-2 in 50 µl of PBS. Mice were anesthetized with a mixture of ketamine/xylazine before each intranasal infection. Mice were weighed daily for signs of pathogenesis.

### Results

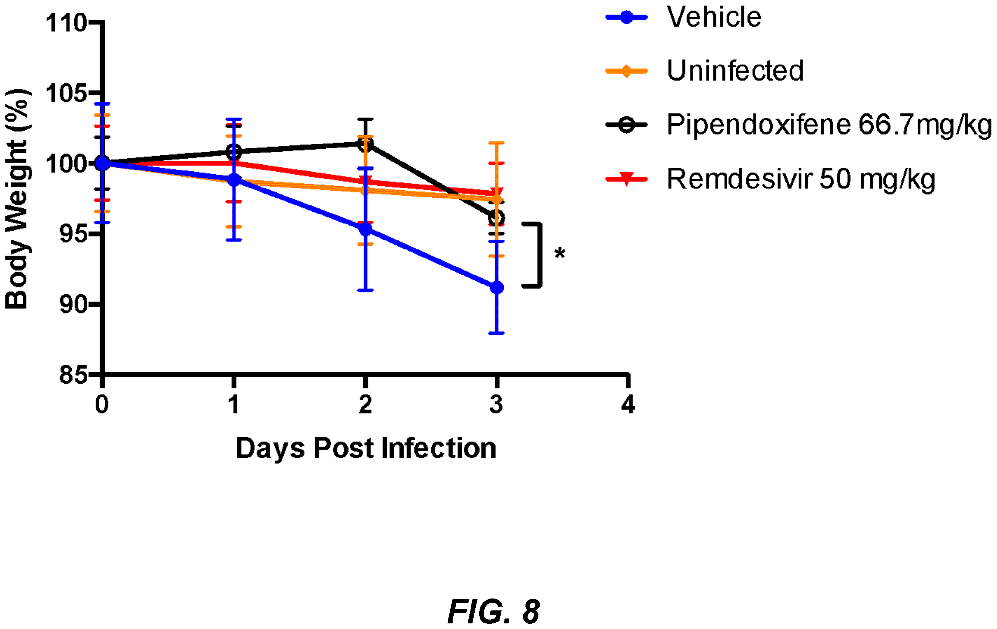

The *in vivo* efficacy of 66.67 mg/kg pipendoxifene in an established mouse-adapted animal model of SARS-CoV-2 infection was tested. First, a variant of SARS-CoV-2 (termed MA-SARS-CoV-2) was obtained after series of passaging in different backgrounds of laboratory mice as well as mACE-2 expressing VeroE6 cells. 129/S mice were dosed prophylactically with pipendoxifene once per day (QD) PO, vehicle, or 50 mg/kg of remdesivir twice per day (BID) SC starting 1 hour prior to infection with MA-SARS-CoV-2. Animal weight was monitored daily for signs of pathogenesis. Vehicle treated mice lost ~10% of body weight over the 3 day experiment, similar to previous results in this model. Treatment with pipendoxifene partially protected mice from MA-SARS-CoV-2 associated weight loss at both day 2 and day 3 post infection, similar to the remdesivir group (FIG. 8).

### Discussion

Unexpectedly, pipendoxifene had a significant impact on SARS-CoV-2 associated weight loss at day 2 and 3 post infection, indicating efficacy. It was found that pipendoxifene protected mice against the primary endpoint of disease measured in the study (weight loss) in a statistically significant manner (p<0.05) and that once a day, oral administration of pipendoxifene at 66.67mg/kg was non-inferior to twice a day, sub-Q administration of remdesivir at 50mg/kg.

### Example 6

### Analysis of 250 mg/kg dosage of pipendoxifene in an in vivo model of SARS-CoV-2 infection

In view of the unexpected results of the pharmacokinetics study above, a higher dose of pipendoxifene was justified and therefore tested in this Example. This example provides additional analysis of pipendoxifene in *in vivo* models of SARS-CoV-2. Mice are not normally susceptible to infection by SARS-CoV-2 since the murine ACE2 protein, which is used by the virus as a receptor for cell entry, is different from the human analogue, hACE2. Therefore, a mouse-adapted SARS-CoV-2 animal model was used for antiviral efficacy experiments.

SARS-CoV-2 isolate USA-WA1/2020 (BEI resources; NR-52281), referred to herein as WT-SARS-CoV-2. A variant of virus (termed MA-SARS-CoV-2) was obtained after series of passaging in different backgrounds of laboratory mice as well as mACE-2 expressing VeroE6 cells. Briefly, the virus was serially passaged every 2 days via intranasal inoculation of the virus in 50 µl volume derived from the spun-down supernatants of lung homogenates. The mouse adaptation of the SARS-CoV-2 variant was studied in C57Bl6, BALB/c and 129S1/SVMJ (termed 129/S for simplicity) mice models. Viral stocks were sequenced after propagation to verify the integrity of the original viral genome. In this mouse model, robust SAR-CoV-2 replication occurs in the lungs, which leads to ~10% weight loss and can be detected in a plaque assay or TCID50 assay.

### Materials and Methods

All the antiviral studies were performed in animal biosafety level 3 (BSL3) facility at the Icahn School of Medicine in Mount Sinai Hospital, New York City. All work was conducted under protocols approved by the Institutional Animal Care and Use Committee

### (IACUC).

Female 10-week-old specific pathogen-free 129 mice (the Jackson laboratory strain 002448) were utilized. The infected pipendoxifene, vehicle, and remdesivir groups each had 11 mice. Six mice were euthanized for lung harvest on day 3 post infection, while the remaining 5 mice were monitored for weight until they recovered from SARS-CoV-2 associated weight loss (Table 28A-Table 28C). Remdesivir was administrated subcutaneously (S.C.) twice per day (BID) for 3 days; and pipendoxifene (0.5% methylcellulose), and vehicle were administered by oral gavage (P.O.) once per day (QD) for 6-8 days. Dosage levels were as follows; Group 1: 66.67 mg/kg pipendoxifene, Group 2: 250 mg/kg pipendoxifene, Group 3: 100 mg/kg remdesivir, Group 4: Vehicle (0.5% methylcellulose). The first dose of all treatments was administered 3 days or 1 hour before (prophylactic) intranasal infection with 2.5 × 10⁴ PFU of MA-SARS-CoV-2 in 50 µl of PBS. Mice were anesthetized with a mixture of ketamine/xylazine before each intranasal infection. Mice were weighed daily for signs of pathogenesis. 3 days post infection (dpi), 6 animals per group were humanely euthanized. The right whole lung of each mouse was harvested and inactivated in 5 µl of acetonitrile per gram of tissue in PBS and homogenized, then frozen at -80°C for later analysis. The right whole lung of each mouse was harvested and homogenized in PBS with silica glass beads then frozen at -80°C for viral titration via TCID₅₀. Infectious titers were quantified by limiting dilution titration using Vero-TMPRSS2 cells. Briefly, Vero-TMPRSS2 cells were seeded in 96-well plates at 20,000 cells/well. The next day, SARS-CoV-2-containing supernatant was applied at serial 10-fold dilutions ranging from 10⁻¹ to 10⁻⁸ and, after 5 days, viral cytopathic effect (CPE) was detected by staining cell monolayers with crystal violet. TCID₅₀/ml were calculated using the method of Reed and Muench. The Prism software (GraphPad) was used to determine differences in lung titers using T tests on log transformed data. Blood was harvested by cheek bleed on day 3 post-infection and UV inactivated for later PK analysis.

**TABLE 28A: ANTIVIRAL EFFICACY STUDY OF PIPENDOXIFENE IN THE MOUSE-ADAPTED MODEL OF SARS-COV-2 INFECTION**

| | | | | Friday | Saturday | Sunday |
|---|---|---|---|---|---|---|
| Group | Dose Level | Cohort | N | Day -3 | Day -2 | Day -1 |
| | mg/kg | | | | | |
| 1 | 66.7 Pipendoxifene PO QD | 1 | 6 | D | D | D |
| | | 2 | 5 | D | D | D |
| 2 | 250 Pipendoxifene PO QD | 1 | 6 | D | D | D |
| | | 2 | 5 | D | D | D |
| 3 | 100 Remdesivir SC BID | 1 | 6 | -- | -- | -- |
| | | 2 | 5 | -- | -- | -- |
| 4 | 0 Vehicle Controls PO QD | 1 | 6 | D | D | D |
| | | 2 | 5 | D | D | D |
| | infected groups (Cohort 1) - Lungs (1 Titers + 1 Acetonitrile) + Blood Day 3 | | | | | |
| | infected group (Cohort 2)- Followed for weight | | | | | |
| Legend: | | | | | | |
| D: Drug or vehicle dose administered, multiple routes, multiple schedules | | | | | | |
| I: Inoculate (intranasally) mice with MA-SARS-CoV-2 (2.5e4 PFU)/per mouse | | | | | | |
| E: euthanize, lung collected for viral titer | | | | | | |
| Design: (sac Day 3 post-infection) | | | | | | |
| Cohort 1 (Groups 1-4) (infected) will be dosed with drug or vehicle from Day 0 to Day 2, euthanized Day 3. | | | | | | |
| Cohort 2 (Groups 1-4) (infected) will be dosed with drug or vehicle from Day 0 to Day 4. | | | | | | |

**TABLE 28B: ANTIVIRAL EFFICACY STUDY OF PIPENDOXIFENE IN THE MOUSE-ADAPTED MODEL OF SARS-COV-2 INFECTION**

| | | | | Monday | Tuesday | Wednesday |
|---|---|---|---|---|---|---|
| Group | Dose Level | Cohort | N | Day 0 | Day 1 | Day 2 |
| | mg/kg | | | | | |
| | | | | | | |
| 1 | 66.7 Pipendoxifene PO QD | 1 | 6 | D, I | D | D |
| | | 2 | 5 | D, I | D | D |
| 2 | 250 Pipendoxifene PO QD | 1 | 6 | D, I | D | D |
| | | 2 | 5 | D, I | D | D |
| 3 | 100 Remdesivir SC BID | 1 | 6 | D, I | D | D |
| | | 2 | 5 | D, I | D | D |
| 4 | 0 Vehicle Controls PO QD | 1 | 6 | D, I | D | D |
| | | 2 | 5 | D, I | D | D |
| | infected groups (Cohort 1) - Lungs (1 Titers + 1 Acetonitrile) + Blood Day 3 | | | | | |
| | infected group (Cohort 2)- Followed for weight | | | | | |
| Legend: | | | | | | |
| D: Drug or vehicle dose administered, multiple routes, multiple schedules | | | | | | |
| I: Inoculate (intranasally) mice with MA-SARS-CoV-2 (2.5e4 PFU)/per mouse | | | | | | |
| E: euthanize, lung collected for viral titer | | | | | | |
| Design: (sac Day 3 post-infection) | | | | | | |
| Cohort 1 (Groups 1-4) (infected) will be dosed with drug or vehicle from Day 0 to Day 2, euthanized Day 3. | | | | | | |
| Cohort 2 (Groups 1-4) (infected) will be dosed with drug or vehicle from Day 0 to Day 4. | | | | | | |

**TABLE 28C: ANTIVIRAL EFFICACY STUDY OF PIPENDOXIFENE IN THE MOUSE-ADAPTED MODEL OF SARS-COV-2 INFECTION**

| | | | | Thursday | Friday | Saturday |
|---|---|---|---|---|---|---|
| Group | Dose Level | Cohort | N | Day 3 | Day 4 | Day 5 |
| | mg/kg | | | | | |
| 1 | 66.7 Pipendoxifene PO QD | 1 | 6 | E | -- | -- |
| | | 2 | 5 | D | D | -- |
| 2 | 250 Pipendoxifene PO QD | 1 | 6 | E | -- | -- |
| | | 2 | 5 | D | D | -- |
| 3 | 100 Remdesivir SC BID | 1 | 6 | E | -- | -- |
| | | 2 | 5 | D | D | -- |
| 4 | 0 Vehicle Controls PO QD | 1 | 6 | E | -- | -- |
| | | 2 | 5 | D | D | -- |
| | infected groups (Cohort 1) - Lungs (1 Titers + 1 Acetonitrile) + Blood Day 3 | | | | | |
| | infected group (Cohort 2)- Followed for weight | | | | | |
| Legend: | | | | | | |
| D: Drug or vehicle dose administered, multiple routes, multiple schedules | | | | | | |
| I: Inoculate (intranasally) mice with MA-SARS-CoV-2 (2.5e4 PFU)/per mouse | | | | | | |
| E: euthanize, lung collected for viral titer | | | | | | |
| Design: (sac Day 3 post-infection) | | | | | | |
| Cohort 1 (Groups 1-4) (infected) will be dosed with drug or vehicle from Day 0 to Day 2, euthanized Day 3. | | | | | | |
| Cohort 2 (Groups 1-4) (infected) will be dosed with drug or vehicle from Day 0 to Day 4. | | | | | | |

### Results

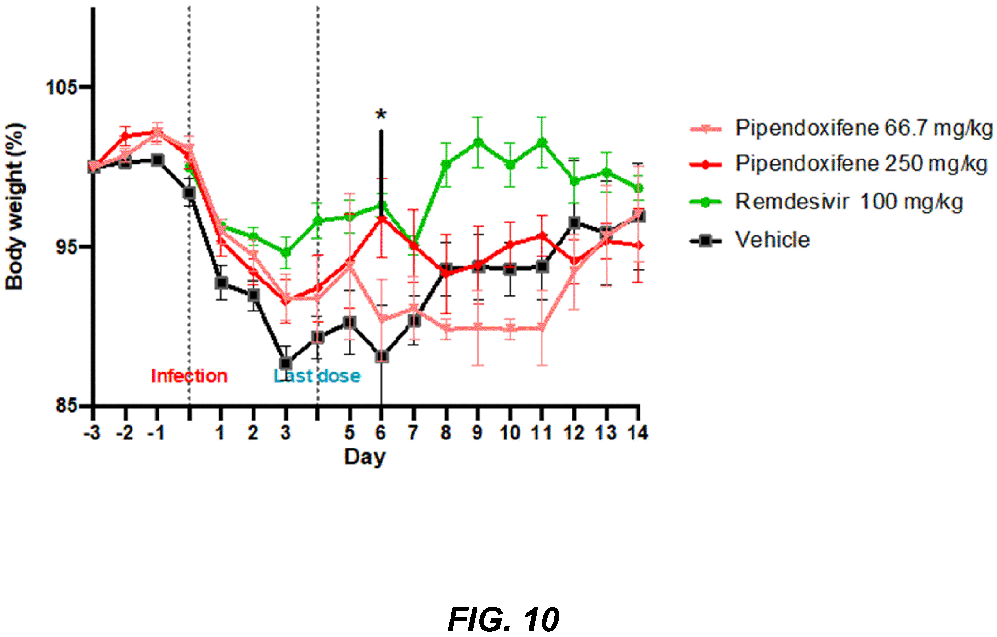

The *in vivo* efficacy of 66.67 or 250 mg/kg pipendoxifene in an established mouse-adapted animal model of SARS-CoV-2 infection was tested. 129/S mice were dosed prophylactically with pipendoxifene QD PO starting 3 days prior to infection with MA-SARS-CoV-2. Lungs were harvested on Day 3 for MA-SARS-CoV-2 lung titers and were quantified by TCID₅₀ assay for the pipendoxifene group and compared to vehicle, and remdesivir controls (FIG. 9). A 3-log reduction in lung titers was observed from remdesivir. Pipendoxifene treatment at 66.67 mg/kg did not have an impact on viral titers. The 250 mg/kg pipendoxifene group reduced viral lung titers by nearly 1 log, which was statistically significant. Animal weight was monitored daily for signs of pathogenesis. Vehicle treated mice lost ~10% of body weight over the experiment, similar to previous results in this model. Treatment with pipendoxifene protected mice from MA-SARS-CoV-2 associated weight loss, similar to the remdesivir group (FIG. 10). This effect was dose-dependent, with the 250 mg/kg pipendoxifene group achieving a statistically significant improvement on day 6 post infection.

### Discussion

Pipendoxifene was able to ameliorate SARS-CoV-2 associated weight loss in a dose dependent manner, which was statistically significant at day 6 post infection in the 250 mg/kg dosage group. The 250 mg/kg pipendoxifene treatment was also associated with a nearly 1 log reduction in viral lung titers compared to vehicle controls, which was also statistically significant. These results indicate that Pipendoxifene shows antiviral properties *in vivo* and can improve SARS-CoV-2 associated pathogenesis in this mouse-adapted SARS-CoV-2 mouse model. This data also indicates that Pipendoxifene is an excellent potential candidate as both a mono and combination therapy with multiple drugs.

### Example 7

### LC/MS/MS Method Development for the Determination of Pipendoxifene in K2EDTA Mouse Plasma and Lung

### Study Objectives

An extraction and LC/MS/MS method was developed to analyze study samples for Pipendoxifene in mouse plasma and lung. An aliquot of the extract was injected onto a LC/MS/MS triple quadrupole mass spectrometer (API4000 MS/MS). An HSC18, 3 µm LC column (2.1 x 50 mm) from Supelco was used to separate Pipendoxifene from interfering compounds that may be present in the sample. No stable label or analog compounds were available to use as internal standards so Berzosertib was used as the internal standard for the Pipendoxifene analysis. The peak area of the product ion of the compound was measured against the peak area of the product ion of the internal standard. A calibration curve ranging from 1.00 to 500 ng/mL (eight concentrations in duplicate) was be used to quantify the compounds in the plasma samples (see Example above). A calibration curve ranging from 1.00 to 500 ng/g (eight concentrations in duplicate) was be used to quantify the compounds in the lung samples (see Example above).

### Conclusions

The data indicates that the developed method is accurate and precise and can be used to analyze study samples (see Example above).

### Example 8

### Docking Study

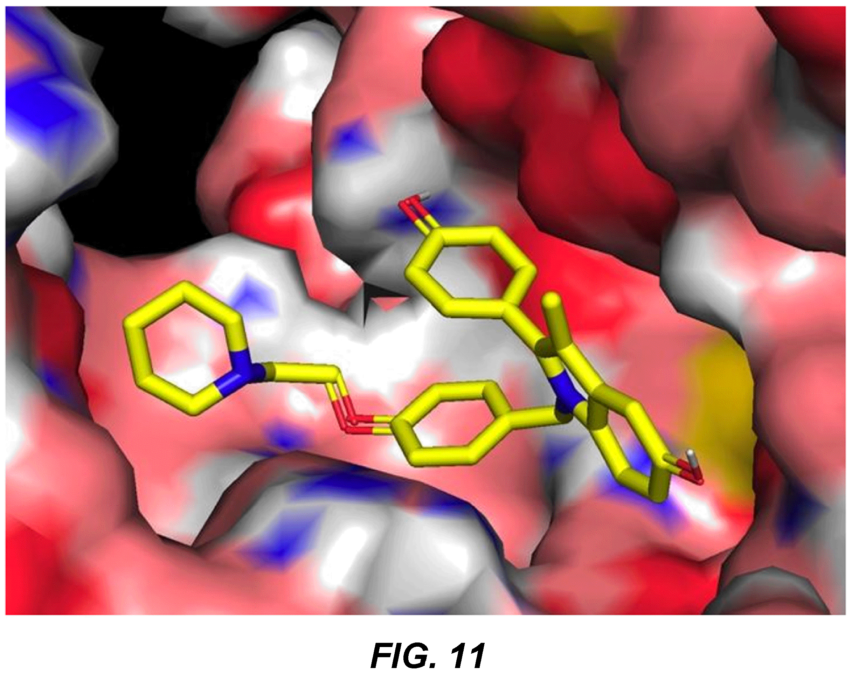

The interactions of MDL-001 to the target protein RdRp were explored through a docking study. Several representations of RdRp were pulled from the Protein Data Bank (PDB) for use in this study. MDL-001 was found to bind to the polymerase active site in PDB-ID: 7b3b. In docking experiments with the active site structure in 7b3d, it was observed that MDL-001 occupies an extended surface area of the target protein, establishing contacts with residues ARG555, ASP452, ARG624, CYS622, THR556, LYS621, TYR619, ASP618, ARG553 of the target. MDL-001 appeared to form 3 hydrogen bonds with RdRp, where the hydroxyl group of the indole ring forms hydrogen bonding interactions with ARG858 of RdRp. There were also extended polar interactions with residues ARG836, ILE548, LYS545, ALA547, ARG858, ASP845 of the target protein along with the polar interactions with the 4 nucleotide bases. In PDB 7bv2, MDL-001 has polar interactions with the residue ASN496 and forms 2 hydrogen bonds with the product and template RNA nucleotide bases. MDL-001 has interactions with the RNA strands and a polar interaction with ASN496. In PDB 7l1f, MDL-001 has interactions with residues ARG624, ARG555, LYS621, ASN691, CYS622, ASP760, ASP618, ARG553, ASP623 of the target protein. FIG. 11 depicts non-limiting exemplary docking study data related to the interactions of MDL-001 with the target protein RdRp. The top docking pose of MDL-001 (yellow) in the SARS-CoV-2 RdRp (PDB-ID: 7L1F) active site binding pocket is depicted. Protein is represented by a colored surface, where C atoms are white, O atoms are red, N atoms are blue, and S atoms are yellow.

### Compounds of Formula (I), Formula (II), and Formula (III)

There are provided, in some embodiments, compounds of Formula (I), Formula (II), and Formula (III). In some embodiments, compounds of Formula (I), Formula (II), or Formula (III) are derived from the results of the docking study described herein. In some embodiments, a compound of the disclosure is generated from the scaffold of a compound of Formula (I), Formula (II), or Formula (III). Cis and trans versions of a compound of Formula (I), Formula (II), or Formula (III) are contemplated therein. In some embodiments, the compound of Formula I, Formula II, or Formula III is or comprises Pipendoxifene.

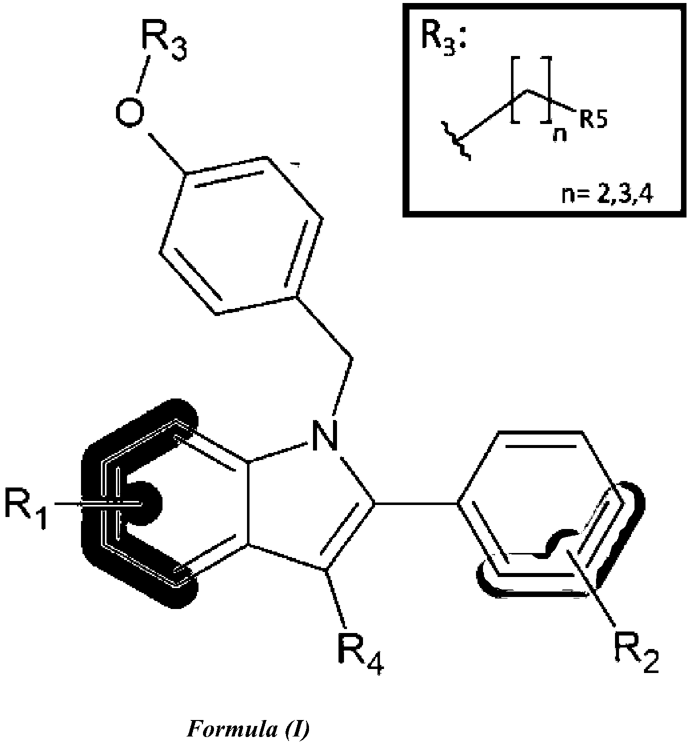

**Formula (I) (e.g., Scaffold 1): (R₁, R₂)** In some embodiments, each of R₁ and R₂ is independently a hydrogen, a halogen atom, a hydroxyl group, a sulfhydryl group, an amino group, an amide group, a carboxyl group, a carbonyl group, a alkoxyalkyl group, or a C1-C3 alkyl group which may be substituted with a halogen atom, a hydroxyl group, a sulfhydryl group, an amino group, an amide group, a carboxyl group, a carbonyl group, or a alkoxyalkyl group. **(R₃)** In some embodiments, R₃ is a C1-C4 alkyl group which may be substituted with a terminal R₅ group. **(R₄)** In some embodiments, R₄ is a hydrogen atom or a C1-C5 alkyl or cycloalkyl group, which may be substituted with a halide, hydroxyl, carboxyl, carbonyl, amino, or thiol groups. **(R₅)** In some embodiments, R₅ is a C1-C10 alkyl, cycloalkylaminoalkyl, aminodialkyl or aminocycloalkyl group, which may be substituted with an amino group, a thiol group, a hydroxyl group, or a carbonyl group.

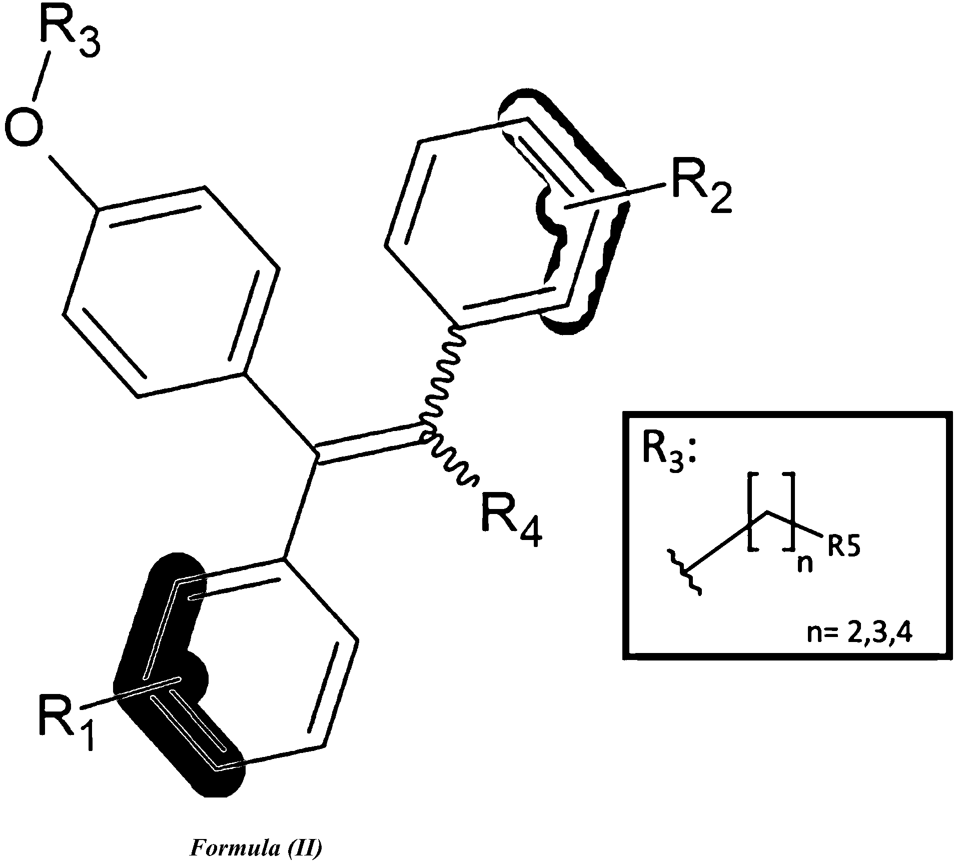

**Formula (II) (e.g., Scaffold 2): (R₁, R₂)** In some embodiments, each of R₁ and R₂ is independently a hydrogen, a halogen atom, a hydroxyl group, a sulfhydryl group, an amino group, an amide group, a carboxyl group, a carbonyl group, a alkoxyalkyl group, or a C1-C3 alkyl group which may be substituted with a halogen atom, a hydroxyl group, a sulfhydryl group, an amino group, an amide group, a carboxyl group, a carbonyl group, or a alkoxyalkyl group. **(R₃)** In some embodiments, R₃ is a C1-C4 alkyl group which may be substituted with a terminal R₅ group. **(R₄)** In some embodiments, R₄ is a hydrogen atom or a C1-C5 alkyl or cycloalkyl group, which may be substituted with a halide, hydroxyl, carboxyl, carbonyl, amino, or thiol groups. **(R₅)** In some embodiments, R₅ is a C1-C10 alkyl or cycloalkyl group which may be substituted with an amino group, a thiol group, a hydroxyl group, or a carbonyl group.

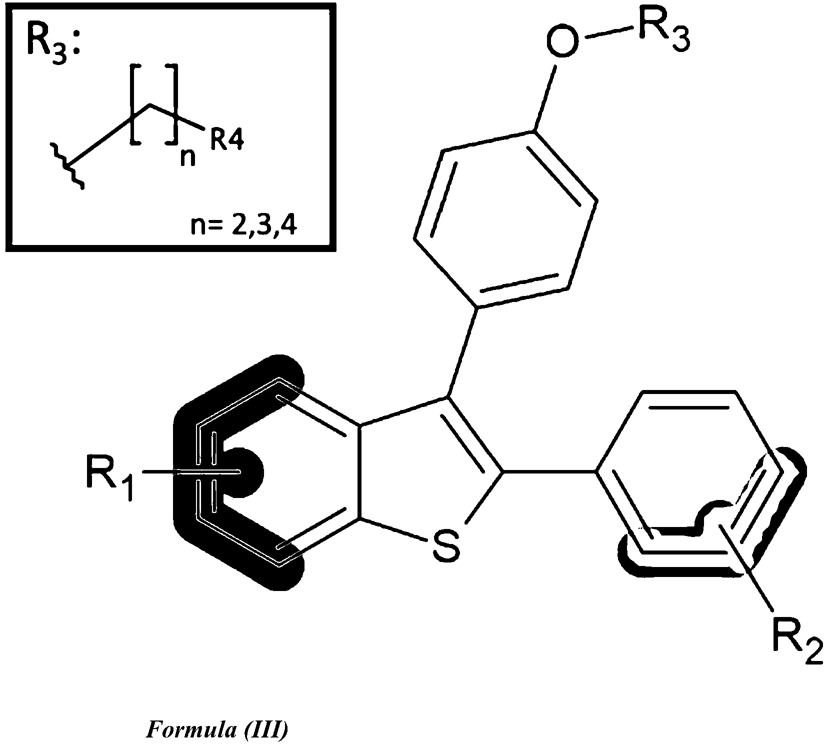

**Formula (III) (e.g., Scaffold 3): (R₁, R₂)** In some embodiments, each of R₁ and R₂ is independently a hydrogen, a halogen atom, a hydroxyl group, a sulfhydryl group, an amino group, an amide group, a carboxyl group, a carbonyl group, a alkoxyalkyl group, or a C1-C3 alkyl group which may be substituted with a halogen atom, a hydroxyl group, a sulfhydryl group, an amino group, an amide group, a carboxyl group, a carbonyl group, or a alkoxyalkyl group. **(R₃)** In some embodiments, R₃ is a C1-C4 alkyl group which may be substituted with a terminal R₄ group. **(R₄)** In some embodiments, R₄ is a C1-C10 alkyl or cycloalkyl group which may be substituted with an amino group, a thiol group, a hydroxyl group, or a carbonyl group.

In some embodiments of the compound of Formula I or Formula III, R₁ has four distinct possible placements (e.g., indicated by shading). In some embodiments of the compound of Formula II, R₁ has three distinct possible placements (e.g., indicated by shading). In some embodiments of the compound of Formula I, Formula II, or Formula III, R₂ has three distinct placements (e.g., indicated by shading). In some embodiments, R₃ of the compound of Formula I, Formula II, or Formula III comprises one of the following structures:

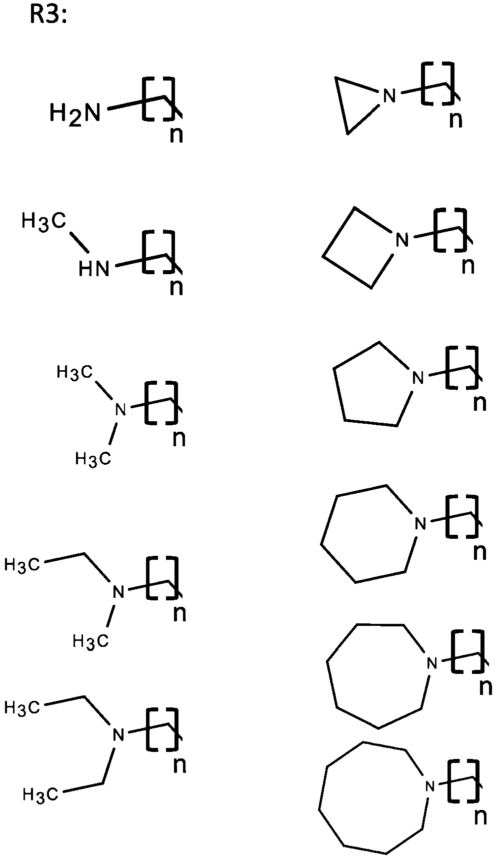

### Formula (e.g., Scaffold) Descriptions

Formula (I) (e.g., Scaffold 1): In some embodiments, the Core of MDL-001 itself, is limited in its entirety, but very amenable to piece-wise construction with R₁, R₂, and R₃ sharing an initial building block, and the R₄ being built onto a building block of its own. In some embodiments, the two building block molecules may be readily joined through a very common alkylation reaction.

Formula (II) (e.g., Scaffold 2): Both cis and trans versions of this scaffold are contemplated herein and, in some embodiments, have members with activity against RdRp. In some embodiments, this scaffold matches the 3D pharmacophore placements in MDL-001 well.

Formula (III) (e.g., Scaffold 3): In some embodiments, this scaffold matches the 3D pharmacophore placements in MDL-001 well.

### Pharmacophore descriptions

### R₁/R₂

In some embodiments, these groups serve the purpose of alternatively Hydrogen Bond Donors (HBD) or Hydrogen Bond Acceptors (HBA). Without being bound by any particular theory, the docking studies on MDL-001 are suggestive of these groups primarily serving the role of HBAs. In some embodiments, Hydrogen bond Donors (decreasing strength): carboxyl, hydroxyl, amide, amino, sulfhydryl. In some embodiments, Hydrogen bond Acceptors (decreasing strength): amino, amide, carbonyl, carboxyl, alkoxyalkyl, sulfhydryl.

In some embodiments, and without being bound by any particular theory, since the distance of each C-C bond in an alkyl chain is ~1.54 Angstroms, the alternate forms of R₁ and R₂ with C1-3 alkyl options primarily serves the purpose of adjusting the relative distances (in Angstroms) between HBAs and HBDs to either access new complementary RdRp residues or closer proximity to known HBD/HBA residues.

In some embodiments, in Formula (I) (e.g., Scaffold 1) and Formula (III) (e.g., Scaffold 3), R₁ has four distinct possible placements and R₂ has three distinct placements (e.g., indicated by shading). In some embodiments, and without being bound by any particular theory, these provide even finer adjustments to the relative orientation of these HDB and HBA groups in the binding pocket of RdRp.

In some embodiments, in Formula (II) (e.g., Scaffold 2), the central vinyl group is either in the (E) or (Z) configuration when the R₁ phenyl and R₂ phenyls are *trans* or *cis* to each other, respectively. Either configuration in certain active compounds against RdRp has been observed. In some embodiments, there are three distinct placement positions for each of R₁ and R₂, again, for purposes of fine-tuning orientation of these groups

### R₃

In some embodiments, R₃ consists of a short alkyl chain connecting R₅ to the phenolic oxygen. In some embodiments, the length of this chain (n) can be either 2, 3, or 4. In some embodiments, and without being bound by any particular theory, it cannot be 1, as that structure would be hydrolytically unstable.

### R₄

In some embodiments, and without being bound by any particular theory, this group serves the purpose of occupying a space in the binding pocket that appears to complement so-far unutilized RdRp residues that may be capable of hydrogen, polar, or hydrophobic bonding. In some embodiments, R₄ is found in Formula (I) (e.g., Scaffold 1) and Formula (II) (e.g., Scaffold 2), but not in Formula (III) (e.g., Scaffold 3).

### R₅

In some embodiments, the core of this structure is a highly substituted amine that is capable of being a hydrogen bond acceptor, in combination with multiple C1-C8 alkyl substituents that can, in some embodiments, and without being bound by any particular theory, serve a purpose of engaging hydrophobic residues in the RdRp binding pocket

In some embodiments, these alkyl substituents are found to be of the form of n-alkyl or branched chains, as well as cycloalkanes (C3-C8) containing the nitrogen as a heteroatom. In some embodiments, and without being bound by any particular theory, the Nitrogen is not critical to the pharmacophore, and the R₅ group comprises other HBA groups instead.

### Example 9

### Docking studies of MDL-001 and other ligands to SARS-CoV-2 RdRp

### Introduction

SARS-CoV-2 (severe acute respiratory syndrome coronavirus-2) is responsible for the pandemic that has affected the world since December of 2019. There has been extensive research since the start of the pandemic on SARS-CoV-2 virus and its effects. With multiple variants, SARS-CoV-2 remains a significant threat to this day, with the latest Omicron variant spreading at an alarming rate worldwide. There is an utmost need to find an oral therapeutic to combat the virus.

SARS-CoV-2 is a single stranded positive sense RNA virus. This virus has been found to bind to the ACE2 protein (Angiotensin Converting Enzyme 2) that is ubiquitously present in the cardiovascular systems, kidneys, lungs, and gastrointestinal tracts of humans. When SARS-CoV-2 binds to ACE2, it enters the host cell and begins to replicate. Based on evidence from literature, drugs can be designed for SARS-CoV-2 that prevent its entry by blocking the binding of SARS-CoV-2 to ACE2, by slowing down/preventing RNA replication by binding to the RNA polymerase/incorporation into the RNA product strand or by binding to the main protease. Remdesivir is a nucleotide analog (Drug name: Veklury, FDA approved in October 2020) developed by Gilead, which was found to prevent RNA replication of SARS-CoV-2 virus by interacting with the RNA polymerase. Remdesivir must be administered intravenously in liquid form once a day for 5 to 10 days depending on the severity of infection. While remdesivir slows down RNA replication of the Sars-CoV-2 virus and is a FDA approved treatment for COVID-19, side effects such as respiratory failure and organ dysfunction are common. Also, clinical trials (Trial registration: ClinicalTrials.gov Identifier: NCT04292730) on moderately affected patients showed no significant clinical status upon 10-day treatment using remdesivir. Molnupiravir and Paxlovid (combination of nirmatrelvir and ritonavir) are two oral drugs recently authorized by FDA for emergency use treatment of Covid19. Molnupiravir tablets are dosed at 2 tablets per day for 5 consecutive days, Paxlovid is a combination of 2 tablets of nirmatrelvir and 1 tablet of ritonavir taken once per day for 5 consecutive days.

Molnupiravir, although authorized by the FDA, has many restrictions based on the clinical trials. It is not recommended for use in pregnant women for fear of embryo-fetal toxicity. Molnupiravir is also not recommended for patients less than 18 years of age as it may affect bone and cartilage growth. Anaphylaxis has also been reported with molnupiravir. Moreover, molnupiravir, being AMES positive, needs to be further evaluated for genotoxicity and mutagenicity. Paxlovid, being a CYP3A inhibitor itself, could lead to significantly adverse reactions in patients using CYP3A inhibitors or inducers causing life-threatening conditions as it may interfere with drugs dependent on CYP3A for clearance. Since paxlovid is a combination drug: nirmatrelvir co-administered with ritonavir, patients with undiagnosed HIV infection could eventually develop resistance to HIV protease inhibitors. Hepatotoxicity has also been reported with paxlovid. The above data suggest the utmost need for a drug that is not only easy to administer (a pill compared to IV fluid) but also has minimal to no toxicology effects. Herein, a compound identified through artificial intelligence, in-vitro efficacy studies, preclinical efficacy studies and docking studies, is proposed as a next-generation COVID-19 therapeutic.

### Methods

### Crystal structure selection

RNA-dependent RNA polymerase (RdRp) crystal structures that had either remdesivir or AMP bound to it or the product RNA strand were chosen for docking studies with MDL-001. The PDB IDs of the crystal structures used for this study are 7b3b, 7b3c, 7b3d, 7bv2 and 7l1f. These specific crystal structures were chosen based on a higher X-ray resolution compared to their previous versions. All but one of the crystal structures have remdesivir bound in one of the binding regions: polymerase active site/position 1, position 2, position 3, position 4. PDB 7b3d alone has AMP bound in position 4. Positions 2,3,4 refer to the 2nd, 3rd and 4th nucleotides downstream from the polymerase active-site respectively. Crystal structures also include 2 RNA strands; the template strand and the product strand.

Remdesivir-bound crystal structures were used for this study to enable comparison of the compound MDL-001 against remdesivir. The crystal structure with PDB ID 7b3d, which has AMP bound to it, was also used for comparison of MDL-001 binding with remdesivir-bound structures.

**TABLE 29: LIST OF PDBS USED FOR THIS STUDY AND THEIR BINDING-SITE(S)**

| PDB | Description |
|---|---|
| 7B3B | Structure of elongating SARS-CoV-2 RNA-dependent RNA polymerase with Remdesivir at position -3 (structure 1) |
| 7B3C | Structure of elongating SARS-CoV-2 RNA-dependent RNA polymerase with Remdesivir at position -4 (structure 2) |
| | **note - for clarity, this title is technically a misnomer as 7B3C in the source paper, i.e. "Structure 2", has RMP bound at the -3 position, with the +1 position filled (pre-translocated) as opposed to 7B3B, i.e. Structure 1, where RMP is bound at the -3 and the +1 position is open (post-translocated) |
| 7B3D | Structure of elongating SARS-CoV-2 RNA-dependent RNA polymerase with AMP at position -4 (structure 3) |
| 7BV2 | The nsp12-nsp7-nsp8 complex bound to the template-primer RNA and triphosphate form of Remdesivir (RTP) |
| | ***note - RMP is covalently incorporated into the primer strand at the +1 position |
| 7L1F | SARS-CoV-2 RdRp in complex with 4 Remdesivir monophosphate |
| | **** note - full incorporation of 3 copies of remdesivir monophosphate (RMP) and a partially incorporated fourth RMP in the active site. |

### Protein Preparation

The crystal structures of SARS-CoV-2 RdRps (PDB IDs: 7b3b, 7b3c, 7b3d, 7l1f and 7bv2) were obtained from the PDB website (www.rcsb.org). The PDB file with the ligand was opened using AutoDock and was separated into the protein and ligand files. This was done by removing the native ligand and saving the protein by itself, deleting any ligand information from the PDB file. Later, all water molecules were deleted, Kollman charges added, AD4 (AutoDock4) atom types were added and finally saved as a PDBQT file. This prepared protein file was used for docking studies

### Ligand Preparation

SMILES representation of compounds tested for COVID19 virus were downloaded from previous literature studies. A dataset of 49 compounds was created from literature reporting compounds found to be effective against Sars-CoV-2 related proteins either in silico, in vitro or in vivo. These compounds were docked against the five crystal structures of SARS-CoV-2 RdRp, mentioned above. The SMILES strings were converted to PDBQT files using an in-house KNIME workflow using RDKit and Open Babel packages.

### Molecular Docking

Molecular docking was performed using AutoDock VINA on a virtual machine using Amazon Elastic Compute Cloud (AWS-EC2).

Binding sites of the proteins were defined from the Remdesivir/AMP bound crystal structures of the proteins. A 50*50*50 grid box was generated centered on the ligand (Remdesivir or AMP). except for PDB 7l1f in a special circumstance as noted below.

The coordinates used for the grid boxes are shown in Table 30.

**TABLE 30: COORDINATES USED FOR THE GRID BOXES**

| **PDB** | **Grid Box Coordinates** |
|---|---|
| 7b3b | 86.253, 91.056, 110.836 |
| 7b3c | 86.525, 89.063, 110.961 |
| 7b3d | 86.525, 89.063, 110.961 |
| 7l1f | 179.567, 181.224, 162.033 (RdRp Active Site/Position 1, Table 31A) |
| 7l1f | 179.968, 184.428, 154.841 (RdRp Active Site/Position 1, Table 31B)) |
| 7l1f | 179.472 183.158 160.051 (Position 2, Table 31B) |
| 7l1f | 177.992 181.660 163.878 (Position 3, Table 31B) |
| 7l1f | 177.982 178.254 166.971 (Position 4, Table 31B) |
| 7bv2 | 91.776, 91.560, 104.863 |

For docking purposes, both the RNA strands (template and product RNA strands) were retained along with the SARS-CoV-2 protein for those crystal structures that had the RNA strands. Docking was performed on the polymerase binding/Position 1 site as well as the regions covering positions 2, 3 and 4, respectively. Grids were separately generated for each possible binding regions and MDL-001 was docked to each of the sites and analyzed for poses within each site.

Docking itself was performed using a perl script as a command line argument. After docking was completed, poses were analyzed using Autodock and/or Pymol for visualization.

### Results and Discussion

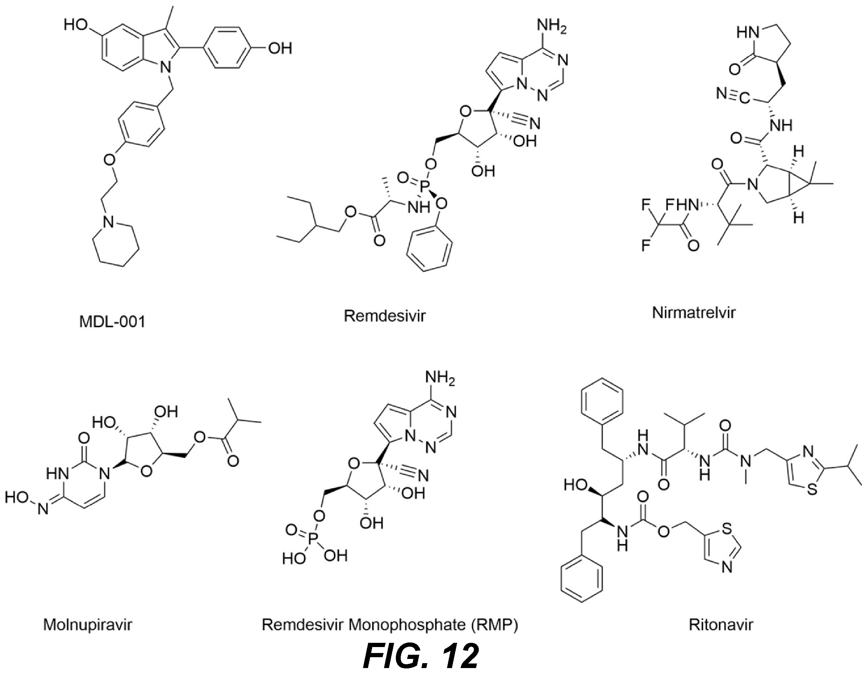

MDL-001 is a compound discovered by AI/ML Drug Discovery platform, CHEMprint^{™}, and validated in in-vitro studies of efficacy and in preclinical animal models of disease to reduce both the symptoms of COVID-19 disease and SARS-CoV-2 viral load. The mechanism of action of the compound is further investigated here using docking studies of MDL-001 versus multiple targets, including SARS-CoV-2 RdRp (SARS-CoV-2 RNA dependent RNA polymerase), other RNA viral RdRp's and other SARS-CoV-2 proteins. FIG. 12 depicts the structures of MDL-001, Remdesivir, Nirmatralvir, Molnupiravir, Remdesivir Monophosphate (RMP) and Ritonavir.

MDL-001 and other compounds were docked to RdRp (nsp12) and/or the RNA present in each respective crystal structure using Auto dock VINA. The docking studies were performed such that MDL-001 was docked in the remdesivir/AMP binding site in each respective crystal structure. This docking study was performed to compare the compound MDL-001 against remdesivir and other drugs being tested for SARS-CoV-2 for binding affinity to RdRp.

Table 31 shows the list of compounds docked against five SARS-CoV-2 RdRp PDBs, their docking scores for the respective PDBs and the average docking score of each compound.

**TABLE 31A: 49-COMPOUND DATASET DOCKED AGAINST 5 AVAILABLE SARS-COV-2-REMDESIVIR BOUND PDBS WITH THE DOCKING SCORES AND THE AVERAGE DOCKING SCORE**

| **Drug Name** | **7b3b** | **7b3c** | **7b3d** | **7l1f*** | **7bv2** | **Average docking score** |
|---|---|---|---|---|---|---|
| **Natamycin** | -10.8 | -10.4 | -10.5 | -9.5 | -10.1 | -10.26 |
| **Olaparib** | -10.9 | -10.1 | -10.1 | -8.4 | -10.5 | -10 |
| **Capastat** | -10 | -10.3 | -10.4 | -8.6 | -10.4 | -9.94 |
| **Pralatrexate** | -9.3 | -11.6 | -9.4 | -8 | -9.4 | -9.54 |
| **Leucal** | -10 | -10.1 | -9.7 | -7.9 | -9.8 | -9.5 |
| **Folinic acid** | -9.6 | -10.7 | -10.2 | -7.3 | -9.7 | -9.5 |
| **Levomefolic acid** | -10.2 | -10 | -9.6 | -7.8 | -9.4 | -9.4 |
| **Isavuconazonium** | -9.1 | -9.6 | -9.9 | -7.2 | -10.5 | -9.26 |
| **Folic acid** | -9.6 | -10.3 | -9.5 | -7.4 | -9.3 | -9.22 |
| **MDL-001** | -9.6 | -8.8 | -9.6 | -8.1 | -9.4 | -9.1 |
| **Berberine** | -9.4 | -9.5 | -9.1 | -8.2 | -8.5 | -8.94 |
| **6lze-11a** | -8.5 | -10.3 | -8.9 | -7.5 | -9.4 | -8.92 |
| **Rolapitant** | -9.4 | -7.7 | -8.3 | -9.3 | -9.1 | -8.76 |
| **Azelastine** | -8.3 | -8.4 | -9.2 | -8 | -9.6 | -8.7 |
| **Nafamostat** | -9.6 | -8.5 | -8.8 | -7.4 | -9 | -8.66 |
| **Remdesivir (RDV)** | -8.2 | -10.1 | -8.9 | -7.2 | -8.7 | -8.62 |
| **Molnupiravir** | -8.6 | -9.4 | -8.1 | -6.9 | -9.9 | -8.58 |
| **Ritonavir** | -8.9 | -8.3 | -9.5 | -7.4 | -8.7 | -8.56 |
| **Lopinavir** | -8.5 | -8.3 | -10.1 | -6.9 | -8.7 | -8.5 |
| **Emetine** | -8.6 | -8.4 | -9.2 | -6.9 | -9.3 | -8.48 |
| **Ciprofloxacin** | -8.5 | -9.6 | -8.5 | -7 | -7.8 | -8.28 |
| **Boceprevie** | -8.5 | -7.7 | -8.6 | -7.4 | -9.1 | -8.26 |
| **Ondansetron** | -8.9 | -9 | -8 | -7.2 | -8 | -8.22 |
| **Homoharringtonine** | -7.8 | -7.8 | -9 | -7.3 | -9.1 | -8.2 |
| **GC376** | -8.4 | -7.8 | -8.7 | -7.7 | -8.4 | -8.2 |
| **Butorfanol** | -7.5 | -9.7 | -7.9 | -7.4 | -8.3 | -8.16 |
| **Perampanel** | -8 | -8.4 | -8.3 | -7.4 | -8.4 | -8.1 |
| **Ketoprofen** | -6.9 | -9.2 | -9.1 | -6.9 | -8.3 | -8.08 |
| **Fluvastatin** | -8.1 | -8 | -8.3 | -6.8 | -9.1 | -8.06 |
| **Nirmatrelvir** | -7.8 | -8.5 | -8.6 | -6.7 | -8.5 | -8.02 |
| **Tetrahydrobiopterin** | -8.4 | -9.2 | -7.7 | -6.8 | -8 | -8.02 |
| **Ribavirin** | -7.8 | -8 | -8.9 | -6.1 | -8.8 | -7.92 |
| **Gatifloxacin** | -8.5 | -7.5 | -8.7 | -6.6 | -8.3 | -7.92 |
| **Vortioxetine** | -8.7 | -6.6 | -7.5 | -7.6 | -9.1 | -7.9 |
| **Carmofur** | -7.8 | -7.7 | -8.1 | -6.6 | -8.7 | -7.78 |
| **Bromfenac** | -7.3 | -9.8 | -7.6 | -6.5 | -7.6 | -7.76 |
| **Remdesivir monophosphate (RMP)** | -7.6 | -8.1 | -7.8 | -6.5 | -7.8 | -7.56 |
| **Penciclovir** | -8 | -8.4 | -7.2 | -6.1 | -8 | -7.54 |
| **Ramelteon** | -8.5 | -6.2 | -6.9 | -6.5 | -9.5 | -7.52 |
| **Nitazoxanide** | -7 | -7.2 | -7.9 | -6.9 | -8.6 | -7.52 |
| **Labetalol** | -7.8 | -9 | -6.7 | -5.6 | -7.2 | -7.26 |
| **Tasimelteon** | -7.6 | -8.4 | -7.1 | -6.2 | -6.6 | -7.18 |
| **Cyclosporine A** | -8 | -7.1 | -7.7 | -7.3 | -5.8 | -7.18 |
| **Modafinil** | -6.5 | -8.6 | -6.5 | -6.8 | -7.1 | -7.1 |
| **Umifenovir** | -6.8 | -6.8 | -7.5 | -6.3 | -7.9 | -7.06 |
| **Hydroxy-chloroquine** | -7.8 | -7.9 | -6.3 | -5.5 | -6.5 | -6.8 |
| **Chloroquine** | -5.9 | -8.4 | -6.3 | -5.3 | -7.6 | -6.7 |
| **Butoconazole** | -6.2 | -6.2 | -7.3 | -6.3 | -7.4 | -6.68 |
| **Favipiravir** | -6.3 | -7 | -6.8 | -5.2 | -7.1 | -6.48 |
| *For PDB 7l1f, the reported binding scores refer to the binding score obtained by blind docking to the polymerase active site/Position 1 of 7l1f using a 50*50*50 grid. However, since 7l1f has 4 copies of Remdesivir bound to it, separate grids were generated for each of the Remdesivir binding sites and all compounds were also docked to each of those sites using a smaller 40*40*40 grid. This data is reported in Table 31B. | | | | | | |

Average docking scores for the 49 compounds evaluated ranged from -10.26 to -6.48 kcal/mol using Autodock VINA. MDL-001 binding scores averaged -9.1 with scores ranging from -9.6 to -8.1 and specific scores of -9.6 (7b3b, RMP -3 Position), -9.6 (7b3d, AMP - 4 Position), -9.4 (7bv2, RMP +1 Position/polymerase active site), -8.8 (7b3c, RMP -3 Position) and -8.1 (7l1f, RMP -3, -2, -1, +1 Positions). Remdesivir (parent) binding scores averaged -8.62 with scores ranging from -10.1 to -7.2 and specific scores of -10.1 (7b3c, RMP -3 Position), -8.9 (7b3d, AMP -4 Position), -8.7 (7bv2, RMP +1 Position), -8.2 (7b3b, RMP -3 Position), and -7.2 (7l1f, RMP -3, -2, -1, +1 Positions). Remdesivir (monophosphate) binding scores averaged -7.56 with scores ranging from -8.1 to -6.5 and specific scores of -8.1 (7b3c, RMP -3 Position), -7.8 (7b3d, AMP -4 Position), -7.8 (7bv2, RMP +1 Position), -7.6 (7b3b, RMP -3 Position), and -6.5 (7l1f, RMP -3, -2, -1, +1 Positions). Molnupiravir binding scores averaged -8.58 with scores ranging from -9.9 to -8.1 and specific scores of -9.9 (7bv2, RMP +1 Position), -9.4 (7b3c, RMP - 3 Position), -8.6 (7b3b, RMP -3 Position), -8.1 (7b3d, AMP -4 Position), and -6.9 (7l1f, RMP -3, -2, -1, +1 Positions). Of the 49 compounds evaluated, MDL-001's average docking score ranked 10/49, remdesivir (parent) ranked 16/49, molnupiravir ranked 17/49 and remdesivir monophosphate ranked 37/49.

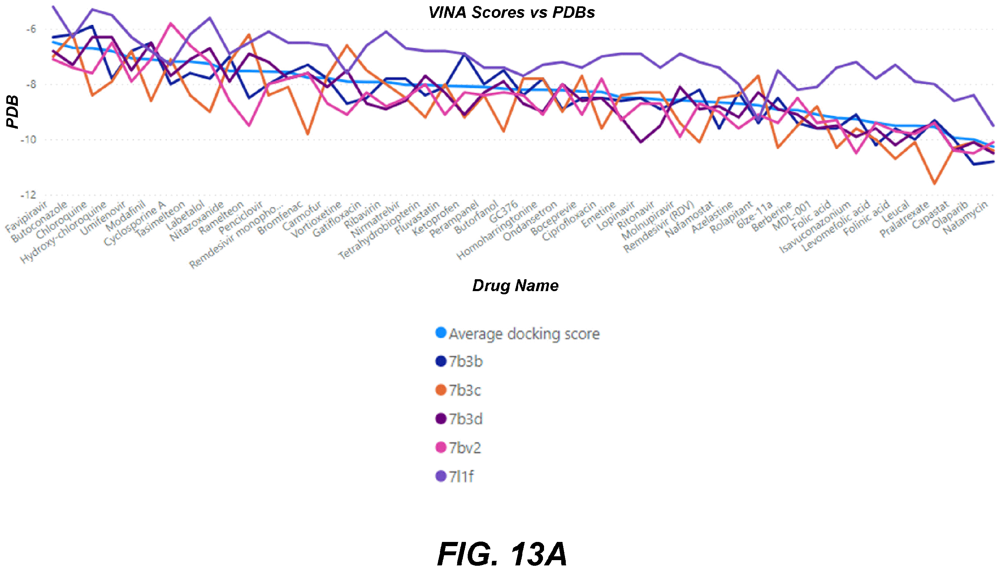

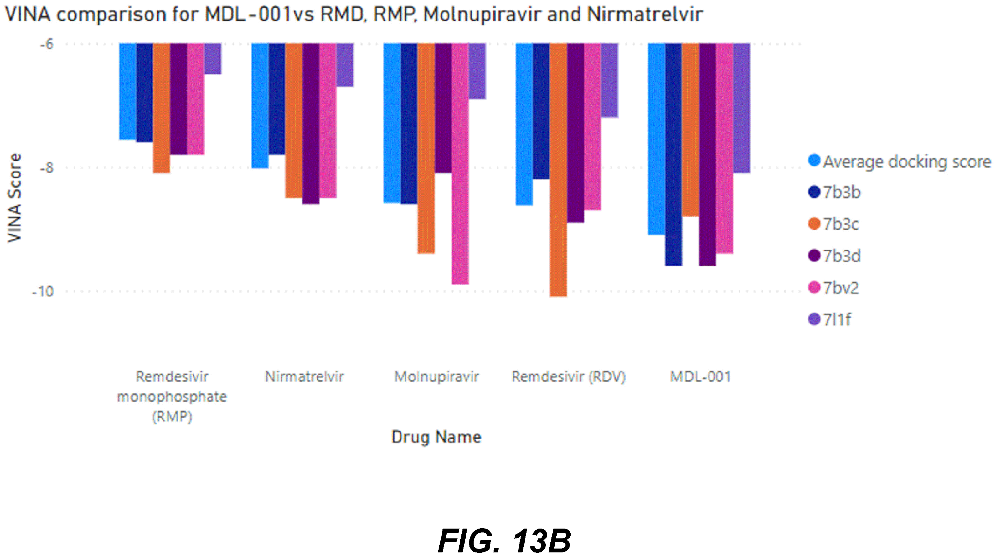

FIGS. 13A-13B depict data related to docking scores. FIG. 13A depicts data related to a comparison of docking scores for the 49 compounds against the 5 different RdRp PDBs. FIG. 13B depicts data related to a comparison of docking scores for RMP, Nirmatrelvir, Remdesivir, Molnupiravir and MDL-001 against the five RdRp PDBs; Blue line indicates the average binding score for each compound.

Remdesivir, lopinavir, ritonavir, chloroquine and hydroxychloroquine were docked to Sars-CoV2 RdRp PDB 7BV2 in a study reported by Hosseini et al. who reported docking scores of -8.1, -10.1, -8.5, -5.7 and -5.7 kcal/mol, respectively, versus PDB 7BV2 docking scores reported here for the same compounds of -8.7/-7.8 (parent/MP), -8.7, -8.7, -7.6 and -6.5 kcal/mol, respectively. Interestingly, Hosseini et al screened 1615 ligands for in silico binding to PDB 7BV2 and identified Isavuconazonium, Leucal, Natamycin, Capastat, Folic Acid and Folinic acid as the 6 highest potential repurposing candidates based on having the greatest free energy reductions (i.e. lowest observed free energies) in their study. Hosseini et al performed their study using a constrained/small grid docking approach (RdRp RMP, coordinates (91.68, 92.49, 103.85) with box sizes of 17, 17, 17 Å) versus the larger grid approach reported here (RdRp RMP, coordinates (91.776, 91.560, 104.863) with box sizes of 50, 50, 50 Å). However, similarly, these 6 drugs report 6 of the 9 greatest free energy reductions on average of the 49 drugs evaluated in this study, further demonstrating consistency across this study and previously published work. In another RdRp docking study reported by Elfiky against a "optimized SARS-CoV-2 RdRp model", remdesivir and ribavirin were reported to have docking scores of -7.6 and -7.8 kcal/mol, respectively as compared to the average docking scores reported in this study: -8.62/- 7.56(parent/MP) and -7.92 kcal/mol, respectively for the same compounds. This demonstrates that the docking scores reported here are generally comparable quantitatively and directionally with *in-silico* studies reported earlier.

**TABLE 31B: VINA SCORES FOR THE 4 DIFFERENT RMP BINDING POSITIONS FOR PDB 7L1F**

| **Drug_Name** | **VINA Score** | | | | |
|---|---|---|---|---|---|
| | **RdRp Active Site/ Position 1** | **Position 2** | **Position 3** | **Position 4** | **Average Docking score** |
| Natamycin | -9.5 | -9.5 | -9.6 | -9.5 | -9.525 |
| Rolapitant | -7.4 | -9.3 | -9.3 | -9.3 | -8.825 |
| Olaparib | -8.8 | -8.8 | -8.8 | -8.8 | -8.8 |
| Capastat | -8.7 | -8.5 | -8.6 | -8.5 | -8.575 |
| Leucal | -8 | -8.3 | -7.5 | -7.9 | -7.925 |
| Folic acid | -8 | -8.1 | -7.7 | -7.9 | -7.925 |
| Levomefolic acid | -8 | -7.8 | -7.8 | -7.9 | -7.875 |
| Nafamostat | -7.8 | -7.5 | -7.8 | -8.2 | -7.825 |
| MDL-001 | -6.9 | -8 | -8 | -7.8 | -7.675 |
| GC376 | -7.5 | -7.6 | -7.7 | -7.7 | -7.625 |
| Isavuconazonium | -7.3 | -8.2 | -7.6 | -7.2 | -7.575 |
| Pralatrexate | -7.4 | -7.5 | -7.5 | -7.6 | -7.5 |
| Butorfanol | -7.4 | -7.4 | -7.8 | -7.3 | -7.475 |
| 6lze-11a | -7.3 | -7.5 | -7.5 | -7.5 | -7.45 |
| Perampanel | -7.4 | -7.4 | -7.5 | -7.4 | -7.425 |
| Folinic acid | -7.3 | -7.5 | -7.3 | -7.5 | -7.4 |
| Remdesivir | -7.1 | -7.2 | -7.7 | -7.3 | -7.325 |
| Azelastine | -6.8 | -7 | -6.9 | -8.5 | -7.3 |
| Cyclosporine A | -7.3 | -7.3 | -7.3 | -7.3 | -7.3 |
| Berberine | -7.2 | -7.3 | -7.1 | -7.2 | -7.2 |
| Boceprevie | -7.1 | -7.5 | -7.2 | -6.9 | -7.175 |
| Homoharringtonine | -7.3 | -7.3 | -7 | -7 | -7.15 |
| Emetine | -7.1 | -6.7 | -7.2 | -7.4 | -7.1 |
| Nirmatrelvir | -7 | -7 | -7.1 | -7 | -7.025 |
| Molnupiravir | -7 | -7 | -7 | -7 | -7 |
| Lopinavir | -6.9 | -6.5 | -6.6 | -7.8 | -6.95 |
| Modafinil | -6.9 | -6.9 | -6.8 | -6.9 | -6.875 |
| Ondansetron | -6.8 | -6.8 | -6.8 | -6.8 | -6.8 |
| Tetrahydrobiopterin | -6.7 | -6.8 | -6.8 | -6.8 | -6.775 |
| Fluvastatin | -6.7 | -6.6 | -6.7 | -6.9 | -6.725 |
| Gatifloxacin | -6.7 | -6.6 | -6.6 | -6.6 | -6.625 |
| Ciprofloxacin | -6.6 | -6.6 | -6.6 | -6.6 | -6.6 |
| Remdesivir Mono phosphate (RMP) | -6.5 | -6.6 | -6.7 | -6.6 | -6.6 |
| Bromfenac | -6.2 | -6.7 | -6.7 | -6.7 | -6.575 |
| Nitazoxanide | -6.4 | -6.4 | -6.5 | -6.7 | -6.5 |
| Ritonavir | -5.8 | -6.7 | -6.3 | -6.4 | -6.3 |
| Ketoprofen | -6.1 | -6.3 | -6.3 | -6.4 | -6.275 |
| Umifenovir | -6.3 | -6.2 | -6.2 | -6.3 | -6.25 |
| Carmofur | -6.5 | -6.6 | -5.9 | -5.9 | -6.225 |
| Ramelteon | -6.2 | -6.2 | -6.2 | -6.2 | -6.2 |
| Vortioxetine | -6.2 | -6.2 | -6.2 | -6.2 | -6.2 |
| Penciclovir | -6.2 | -6.1 | -6.2 | -6.1 | -6.15 |
| Ribavirin | -6 | -6.2 | -6.2 | -6.2 | -6.15 |
| Tasimelteon | -5.8 | -6.1 | -5.9 | -6.1 | -5.975 |
| Labetalol | -5.4 | -5.6 | -5.3 | -6.9 | -5.8 |
| Chloroquine | -5.4 | -6.4 | -5.1 | -5.4 | -5.575 |
| Hydroxy-chloroquine | -5.2 | -5.6 | -5.8 | -5.4 | -5.5 |
| Butoconazole | -5.4 | -5.4 | -5.6 | -5.5 | -5.475 |
| Favipiravir | -5.6 | -5.1 | -5.4 | -5.3 | -5.35 |

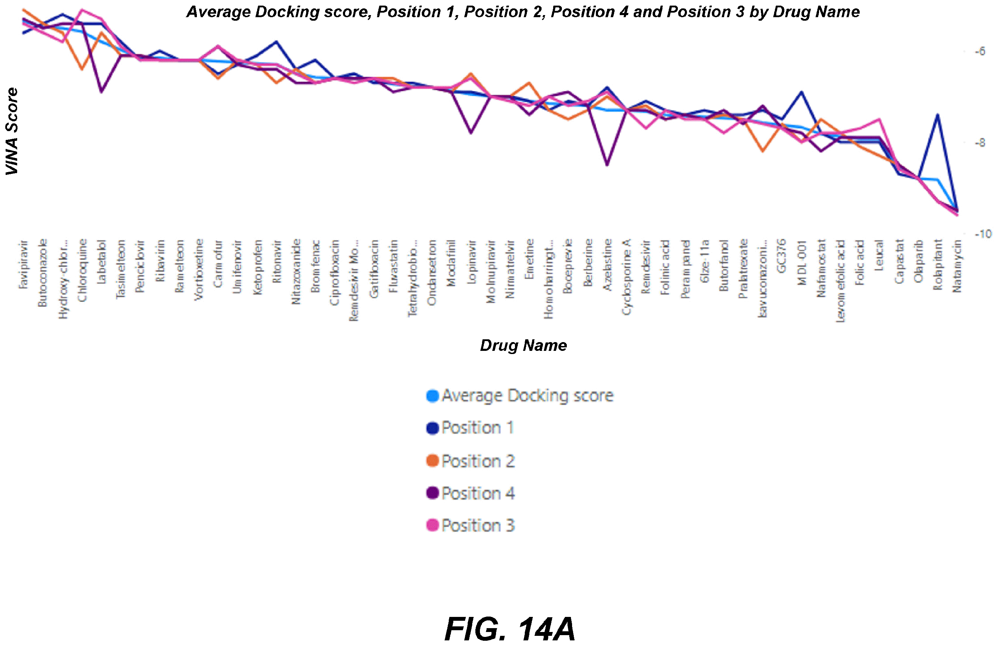

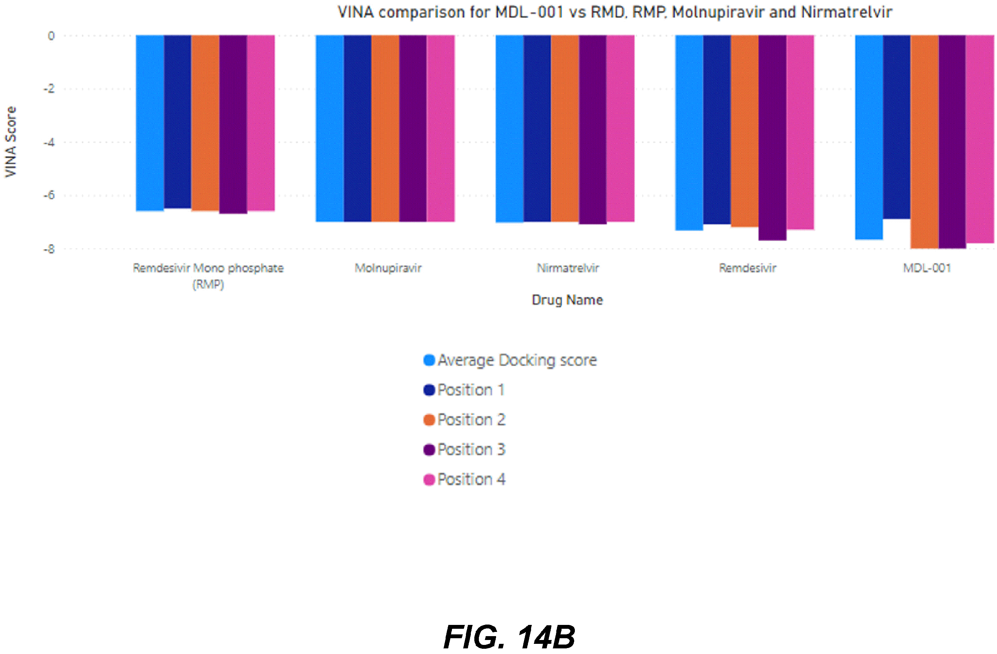

FIGS. 14A-14B depict data related to docking scores. FIG. 14A depicts data related to a comparison of docking scores for the 49 compounds against the 4 different positions in PDB 7l1f. FIG. 14B depicts data related to a comparison of docking scores for RMP, Nirmatrelvir, Remdesivir, Molnupiravir and MDL-001 against the 4 positions in PDB 7l1f; Blue line indicates the average binding score for each compound.

PDB 711f has 4 copies of RMP incorporated into the polymerase active site/Position 1, position 2, position 3 and position 4 of the nucleotide base respectively. Individual grid boxes were created for each of the RMP binding regions to compare the MDL-001 docking in those individual positions. Here the grid box was slightly smaller than the ones used for the PDBs in Table 29. A 40*40*40 grid box was used for each of these positional binding study compared to the 50*50*50 grid used for the PDBs in Table 29.

Of the 49 compounds evaluated, MDL-001's average docking score ranked 9/49, remdesivir (parent) ranked 17/49, molnupiravir ranked 25/49 and remdesivir monophosphate ranked 33/49. These results are compared to the results reported in Table 31A: MDL-001's average docking score ranked 10/49, remdesivir (parent) ranked 16/49, molnupiravir ranked 17/49 and remdesivir monophosphate ranked 37/49. Although there is some expected binding position score variability and absolute score variability between data reported in Table 31A and Table 31B, which can be attributed to differences in RdRp crystal structures, grid size and coordinates, a significant directional correlation of binding score rankings was observed in each Table. For instance, again, it was observed that the 6 compounds identified as potent potential binders by Hosseini et al in their screen of 1615 ligands (Isavuconazonium, Leucal, Natamycin, Capastat, Folic Acid and Folinic acid) can all be found in the top 17 compounds reported here in Table 31B and 4 of those compounds can be found in the top 6 ranked compounds reported in Table 31B. Furthermore, it was again observed that MDL-001's average docking score to RdRp is in the top 20% of all evaluated compounds, and it has a greater binding energy reduction on average than remdesivir (parent), molnupiravir and remdesivir monophosphate.

Given that a) MDL-001's average docking score, across 5 distinct PDB's, is in the top 20% of the 49 evaluated compounds in terms of free energy reduction, b) MDL-001's average docking scores were lower than that found for remedesivir (parent), remdesivir (mono phosphate) and molnupiravir, and c) there is good agreement between the binding scores, both quantitatively and directionally, reported here with the literature, it can be d) concluded that MDL-001 therapeutic activity via RdRp in COVID-19 is generally supported via this in silico binding study.

### Example 10

### Dose-escalation study of in vivo antiviral activity of pipendoxifene against a mouse-adapted SARS-CoV-2 virus in 129/S mice

An aim was to confirm the activity of pipendoxifene in in vivo models of SARS-CoV-2. However, mice are not normally susceptible to infection by CoV-2 since the murine ACE2 protein, which is used by the virus as a receptor for cell entry, is different from the human analogue, hACE2. A mouse-adapted SARS-CoV-2 animal model was therefore used for antiviral efficacy experiments.

SARS-CoV-2 isolate USA-WA1/2020 (BEI resources; NR-52281), referred in this report as WT-SARS-CoV-2. A variant of this virus (termed MA-SARS-CoV-2) was obtained after series of passaging in different backgrounds of laboratory mice as well as mACE-2 expressing VeroE6 cells. Briefly, the virus was serially passaged every 2 days via intranasal inoculation of the virus in 50 ul volume derived from the spun-down supernatants of lung homogenates. The mouse adaptation of the SARS-CoV-2 variant was studied in C57Bl6, BALB/c and 129S1/SVMJ (termed 129/S for simplicity) mice models. Viral stocks were sequenced after propagation to verify the integrity of the original viral genome. In this mouse model, robust SAR-CoV-2 replication occurs in the lungs, which leads to ~10% weight loss and can be detected in a plaque assay or TCID₅₀ assay.

### Methods

All the antiviral studies were performed in conventional and animal biosafety level 3 (BSL3) facilities. All work was conducted under protocols approved by the Institutional Animal Care and Use Committee (IACUC).

### SARS-CoV-2 isolates and mouse adaptation

SARS-CoV-2 isolate USA-WA1/2020 (BEI resources; NR-52281), referred in this report as SARS-CoV-2/WA1, was used to challenge mice intranasally. A variant of virus (termed MA- SARS-CoV-2) was obtained after series of passaging in different backgrounds of laboratory mice as well as mACE-2 expressing VeroE6 cells. Briefly, the virus was serially passaged every 2 days via intranasal inoculation of the virus in 50 ul volume derived from the spun-down supernatants of lung homogenates. The mouse adaptation of the SARS-CoV-2 variant was studied in C57Bl6, BALB/c and 129S1/SVMJ (termed 129 for simplicity) mice models. Viral stocks were sequenced after propagation to verify the integrity of the original viral genome.

Natural SARS-CoV-2 variants used: Nasopharyngeal swab specimens were collected as part of the routine SARS-CoV-2 surveillance conducted by Viviana Simon and the Mount Sinai Pathogen Surveillance program (IRB approved, HS#13-00981). Specimens were selected for viral culture on Vero-E6 cells based on the complete viral genome sequence information [1]. The SARS-CoV-2 virus USA-WA1/2020 was obtained from BEI resources (NR-52281) and used as wild-type reference. Viruses were grown in Vero-TMPRSS2 cells (BPS Bioscience) for 4-6 days; the supernatant was clarified by centrifugation at 4,000 g for 5 min and aliquots were frozen at -80°C for long term use. Expanded viral stocks were sequence-verified to be the identified SARS-CoV-2 variant and tittered on Vero-TMPRSS2 cells prior to use in antiviral assays.

### SARS-CoV-2 mouse-adapted model

Female 10-week-old specific pathogen-free 129/S mice (the Jackson laboratory strain 002448) were utilized. The infected pipendoxifene, vehicle, and remdesivir groups each had 9 mice split into two cohorts (Table 32). All mice were monitored for weight daily and euthanized for lung harvest on day 3 post infection. Cohort 1 (n=6) had the left lung harvested for pharmacokinetics analysis and the right lung harvested for viral titer quantification. Cohort 2 (n=3) had the left lung harvested for histopathology analysis (Histowiz procured by the White lab) and the right lung harvested for viral titer quantification. Remdesivir were administrated subcutaneously (S.C.) twice per day (BID) for 3 days; and pipendoxifene (0.5% methylcellulose), and vehicle (0.5% methylcellulose) were administered by oral gavage (P.O.) once (QD) or twice (BID) per day for 3 days. Dosage levels were as follows; Group 1: 250 mg/kg pipendoxifene PO QD, Group 2: 125 mg/kg pipendoxifene PO BID, Group 3: 250 mg/kg pipendoxifene PO BID, Group 4: 375 mg/kg pipendoxifene PO BID, Group 5: 100 mg/kg remdesivir SC BID, and Group 6: Vehicle (0.5% methylcellulose) PO BID. The first dose of all treatments was administered 1 hour before (prophylactic) intranasal infection with 2.5 × 10⁴ PFU of MA-SARS-CoV-2 in 50 µl of PBS. Mice were anesthetized with a mixture of ketamine/xylazine before each intranasal infection. Mice were weighed daily for signs of pathogenesis. 3 days post infection (dpi), 6 animals per group were humanely euthanized. The right whole lung of each mouse was harvested and inactivated in 5 ul of acetonitrile per gram of tissue in PBS and homogenized then frozen at -80°C for later PK analysis. The right whole lung of each mouse was harvested and homogenized in PBS with silica glass beads then frozen at -80°C for viral titration via TCID50. Infectious titers were quantified by limiting dilution titration using Vero-TMPRSS2 cells. Briefly, Vero-TMPRSS2 cells were seeded in 96-well plates at 20,000 cells/well. The next day, SARS-CoV-2-containing supernatant was applied at serial 10-fold dilutions ranging from 10⁻¹ to 10⁻⁸ and, after 5 days, viral cytopathic effect (CPE) was detected by staining cell monolayers with crystal violet. TCID₅₀/ml were calculated using the method of Reed and Muench. The Prism software (GraphPad) was used to determine differences in lung titers using T tests on log transformed data. Blood was harvested by cheek bleed on day 3 post-infection and UV inactivated for later PK analysis.

### Mouse Lung Histological Analysis

Paraffin-embedded lung tissue blocks for mouse lungs were cut into 5µm sections. Sections were stained with hematoxylin and eosin (H&E) and analyzed by Histowiz (Brooklyn, NY). Digital light microscopic scans of whole lung processed *in toto* were examined by an experienced veterinary pathologist. Hematoxylin Eosin stained sections of lung from K18 hACE2 mice were examined by implementing a semi quantitative, 5 point grading scheme (0 - within normal limits, 1 - mild, 2 - moderate, 3 - marked, 4 - severe). That took into account four different histopathological parameters: 1) perivascular inflammation 2) bronchial or bronchiolar epithelial degeneration or necrosis 3) bronchial or bronchiolar inflammation and 4) alveolar inflammation.

**TABLE 32: IN VIVO EFFICACY OF MDL-001 AND OTHER COMPOUNDS**

| | | | | | | | |
|---|---|---|---|---|---|---|---|
| | D: Drug or vehicle dose administered, multiple routes, multiple schedules | | | | | | |
| | I: Inoculate (intranasally) mice with MA-SARS-CoV-2 (2.5e4 PFU)/per mouse | | | | | | |

| | E: euthanize, lung collected for viral titer | | | | | | |
|---|---|---|---|---|---|---|---|
| | **Design: (sac Day 3 post-infection)** | | | | | | |
| | Cohorts ½ (Groups 1-6) (infected) will be dosed with drug or vehicle from Day 0 to Day 2, euthanized Day 3. | | | | | | |
| | | | | Tuesday | Wednesday | Thursday | Friday |
| Group | Dose Level | Cohort | N | Day 0 | Day 1 | Day 2 | Day 3 |
| | mg/kg | | | | | | |
| 1 | 250 Pipendoxifene | 1* | 6 | D, I | D | D | E |
| | PO QD | 2** | 3 | D, I | D | D | E |
| 2 | 125 Pipendoxifene | 1* | 6 | D, I | D | D | E |
| | PO BID | 2** | 3 | D, I | D | D | E |
| 3 | 250 Pipendoxifene | 1* | 6 | D, I | D | D | E |
| | PO BID | 2** | 3 | D, I | D | D | E |
| 4 | 375 Pipendoxifene | 1* | 6 | D, I | D | D | E |
| | PO BID | 2** | 3 | D, I | D | D | E |
| 5 | 100 Remdesivir | 1* | 6 | D, I | D | D | E |
| | SC BID | 2** | 3 | D, I | D | D | E |
| 6 | 0 Vehicle Controls | 1* | 6 | D, I | D | D | E |
| | PO BID | 2** | 3 | D, I | D | D | E |
| | *infected groups - Lungs Day 3 - Viral titers and pharmacokinetics | | | | | | |
| | **infected groups - Lungs Day 3 - Viral Titers and histopathology | | | | | | |

### Results and Discussion

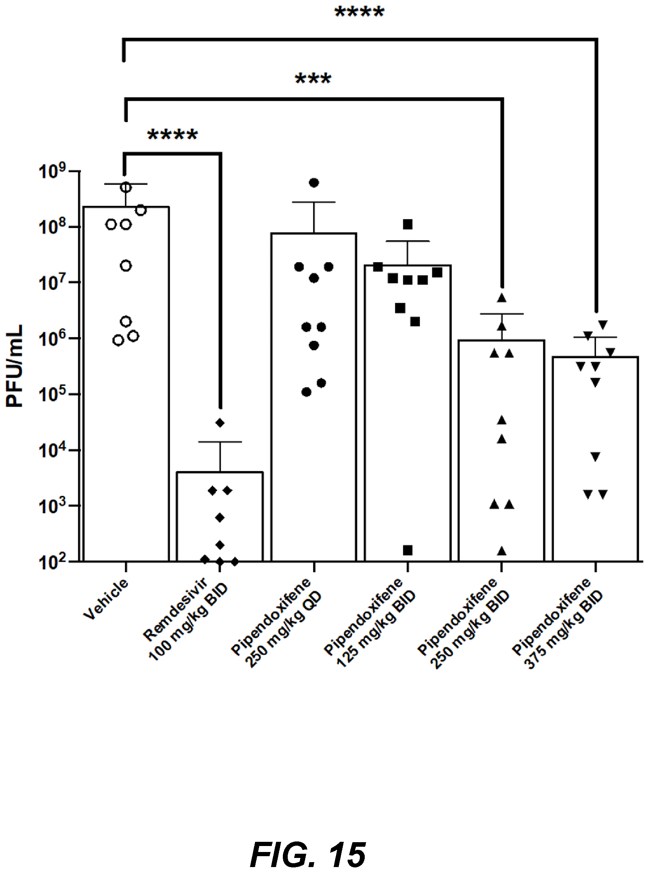

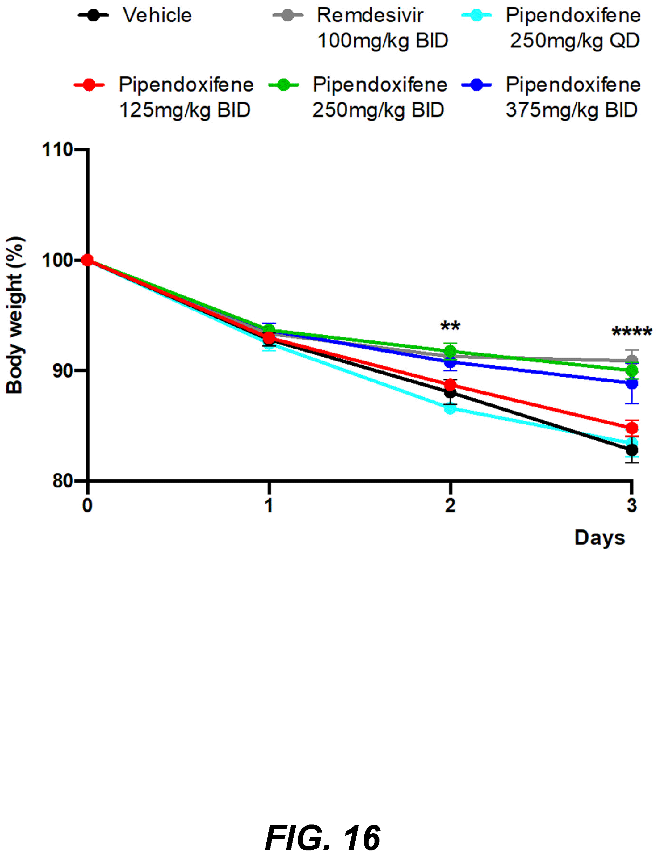

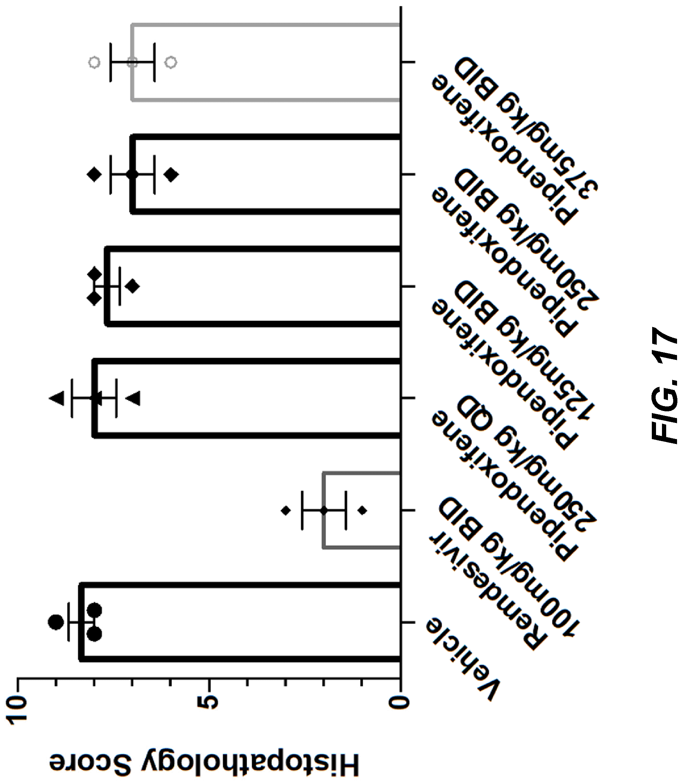

The in vivo efficacy of 250 mg/kg QD, 125 mg/kg BID, 250 mg/kg BID, or 375 mg/kg BID of pipendoxifene was tested in an established mouse-adapted animal model of SARS-CoV-2 infection. 129/S mice were dosed prophylactically with pipendoxifene PO starting 1 hour prior to infection with MA-SARS-CoV-2. Lungs were harvested on Day 3 for MA-SARS-CoV-2 lung titers and were quantified by TCID₅₀ assay for the pipendoxifene group and compared to vehicle, and remdesivir controls (FIG. 15). A 4-log reduction in lung titers was observed from remdesivir. Pipendoxifene treatment at 250 mg/kg QD and 125 mg/kg BID reduced viral lung titers, in agreement with previous results, but the reductions did not achieve statistical significance. The 250 and 375 mg/kg BID pipendoxifene groups did reduce viral lung titers by over 2 orders of magnitude, which was statistically significant (2.39 reduction, p<0.001 and 2.70 reduction, p<0.0001, respectively). Animal weight was monitored daily for signs of pathogenesis. Vehicle treated mice lost ~15% of body weight over the experiment, similar to previous results in this model. Treatment with 250 and 375 mg/kg BID pipendoxifene protected mice from MA-SARS-CoV-2 associated weight loss with statistical significance at day 2 and 3, similar to the remdesivir group (FIG. 16, P<0.01 and P<0.0001, respectively). This effect was dose-dependent. Histopathology analysis of infected lung samples also showed a small, but dose-dependent improvement in the histopathology scores of inflammation and damage due to pipendoxifene treatment (FIG. 17).

### Mouse Plasma and Lung Concentrations (MA-SARS-CoV-2 Infected)

A limited and preliminary MDL-001 plasma and lung exposure assessment in MA-SARS-CoV-2 infected mice was conducted within the study of Example 10. In this study, MDL-001 dose groups were 250 mg/kg QD, 125 mg/kg BID, 250 mg/kg BID, or 375 mg/kg BID. Plasma and lung samples were obtained at 24 h (Day 4) following 3 days of MDL-001 administration. The purpose was to assess dose response and the effect of QD vs BID. Administration (Table 33). In addition, a limited comparison of differences in exposure between infected and non-infected mice (Example 4) was performed and is presented in Table 34.

**TABLE 33: MEAN PLASMA AND LUNG TISSUE CONCENTRATIONS (NG/ML) OF MDL-001 AT 24 H AFTER 3 DAYS ORAL ADMINISTRATION**

| **MDL-001 Concentrations (ng/mL) in the Mouse at 24 h after 3 Day Administration** | | | | | | | | |
|---|---|---|---|---|---|---|---|---|
| **Parameter** | **250 QD** | | **125 BID** | | **250 BID** | | **375 BID** | |
| | **Mean** | **SD** | **Mean** | **SD** | **Mean** | **SD** | **Mean** | **SD** |
| Lung | 784 | 535 | 947 | 779 | 644 | 485 | 573 | 476 |
| Plasma | 194 | 136 | 290 | 202 | 134 | 120 | 197 | 148 |
| L/P Ratio | 4.03 | NA | 3.27 | NA | 4.80 | NA | 2.90 | NA |
| QD: Once daily; BID: Twice daily; NA: Not applicable | | | | | | | | |

**TABLE 34: PLASMA AND LUNG TISSUE CONCENTRATIONS (NG/ML) OF MDL-001 (250 MG/KG) AFTER ORAL ADMINISTRATION IN NON-INFECTED (1 AND 5 DAYS) OR IN INFECTED (3 DAYS) MICE**

| **Comparison of MDL-001 Concentrations (ng/mL) at 24 h after 250 mg/kg QD** | | | |
|---|---|---|---|
| **Parameter** | **Non-Infected** | | **Infected** |
| | **Day 1** | **Day 5** | **Day 3** |
| Lung | 3888 | 1770 | 784 |
| Plasma | 34 | 10 | 194 |
| L/P Ratio | 114.35 | 177.00 | 4.04 |
| Non-infected: Example 4; Infected: Study 3 | | | |

In infected mice, MDL-001 C₂₄ₕ plasma concentrations were highest in the 125 mg/kg BID group (290 ng/mL), followed by the 250 mg/kg QD group (194 ng/mL). There were no dose-dependent increases in C₂₄ₕ plasma concentrations from 125 through 375 mg/kg BID. It should be noted that these are sparse data; that is, it is possible that Cₘₐₓ and AUC values, if available, could have demonstrated differential exposure in these groups. MDL-001 C₂₄ₕ lung concentrations were in general about 3- to 4-fold higher than plasma concentrations, demonstrating preferential distribution or retention in the target tissue (Table 33).

Comparing exposure in non-infected and infected mice at the common dose of 250 mg/kg QD, MDL-001 C₂₄ₕ plasma concentrations were significantly higher (6- to 19-fold) in infected mice. The reason for this difference is not known but suggests decreased plasma clearance in infected mice. Conversely, C₂₄ₕ lung concentrations were 2- to 5-fold lower in infected compared to non-infected mice. As a result, apparent lung/plasma C₂₄ₕ ratios are significantly lower in infected compared to non-infected mice. It should be noted again that these are sparse data, and a more comprehensive pharmacokinetic study in infected mice, to include Cₘₐₓ and AUC determinations, would provide for a more definitive assessment.

### Conclusions

Pipendoxifene dosed at 250 and 375 mg/kg twice per day orally was able to ameliorate SARS-CoV-2 associated weight loss in a dose dependent manner, which was statistically significant at day 2 and 3 post infection. Both the 250 and 375 mg/kg BID pipendoxifene treatments were also associated with an over 2-log reduction in viral lung titers compared to vehicle controls, which were also statistically significant. Finally, a small, but dose-dependent improvement in histopathology scores of infected mouse lungs was observed during pipendoxifene treatment. The histopathology changes due to pipendoxifene treatment were not statistically significant, possible due to the small sample size (n=3). Taken together, these results indicate that Pipendoxifene shows antiviral properties *in vivo* and can improve SARS-CoV-2 associated pathogenesis in this mouse-adapted SARS-CoV-2 mouse model.

### Example 11

### In vitro antiviral activity of Pipendoxifene and Berzosertib against a panel of SARS-CoV-2 variants in HeLa-ACE2 cells

The object of this study was to evaluate the *in vitro* antiviral efficacy of Pipendoxifene and Berzosertib against the omicron variant (B.1.1.529) of SARS-CoV-2. Antiviral activity of compounds against SARS-CoV-2/WA1, (mouse-adapted) MA-SARS-CoV-2/WA1, the Alpha variant (B.1.1.7), the Beta variant (B.1.351), the Delta variant (B.1.617.2), and the Omicron variant (B.1.1.529) was assessed in HeLa-ACE2 cells. Full 6-point SARS-CoV-2 antiviral curves using our immunostaining-based protocol with concurrent cytotoxicity curves (MTT Assay) were generated for all compounds and IC₅₀/IC₉₀/CC₁₀/CC₅₀ were calculated. Experiments were performed twice in triplicate. Nirmatrelvir and DMSO controls were included with all experiments.

### Methods

All the antiviral studies were performed in conventional and animal biosafety level 3 (BSL3) facilities. All work was conducted under protocols approved by the Institutional Animal Care and Use Committee (IACUC).

### SARS-CoV-2 isolates and mouse adaptation

SARS-CoV-2 isolate USA-WA1/2020 (BEI resources; NR-52281), referred in this report as SARS-CoV-2/WA1, was used to challenge mice intranasally. A variant of virus (termed MA- SARS-CoV-2) was obtained after series of passaging in different backgrounds of laboratory mice as well as mACE-2 expressing VeroE6 cells. Briefly, the virus was serially passaged every 2 days via intranasal inoculation of the virus in 50 ul volume derived from the spun-down supernatants of lung homogenates. The mouse adaptation of the SARS-CoV-2 variant was studied in C57Bl6, BALB/c and 129S1/SVMJ (termed 129 for simplicity) mice models. Viral stocks were sequenced after propagation to verify the integrity of the original viral genome.

Natural SARS-CoV-2 variants used: Nasopharyngeal swab specimens were collected as part of the routine SARS-CoV-2 surveillance conducted by Viviana Simon and the Mount Sinai Pathogen Surveillance program (IRB approved, HS#13-00981). Specimens were selected for viral culture on Vero-E6 cells based on the complete viral genome sequence information. The SARS-CoV-2 virus USA-WA1/2020 was obtained from BEI resources (NR-52281) and used as wild-type reference. Viruses were grown in Vero-TMPRSS2 cells (BPS Bioscience) for 4-6 days; the supernatant was clarified by centrifugation at 4,000 g for 5 min and aliquots were frozen at -80°C for long term use. Expanded viral stocks were sequence-verified to be the identified SARS-CoV-2 variant and tittered on Vero-TMPRSS2 cells prior to use in antiviral assays.

### SARS-CoV-2 Antiviral Assays

Two thousand Vero-TMPRSS2 or HeLa-ACE2 cells were seeded into 96-well plates in DMEM (10% FBS) and incubated for 24 hours at 37°C, 5% CO2. Two hours before infection, the medium was replaced with 100 µL of DMEM (2% FBS) containing the compound of interest at concentrations 50% greater than those indicated, including a DMSO control. Plates were then transferred into the BSL3 facility and 100 PFU (Vero-TMPRSS2 MOI = 0.025) or 1000 PFU (HeLa-ACE2 MOI = 0.25) of indicated variant was added in 50 µL of DMEM (2% FBS), bringing the final compound concentration to those indicated. Plates were then incubated for 48 hours at 37°C. After infection, supernatants were removed and cells were fixed with 4% formaldehyde for 24 hours prior to being removed from the BSL3 facility. The cells were then immunostained for the viral NP protein (an inhouse mAb 1C7, provided by Dr. Thomas Moran) with a DAPI counterstain. Infected cells (488 nm) and total cells (DAPI) were quantified using the Celigo (Nexcelcom) imaging cytometer. Infectivity was measured by the accumulation of viral N protein (fluorescence accumulation). Percent infection was quantified as ((Infected cells/Total cells) - Background) *100 and the DMSO control was then set to 100% infection for analysis. Data was fit using nonlinear regression and IC50s for each experiment were determined using GraphPad Prism version 8.0.2 (San Diego, CA). Cytotoxicity was also performed using the MTT assay (Roche), according to the manufacturer's instructions. Cytotoxicity was performed in uninfected Vero-TMPRSS2 or HeLa-ACE2 cells with same compound dilutions and concurrent with viral replication assay. All assays were performed in biologically independent triplicates.

### Influenza Antiviral Assays:

A549 cells were infected with each viral strain at MOI 0.05. After 24 h post-infection with A/WSN/33 cells were fixed with 4% formaldehyde for 30 min. Cells were briefly washed with PBS, then permeabilized with 0.1% Triton X-100 in PBS for 15 minutes. Blocking occurred at room temperature for 1 hour with 0.5% BSA in PBS followed by incubation with the NP antibody (HT103, a gift from Thomas Moran) in 0.5% BSA in PBS for 1 h at room temperature. Cells were washed with PBS 2x and incubated with a fluorescently-labeled secondary antibody, alexa-fluor-488 (Invitrogen), in 0.5% BSA in PBS with DAPI for 45 min at room temperature. Two washes with PBS were performed before imaging the cells on a Celigo Image Cytometer. Infected cells (488 nm) and total cells (DAPI) were quantified using the Celigo (Nexcelcom) imaging cytometer. Infectivity was measured by the accumulation of viral NP protein (fluorescence accumulation). Percent infection was quantified as ((Infected cells/Total cells) - Background) *100 and the DMSO control was then set to 100% infection for analysis. Data was fit using nonlinear regression and IC₅₀ₛ for each experiment were determined using GraphPad Prism version 8.0.2 (San Diego, CA). Cytotoxicity was also performed using the MTT assay (Roche), according to the manufacturer's instructions. Cytotoxicity was performed in uninfected A549 cells with same compound dilutions and concurrent with viral replication assay. All assays were performed in biologically independent triplicates.

### Results and Discussion

The *in vitro* efficacy of pipendoxifene and berzosertib against a panel of SARS-CoV-2 variants was assessed. Antiviral activity of compounds against SARS-CoV-2/WA1, (mouse-adapted) MA-SARS-CoV-2/WA1, the Alpha variant (B.1.1.7), the Beta variant (B.1.351), the Delta variant (B.1.617.2), and the Omicron variant (B.1.1.529) was assessed in HeLa-ACE2 cells. Full 6-point SARS-CoV-2 antiviral curves using our immunostaining-based protocol with concurrent cytotoxicity curves (MTT Assay) were generated for all compounds and IC₅₀/IC₉₀/CC₁₀/CC₅₀ were calculated. Experiments were performed twice in triplicate. Nirmatrelvir and DMSO controls were included with all experiments.

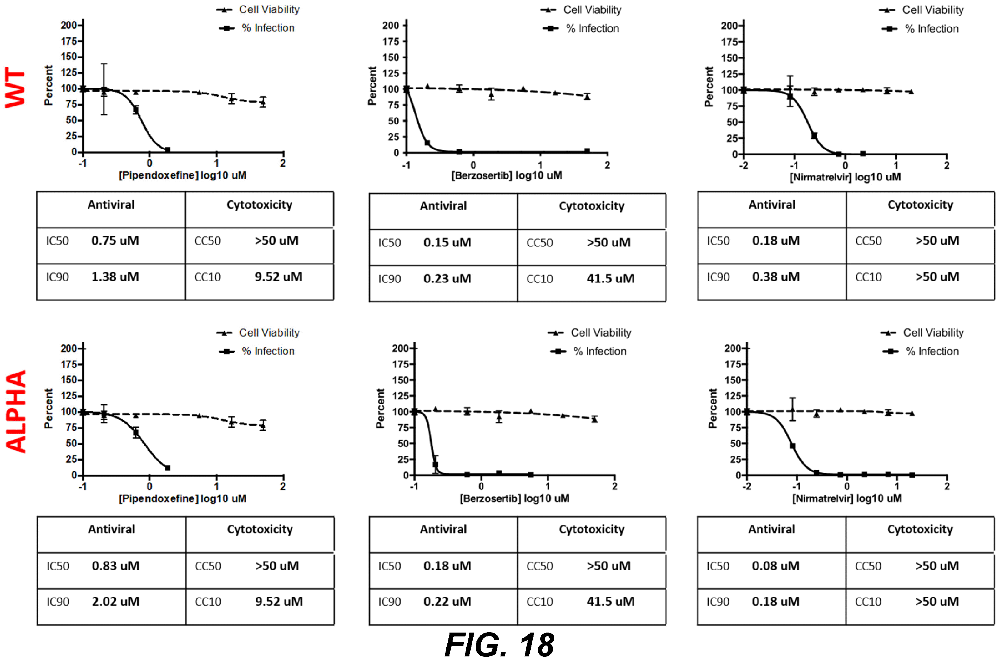

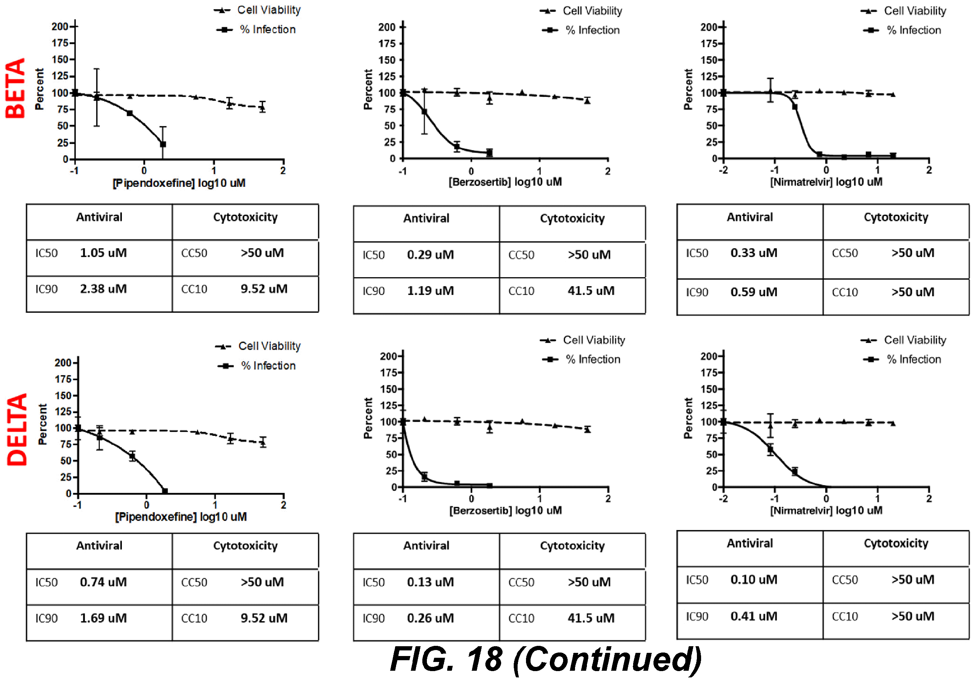

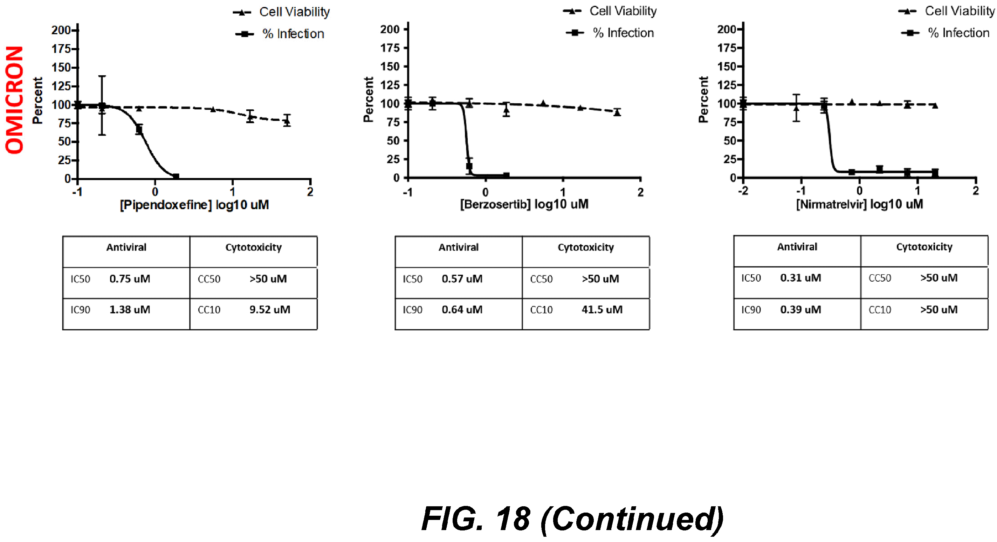

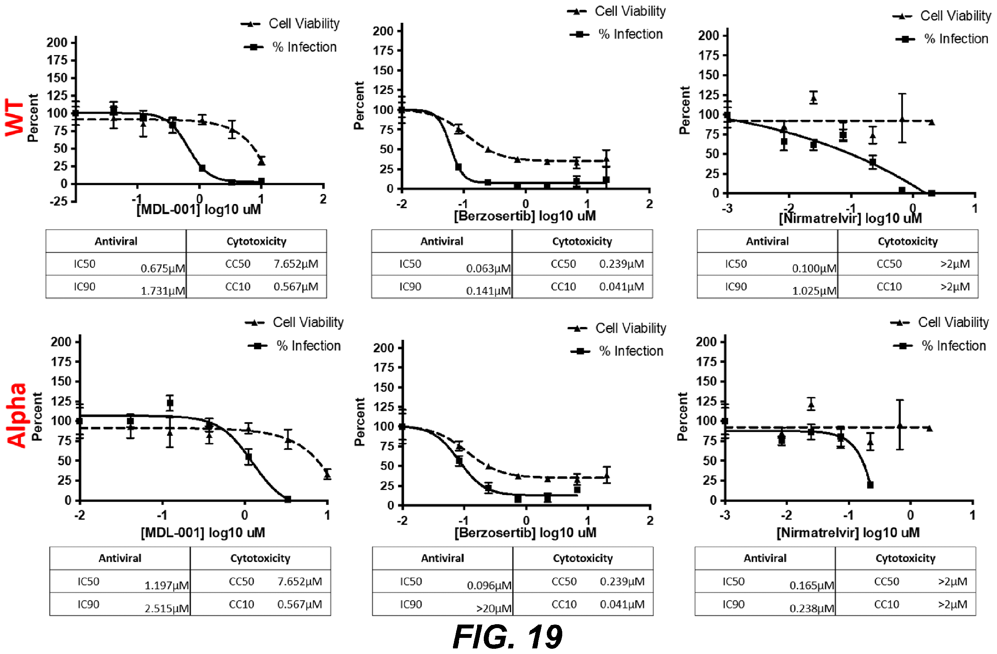

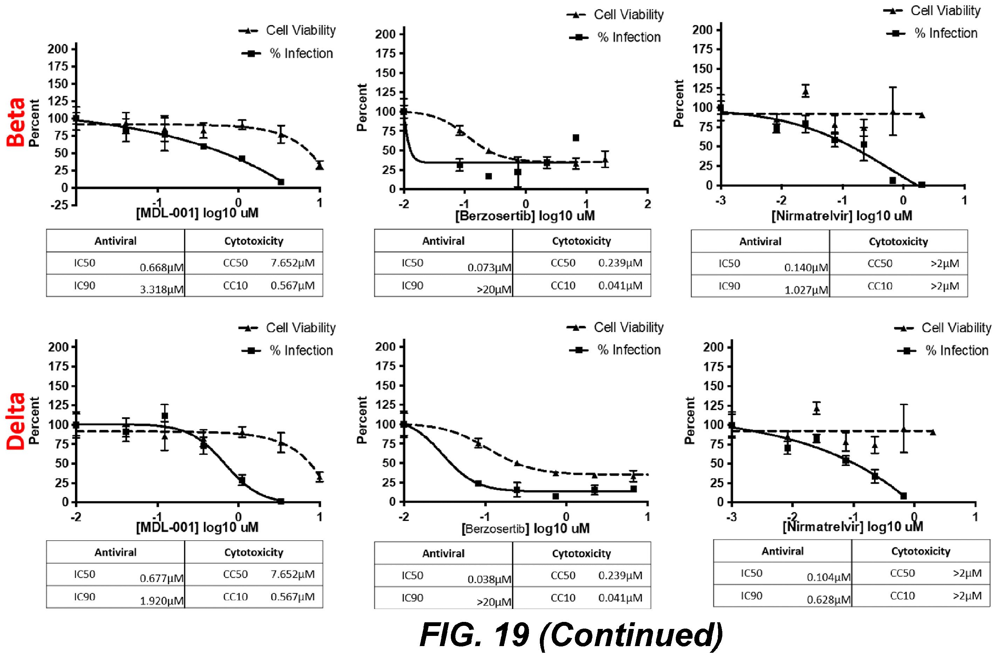

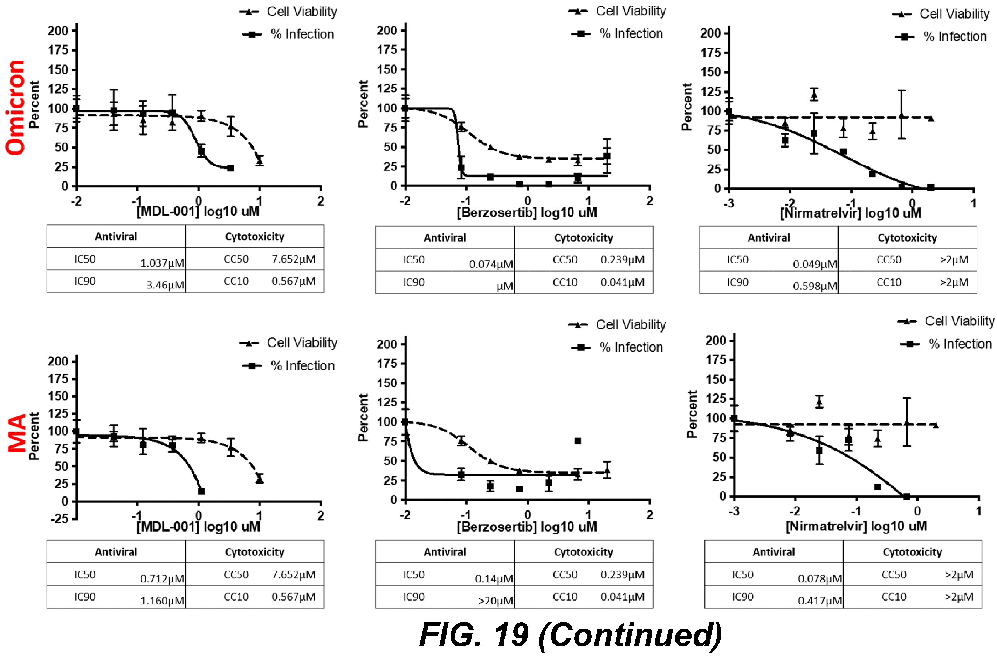

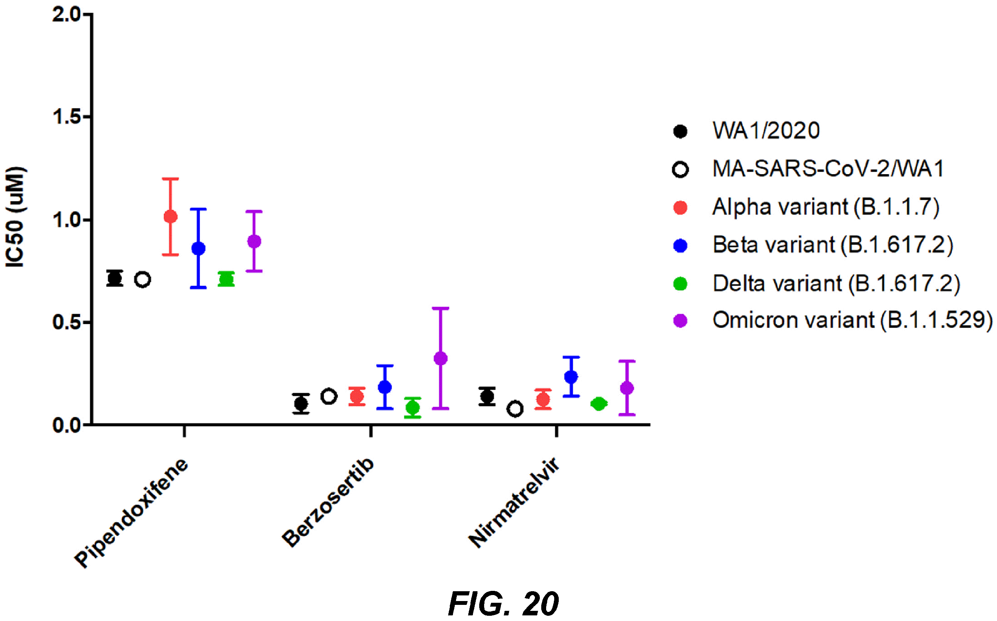

Pipendoxifene was calculated to have an IC50 of 0.72uM and berzosertib was calculated to have an IC₅₀ of 0.11uM against SARS-CoV-2/WA1 (WT) across two replicates performed in biological triplicate. Both pipendoxifene and berzosertib maintained a similar IC₅₀/IC₉₀ against all variants compared to the parental SARS-CoV-2/WA1 in HeLa-ACE2 cells (FIG. 18, FIG. 19, and FIG. 20).

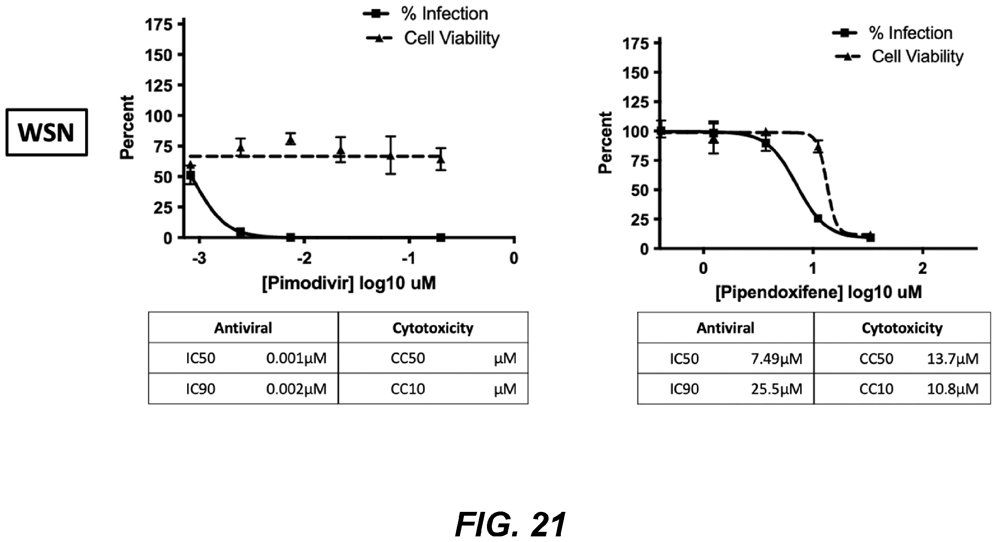

The broad-spectrum antiviral activity of MDL-001was assessed against influenza viruses based on in silico modeling on the viral RdRp (FIG. 21). Full 6-point influenza A/WSN/33 antiviral curves using our immunostaining-based protocol with concurrent cytotoxicity curves (MTT Assay) were generated for all compounds and IC₅₀/IC₉₀/CC₁₀/CC₅₀ were calculated. Experiments were performed in triplicate Nirmatrelvir and DMSO controls were included with all experiments.

MDL-001 has an IC₅₀ of 7.49uM against A/WSN/33 indicating it has detectable antiviral activity against an H1N1 influenza A virus.

### Conclusions

Pipendoxifene and berzosertib antiviral activity was maintained for the omicron variant as compared to the parental SARS-CoV-2/WA1, or any other variant tested. No differences were observed in the nirmatrelvir controls between variants. This was confirmed by antiviral assays performed in HeLa-ACE2 cells. This indicates that the omicron variant has not gained any resistance to pipendoxifene or berzosertib, and potency should be maintained in the clinic during the ongoing omicron wave of SARS-CoV-2 infection.

Furthermore, the antiviral activity of pipendoxifene against an influenza A virus indicates that it has potential broad-spectrum antiviral activity across viral families. This is consistent with the proposed hypothesis of an RdRp target, which is partial conserved across RNA viruses.

### Example 12

### Determination of Pharmacokinetics of Mdl-001 Following Oral Dosing in Male Sprague-Dawley Rats

In this study, the plasma and lung tissue concentrations and pharmacokinetics of MDL-001 for plasma samples were evaluated in male SD rat following single oral administrations at 250 mg/kg, 500 mg/kg 750 mg/kg or 1000 mg/kg. Blood samples were collected via jugular vein cannula (JVC). The generated rat plasma and lung tissue underwent bioanalysis. The measured plasma and lung tissue concentrations of MDL-001 were employed for pharmacokinetic (PK) analysis. PK parameters were determined using Phoenix WinNonlin (v8.3.1) software. The PK summary data are presented in Table 35. The average plasma and lung tissue concentrations at 24-hour time point are provided in Table 36.

**TABLE 35: PK SUMMARY DATA FOR PLASMA**

| **PK Parameter** | **Group 1,** | **Group 2,** | **Group 3,** | **Group 4,** | **Group 4,** |
|---|---|---|---|---|---|
| | **250 mg/kg** | **500 mg/kg** | **750 mg/kg** | **1000 mg/kg** | **1000 mg/kg*** |
| Cₘₐₓ (ng/mL) | 311 1 42 | 462 ± 87 | 415 ± 72 | 452 ± 75 | 452 ± 75 |
| tₘₐₓ (hr) | 5.33 ± 1.15 | 6.67 ± 1.15 | 6.67 ± 1.15 | 6.00 ± 0.00 | 6.00 ± 0.00 |
| t_{1/2} (hr) | 6.06 ± 2.62 | 5.20 ± 1.34 | 4.65 ± 0.37 | 4.88 ± 0.50 | 4.73 |
| MRTₗₐₛₜ (hr) | 9.02 ± 1.28 | 9.20 ± 0.82 | 9.38 ± 0.43 | 10.3 ± 1.38 | 8.44 ± 1.87 |
| AUCₗₐₛₜ (hr·ng/mL) ± SE | 3515 ± 980 | 5420 ± 1032 | 5260 ± 1970 | 6762 ± 930 | 5710 ± 1731 |
| AUCₗₐₛₜ_D (hr·kg·ng/mL/mg) | 14.1 ± 3.9 | 10.8 ± 2.1 | 7.01 ± 2.63 | 6.76 ± 0.93 | 5.71 ± 1.73 |
| *One outlier was excluded from the calculations | | | | | |

**TABLE 36: LUNG CONCENTRATION AND DATA FOR LUNG₂₄/PLASMA₂₄ RATIO**

| MDL-001 Concentration in (ng/mL or ng/g) | **Group 1, 250 mg/kg** | **Group 2, 500 mg/kg** | **Group 3, 750 mg/kg** | **Group 4, 1000 mg/kg** | **Group 4, 1000 mg/kg*** |
|---|---|---|---|---|---|
| Plasma at 24 hr | 38.5 | 51.7 | 38.0 | 131 | 44.1 |
| Lung at 24 hr | 3177 | 4520 | 4373 | 13427 | 5890 |
| Lung₂₄/Plasma₂₄ | 96.7 | 98.8 | 122 | 121 | 134 |
| *One outlier was excluded from the calculations | | | | | |

### Objective & Introduction

The objective of this study was to determine the PK of MDL-001 following PO administrations in male Sprague-Dawley rats.

In this study, the plasma and lung concentrations verses time profile of MDL-001 were determined in male SD rat following single PO dose administrations at 250 mg/kg, 500 mg/kg, 750 mg/kg and 1000 mg/kg. The measured plasma and lung tissue concentrations of MDL-001 were used for PK analysis.

### Methods

### Test System and Animal Description

The male SD rat, a total of 12 (3 per group), utilized for this study, were obtained from Hilton Lab. Animals were identified by cage labels. A single room was used for all the animals. Animals were healthy at the start of the trial and were randomly assigned to treatment groups. The study was not blinded. Animals were fasted overnight prior dosing with food returned 4 hours post dose. Water was offered ad libitum.

### Study Design

The study design is presented in Table 37.

**TABLE 37: RAT PK STUDY DESIGN**

| **Group #** | **Test Article** | **Dosing Route** | **Animals N=** | **Dose (mg/kg)** | **Dosing Solution Conc. (mg/mL)** | **Dosing Volume (mL/kg)** | **Vehicle** | **Blood Sampling Time Points** | **Lung Removal Time Point** |
|---|---|---|---|---|---|---|---|---|---|
| 1 | | PO | 3 | 250 | 31.25 | 8.0 | 0.5% MC (4000 cP) in water | 0.25, 0.5, 1, 2, 4, 6, 8, 12, and 24 hours | |
| 2 | MDL-001 | PO | 3 | 500 | 62.50 | 8.0 | | | 24-hour |
| 3 | | PO | 3 | 750 | 93.75 | 8.0 | | | |
| 4 | | PO | 3 | 1000 | 128 | 8.0 | | | |

### Dosing Formulations

The test article was MDL-001. All dosing formulations were prepared fresh on the day of dosing.

### Animal Dosing and Sampling

Freshly prepared dosing solutions of MDL-001 were administered to the male rats as a single PO dose at 250 mg/kg (Group 1), 500 mg/kg (Group 2), 750 mg/kg (Group 3), or 1000 mg/kg (Group 4). Blood samples were collected at assigned time points up to 24-hour post dose. Lungs were removed at the last time point of 24-hour post dose.

### Plasma and Lung Samples Preparation

Blood samples were collected via JVC, placed into chilled tubes containing K₂EDTA as anticoagulant, inverted several times, and kept on ice until centrifugation. The blood samples were centrifuged at temperatures of 2°C to 8°C, at 3,000xg, for 5 minutes. The plasma samples were transferred to labeled clear polypropylene tubes.

The lung tissue samples were collected at the selected time points, rinsed with saline, patted dry, and weighed to at least 3 significant figures. The tissue samples were placed into chilled tubes.

### PK Parameters

PK parameters were calculated from the time course of the plasma concentrations with Phoenix WinNonlin (v8.3.1) software using a non-compartmental model. The maximum plasma concentration (Cₘₐₓ) and the time to reach maximum plasma drug concentration (tₘₐₓ) after PO dosing were observed from the data. The area under the time concentration curve (AUC) was calculated using the linear trapezoidal rule with calculation to the last quantifiable data point, and with extrapolation to infinity if applicable. Plasma half-life (t_{1/2}) was calculated from 0.693/slope of the terminal elimination phase. Mean residence time (MRT) was calculated by dividing the area under the moment curve (AUMC) by the AUC.

### Results

### Observations and Adverse Reactions

All animals behaved normally during the experiment. No adverse reactions were observed following the PO administrations of MDL-001 in male SD rat in this study.

### Analytical Data and PK Results

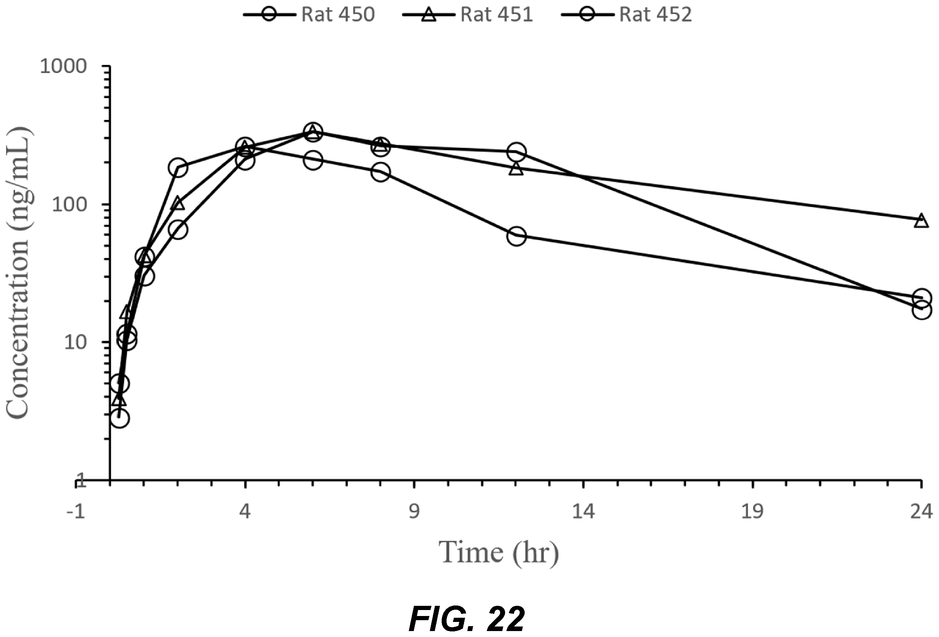

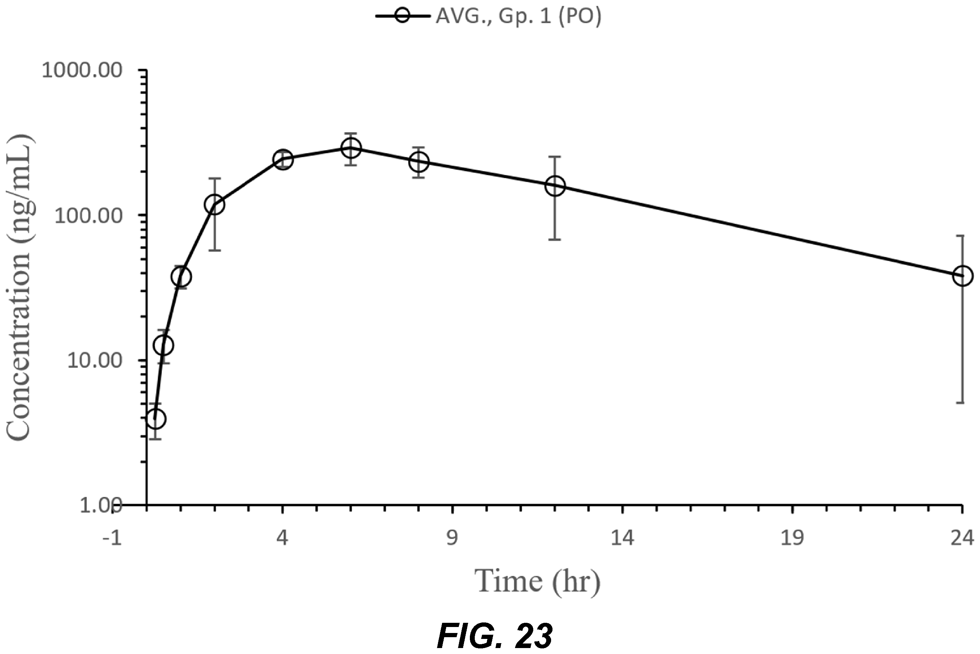

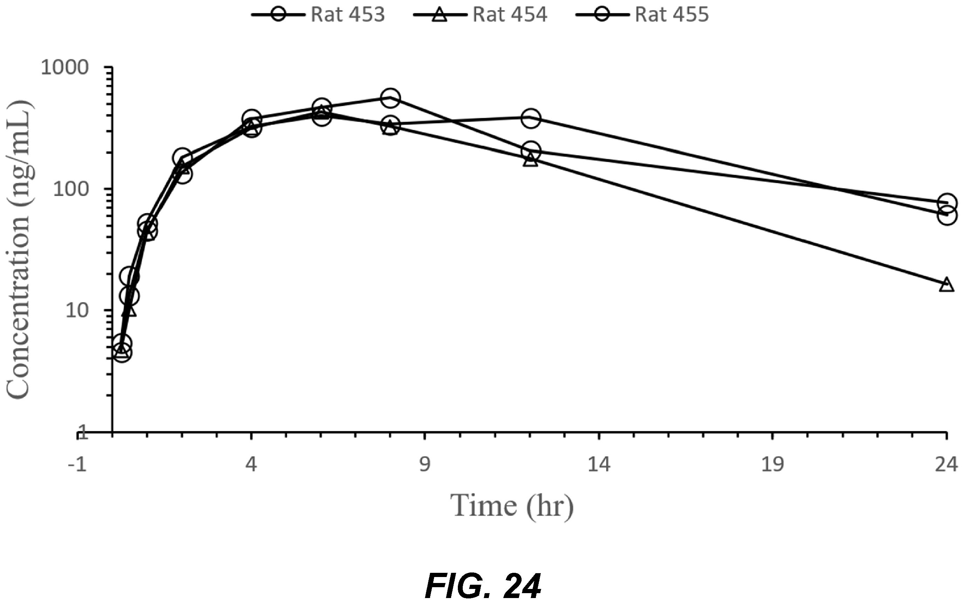

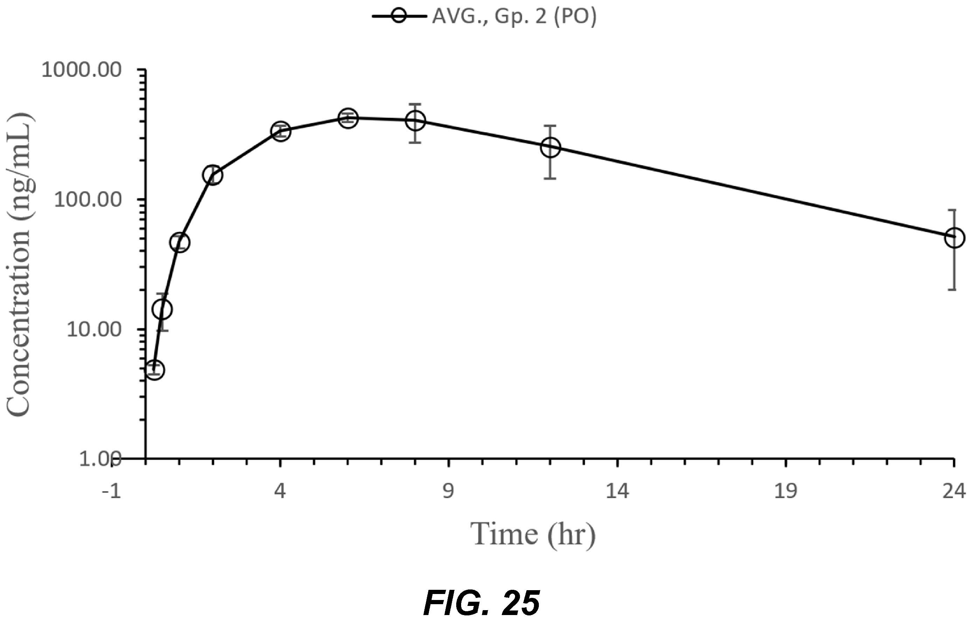

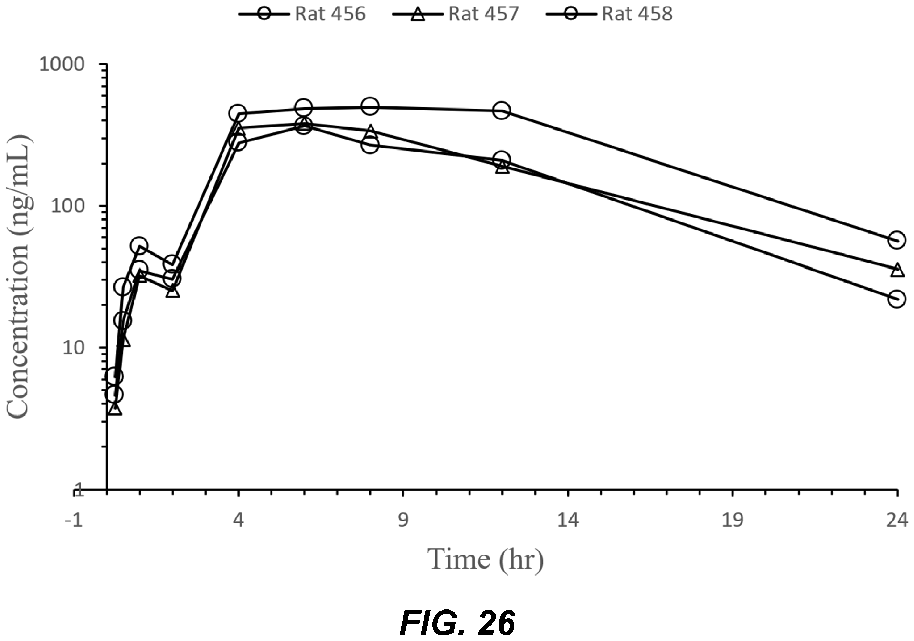

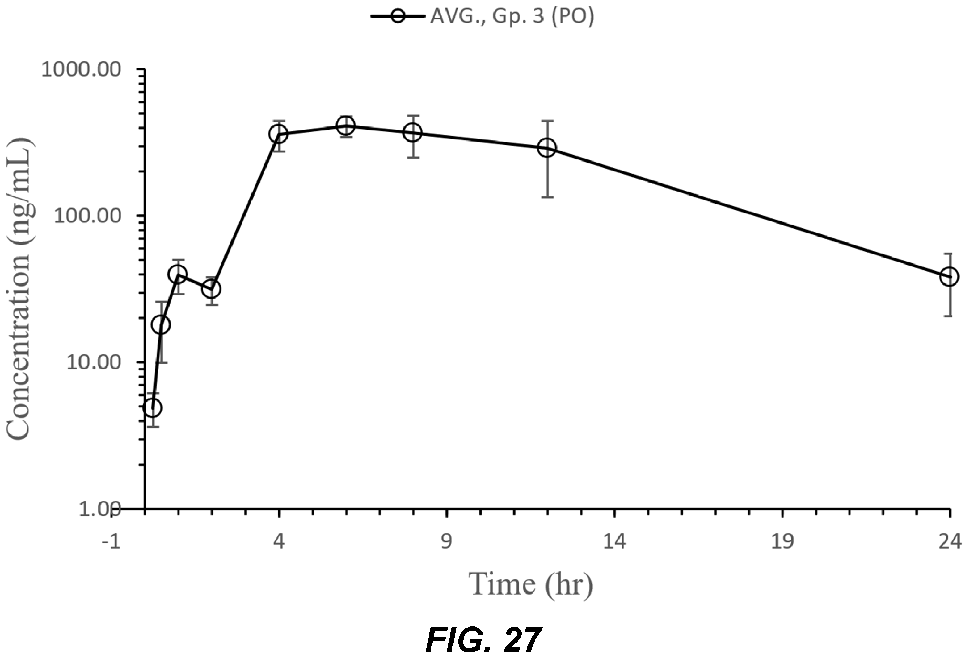

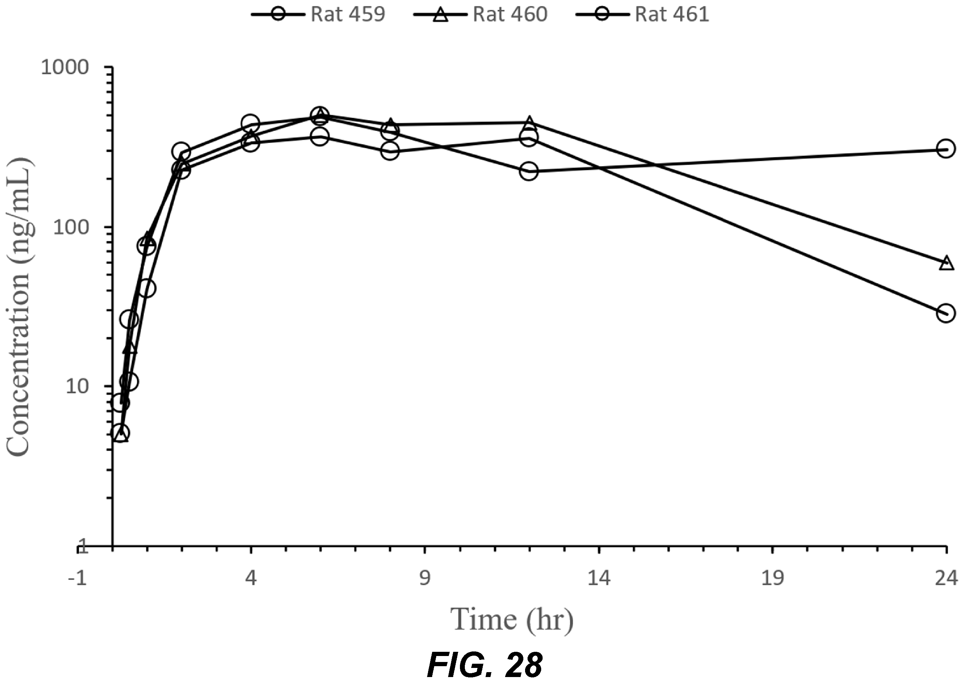

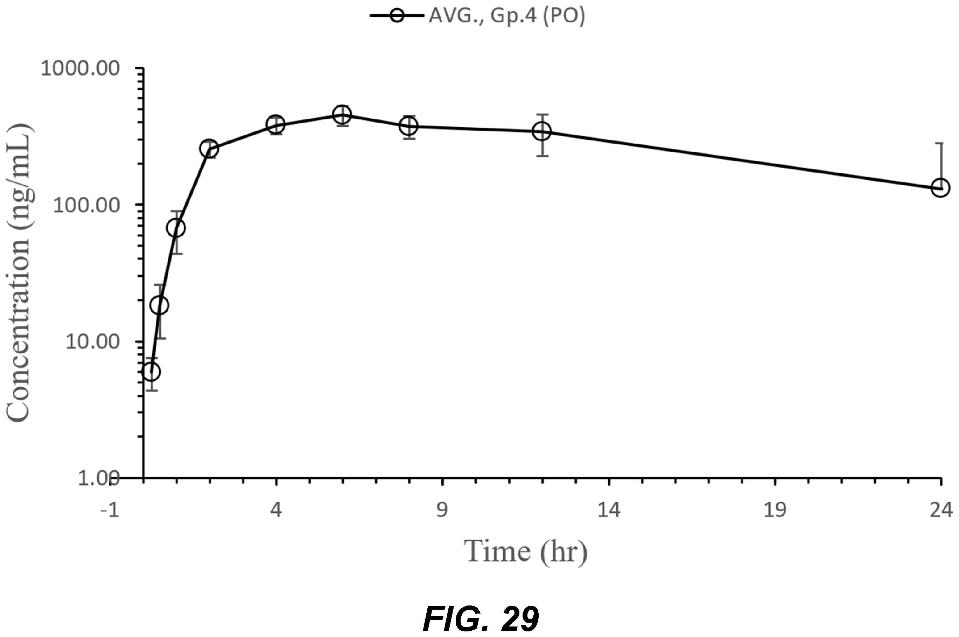

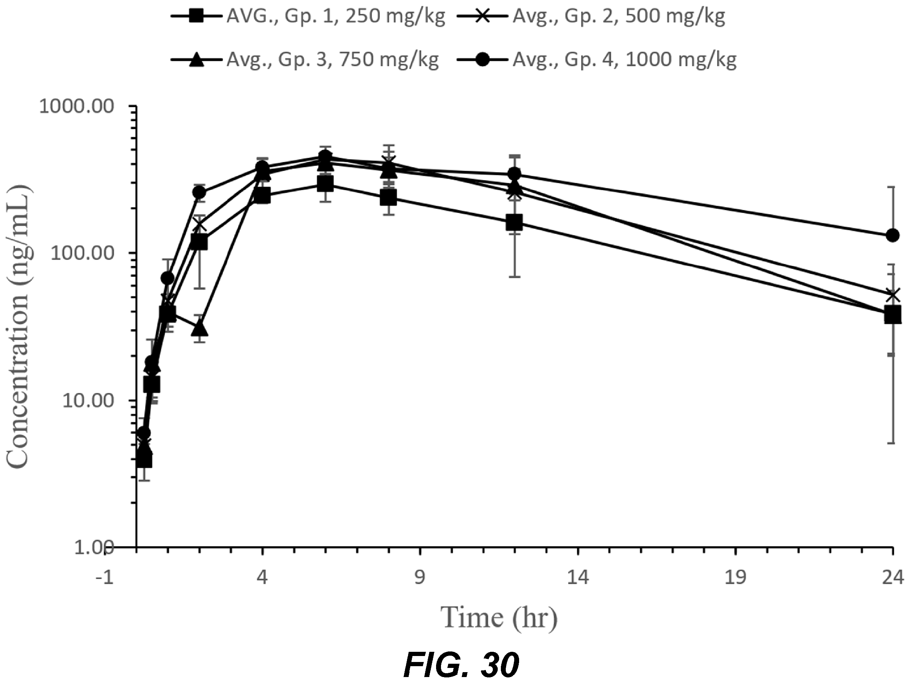

Individual and average plasma and lung concentrations and PK data are shown in Table 38 through Table 42 and in FIGS. 22-29. All data are expressed as ng/mL of the free base for plasma or ng/g for lung tissue. Comparison of average drug concentration in plasma is presented in FIG. 30.

**TABLE 38: INDIVIDUAL AND AVERAGE PLASMA CONCENTRATIONS (NG/ML) AND PK PARAMETERS FOR MDL-001 AFTER SINGLE ORAL ADMINISTRATION AT 250 MG/KG IN MALE SD RAT (GROUP 1)**

| **Oral (250 mg/kg MDL-001; Group 1)** | | | | | |
|---|---|---|---|---|---|
| | **Concentration of MDL-001 (ng/mL)** | | | | |
| **Time Plasma (hr)** | | **Rat #** | | **Mean** | **SD** |
| | **450** | **451** | **452** | | |
| 0.25 | 2.86 | 3.91 | 5.04 | 3.94 | 1.1 |
| 0.5 | 10.4 | 16.6 | 11.5 | 12.8 | 3.31 |
| 1 | 30.5 | 42.5 | 41.5 | 38.2 | 6.66 |
| 2 | 66.7 | 103 | 186 | 119 | 61 |
| 4 | 212 | 259 | 262 | 244 | 28 |
| 6 | 334 | 336 | **211** | 294 | 72 |
| 8 | **264** | **274** | **173** | 237 | 56 |
| 12 | **240** | **182** | **59.6** | 161 | 92 |
| 24 | **17.4** | **77.1** | **21.1** | 38.5 | 33.5 |
| 24 (Lung) | 2290 | 5360 | 1880 | 3177 | 1902 |
| Lung₂₄/Plasma₂₄ | 132 | 69.5 | 89.1 | 96.7 | 31.7 |
| **t_{1/2} (hr)** | 3.83 | 8.95 | 5.42 | 6.06 | 2.62 |
| **tₘₐₓ (hr)** | 6.00 | 6.00 | 4.00 | 5.33 | 1.15 |
| **Cₘₐₓ (ng/mL)** | 334 | 336 | 262 | 311 | 42 |
| **MRTₗₐₛₜ (hr)** | 9.37 | 10.1 | 7.61 | 9.02 | 1.28 |
| **AUCₗₐₛₜ (hr·ng/mL)** | 4036 | 4124 | 2384 | 3515 | 980 |
| **AUC_{∞} (hr·ng/mL)** | 4132 | 5119 | 2549 | 3933 | 1297 |
| **Dose-normalized Values¹** | | | | | |
| **AUCₗₐₛₜ (hr·kg·ng /mL/mg)** | 16.1 | 16.5 | 9.54 | 14.1 | 3.9 |
| **AUC_{∞} (hr·kg·ng /mL/mg)** | 16.5 | 20.5 | 10.2 | 15.7 | 5.2 |
| t_{1/2}: half-life, time points in bold used for half-life calculation; Cₘₐₓ: maximum plasma concentration; tₘₐₓ: time of maximum plasma concentration; MRTₗₐₛₜ: mean residence time, calculated to the last observable time point; AUCₗₐₛₜ: area under the curve, calculated to the last observable time point; AUC_{∞}: area under the curve, extrapolated to infinity | | | | | |
| ¹Dose-normalized by dividing the parameter by the measured dose in mg/kg. | | | | | |

**TABLE 39: INDIVIDUAL AND AVERAGE PLASMA CONCENTRATIONS (NG/ML) AND PK PARAMETERS FOR MDL-001 AFTER SINGLE ORAL ADMINISTRATION AT 500 MG/KG IN MALE SD RAT (GROUP 2)**

| **Oral (500 mg/kg MDL-001; Group 2)** | | | | | |
|---|---|---|---|---|---|
| | **Concentration of MDL-001 (ng/mL)** | | | | |
| **Time (hr)** | | **Rat #** | | **Mean** | **SD** |
| | **453** | **454** | **455** | | |
| | | | | | |
| 0.25 | 4.63 | 4.73 | 5.39 | 4.92 | 0.4 |
| 0.5 | 13.5 | 10.3 | 19.2 | 14.3 | 4.51 |
| 1 | 45.3 | 43.5 | 53 | 47.3 | 5.05 |
| 2 | 136 | 154 | 181 | 157 | 23 |
| 4 | 376 | 319 | 325 | 340 | 31 |
| 6 | 465 | 428 | 398 | 430 | 34 |
| 8 | **561** | **325** | **341** | 409 | 132 |
| 12 | **207** | **179** | **388** | 258 | 113 |
| 24 | **77.5** | **16.4** | **61.3** | 51.7 | 31.7 |
| 24 (Lung) | 4940 | 2060 | 6560 | 4520 | 2279 |
| Lung₂₄/Plasma₂₄ | 64 | 126 | 107 | 98.8 | 31.7 |
| **t_{1/2} (hr)** | 6.08 | 3.66 | 5.87 | 5.20 | 1.34 |
| **tₘₐₓ (hr)** | 8.00 | 6.00 | 6.00 | 6.67 | 1.15 |
| **Cₘₐₓ (ng/mL)** | 561 | 428 | 398 | 462 | 87 |
| **MRTₗₐₛₜ (hr)** | 9.34 | 8.32 | 9.94 | 9.20 | 0.82 |
| **AUCₗₐₛₜ (hr·ng/mL)** | 5730 | 4268 | 6261 | 5420 | 1032 |
| **AUC_{∞} (hr·ng/mL)** | 6410 | 4355 | 6780 | 5848 | 1307 |
| **Dose-normalized Values¹** | | | | | |
| **AUCₗₐₛₜ (hr·kg·ng /mL/mg)** | 11.5 | 8.54 | 12.5 | 10.8 | 2.1 |
| **AUC_{∞} (hr·kg·ng /mL/mg)** | 12.8 | 8.71 | 13.6 | 11.7 | 2.6 |
| t_{1/2}: half-life, time points in bold used for half-life calculation; Cₘₐₓ: maximum plasma concentration; tₘₐₓ: time of maximum plasma concentration; MRTₗₐₛₜ: mean residence time, calculated to the last observable time point; AUCₗₐₛₜ: area under the curve, calculated to the last observable time point; AUC_{∞}: area under the curve, extrapolated to infinity | | | | | |
| ¹Dose-normalized by dividing the parameter by the measured dose in mg/kg. | | | | | |

**TABLE 40: INDIVIDUAL AND AVERAGE PLASMA CONCENTRATIONS (NG/ML) AND PK PARAMETERS FOR MDL-001 AFTER SINGLE ORAL ADMINISTRATION AT 750 MG/KG IN MALE SD RAT (GROUP 3)**

| **Oral (750 mg/kg MDL-001; Group 3)** | | | | | |
|---|---|---|---|---|---|
| | **Concentration of MDL-001 (ng/mL)** | | | | |
| **Time Plasma (hr)** | | **Rat #** | | **Mean** | **SD** |
| | **456** | **457** | **458** | | |
| 0.25 | 6.21 | 3.75 | 4.62 | 4.86 | 1 |
| 0.5 | 26.7 | 11.4 | 15.4 | 17.8 | 7.93 |
| 1 | 51.7 | 32.2 | 35 | 39.6 | 10.5 |
| 2 | 38.4 | 25.2 | 30.4 | 31.3 | 6.6 |
| 4 | 445 | 352 | 278 | 358 | 84 |
| 6 | 486 | 381 | 366 | 411 | 65 |
| 8 | **497** | **338** | **267** | 367 | 118 |
| 12 | **466** | **190** | **209** | 288 | 154 |
| 24 | **56.4** | **35.6** | **21.9** | 38.0 | 17.4 |
| 24 (Lung) | 6390 | 3160 | 3570 | 4373 | 1758 |
| Lung₂₄/Plasma₂₄ | 113 | 88.8 | 163 | 122 | 38 |
| **t_{1/2} (hr)** | 4.77 | 4.94 | 4.24 | 4.65 | 0.37 |
| **tₘₐₓ (hr)** | 8.00 | 6.00 | 6.00 | 6.67 | 1.15 |
| **Cₘₐₓ (ng/mL)** | 497 | 381 | 366 | 415 | 72 |
| **MRTₗₐₛₜ (hr)** | 9.88 | 9.10 | 9.16 | 9.38 | 0.43 |
| **AUCₗₐₛₜ (hr·ng/mL)** | 7527 | 4281 | 3971 | 5260 | 1970 |
| **AUC_{∞} (hr·ng/mL)** | 7916 | 4534 | 4105 | 5518 | 2087 |
| **Dose-normalized Values¹** | | | | | |
| **AUCₗₐₛₜ (hr·kg·ng /mL/mg)** | 10.0 | 5.71 | 5.29 | 7.01 | 2.63 |
| **AUC_{∞} (hr·kg·ng /mL/mg)** | 10.6 | 6.05 | 5.47 | 7.36 | 2.78 |
| t_{1/2}: half-life, time points in bold used for half-life calculation; Cₘₐₓ: maximum plasma concentration; tₘₐₓ: time of maximum plasma concentration; MRTₗₐₛₜ: mean residence time, calculated to the last observable time point; AUCₗₐₛₜ: area under the curve, calculated to the last observable time point; AUC_{∞}: area under the curve, extrapolated to infinity | | | | | |
| ¹Dose-normalized by dividing the parameter by the measured dose in mg/kg. | | | | | |

**TABLE 41: INDIVIDUAL AND AVERAGE PLASMA CONCENTRATIONS (NG/ML) AND PK PARAMETERS FOR MDL-001 AFTER SINGLE ORAL ADMINISTRATION AT 1000 MG/KG IN MALE SD RAT (GROUP 4), (ALL DATA INCLUDED)**

| **Oral (1000 mg/kg MDL-001; Group 4)** | | | | | |
|---|---|---|---|---|---|
| | **Concentration of MDL-001 (ng/mL)** | | | | |
| **Time (hr)** | **Rat #** | | | **Mean** | **SD** |
| | **459** | **460** | **461** | | |
| 0.25 | 5.05 | 5 | 7.81 | 5.95 | 1.61 |
| 0.5 | 10.6 | 17.9 | 26 | 18.2 | 7.7 |
| 1 | 40.7 | 85.1 | 74.9 | 66.9 | 23.3 |
| 2 | 226 | 248 | 293 | 256 | 34 |
| 4 | 335 | 370 | 438 | 381 | 52 |
| 6 | 365 | 501 | 489 | 452 | 75 |
| 8 | **295** | **436** | **393** | 375 | 72 |
| 12 | **359** | **449** | **221** | 343 | 115 |
| 24 | **28.4** | **59.7** | **305** | 131.0 | 151.5 |
| 24 (Lung) | 3850 | 7930 | 28500 | 13427 | 13212 |
| Lung₂₄/Plasma₂₄ | 136 | 132.8 | 93.4 | 121 | 24 |
| **t_{1/2} (hr)** | 4.30 | 5.16 | 5.17 | 4.88 | 0.50 |
| **tₘₐₓ (hr)** | 6.00 | 6.00 | 6.00 | 6.00 | 0.00 |
| **Cₘₐₓ (ng/mL)** | 365 | 501 | 489 | 452 | 75 |
| **MRTₗₐₛₜ (hr)** | 9.37 | 9.67 | 11.9 | 10.3 | 1.38 |
| **AUCₗₐₛₜ (hr·ng/mL)** | 5702 | 7444 | 7138 | 6762 | 930 |
| **AUC_{∞} (hr·ng/mL)** | 5878 | 7888 | 9414 | 7727 | 1774 |

| **Dose-normalized Values¹** | | | | | |
|---|---|---|---|---|---|
| **AUCₗₐₛₜ (hr·kg·ng /mL/mg)** | 5.70 | 7.44 | 7.14 | 6.76 | 0.93 |
| **AUC_{∞} (hr·kg·ng /mL/mg)** | 5.88 | 7.89 | 9.41 | 7.73 | 1.77 |
| t_{1/2}: half-life, time points in bold used for half-life calculation; Cₘₐₓ: maximum plasma concentration; tₘₐₓ: time of maximum plasma concentration; MRTₗₐₛₜ: mean residence time, calculated to the last observable time point; AUCₗₐₛₜ: area under the curve, calculated to the last observable time point; AUC_{∞}: area under the curve, extrapolated to infinity | | | | | |
| ¹Dose-normalized by dividing the parameter by the measured dose in mg/kg. | | | | | |

**TABLE 42: INDIVIDUAL AND AVERAGE PLASMA CONCENTRATIONS (NG/ML) AND PK PARAMETERS FOR MDL-001 AFTER SINGLE ORAL ADMINISTRATION AT 1000 MG/KG IN MALE SD RAT (GROUP 4), (ONE DATA IS EXCLUDED AS AN OUTLIER)**

| **Oral (1000 mg/kg MDL-001; Group 4)** | | | | | |
|---|---|---|---|---|---|
| | **Concentration of MDL-001 (ng/mL)** | | | | |
| **Time (hr)** | **Rat #** | | | **Mean** | **SD** |
| | **459** | **460** | **461** | | |
| 0.25 | 5.05 | 5 | 7.81 | 5.95 | 1.61 |
| 0.5 | 10.6 | 17.9 | 26 | 18.2 | 7.7 |
| 1 | 40.7 | 85.1 | 74.9 | 66.9 | 23.3 |
| 2 | 226 | 248 | 293 | 256 | 34 |
| 4 | 335 | 370 | 438 | 381 | 52 |
| 6 | 365 | 501 | 489 | 452 | 75 |
| 8 | **295** | **436** | 393 | 375 | 72 |
| 12 | **359** | **449** | 221 | 343 | 115 |
| 24 | **28.4** | **59.7** | 305* | 44.5 | NA |
| 24 (Lung) | 3850 | 7930 | 28500* | 5890 | NA |
| Lung₂₄/Plasma₂₄ | 136 | 133 | ND | 135 | NA |
| **t_{1/2} (hr)** | 4.30 | 5.16 | ²ND | 4.73 | NA |
| **tₘₐₓ (hr)** | 6.00 | 6.00 | 6.00 | 6.00 | 0.00 |
| **Cₘₐₓ (ng/mL)** | 365 | 501 | 489 | 452 | 75 |
| **MRTₗₐₛₜ (hr)** | 9.37 | 9.67 | 6.29 | 8.44 | 1.87 |
| **AUCₗₐₛₜ (hr·ng/mL)** | 5702 | 7444 | 3982 | 5710 | 1731 |
| **AUC_{∞} (hr·ng/mL)** | 5878 | 7888 | ²ND | 6883 | NA |

| **Dose-normalized Values¹** | | | | | |
|---|---|---|---|---|---|
| **AUCₗₐₛₜ (hr·kg·ng /mL/mg)** | 5.70 | 7.44 | 3.98 | 5.71 | 1.73 |
| **AUC_{∞} (hr·kg·ng /mL/mg)** | 5.88 | 7.89 | ²ND | 6.88 | NA |
| t_{1/2}: half-life, time points in bold used for half-life calculation; Cₘₐₓ: maximum plasma concentration; tₘₐₓ: time of maximum plasma concentration; MRTₗₐₛₜ: mean residence time, calculated to the last observable time point; AUCₗₐₛₜ: area under the curve, calculated to the last observable time point; AUC_{∞}: area under the curve, extrapolated to infinity; NA: not applicable | | | | | |
| *Outlier, excluded from calculations | | | | | |
| ¹Dose-normalized by dividing the parameter by the measured dose in mg/kg. | | | | | |
| ²ND: not determined because not enough data points on an elimination stage | | | | | |
| NA: not applicable | | | | | |

### Example 13

### MDL-001 development program

The overall objective of the MDL-001 development program is to examine the safety, tolerability and efficacy of MDL-001 in treating patients with COVID-19 and other influenza-like infections (ILI's). Initial trials will focus on the COVID-19 indication and will be expanded to other ILI's following initial proof-of-concept in COVID-19.

The completed nonclinical studies are described in the foregoing Examples. The studies that will be completed prior to the start of the clinical trial(s) are included in Example 13 and the studies that will be completed subsequently are included in Example 13. The clinical dosing duration for this indication is not anticipated to exceed 10-14 days, which is significantly shorter than the 28 days pipendoxifene was evaluated in clinical studies in healthy subjects.

### MDL-001 Nonclinical Studies

### In vitro and In vivo Pharmacology Studies

The following studies regarding additional SAR-CoV-2 experiments and further exploration of an indication for treatment of ILI's will be performed. First, an in vitro study evaluating MDL-001 influenza antiviral activity in the HeLa-ACE2 cells. Second, an in vitro study evaluating MDL-001 against influenza H1N1 and H3N2. Third, a study to evaluate MDL-001 antiviral activity against influenza virus and SARS-CoV-2 in primary cell models (iPSC pneumocyte model, HTBE/ALI model). Fourth, MDL-001 Antiviral Resistance studies for SARS-CoV-2 and influenza to reconfirm RdRp target for both viruses. Fifth, in vivo animal studies in well-established influenza animal models.

### Safety Pharmacology Studies

The following safety pharmacology studies will be conducted.

(1) Broad receptor (CEREP) panel screen. A broad receptor/enzyme screening panel will be conducted to assess the potential for off-target activity.

(2) CardioPrime Assay. Anabios' CardioPrime assay will be conducted to evaluate potential for Drug-Induced Pro-Arrhythmia and Inotropic Risk, as an alternative to a dog CV telemetry study. This assay has been shown to be a better predictor of clinical risk than the dog model.

### Pharmacokinetics/ADME Studies

The following PK/ADME studies will be conducted.

(1) Rat Oral Pharmacokinetic Study. The goal of this study is to identify a maximum feasible dose (MFD) or maximum tolerated dose (MTD) in order to identify dose levels and API supplies for subsequent rat toxicology studies. Male and female rats (n=4/group) will be dosed at 250, 500, 750 and 1000 mg/kg for 3 days. Following the 3rd dose, blood samples will be collected at pre-dose, 0.5, 1, 1.5, 2, 3, 4, 8, 12 and 24 h, and plasma prepared. Lung tissue samples will also be collected at 24 h and analyzed by LC-MS/MS for MDL-001. Exposure (Cₘₐₓ and AUC) will be used to determine if an MFD is attained; tolerability will be used to determine if an MTD is attained. Depending upon these data, higher doses will be investigated if neither an MFD or MTD was achieved.

(2) In vitro metabolite profiling and ID in rat, dog, monkey and human hepatocytes. MDL-001 will be incubated in rat, dog, monkey or human pooled hepatocytes for determination of in vitro hepatic clearance as well as metabolite profiling and ID. Nonradiolabeled MDL-001 will be used, with quantitation and metabolite ID by HRAMS. Qualitative and quantitative evaluation of the metabolite profiles will be performed to ensure that any significant human metabolites are represented in the rodent and non-rodent species planned for

### GLP toxicology studies.

(3) Plasma protein binding in mouse, rat, dog, monkey and human plasma. Plasma protein binding will be determined by rapid equilibrium dialysis (RED). Any interspecies differences in free fraction will be used to adjust total drug concentrations when comparing exposure between species in efficacy and toxicity studies.

### Genotoxicity Studies

The following genotoxicity studies will be performed. Additional standard battery of genotoxicity studies will be assessed based upon the data from the Ames and micronucleus studies and conducted as per regulatory Guidance: Bacterial assay (Ames) test and micronucleus assay.

### Repeat Dose Toxicology Studies

Based upon the FDA and ICH guidance documents and the projected 10-14 day clinical study duration and exposure to patients, a 14-day GLP rat study will be conducted. This study will include all required toxicology assessments including toxicokinetics. Non-rodent general toxicology studies will be conducted.

### MDL-001 Studies for Completion Prior to the Start of Pivotal Trials

### Safety Pharmacology Studies

(1) CNS Safety Pharmacology. A GLP rat Irwin test will be performed.
(2) Respiratory Safety Pharmacology. A GLP rat respiratory study will be conducted.

### Genotoxicity Studies

Additional standard battery of genotoxicity studies will be assessed based upon the data from the Ames and micronucleus studies and will conducted as per regulatory Guidance.

### GLP Toxicology Study(ies)

GLP toxicology studies will be performed in a non-rodent species.

### Nonclinical Development Plan Summary

The completed and proposed nonclinical studies in the foregoing Examples, further supported by the previous studies conducted by Wyeth/Pfizer on pipendoxifene, will be sufficient to support the safety of a MDL-001 clinical program.

Prior to starting the clinical study(ies) with MDL-001, the studies outlined in herein will be completed. The data from these studies will be used in conjunction with the information from the completed nonclinical pharmacology studies (*See* above Examples) and the available data from the previously conducted pipendoxifene clinical studies to assess the starting dose.

### Clinical

The overall objective of the MDL-001 clinical development program to be conducted will be to examine the safety, tolerability and efficacy of MDL-001 in treating patients with COVID-19 and other influenza-like infections (ILI's). Initial trials will focus on the COVID-19 indication, and will be expanded to other ILI's following initial proof-of-concept in COVID-19.

Clinical studies will be conducted, which are described in brief below.
(1) Healthy Volunteers - A Single Ascending Dose (SAD), Open-label, Study to Evaluate Safety, Tolerability and Pharmacokinetics of Orally Administered MDL-001 for the Treatment of SARS-CoV-2 infection (N=30).
(2) Patients - A Randomized, Placebo-controlled Study of Orally Administered MDL-001 for the Treatment of SARS-CoV-2 infection (N=45).

In at least some of the previously described embodiments, one or more elements used in an embodiment can interchangeably be used in another embodiment unless such a replacement is not technically feasible. It will be appreciated by those skilled in the art that various other omissions, additions and modifications may be made to the methods and structures described above without departing from the scope of the claimed subject matter. All such modifications and changes are intended to fall within the scope of the subject matter, as defined by the appended claims.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. As used in this specification and the appended claims, the singular forms "a," "an," and "the" include plural references unless the context clearly dictates otherwise. Any reference to "or" herein is intended to encompass "and/or" unless otherwise stated.

It will be understood by those within the art that, in general, terms used herein, and especially in the appended claims (*e.g.*, bodies of the appended claims) are generally intended as "open" terms (*e.g.*, the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (*e.g.*, "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (*e.g.*, the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (*e.g.*, " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (*e.g.*, " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

While various aspects and embodiments have been disclosed herein, other aspects and embodiments will be apparent to those skilled in the art. The various aspects and embodiments disclosed herein are for purposes of illustration and are not intended to be limiting, with the true scope and spirit being indicated by the following claims.

### CLAUSES:

1. A method for preventing, delaying the onset of, or treating an infection or a disease caused by a RNA virus, comprising administering to a subject in need thereof a composition comprising a compound of Formula (I), Formula (II), or Formula (III), or a pharmaceutically acceptable salt, ester, solvate, stereoisomer, tautomer, or prodrug thereof, thereby preventing, delaying the onset of, or treating the infection or the disease.
2. A method for preventing, delaying the onset of, or treating an inflammatory effect of an infection or a disease caused by a RNA virus, comprising administering to a subject in need thereof a composition comprising a compound of Formula (I), Formula (II), or Formula (III), or a pharmaceutically acceptable salt, ester, solvate, stereoisomer, tautomer, or prodrug thereof, thereby preventing, delaying the onset of, or treating the inflammatory effect.
3. The method of any one of clauses 1-2, wherein for the compound of Formula (I):
   each of R₁ and R₂ is independently a hydrogen, a halogen atom, a hydroxyl group, a sulfhydryl group, an amino group, an amide group, a carboxyl group, a carbonyl group, a alkoxyalkyl group, or a C1-C3 alkyl group which may be substituted with a halogen atom, a hydroxyl group, a sulfhydryl group, an amino group, an amide group, a carboxyl group, a carbonyl group, or a alkoxyalkyl group;
   R₃ is a C1-C4 alkyl group which may be substituted with a terminal R₅ group;
   R₄ is a hydrogen atom or a C1-C5 alkyl or cycloalkyl group, which may be substituted with a halide, hydroxyl, carboxyl, carbonyl, amino, or thiol groups; and/or
   R₅ is a C1-C10 alkyl, cycloalkylaminoalkyl, aminodialkyl or aminocycloalkyl group, which may be substituted with an amino group, a thiol group, a hydroxyl group, or a carbonyl group.
4. The method of any one of clauses 1-3, wherein for the compound of Formula (II):
   each of R₁ and R₂ is independently a hydrogen, a halogen atom, a hydroxyl group, a sulfhydryl group, an amino group, an amide group, a carboxyl group, a carbonyl group, a alkoxyalkyl group, or a C1-C3 alkyl group which may be substituted with a halogen atom, a hydroxyl group, a sulfhydryl group, an amino group, an amide group, a carboxyl group, a carbonyl group, or a alkoxyalkyl group;
   R₃ is a C1-C4 alkyl group which may be substituted with a terminal R₅ group;
   R₄ is a hydrogen atom or a C1-C5 alkyl or cycloalkyl group, which may be substituted with a halide, hydroxyl, carboxyl, carbonyl, amino, or thiol groups; and/or
   R₅ is a C1-C10 alkyl or cycloalkyl group which may be substituted with an amino group, a thiol group, a hydroxyl group, or a carbonyl group.
5. The method of any one of clauses 1-4, wherein for the compound of Formula (III):
   each of R₁ and R₂ is independently a hydrogen, a halogen atom, a hydroxyl group, a sulfhydryl group, an amino group, an amide group, a carboxyl group, a carbonyl group, a alkoxyalkyl group, or a C1-C3 alkyl group which may be substituted with a halogen atom, a hydroxyl group, a sulfhydryl group, an amino group, an amide group, a carboxyl group, a carbonyl group, or a alkoxyalkyl group;
   R₃ is a C1-C4 alkyl group which may be substituted with a terminal R₄ group; and/or
   R₄ is a C1-C10 alkyl or cycloalkyl group which may be substituted with an amino group, a thiol group, a hydroxyl group, or a carbonyl group.
6. The method of any one of clauses 1-5, wherein the compound of Formula I, Formula II, or Formula III is Pipendoxifene.
7. The method of any one of clauses 1-6, wherein the inflammatory effect comprises respiratory failure, a sequela of respiratory failure, acute lung injury, or acute respiratory distress syndrome, optionally the sequela of respiratory failure comprises multi-organ failure.
8. The method of any one of clauses 1-7, wherein the composition comprises a therapeutically or prophylactically effective amount of the compound of Formula (I), Formula (II), or Formula (III), or a pharmaceutically acceptable salt, ester, solvate, stereoisomer, tautomer, or prodrug thereof.
9. The method of any one of clauses 1-8, wherein the subject in need thereof is a subject that is suffering from the infection or the disease, or a subject that is at a risk for the infection or the disease.
10. The method of any one of clauses 1-9, wherein the compound of Formula (I), Formula (II), or Formula (III), or the pharmaceutically acceptable salt, ester, solvate, stereoisomer, tautomer, or prodrug thereof, is administered at a daily dose of at least about 600 mg, 620 mg, 640 mg, 660 mg, 680 mg, 700 mg, 720 mg, 740 mg, 760 mg, 780 mg, 800 mg, 820 mg, 840 mg, 860 mg, 880 mg, 900 mg, 920 mg, 940 mg, 960 mg, 980 mg, 1000 mg, 1020 mg, 1040 mg, 1060 mg, 1080 mg, 1100 mg, 1120 mg, 1140 mg, 1160 mg, 1180 mg, 1200 mg, 1220 mg, 1240 mg, 1260 mg, 1280 mg, 1300 mg, 1320 mg, 1340 mg, 1360 mg, 1380 mg, 1400 mg, 1420 mg, 1440 mg, 1460 mg, 1480 mg, 1500 mg, 1520 mg, 1540 mg, 1560 mg, 1580 mg, 1600 mg, 1620 mg, 1640 mg, 1660 mg, 1680 mg, 1700 mg, 1720 mg, 1740 mg, 1760 mg, 1780 mg, 1800 mg, 1820 mg, 1840 mg, 1860 mg, 1880 mg, 1900 mg, 1920 mg, 1940 mg, 1960 mg, 1980 mg, 2000 mg, 2020 mg, 2040 mg, 2060 mg, 2080 mg, 2100 mg, 2120 mg, 2140 mg, 2160 mg, 2180 mg, 2200 mg, 2220 mg, 2240 mg, 2260 mg, 2280 mg, 2300 mg, 2320 mg, 2340 mg, 2360 mg, 2380 mg, 2400 mg, 2420 mg, 2440 mg, 2460 mg, 2480 mg, or 2500 mg, optionally the administering comprises once daily or twice daily oral administration.
11. The method of any one of clauses 1-10, wherein the administering is prophylaxis administration.
12. The method of any one of clauses 1-11, wherein the administration is 3 hours, 6 hours, 12 hours, 18 hours, 24 hours, 36 hours, 47 hours, 72 hours, 96 hours, 4 days, 5 days, 6 days, or 7 days before commencement of the infection or the disease.
13. The method of any one of clauses 1-12, wherein the administration is repeated once or more times per day.
14. The method of any one of clauses 1-13, wherein the administration is repeated hourly, daily, or weekly.
15. The method of any one of clauses 1-14, wherein the administering comprises administering one or more loading doses and one or more maintenance doses of the compound of Formula (I), Formula (II), or Formula (III), or the pharmaceutically acceptable salt, ester, solvate, stereoisomer, tautomer, or prodrug thereof.
16. The method of any one of clauses 1-15, wherein the subject is a low-risk patient, optionally a low-risk patient exposed to an RNA virus or suspected of being exposed to an RNA virus.
17. The method of any one of clauses 1-16, wherein the subject is a high-risk and/or severe disease patient post-infection with a RNA virus.
18. The method of any one of clauses 1-17, wherein the administration does not cause an adverse event in the subject.
19. The method of any one of clauses 1-18, wherein the administration does not cause any significant drug-drug interactions and/or genotoxicity in the subject.
20. The method of any one of clauses 1-19, wherein therapeutic levels of the compound of Formula (I), Formula (II), or Formula (III), or the pharmaceutically acceptable salt, ester, solvate, stereoisomer, tautomer, or prodrug thereof, are achieved in the subject with a dose at least 1.1-fold, 1.3-fold, 1.5-fold, 1.7-fold, 1.9-fold, 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, or 100-fold, below the LD₅₀.
21. The method of any one of clauses 1-20, wherein the administration of the composition prevents, delays the onset of, and/or treats the infection, the disease and/or inflammatory effect in the subject comparable to or better than administration of a composition comprising Remdesivir, optionally the composition comprising Remdesivir is subcutaneously administered twice a day at a dose of 150 mg.
22. The method of any one of clauses 1-21, wherein the administration of the composition produces an improvement in one or more clinical endpoints in the subject equal to or greater than the improvement in said one or more clinical endpoints in a subject administered a composition comprising Remdesivir, optionally the composition comprising Remdesivir is subcutaneously administered twice a day at a dose of 150 mg, further optionally a clinical end point comprises body weight.
23. The method of any one of clauses 1-22, wherein a significant amount of the compound of Formula (I), Formula (II), or Formula (III), or the pharmaceutically acceptable salt, ester, solvate, stereoisomer, tautomer, or prodrug thereof, accumulates in the plasma and/or lung tissue of the subject following administration, optionally the lung tissue is the primary site of the infection and/or disease.
24. The method of any one of clauses 1-23, wherein the administration achieves lung concentrations of the compound of Formula (I), Formula (II), or Formula (III), or the pharmaceutically acceptable salt, ester, solvate, stereoisomer, tautomer, or prodrug thereof, of greater than 30 ug/g, optionally the administration comprises once daily oral administration.
25. The method of any one of clauses 1-24, wherein the administration achieves an at least 1.1-fold, 1.3-fold, 1.5-fold, 1.7-fold, 1.9-fold, 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, or 100-fold, enrichment in lung to plasma concentrations of the compound of Formula (I), Formula (II), or Formula (III), or the pharmaceutically acceptable salt, ester, solvate, stereoisomer, tautomer, or prodrug thereof.
26. The method of any one of clauses 1-25, wherein the compound of Formula (I), Formula (II), or Formula (III), or the pharmaceutically acceptable salt, ester, solvate, stereoisomer, tautomer, or prodrug thereof, achieves an at least 1.1-fold, 1.3-fold, 1.5-fold, 1.7-fold, 1.9-fold, 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, or 100-fold, greater lung tissue concentration than the minimum therapeutic concentration in the lung tissue.
27. The method of any one of clauses 1-26, wherein the C_{Lung}/EC₅₀ ratio of the compound of Formula (I), Formula (II), or Formula (III), or the pharmaceutically acceptable salt, ester, solvate, stereoisomer, tautomer, or prodrug thereof, exceeds about 1, 2, 3, 4, 5, 6, 7, 8, 9, 10, 12, 14, 16, 18, or 20 at a time point of 1, 2, 3, 4, 5, 6, 7, 8, 9, 10, 12, 14, 16, 18, 20, 22, or 24 hours after one or more administrations of the composition.
28. The method of any one of clauses 1-27, wherein the C_{Lung}/EC₉₀ ratio of the compound of Formula (I), Formula (II), or Formula (III), or the pharmaceutically acceptable salt, ester, solvate, stereoisomer, tautomer, or prodrug thereof, exceeds about 1, 2, 3, 4, 5, 6, 7, 8, 9, 10, 12, 14, 16, 18, or 20 at a time point of 1, 2, 3, 4, 5, 6, 7, 8, 9, 10, 12, 14, 16, 18, 20, 22, or 24 hours after one or more administrations of the composition.
29. The method of any one of clauses 1-28, wherein the administering provides a C_{Lung}/EC₉₀ of the compound of Formula (I), Formula (II), or Formula (III), or the pharmaceutically acceptable salt, ester, solvate, stereoisomer, tautomer, or prodrug thereof, greater than 1 coverage for at least about 24 hours, optionally the administering comprises b.i.d. dosing.
30. The method of any one of clauses 1-29, wherein the infection or the disease is in the respiratory tract of the subject.
31. The method of any one of clauses 1-30, wherein the subject has been exposed to the RNA virus, is suspected to have been exposed to the RNA virus, or is at a risk of being exposed to the RNA virus.
32. The method of any one of clauses 1-31, wherein the subject is a mammal, optionally the subject is a human.
33. The method of any one of clauses 1-32, wherein the RNA virus is a doublestranded RNA virus.
34. The method of any one of clauses 1-32, wherein the RNA virus is a positive-sense single-stranded RNA virus.
35. The method of clause 34, wherein the positive-sense single-stranded RNA virus is a coronavirus, optionally the coronavirus is an alpha coronavirus, a beta coronavirus, a gamma coronavirus, or a delta coronavirus.
36. The method of clause 35, wherein the coronavirus is Middle East respiratory coronavirus (MERS-CoV), severe acute respiratory syndrome coronavirus (SARS-CoV), or SARS-CoV-2, optionally a SARS-CoV-2 variant selected from the group comprising B.1.1.7 (Alpha), B.1.351 (Beta), B.1.525 (Eta), B.1.427/B.1.429 (Epsilon), B.1.526 (Iota), B.1.617.1 (Kappa), B.1.617.2 (Delta), C.37 (Lambda), P.1 (Gamma), P.2 (Zeta), P.3 (Theta), B.1.1.529 (Omicron), derivatives thereof, of any combination thereof.
37. The method of any one of clauses 1-36, wherein the infection or disease caused by the RNA virus is common cold, influenza, SARS, coronaviruses, COVID-19, hepatitis C, hepatitis E, West Nile fever, Ebola virus disease, rabies, polio, or measles.
38. The method of any one of clauses 1-37, wherein the composition is a pharmaceutical composition comprising the compound of Formula (I), Formula (II), or Formula (III), or a pharmaceutically acceptable salt, ester, solvate, stereoisomer, tautomer, or prodrug thereof, and one or more pharmaceutically acceptable excipients.
39. The method of any one of clauses 1-38, comprising administering to the subject one or more additional antiviral agents.
40. The method of clause 39, wherein at least one of the one or more additional antiviral agents is co-administered to the subject with the composition.
41. The method of clause 39, wherein at least one of the one or more additional antiviral agents is administered to the subject before the administration of the composition, after the administration of the composition, or both.
42. The method of any one of clauses 1-41, wherein the composition comprises one or more additional therapeutic agents.
43. The method of clause 42, wherein the one or more additional therapeutic agents comprise one or more antiviral agents.
44. The method of any one of clauses 39-43, wherein the antiviral agent is selected from the group consisting of a nucleoside or a non-nucleoside analogue reverse-transcriptase inhibitor, a nucleotide analogue reverse-transcriptase inhibitor, a NS3/4A serine protease inhibitor, a NS5B polymerase inhibitor, and interferon alpha.
45. The method of any one of clauses 1-44, wherein the composition is administered to the subject by intravenous administration, nasal administration, pulmonary administration, oral administration, parenteral administration, or nebulization.
46. The method of any one of clauses 1-44, wherein the composition is aspirated into at least one lung of the subject.
47. The method of any one of clauses 1-44, wherein the composition is in the form of powder, pill, tablet, microtablet, pellet, micropellet, capsule, capsule containing microtablets, liquid, aerosols, or nanoparticles.
48. The method of any one of clauses 1-44, wherein the composition is in a formulation for administration to the lungs.
49. The method of any one of clauses 1-48, wherein the composition is administered to the subject once, twice, or three times a day.
50. The method of any one of clauses 1-48, wherein the composition is administered to the subject once every day, every two days, or every three days.
51. The method of any one of clauses 1-50, wherein the composition is administered to the subject over the course of at least two weeks, at least three weeks, at least four weeks, or at least five weeks.
52. The method of any one of clauses 1-51, further comprising measuring the viral titer of the RNA virus in the subject before administering the composition to the subject, after administering the composition to the subject, or both, optionally the viral titer is lung bulk virus titer.
53. The method of any one of clauses 1-52, wherein administrating the composition results in reduction of the viral titer of the RNA virus in the subject as compared to that in the subject before administration of the composition.
54. The method of any one of clauses 1-53, wherein the administration of the composition achieves an at least 1.1-fold, 1.3-fold, 1.5-fold, 1.7-fold, 1.9-fold, 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, or 100-fold, reduction in viral titer in the subject as compared to a subject administered a vehicle control, optionally the viral titer is viral lung titer, optionally viral lung titer is measured from whole lung homogenates.
55. The method of any one of clauses 1-54, wherein the viral titer is measured 3 hours, 6 hours, 12 hours, 18 hours, 24 hours, 36 hours, 47 hours, 72 hours, 96 hours, 4 days, 5 days, 6 days, 7 days, 8 days, 9 days, 10 days, 11 days, 12 days, 13 days, and/or 14 days post-infection.
56. The method of any one of clauses 1-55, further comprising determining global virus distribution in the lungs of the subject.
57. The method of any one of clauses 1-56, further comprising measuring the body weight of the subject, optionally administering the composition ameliorates disease-associated and/or infection-associated weight loss, optionally in a dose-dependent manner, further optionally the disease-associated and/or infection-associated loss in body weight is less than about 20%, 15%, 14%, 13%, 12%, 11%, 10%, 9%, 8%, 7%, 6%, 5%, 4%, 3%, 2%, 1%, 0.9%, 0.8%, 0.7%, 0.6%, 0.5%, 0.4%, or 0.3%.
58. The method of any one of clauses 1-57, further comprising measuring a neutrophil density within the lungs of the subject, optionally administering the composition results in reduction of the neutrophil density within the lungs of the subject as compared to that in the subject before administration of the composition.
59. The method of any one of clauses 1-58, further comprising measuring a total necrotized cell count within the lungs of the subject, optionally administering the composition results in reduction of the total necrotized cell count in the subject as compared to that in the subject before administration of the composition.
60. The method of any one of clauses 1-59, further comprising measuring a total protein level within the lungs of the subject, optionally administering the composition results in reduction of the total protein level within the lungs of the subject as compared to that in the subject before administration of the composition.
61. A kit, comprising
   a compound of Formula (I), Formula (II), or Formula (III), or a pharmaceutically acceptable salt, ester, solvate, stereoisomer, tautomer, or prodrug thereof; and
   a label indicating that the kit is for preventing, delaying the onset of, or treating an infection or a disease caused by a RNA virus.
62. A kit, comprising
   a compound of Formula (I), Formula (II), or Formula (III), or a pharmaceutically acceptable salt, ester, solvate, stereoisomer, tautomer, or prodrug thereof; and
   a label indicating that the kit is for preventing, delaying the onset of, or treating an inflammatory effect of an infection or a disease caused by a RNA virus.
63. The kit of any one of clauses 61-62, wherein for the compound of Formula (I):
   each of R₁ and R₂ is independently a hydrogen, a halogen atom, a hydroxyl group, a sulfhydryl group, an amino group, an amide group, a carboxyl group, a carbonyl group, a alkoxyalkyl group, or a C1-C3 alkyl group which may be substituted with a halogen atom, a hydroxyl group, a sulfhydryl group, an amino group, an amide group, a carboxyl group, a carbonyl group, or a alkoxyalkyl group;
   R₃ is a C1-C4 alkyl group which may be substituted with a terminal R₅ group;
   R₄ is a hydrogen atom or a C1-C5 alkyl or cycloalkyl group, which may be substituted with a halide, hydroxyl, carboxyl, carbonyl, amino, or thiol groups; and/or
   R₅ is a C1-C10 alkyl, cycloalkylaminoalkyl, aminodialkyl or aminocycloalkyl group, which may be substituted with an amino group, a thiol group, a hydroxyl group, or a carbonyl group.
64. The kit of any one of clauses 61-63, wherein for the compound of Formula (II):
   each of R₁ and R₂ is independently a hydrogen, a halogen atom, a hydroxyl group, a sulfhydryl group, an amino group, an amide group, a carboxyl group, a carbonyl group, a alkoxyalkyl group, or a C1-C3 alkyl group which may be substituted with a halogen atom, a hydroxyl group, a sulfhydryl group, an amino group, an amide group, a carboxyl group, a carbonyl group, or a alkoxyalkyl group;
   R₃ is a C1-C4 alkyl group which may be substituted with a terminal R₅ group;
   R₄ is a hydrogen atom or a C1-C5 alkyl or cycloalkyl group, which may be substituted with a halide, hydroxyl, carboxyl, carbonyl, amino, or thiol groups; and/or
   R₅ is a C1-C10 alkyl or cycloalkyl group which may be substituted with an amino group, a thiol group, a hydroxyl group, or a carbonyl group.
65. The kit of any one of clauses 61-64, wherein for the compound of Formula (III):
   each of R₁ and R₂ is independently a hydrogen, a halogen atom, a hydroxyl group, a sulfhydryl group, an amino group, an amide group, a carboxyl group, a carbonyl group, a alkoxyalkyl group, or a C1-C3 alkyl group which may be substituted with a halogen atom, a hydroxyl group, a sulfhydryl group, an amino group, an amide group, a carboxyl group, a carbonyl group, or a alkoxyalkyl group;
   R₃ is a C1-C4 alkyl group which may be substituted with a terminal R₄ group; and/or
   R₄ is a C1-C10 alkyl or cycloalkyl group which may be substituted with an amino group, a thiol group, a hydroxyl group, or a carbonyl group.
66. The kit of any one of clauses 61-65, wherein the compound of Formula I, Formula II, or Formula III is Pipendoxifene.
67. The kit of any one of clauses 61-66, wherein the label indicates that the kit is for prophylaxis administration.
68. The kit of any one of clauses 61-67, wherein the label indicates that the kit is for low-risk patients, optionally low-risk patients exposed to an RNA virus or suspected of being exposed to an RNA virus.
69. The kit of any one of clauses 61-68, wherein the label indicates that the kit is for high-risk and/or severe disease patients post-infection with a RNA virus.
70. The kit of any one of clauses 61-69, wherein the label indicates the compound of Formula (I), Formula (II), or Formula (III), or the pharmaceutically acceptable salt, ester, solvate, stereoisomer, tautomer, or prodrug thereof, is administered at a daily dose of at least about 600 mg, 620 mg, 640 mg, 660 mg, 680 mg, 700 mg, 720 mg, 740 mg, 760 mg, 780 mg, 800 mg, 820 mg, 840 mg, 860 mg, 880 mg, 900 mg, 920 mg, 940 mg, 960 mg, 980 mg, 1000 mg, 1020 mg, 1040 mg, 1060 mg, 1080 mg, 1100 mg, 1120 mg, 1140 mg, 1160 mg, 1180 mg, 1200 mg, 1220 mg, 1240 mg, 1260 mg, 1280 mg, 1300 mg, 1320 mg, 1340 mg, 1360 mg, 1380 mg, 1400 mg, 1420 mg, 1440 mg, 1460 mg, 1480 mg, 1500 mg, 1520 mg, 1540 mg, 1560 mg, 1580 mg, 1600 mg, 1620 mg, 1640 mg, 1660 mg, 1680 mg, 1700 mg, 1720 mg, 1740 mg, 1760 mg, 1780 mg, 1800 mg, 1820 mg, 1840 mg, 1860 mg, 1880 mg, 1900 mg, 1920 mg, 1940 mg, 1960 mg, 1980 mg, 2000 mg, 2020 mg, 2040 mg, 2060 mg, 2080 mg, 2100 mg, 2120 mg, 2140 mg, 2160 mg, 2180 mg, 2200 mg, 2220 mg, 2240 mg, 2260 mg, 2280 mg, 2300 mg, 2320 mg, 2340 mg, 2360 mg, 2380 mg, 2400 mg, 2420 mg, 2440 mg, 2460 mg, 2480 mg, or 2500 mg, optionally the administering comprises once daily or twice daily oral administration.
71. The kit of any one of clauses 61-70, wherein the RNA virus is a coronavirus.
72. The kit of any one of clauses 61-71, wherein the coronavirus is Middle East respiratory coronavirus (MERS-CoV), severe acute respiratory syndrome coronavirus (SARS-CoV), or SARS-CoV-2, optionally a SARS-CoV-2 variant selected from the group comprising B.1.1.7 (Alpha), B.1.351 (Beta), B.1.525 (Eta), B.1.427/B.1.429 (Epsilon), B.1.526 (Iota), B.1.617.1 (Kappa), B.1.617.2 (Delta), C.37 (Lambda), P.1 (Gamma), P.2 (Zeta), P.3 (Theta), B.1.1.529 (Omicron), derivatives thereof, of any combination thereof.
73. A composition comprising a compound of Formula (I), Formula (II), or Formula (III), or a pharmaceutically acceptable salt, ester, solvate, stereoisomer, tautomer, or prodrug thereof, for use in preventing, delaying the onset of, or treating an infection or a disease caused by a RNA virus.
74. A composition comprising a compound of Formula (I), Formula (II), or Formula (III), or a pharmaceutically acceptable salt, ester, solvate, stereoisomer, tautomer, or prodrug thereof, for use in preventing, delaying the onset of, or treating an inflammatory effect of an infection or a disease caused by a RNA virus.
75. The composition of any one of clauses 73-74, wherein for the compound of Formula (I):
   each of R₁ and R₂ is independently a hydrogen, a halogen atom, a hydroxyl group, a sulfhydryl group, an amino group, an amide group, a carboxyl group, a carbonyl group, a alkoxyalkyl group, or a C1-C3 alkyl group which may be substituted with a halogen atom, a hydroxyl group, a sulfhydryl group, an amino group, an amide group, a carboxyl group, a carbonyl group, or a alkoxyalkyl group;
   R₃ is a C1-C4 alkyl group which may be substituted with a terminal R₅ group;
   R₄ is a hydrogen atom or a C1-C5 alkyl or cycloalkyl group, which may be substituted with a halide, hydroxyl, carboxyl, carbonyl, amino, or thiol groups; and/or
   R₅ is a C1-C10 alkyl, cycloalkylaminoalkyl, aminodialkyl or aminocycloalkyl group, which may be substituted with an amino group, a thiol group, a hydroxyl group, or a carbonyl group.
76. The composition of any one of clauses 73-75, wherein for the compound of Formula (II):
   each of R₁ and R₂ is independently a hydrogen, a halogen atom, a hydroxyl group, a sulfhydryl group, an amino group, an amide group, a carboxyl group, a carbonyl group, a alkoxyalkyl group, or a C1-C3 alkyl group which may be substituted with a halogen atom, a hydroxyl group, a sulfhydryl group, an amino group, an amide group, a carboxyl group, a carbonyl group, or a alkoxyalkyl group;
   R₃ is a C1-C4 alkyl group which may be substituted with a terminal R₅ group;
   R₄ is a hydrogen atom or a C1-C5 alkyl or cycloalkyl group, which may be substituted with a halide, hydroxyl, carboxyl, carbonyl, amino, or thiol groups; and/or
   R₅ is a C1-C10 alkyl or cycloalkyl group which may be substituted with an amino group, a thiol group, a hydroxyl group, or a carbonyl group.
77. The composition of any one of clauses 73-76, wherein for the compound of Formula (III):
   each of R₁ and R₂ is independently a hydrogen, a halogen atom, a hydroxyl group, a sulfhydryl group, an amino group, an amide group, a carboxyl group, a carbonyl group, a alkoxyalkyl group, or a C1-C3 alkyl group which may be substituted with a halogen atom, a hydroxyl group, a sulfhydryl group, an amino group, an amide group, a carboxyl group, a carbonyl group, or a alkoxyalkyl group;
   R₃ is a C1-C4 alkyl group which may be substituted with a terminal R₄ group; and/or
   R₄ is a C1-C10 alkyl or cycloalkyl group which may be substituted with an amino group, a thiol group, a hydroxyl group, or a carbonyl group.
78. The composition of any one of clauses 73-77, wherein the compound of Formula I, Formula II, or Formula III is Pipendoxifene.
79. The composition of any one of clauses 73-78, wherein the inflammatory effect comprises respiratory failure, a sequela of respiratory failure, acute lung injury, or acute respiratory distress syndrome, optionally the sequela of respiratory failure comprises multi-organ failure.
80. The composition of any one of clauses 73-79,wherein the composition comprises a therapeutically or prophylactically effective amount of the compound of Formula (I), Formula (II), or Formula (III), or a pharmaceutically acceptable salt, ester, solvate, stereoisomer, tautomer, or prodrug thereof.
81. The composition of any one of clauses 73-80, wherein the RNA virus is a doublestranded RNA virus.
82. The composition of any one of clauses 73-80, wherein the RNA virus is a positive-sense single-stranded RNA virus.
83. The composition of clause 82, wherein the positive-sense single-stranded RNA virus is a coronavirus, optionally the coronavirus is an alpha coronavirus, a beta coronavirus, a gamma coronavirus, or a delta coronavirus.
84. The composition of clause 83, wherein the coronavirus is Middle East respiratory coronavirus (MERS-CoV), severe acute respiratory syndrome coronavirus (SARS-CoV), or SARS-CoV-2, optionally a SARS-CoV-2 variant selected from the group comprising B.1.1.7 (Alpha), B.1.351 (Beta), B.1.525 (Eta), B.1.427/B.1.429 (Epsilon), B.1.526 (Iota), B.1.617.1 (Kappa), B.1.617.2 (Delta), C.37 (Lambda), P.1 (Gamma), P.2 (Zeta), P.3 (Theta), B.1.1.529 (Omicron), derivatives thereof, of any combination thereof.
85. The composition of any one of clauses 73-84, wherein the composition is a pharmaceutical composition comprising the compound of Formula (I), Formula (II), or Formula (III), or a pharmaceutically acceptable salt, ester, solvate, stereoisomer, tautomer, or prodrug thereof, and one or more pharmaceutically acceptable excipients.
86. The composition of any one of clauses 73-85, wherein the composition comprises one or more additional therapeutic agents, optionally the one or more additional therapeutic agents comprise one or more antiviral agents.
87. The composition of clause 86, wherein the one or more antiviral agents is selected from the group consisting of a nucleoside or a non-nucleoside analogue reverse-transcriptase inhibitor, a nucleotide analogue reverse-transcriptase inhibitor, a NS3/4A serine protease inhibitor, a NS5B polymerase inhibitor, and interferon alpha.
88. The composition of any one of clauses 73-87, wherein the composition is in the form of powder, pill, tablet, microtablet, pellet, micropellet, capsule, capsule containing microtablets, liquid, aerosols, or nanoparticles.
89. The composition of any one of clauses 73-88, wherein the composition is in a formulation for administration to the lungs.

## Claims

1. An antiviral compound of Formula (I): 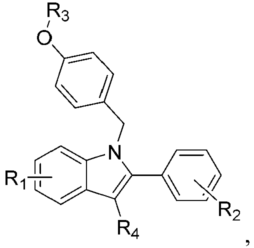 or a pharmaceutically acceptable salt, ester, solvate, stereoisomer, tautomer, or prodrug thereof, wherein:
each of R₁ and R₂ is independently a hydrogen, a halogen atom, a hydroxyl group, a sulfhydryl group, an amino group, an amide group, a carboxyl group, a carbonyl group, a alkoxyalkyl group, or a C₁-C₃ alkyl group which may be substituted with a halogen atom, a hydroxyl group, a sulfhydryl group, an amino group, an amide group, a carboxyl group, a carbonyl group, or a alkoxyalkyl group;
R₃ is a C₂₋₄ alkyl group substituted with a terminal R₅ group;
R₄ is a hydrogen atom or a C₁-C₅ alkyl or cycloalkyl group, which may be substituted with a halide, hydroxyl, carboxyl, carbonyl, amino, or thiol groups; and
R₅ is C₃-C₈ aminocycloalkyl;
wherein the compound is not Pipendoxifene or Bazedoxifene.

2. The antiviral compound of claim 1, wherein R₃ is 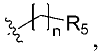 wherein n is 2, 3, or 4.

3. The antiviral compound of claim 1, wherein R₃ is an ethyl group substituted with a terminal R₅ group.

4. The antiviral compound of claim 1, wherein R₅ is

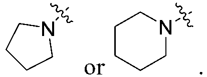

5. The antiviral compound of claim 1, wherein R₁ is a halogen atom or a hydroxyl group.

6. The antiviral compound of claim 1, wherein R₂ is a hydroxyl group or a carboxyl group.

7. The antiviral compound of claim 1, wherein R₄ is a C₁-C₅ alkyl or cycloalkyl group.

8. The antiviral compound of claim 1, wherein the antiviral compound is configured to engage a ribonucleic acid (RNA)-dependent RNA polymerase (RdRp) encoded by an RNA virus.

9. An antiviral compound of Formula (I) or a pharmaceutically acceptable salt, ester, solvate, stereoisomer, tautomer, or prodrug thereof, according to any one of claims 1-8, for use in preventing, delaying the onset of, or treating an infection or a disease caused by a ribonucleic acid (RNA) virus.

10. The antiviral compound of claim 8 or the antiviral compound for use of claim 9, wherein the RNA virus is a coronavirus.

11. The antiviral compound of claim 8 or the antiviral compound for use of claim 9 or 10, wherein the RNA virus is a severe acute respiratory syndrome coronavirus 2 (SARS-CoV-2) virus.

12. A kit, comprising
the antiviral compound according to any one of claims 1-8, or a pharmaceutically acceptable salt, ester, solvate, stereoisomer, tautomer, or prodrug thereof; and
a label indicating that the kit is for preventing, delaying the onset of, or treating an infection or a disease caused by an RNA virus.
